# EUROPÄISCHE PATENTSCHRIFT

(11) **EP 1 725 572 B1**
(45) Veröffentlichungstag und Bekanntmachung des Hinweises auf die Patenterteilung: **31.05.2017**
(21) Anmeldenummer: 04797663.4
(22) Anmeldetag: 05.11.2004
(51) Int. Cl.: C07H 19/10, C07H 19/16, C07H 19/20, C07H 21/00, C12Q 1/68

(54) **MAKROMOLEKULARE NUKLEOTIDVERBINDUNGEN UND METHODEN ZU DEREN ANWENDUNG**
MACROMOLECULAR NUCLEOTIDE COMPOUNDS AND METHODS FOR USING THE SAME
COMPOSES NUCLEOTIDIQUES MACROMOLECULAIRES ET LEURS PROCEDES D'UTILISATION

(30) Priorität: 05.11.2003 DE 10351636; 05.12.2003 DE 10356837
(43) Veröffentlichungstag der Anmeldung: 29.11.2006
(73) Patentinhaber: AGCT GmbH, 23562 Lübeck (DE)
(72) Erfinder: CHERKASOV, Dmitry, 35039 Marburg (DE); HENNIG, Christian, 30659 Hannover (DE)
(86) Internationale Anmeldenummer: PCT/EP2004/012556
(87) Internationale Veröffentlichungsnummer: WO 2005/044836

(56) Entgegenhaltungen:
- WO-A-03/020968
- US-A- 5 256 535
- LEMAITRE M ET AL: "BIOLOGICAL ACTIVITIES OF OLIGONUCLEOTIDES LINKED TO POLY(L-LYSINE)" NUCLEOSIDES & NUCLEOTIDES, DEKKER, NEW YORK,NY,, US, Bd. 1/2, Nr. 6, 1987, Seiten 311-315, XP000775556 ISSN: 0732-8311
- JAESCHKE A ET AL: "HYBRIDIZATION-BASED AFFINITY PARTITIONING OF NUCLEIC ACIDS USING PEG-COUPLED OLIGONUCLEOTIDES" NUCLEIC ACIDS RESEARCH, OXFORD UNIVERSITY PRESS, SURREY, GB, Bd. 22, Nr. 10, 25. Mai 1994 (1994-05-25), Seiten 1880-1884, XP001097519 ISSN: 0305-1048
- SEELIG B ET AL: "Site-Specific Modification of Enzymatically Synthesized RNA: Transcription Initiation and Diels-Alder Reaction", TETRAHEDRON LETTERS, ELSEVIER, AMSTERDAM, NL, vol. 38, no. 44, 3 November 1997 (1997-11-03), pages 7729-7732, XP004093407, ISSN: 0040-4039, DOI: 10.1016/S0040-4039(97)10151-4

## Beschreibung

### 1. Einführung

### 1.1 Einordnung in das technologische Feld

In einer Ausführung bezieht sich die Erfindung auf die Struktur, die Herstellung und die Verwendung modifizierter Nukleotid- und Nukleosidbausteine, im folgenden "Nuk-Makromoleküle".

In einer weiteren Ausführung bezieht sich die Erfindung auf die Struktur, die Herstellung und die Verwendung modifizierter Nukleinsäuremolekülen, z.B. Oligonukleotide.

### 1.2 Stand der Technik:

Niedermolekulare Stoffe spielen eine wichtige Rolle in lebendigen Organismen. Sie dienen Beispielsweise als Bausteine von Polymeren, als Botenstoffe, als Energieträger. In der Diagnostik werden sie eingesetzt zur Bestimmung von medizinisch relevanter Parameter. Dabei werden diese Stoffe oft mit bestimmten Signal-Trägern markiert. Ein Beispiel für die niedermolekularen Stoffe bieten Nukleotide und Nukleoside.

Sie spielen ebenfalls eine zentrale Rolle in unterschiedlichen Stoffwechselvorgängen der lebenden Organismen ("Biochemie und Pathobiochemie", G. Löffler, 2003) und stellen in der modernen Biotechnologie verbreitete Elemente dar ("Molecular-Cloning", J. Sambrook, band 1-3, 2001, ISBN 0-87969-576-5), beispielsweise für künstliche Detektionssysteme ("DNA Microarrays", Bowtell, 2003, ISBN 0-87969-624-9, "Microarray-Biochip Technology" M Schena, 2000, ISBN 1-881299-37-6). Aus diesem Grund werden modifizierte Nukleotide, Nukleotid-Analoga, in unterschiedlichen Bereichen der Biotechnologie, Medizin und Pharmakologie eingesetzt ("Nucleotide Analogs" Scheit, 1980, ISBN 0-471-04854-2, "Nucleoside and Nucleic Acid Chemistry", Kisakürek 2000, "Anti-HIV Nucleosides" Mitsuya, 1997, "Nucleoside Analogs in cancer therapy", Cheson, 1997).

Eines der wichtigsten Felder der modernen Life-Science sind Analysen der Nukleinsäuren. Ein großer Bereich dieser Analysen befasst sich mit dem Nachweis der Nukleinsäuren oder deren Bestandteilen im biologischen Präparat. In vielen Fällen wird zu diesem Zweck ein Reaktionspartner markiert, der mit der zu analysierenden Nukleinsäure reagieren kann. Dem Fachmann sind unterschiedliche Möglichkeiten zur Markierung der Nukleinsäuren bekannt. Einerseits können einzelne Nukleinsäurebausteine, Nukleotide bzw. Nukleoside modifiziert sein, andererseits können kurze Fragmente von Nukleinsäuren, Oligonukleotide oder Polynukleotide zum Nachweis eingesetzt werden.

Konventionell modifizierte Nukleotide sind beispielsweise in Lee et al. Nucleic acid research 1992, V. 20, S.2471; Augustin et. al. J. Biotechnology 2001 V. 86, S.289-301; US Patent 4828979; Held et al. Nucleic acid research, 2002, v. 30, S. 3857 beschrieben. Diese Nukleotide haben einen niedermolekularen detektierbaren Teil, der direkt (wie beispielsweise ein Fluoreszenzfarbstoffmolekül) oder indirekt detektiert werden kann (wie beispielsweise bei einem Biotin-Molekül, das erst nach Kopplung mit einem Streptavidin-Farbstoff-Konjugat nachgewiesen wird)). Solche Nukleotide stellen Beispiele für die Stand der Technik der Nukleotid-Modifikationen dar. Viele modifizierte Nukleotide können kommerziell erworben werden, z.B. von NEN Life Science Products (Biotin-11-dUTP, DNP-11-dATP, Fluorescein-12-dCTP), von Amersham Bioscience ( dCTP-Cy3, dCTP-Cy5) oder von Roche (Biotin-16-dUTP). Entsprechende Nachweisreagenzien, z.B. markiertes Streptavidin, markierte Antikörper, können von den selben Anbietern bezogen werden. Auch modifizierte Nukleoside werden für den Nachweis von Nukleinsäuren eingesetzt (Trilink Biotechnologies, Eurogentec, MWG-Biotech).

Zur Vereinfachung der Darstellung werden im Folgenden modifizierte Nukleotide besprochen. Einem Fachmann sollte es naheliegend erscheinen, dass auch modifizierte Nukleoside für die enzymatischen und nicht enzymatischen Synthesen eingesetzt werden können, konventionell modifizierte Nukleoside sind erhältlich beispielsweise bei Trilink Biotechnologies oder Eurogentec.
Konventionell modifizierte Nukleotide haben ein äquimolares Verhältnis zwischen der Nukleotid-Komponente und dem niedermolekularen detektierbaren Teil, z.B. Farbstoff-Molekül bzw. Biotin-Molekül. Zwischen beiden Teilen liegt ein Linker mit einer durchschnittlichen Länge von 5 - 30 Atomen. Solche Nukleotide können von Polymerasen in den wachsenden Strang eingebaut werden und somit ein signaltragendes Molekül (Farbstoff) oder signalvermittelndes Molekül (Biotin oder Digoxigenin) in die Nukleinsäurekette einführen. Nach dem Einbau von modifizierten Nukleotiden kann die Signaldetetkion im Fall von farbstoffmarkierten NT direkt erfolgen oder nach Inkubation mit einem sekundären signaltragenden Molekül (z.B. Strepavidin-Farbstoff-Konjugat im Fall von Biotin), wenn signalvermittelnde Moleküle verwendet werden.

Zum Stand der Technik gehört weiterhin Ylikoski et al (D2; US Pat No.: 5,256,535), sowie Seelig et al (D5; Tetrahedron Letters, 1997, V. 38, p7729 - 7732).

In Ylikoski et al (D2) werden Herstellung und Verwendung von fluoreszent markierten Polymeren beschrieben, die an Nukleinsäureketten gekoppelt werden können. Weiterhin wird Modifizierung von bereits synthetisieren Nukleinsäureketten mit solchen aktivierten Polymeren beschrieben. Diese Herstellung / Modifizierung basiert auf chemischer Kopplung von aktivierten Polymeren an Nukleinsäureketten und stellt somit einen der bekannten Wege der Nukleinsäure-Modifizierung dar, nämlich Kopplung nach einer bereits stattgefundenen Synthese von Nukleinsäureketten.

In Seelig et al (D5) werden Herstellung und Verwendung eines Nukleotides beschrieben, welches an seinem 5'-Phosphat einen relativ kurzen Linker (PEG 600, enthält Linker mit 6 bis 15 PEG-Einheiten, Seite 7731, 2. Absatz) und einen niedermolekularen Marker, in diesem Beispiel Anthrazene oder Biotin hat. Dieses modifizierte Nukleotid wird von einer RNA-Polymerase als Initiator der RNA-Synthese beschrieben. Dieses Nukleotid wird in der enzymatischen Synthesereaktion ohne einen makromolekularen Marker verwendet. Die Kopplung des Streptavidins erfolgt erst nach der Synthese (Seite 7731, 3. Absatz "immobilization on streptavidin agarose"). Der Linker hat eine beschriebene Maximal-Länge von ca. 15 PEG-Einheiten (entspricht 45 Kettenatome), was einem Molekular-Gewicht von ca. 660 Da entspricht (PEG 600). Die Masse der niedermolekularen Marker (Anthrazene und Biotin liegt unter 2000 Da. Das modifizierte Nukleotid-Molekül passt somit in das Schema für Strukturen von konventionell modifizierten Nukleotiden.

Seelig et al (D5) beschreibt somit allgemeine Struktur (Bauplan) von konventionell modifizierten Nukleotiden, welche seit Mitte der 80er Jahre bekannt ist: modifizierte Nukleotide schließen einen kleinmolekularen Marker mit einer Masse von ca. 500 - 2000 Da (z.B. Botin, Farbstoff) und an einem relativ kurzen Linker (meisten deutlich unter 50 Kettenatome, z.B. ca. 12 bis 20 Kettenatome) ein. Verwendung von solch modifizierten Nukleotiden für Einbau in die Nukleinsäureketten wurde mehrfach beschrieben. Während der Synthese werden meistens mehrere markierte Nukleotide mit kleinen Markern in die wachsende Nukleinsäurekette eingebaut. Falls eine Modifizierung mit makromolekularen Liganden angestrebt wird, z.B. mit Streptavidin, so erfolgt eine solche Modifizierung stetts erst nach der Synthese von Nukleinsäureketten.

### Nachteile des Standes der Technik:

### Die Nachteile des Standes der Technik umfassen mehrere Aspekte:

Zum einen, Signalstabilität und Signalintensität einzelner Farbstoff-Moleküle sind häufig mangelhaft, um im wachsenden Feld der NanoBioTechnologie, Einbau-Ereignisse der Nukleotide eindeutig zu detektieren. Einzelne Fluorophore können beispielsweise während der Detektion ausbleiben und dadurch zum Verlust des Signals führen.
Bestimmte Verfahren, beschrieben beispielsweise in Seeger WO 0018956, Kartalov WO02072892, sind auf die Detektion der Signale von einzelnen Nukleotid-Molekülen angewiesen. Beim Einsatz konventioneller Nukleotide beeinflüssen mehrere Phänomene wie z.B. das Ausbleichen der Farbstoffe oder Blinking die Ergebnisse der Einzelmoleküldetektion. Für solche Verfahren bedeutet eine Steigerung von Signalstärke und Intensität der Nukleotidmarkierung eine Verringerung der Fehlerrate bei der Detektion.

Zum anderen, gebräuchliche Verfahren einer nachträglichen Markierung / Signal-Verstärkung mittels Konjugation mit signalgebenden Elementen (z.B. Fluoreszent markierten Polymeren etc.) leiden häufig an mageren Ausbeuten im Kopplungs-Schritt / Markierungsschritt: In jedem Verfahrensschritt bleibt ein signifikanter Anteil von eingebauten Nukleotiden ohne Signal. Dies führt ebenfalls zu Signal-Verlusten in der Detektion.

Weiterhin, werden bei der Markierung der Nukleinsäuren Signalvervielfältigungsschritte_eingesetzt. Diese können auf unterschiedlichen Stufen der Analyse durchgeführt werden. Beispiele dafür stellen Materialvervielfältigung (PCR), mehrfacher Einbau von markierten Nukleotiden, mehrstufige nachträgliche Markierung von Biotin-Nukleotiden ("Molecular-Cloning", J. Sambrook, Band 1-3, 2001, ISBN 0-87969-576-5). Diese Verfahren führen zu einer Verzerrung der Signale, da sie aus mehreren, oft ungenügend kontrollierten Schritten bestehen, die mit unterschiedlichen Ausbeuten ablaufen und von vielen Faktoren beeinflusst werden können.

Die gewünschte und für den Fachmann naheliegende Signalverstärkung durch eine mehrfache Markierung eines Nukleotids bereits während der Nukleotid-Synthese erweist sich in Kombination mit herkömmlichen Nukleotidstrukturen als sehr großer Nachteil. Zwar können solche Nukleotide ohne größeren Aufwand hergestellt werden (Beispiel 25), verlieren aber ihre Funktion als Substrat für Polymerasen (Beispiel 34B) und andere Enzyme. Die Ursache dafür liegt in der Veränderung der Eigenschaften der Nukleotide in Folge der Kopplung an ein wesentlich größeres Molekül.

Es besteht somit ein Bedarf an modifizierten Nukleotiden oder Nukleosiden mit einer verbesserten signalgebenden oder signalvermittelnden Wirkung, insbesondere für deren Einsatz zur Markierung von Nukleinsäuren bei der Nukleinsäureanalyse.

### Aufgaben der Erfindung:

Eine Aufgabe der vorliegenden Erfindung ist folglich die Bereitstellung von modifizierten Nukleotiden oder Nukleosiden, die ihre Substrateigenschaften für Polymerasen oder andere Enzyme beibehalten und nach ihrem Einbau in die Nukleinsäuren eine verbesserte Signalintensität bei deren Analyse gewährleisten.

Eine weitere Aufgabe der Erfindung ist die Bereitstellung von Verfahren zur Markierung von Nukleinsäuren mit erfindungsgemäß modifizierten Nukleotiden.

Die vorliegende Erfindung beschreibt in einer Ausführungsform eine neue Klasse an modifizierten Nukleotiden, die Nuk-Makromoleküle. Nuk-Makromoleküle sind dadurch gekennzeichnet, dass eine oder mehrere Nukleotid-Komponenten mit einem oder mehreren signalgebenden oder signalvermittelnden makromolekularen Marker-Komponenten (Marker) über einen langen Linker verbunden sind. Die jeweilige Nuk-Komponente bewahrt in einem Nuk-Makromolekül seine Substratfunktion und kann z.B. durch Polymerasen in einen wachsenden Strang eingebaut werden. Eine signalgebende makromolekulare Komponente trägt beispielsweise mehrere Farbstoffmoleküle.

Dadurch können mehrere Nachteile des Standes der Technik überwunden werden:
Zunächst, dank diesem makromolekularen Marker kann jedes Nukleotid-Konjugat ein signifikant stärkeres und stabileres Signal entwickeln, als sogenannte konventionell markierte Nukleotide. Vorteile einer starken und stabilen Signalgebung sind insbesondere im wachsenden Feld der Einzelmolekül-Analysen hervorzuheben.

Weiterhin, ein makromolekularer Marker kann direkt beim Einbauereignis des Nukleotid-Konjugates in die wachsende Nukleinsäurekette eingeführt werden. Somit können Unsicherheiten einer "nachträglichen Markierung", d.h. Markierung welche erst durch nachträgliche Verfahrensschritte eingeführt wird, ausgeschlossen werden.

Zusammenfassend, Nuk-Makromoleküle besitzen neuartige Eigenschaften verglichen mit konventionell modifizierten Nukleotiden. Diese Eigenschaften ermöglichen deutliche Verbesserungen im Bereich der Modifizierung von Nukleinsäureketten:
- Es können deutlich höhere Signalstärken bei Markierung erreicht werden.
- Es können Marker mit vielseitigen Eigenschaften verwendet werden
- Es können neue Verbindungen von Nukleinsäureketten synthetisiert werden

Nuk-Makromoleküle können wie konventionell modifizierte Nukleotide in verschiedenen Bereichen der Biotechnologie und Medizin verwendet werden. Im Folgenden sollte lediglich als Beispiel deren Einsatz für die Nukleinsäuremarkierung dargestellt werden. Andere Einsatzbereiche der Nuk-Makromoleküle sind dem Fachmann bereits bekannt s.o.
den natürlichen Nukleotiden oder Nukleosiden ähnlich, trägt aber beispielsweise zusätzliche chemische Gruppen. Beispiele für Kombinationen aus verschiedenen Komponenten sind dem Fachmann bekannt. Solche Nuk-Komponenten können in vielen enzymatischen und chemischen Reaktionen eingesetzt werden (G. Wright et al. Pharmac.Ther. 1990, V. 47, S. 447-).

In einer anderen Ausführungsform ist eine Nuk-Komponente mit weiteren Nukleotiden gekoppelt, z.B in einer Nukleinsäurekette. Dabei tritt die Nuk-Komponente als ein Monomer eines Polymers auf.

### 1.3.3.1.1 Variationen am Phosphat

In einer Ausführungsform stellt die Nuk-Komponente ein Nukleosid dar. In einer anderen Ausführungsform stellt die Nuk-Komponente ein Nukleosid-Monophosphat dar. In einer anderen Ausführungsform stellt die Nuk-Komponente ein Nukleosid-Diphosphat dar. In einer weiteren Ausführungsform stellt die Nuk-Komponente ein Nukleosid-Triphosphat dar. Auch höhere Phosphat-Derivate (Tetraphosphat usw.) können verwendet werden.

Die genannten Phosphatmodifikationen können wie bei Nukleosid-Triphosphaten an der 5' -Position oder auch an anderen Positionen des Zucker-Teils des Nukleotides sitzen, beispielsweise an der 3' -Position.

Wahlweise kann die Phosphat-Komponente Modifikationen einschließen, solche Modifikationen schließen beispielsweise in einer Ausführungsform einen Linker ein (D. Jameson et al. Methods in Enzymology 1997, V. 278, S. 363-, A. Draganescu et al. J. Biol.Chem. 2000 v.275, 4555-). In einer werteren Ausführungsform schließt die Phosphat-Komponente der Nuk-Komponente Thiotriphosphat-Verbindungen ein (Burges et al. PNAS 1978 v. 75, S. 4798-).

In einer weiteren Ausführungsform schließt die Phosphat-Komponente der Nuk-Komponente geschützte Phosphat-Gruppen (z.B. Phosphoroamidite) ein.

In einer Ausführungsform stellt die Phosphat-Komponente die Verbindung zwischen der Nuk-Komponente und der Linker-Komponente der Nuk-Makromoleküle dar.

### 1.3.3.1.2 Variationen an der Base

Die Nuk-Komponente kann ein in der Natur in den Nukleinsäuren vorkommendes Nukleotid oder Nukleosid oder seine Analoga darstellen, vorzugsweise die an Watson-Crick-Paarbildung teilnehnem, beispielsweise Adenin, Guanin, Thymin, Cytosin, Uracil, Inosin, oder eine modifizierte Base, wie z.B. 7-Deazaadenin, 7-Deazaguanin, 6-Thio-adenin, einschließen, Literatur s. oben. Wahlweise kann die Base Modifikationen einschließen, solche Modifikationen schließen beispielsweise in einer Ausführungsform einen an die Base gekoppelten Linker wie beispielsweise ein Amino-propargyl-Linker oder ein Amino-Allyl-Linker ein, weitere Beispiele für die Linker sind bekannt (G. Wright et al. Pharmac.Ther. 1990, V. 47, S. 447-, Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nnucleic acids, 2003, v. 22, S. 391, Short US Pat. 6579704, Odedra WO 0192284). In einer Ausführungsform stellt der an die Base gekoppelte Linker die Verbindung zwischen der Nuk-Komponente und der Linker-Komponente der Nuk-Makromoleküle dar. Weitere Modifikationen an der Base sind beispielsweise im Katalog von Trilink Biotechnologies, Inc. San Diego, USA, Ausgabe 2003 auf Seite 38 aufgeführt.

### 1.3.3.1.3 Variationen am Zucker

Dem Fachmann sind verschiedene Variationen der Zucker-Komponente der Nukleotide bekannt, die z.B. in der Diagnostik, Therapie oder Forschung eingesetzt werden. Solche Variationen schließen z.B. Ribose , 2' -Deoxyribose oder 2',3'-Dideoxyribose ein. Wahlweise kann die Zucker-Komponente Modifikationen einschließen (M. Metzger et al. Nucleic Acid Research 1994, V. 22, 4259-), solche Modifikationen schließen beispielsweise in einer Ausführungsform einen Linker ein. Die modifizierende Gruppe oder Linker kann beispielsweise reversibel an die Zucker-Komponente gekoppelt sein (Hovinen et al. J.Chem.Soc.Prking Trans. 1994, S. 211-, Canard US Pat. 5798210, Kwiatkowski US Pat. 6255475, Kwiatkowski WO 01/25247, Ju et al. US Pat. 6664079, Fahnestock et al. WO 91066678, Cheeseman US Pat. 5302509, Parce et al. WO 0050642, Milton et al. WO 2004018493, Milton et al. 2004018497).
In einer Ausführungsform stellt der an die Zucker-Komponente gekoppelte Linker die Verbindung zwischen der Nuk-Komponente und der Linker-Komponente der Nuk-Makromoleküle dar.

In einer weiteren Ausführungsform schließt die Zuker-Komponente z.B. folgende Modifikationen ein: wahlweise kann die 3'- oder die 2'-OH-Gruppen durch folgende Atome oder Gruppen ersetzt werden: Halogenatome, Wasserstoff, Amino-, Merkapto- oder Azido-Gruppe (Beabealashvilli et al. Biochem Biophys Acta 1986, v.868, 136-, Yuzhanov et al. FEBS Lett. 1992 v. 306, 185-).

In einer weiteren Ausführungsform schließt die Nuk-Komponente azyklische Nukleotid- oder Nukleosid-Modifikationen ein (A. Holy Current Pharmaceutical Design 2003 v. 9, 2567-, G. Wright et al. Pharmac.Ther. 1990, V. 47, S. 447-). In einer weiteren Ausführung kann die Zucker-Komponente eine Doppeltbindung einschließen.
In der Anmeldung werden 2'-Deoxynukleotide, beispielsweise 2'-Deoxyuridin-Triphosphat, 2'-Deoxycytidin-Triphosphat, 2'-Deoxyadenosin-Triphosphat, 2'-Deoxyguanosin-Triphosphat als dUTP, dCTP, dATP und dGTP bezeichnet.

### 1.3.3.1.4 Kopplung von NT und Linker

Die Nuk-Komponente ist an einer Kopplungsstelle mit dem Linker verbunden. Die Kopplungsstelle des Linkers an die Nuk-Komponente kann sich an der Base, am Zucker (Ribose bzw. Deoxyribose), oder am Phosphat-Teil befinden.

Die Verbindung zwischen der Linker-Komponente und der Nuk-Komponente ist vorzugsweise kovalent.
Wenn die Kopplungsstelle an der Base liegt, so befindet sie sich vorzugsweise an den Positionen 4 oder 5 bei Pyrimidin-Basen und an den Positionen 6,7,8 bei den Purin-Basen. Am Zucker können die Positionen 2', 3', 4' oder 5' die Kopplungsstellen darstellen. Die Kopplung an die Phosphatgruppen kann an der alpha, beta, oder gamma-Phosphatgruppe erfolgen. Beispiele für die Kopplungsstelle an der Base sind in Short WO 9949082, Balasubramanian WO 03048387, Tcherkassov WO 02088382 (siehe auch kommerziell erhältliche Nukleotide (Amersham, Roche), an der Ribose in Herrlein et al. Helvetica Chimica Acta, 1994, V. 77, S. 586, Jameson et al. Method in Enzymology, 1997, V. 278, S. 363, Canard US Pat. 5798210, Kwiatkowski US Pat. 6255475, Kwiatkowski WO 01/25247, Parce WO 0050642., an Phosphatgruppen in Jameson et al. Method in Enzymology, 1997, V. 278, S. 363 beschrieben.

Die Position der Kopplungsstelle hängt vom Einsatzgebiet der NT-Makromoleküle ab. So werden beispielsweise für die Markierung von Nukleinsäuren, die am Nukleinsäurestrang verbleiben soll, vorzugsweise Kopplungsstellen am Zucker oder an der Base verwendet. Die Kopplung an die Gamma- oder Beta-Phosphatgruppen kann beispielsweise dann erfolgen, wenn die Markierung beim Einbau des Nuk-Makromoleküls freigesetzt werden soll.

Die Verbindung zwischen der Nuk-Komponente und der Linker-Komponente erfolgt über eine Kopplungseinheit (L), die ein Teil der Linker-Komponente ist.

Die Verbindung zwischen der Nuk-Komponente und dem Linker kann in einer Ausführungsform widerstandsfähig sein, z.B. bei Temperaturen bis zu 130°C, für pH-Bereiche zwischen 1 und 14, und/oder resistent gegen hydrolytische Enzyme (z.B. Proteasen, Esterasen)sein . In einer anderen Ausführungsform der Erfindung ist die Verbindung zwischen der Nuk-Komponente und dem Linker unter milden Bedingungen spaltbar.

Diese spaltbare Verbindung ermöglicht die Entfernung der Linker- und der Marker-Komponenten. Dies kann beispielsweise in den Verfahren der Sequenzierung durch die Synthese von Bedeutung sein, wie Pyrosequencing, BASS (Canard et al. US Patent 5.798.210, Rasolonjatovo Nucleosides & Nucleotides 1999, V.18 S.1021, Metzker et al. NAR 1994, V.22, S.4259, Welch et al. Nucleosides & Nucleotides 1999, V.18, S.19, Milton et al. WO 2004018493, Odedra at al. WO 0192284) oder single molecule sequencing, Tcherkassov WO 02088382. Ihre Wahl ist nicht eingeschränkt, sofern sie unter den Bedingungen der enzymatischen Reaktion stabil bleibt, keine irreversible Störung der Enzyme (z.B. Polymerase) verursacht und unter milden Bedingungen abgespalten werden kann. Unter "milden Bedingungen" sind solche Bedingungen zu verstehen, die zum Beispiel Nukleinsäure-Primer-Komplexe nicht zerstören, wobei z.B. der pH-Wert vorzugsweise zwischen 3 und 11 und die Temperatur zwischen 0°C und einem Temperaturwert (x) liegt. Dieser Temperaturwert (x) hängt von der Tm des Nukleinsäure-Primer-Komplexes (Tm ist der Schmelzpunkt) und wird beispielsweise als Tm (Nukleinsäure-Primer-Komplex) minus 5°C errechnet (z.B. Tm ist 47°C, dann liegt die maximale Temperatur bei 42°C; unter diesen Bedingungen eignen sich besonders Ester-, Thioester-, Acetale, Phosphoester-, Disulfid-Verbindungen und photolabile Verbindungen als spaltbare Verbindungen).

Vorzugsweise gehört die genannte spaltbare Verbindung zu chemisch oder enzymatisch spaltbaren oder photolabilen Verbindungen. Als Beispiele von chemisch spaltbaren Gruppen sind Ester-, Thioester-, Disulfid-, Acetal-Verbindungen bevorzugt (Short WO 9949082, "Chemistry of protein conjugation and crosslinking" Shan S. Wong 1993 CRC Press Inc., Herman et al. Method in Enzymology 1990 V.184 S.584, Lomant et al. J.Mol.Biol. 1976 V.104 243, "Chemistry of carboxylic acid and esters" S.Patai 1969 Interscience Publ.). Beispiele für photolabile Verbindungen können in folgenden Literaturstellen gefunden werden: Rothschild WO 9531429, "Protective groups in organic synthesis" 1991 John Wiley & Sons, Inc., V. Pillai Synthesis 1980 S.1, V. Pillai Org. Photochem. 1987 V.9 S.225, Dissertation "Neue photolabile Schutzgruppen für die lichtgesteuerte Oligonucleotidsynthese" H.Giegrich, 1996, Konstanz, Dissertation "Neue photolabile Schutzgruppen für die lichtgesteuerte Oligonucleotidsynthese" S.M.Bühler, 1999, Konstanz)..

### 1.3.3.1.5 Zahl der gekoppelten Nuk-Komponenten

In einer Ausführungsform der Erfindung ist pro ein Nuk-Makromolekül nur eine Nuk-Komponente gekoppelt. In einer anderen Ausführungsform der Erfindung sind mehrere Nuk- Komponenten pro Nuk-Makromolekül gekoppelt. Mehrere Nuk-Komponenten können einheitlich oder unterschiedlich sein, wobei beispielsweise durchschnittlich 2 bis 5, 5 bis 10, 10 bis 25, 25 bis 50, 50 bis 100, 100 bis 250, 250 bis 500, 500 bis 1000 Nuk-Komponenten pro ein Nuk-Makromolekül gekoppelt sein können.

### 1.3.3.2 Linker-Komponente

Die Bezeichnung Linker oder Linker-Komponente wird synonym in der Anmeldung verwendet und bezieht sich auf den gesamten strukturellen Abschnitt des Nuk-Makromoleküls zwischen der Nuk-Komponente und der Marker-Komponente.

### 1.3.3.2.1 Linker-Bestandteile

Die Linker-Komponente stellt einen Teil des Nuk-Markromoleküls dar, das zwischen der jeweiligen Nuk-Komponente und der Marker-Komponente liegt. Die Linker-Komponente hat in ihrer Struktur vorzugsweise folgende Bestandteile:
1) Kopplungseinheit L
2) wasserlösliches Polymer
3) Kopplungseinheit T

Die Aufteilung der Linker-Komponente in einzelne Bestandteile ist rein funktionell und soll lediglich der Anschaulichkeit der Struktur dienen. Je nach Betrachtungsweise können einzelne Strukturen des Linkers zum einen oder zum anderen funktionellen Bestandteil gerechnet werden.

Die Kopplungseinheit L hat die Funktion, die Linker-Komponente und die Nuk-Komponente zu verbinden. Bevorzugt sind kurze, unverzweigte Verbindungen, bis max. 20 Atome in der Länge. Die jeweilige Struktur der Kopplungseinheit L hängt von der Kopplungsstelle des Linkers an das Nukleotid und von dem jeweiligen Polymer des Linkers. Einige Beispiele für die Kopplungseinheiten L sind in Beispielen 1 bis 33 angegeben. Viele konventionell modifizierte Nukleotide tragen einen kurzen Linker, diese kurzen Linker dienen als weitere Beispiele für die Kopplungseinheit L, z.B. kurze Linker an der Base: Short WO 9949082, Balasubramanian WO 03048387, Tcherkassov WO 02088382(siehe auch kommerziell erhältliche Nukleotide (Amersham, Roche), kurze Linker an der Ribose in Herrlein et al. Helvetica Chimica Acta, 1994, V. 77, S. 586, Jameson et al. Method in Enzymology, 1997, V. 278, S. 363, Canard US. Pat. 5798210, Kwiatkowski US Pat. 6255475, Kwiatkowski WO 01/25247, Ju et al. US Pat. 6664079, Parce WO 0050642., kurze Linker an Phosphatgruppen in Jameson et al. Method in Enzymology, 1997, V. 278, S. 363 .
Noch weitere Beispiele für die Kopplungseinheit L sind nachfolgend angegeben:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇,
   R₆-(CH=CH-)ₙ-R₇, R₆-(A-CH=CH-)ₙ-R₇, R₆-(CH=CH-B-)ₙ-R₇,
   R₆-A-CH=CH-(CH₂-)ₙ-R₇, R₆-(-CH=CH-CH₂)ₙ-B-R₇, R₆-(-CH=CH-CH₂-CH₂)ₙ-B-R₇, R₆-(C≡C-)ₙ-R₇, R₆-(A-C≡C-)ₙ-R₇, R₆-(C≡C-B-)ₙ-R₇,
   R₆-A-C≡C-(CH₂-)ₙ-R₇, R₆-(-C≡C-CH₂)ₙ-B-R₇, R₆-(-C≡C-CH₂-CH₂)ₙ-B-R₇,
wobei R₆ - die Nuk-Komponente ist, R₇ - de Polymer ist und A und B folgende strukturelle Elemente einschließen: -NH-, -O-, -S-, -SS-, -CO-NH-, -NH-CO-, - CO-O-, -O-CO-, -CO-S-, -S-CO-, -P(O)₂-, -Si-, -(CH₂)ₙ-, eine photolabile Gruppe, wobei n - gleich 1 bis 5 ist

Die Kopplungseinheit L ist auf einer Seite mit der Nuk-Komponente, auf der anderen mit Polymer kovalent verbunden. Die Art der Kopplung hängt von der Art des Polymers ab. In bevorzugter Form hat das Polymer an seinen Enden reaktive Gruppen, beispielsweise NH2 (Amino), OH (Hydroxy), SH (Mercapto), COOH (Carboxy), CHO (Aldehyd), Acryl- oder Maleimid, Halogen-Gruppen. Solche Polymere sind käuflich erwerblich (z.B. Fluka). Einige Varianten für die Kopplung von Polymeren an die Kopplungseinheiten sind unter Beispielen 1 bis 33 angegeben.

Das wasserlösliche Polymer bildet in einer bevorzugten Ausführungsform den Hauptanteil der Linker-Komponente. Es ist ein Polymer, vorzugsweise hydrophil, bestehend aus gleichen oder unterschiedlichen Monomeren. Beispiele für geeignete Polymere stellen Polyethylen-glycol (PEG), Polyamide (z.B. Polypeptide), Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acrylsäure und ihre Derivate, Poly-Acrylamide in ihre derivate, Poly(Vinylalkohol), Polylactat Säure, polyglycolic Säure, Poly(epsilon-caprolactone), Poly(beta-hydroxybutyrate), Poly(beta-hydroxyvalerate), Polydioxanone, Poly(ethylene terephthalate), Poly(malic Säure), Poly(tartronic Säure), Poly(ortho ester), Polyanhydride, Polycyanoacrylate, Poly(phosphoester), Polyphosphazene, Hyaluronidate, Polysulfones.

Dieses Polymer schließt in einer Ausführungsform verzweigte oder weiteren Ausführungsform nicht verzweigte Polymere ein. Das Polymer kann aus mehreren unterschiedlich langen Abschnitten bestehen, wobei jeder Abschnitt aus gleichen Monomeren besteht und Monomere in unterschiedlichen Abschnitten unterschiedlich sind. Einem Fachmann sollte naheliegend erscheinen, dass für einen makromolekularen Linker meistens nur eine durchschnittliche Masse bestimmt werden kann, so dass die Angaben zu den Molmassen einen Mittelwert darstellen ("Makromoleküle, Chemische Struktur und Synthesen", Band 1, 4, H. Elias, 1999, ISBN 3-527-29872-X). Aus diesem Grund kann für Nuk-Makromoleküle keine exakte Massenangabe gemacht werden.

In einer bevorzugten Ausführungsform der Erfindung schließt die Linker-Komponente ein lineares, nicht verzweigtes Polymer ein und trägt keine sterisch anspruchsvollen chemischen Strukturen, wie beispielsweise Farbstoffe, Fluoreszenzfarbstoffe, Liganden. Solche Linker ermöglichen eine geringe sterische Hinderung der enzymatischen Erkennung der Nuk-Komponenten.

In einer weiteren bevorzugten Ausführungsform der Erfindung ist das Polymer der Linker-Komponente linear, die Linker-Komponente trägt jedoch eine oder mehrere sterisch anspruchsvollen chemischen Strukturen, wie beispielsweise Farbstoffe. Die Einführung der sterisch anspruchsvollen Gruppen erlaubt bei manchen Verfahren eine Kontrolle der enzymatischen Reaktion (Tcherkassov WO 02088382).

### 1.3.3.2.2 Linker-Länge

Die Linkerlänge beträgt zwischen 50 bis 60, 60 bis 70, 70 bis 80, 80 bis 90, 90 bis 100, 50 bis 100, 100 bis 200, 200 bis 500, 500 bis 1000, 1000 bis 2000, 2000 bis 10.000, 10000 bis 100000 Atome (gezählt werden Ketten-Atome), somit beträgt die durchschnittliche Linker-Länge zwischen 50 bis 60, 60 bis 70, 70 bis 80, 80 bis 90, 90 bis 100, 50 bis 100, 100 bis 200, 200 bis 500, 500 bis 1000, 1000 bis 2000, 2000 bis 10.000, 10000 bis 100000 Angström (gemessen an einem potenziell maximal ausgestrecktem Molekül).

Falls ein Nuk-Makromolekül mehrere Linker-Komponenten einschließt, können diese Linker-Komponenten untereinander gleiche oder auch unterschiedlich lang sein.

Einige Abschnitte des Linkers können rigide Bereiche enthalten und andere Abschnitte können flexible Bereiche enthalten.

Es wird einem Fachmann naheliegend erscheinen, dass die angeführten Linker-Längen eine beträchtlich größere Molekül-Größe und -Masse haben können, als die jeweilige Nuk-Komponente selbst. Die Angaben der Linker-Längen betreffen eine durchschnittliche Zahl der Ketten-Atome.

### 1.3.3.2.3 Linker-Kopplung in einem Nuk-Makromolekül

Der Linker ist an einer Seite an die Nuk-Komponente und auf der anderen Seite an die Marker-Komponente gebunden. Dazu hat der Linker an seinen Enden Kopplungseinheiten, die diese Funktion erfüllen. Die Verbindung mit der Nuk-Komponenten wurde oben erörtert. Die Verbindung zwischen dem Linker und der Marker-Komponenten erfolgt über Kopplungseinheit T. Bevorzugt sind kurze, unverzweigte Verbindungen, bis max. 20 Atome in der Länge. Die jeweilige Struktur der Kopplungseinheit T hängt von der Kopplungsstelle an der Marker-Komponente und von dem jeweiligen Polymer des Linkers. Die Kopplungseinheit T ist mit dem Polymer kovalent verbunden. Die Art der Kopplung hängt von der Art des Polymers ab. In bevorzugter Form hat das Polymer an seinen Enden reaktive Gruppen, beispielsweise NH2 (Amino), OH (Hydroxy), SH (Mercapto), COOH (Carboxy), CHO (Aldehyd), Acryl- oder Maleimid, Halogen-Gruppen. Solche Polymere sind kommerziell erhältlich (z.B. Fluka). Einige Beispiele für die Kopplungseinheiten L sind in Beispielen 1 bis 33 angegeben, Weitere Beispiele für die chemische und affine Kopplungen s. in der Literatur: "Chemistry of protein conjugation and crosslinking" Shan S. Wong 1993, "Bioconjugation: protein coupling techniques for the biomedical sciences", M. Aslam, 1996.

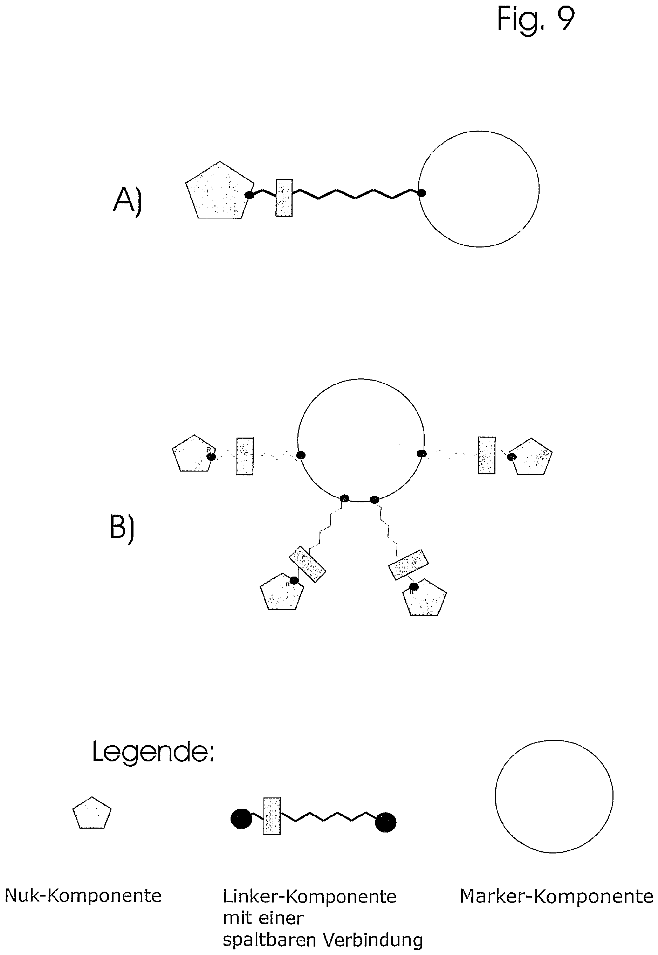

Der Linker kann auch andere funktionelle Gruppen, bzw.Abschnitte enthalten, beispielsweise eine oder mehrere unter milden Bedingungen spaltbare Gruppen (Fig.9) s. Beispiele 22, 23, 24,31.

Eine spaltbare Verbindung innerhalb des Linkers ermöglicht eine Entfernung eines Teils des Linkers und der Marker-Komponente. Nach der Spaltung verbleibt ein LinkerRest an der Nuk-Komponente, Beispiele für spaltbare Verbindungen sind im Abschnitt 1.3.3.1.4 angegeben.

### 1.3.3.3 Marker-Komponente

Die Marker-Komponente kann unterschiedliche Strukturen haben. Die Strukturen im einzelnen sind nicht eingeschränkt, solange sie die Substrateigenschaften der Nuk-Komponenten für Enzyme nicht aufheben. Diese Struktur hat vorzugsweise eine signalgebende oder eine signalvermittelnde Funktion. Der Marker kann auch andere Funktionen haben, beispielsweise strukturturelle, antitoxische oder affine Funktion (beispielsweise in Arzneimittel oder Zuberetungen).

### 1.3.3.3.1 Die Zusammensetzung der Marker-Komponente (Marker)

Der Marker schließt in einer Ausführung eine niedermolekulare Marker-Einheit ein. In einer weiteren Ausführungsform schließt der Marker eine makromolekulare Marker-Einheit ein. In einer weiteren Ausführungsform schließt der Marker mehrere niedermolekulare Marker-Einheiten ein. In einer weiteren Ausführungsform schließt der Marker mehrere makromolekulare Marker-Einheiten ein. In einer weiteren Ausführungsform schließt der Marker eine Kombination aus niedermolekularen und makromolekularen Einheiten ein. Die Marker-Einheiten können eine signalgebende oder signalvermittelnde Funktion haben.

Diese Einheiten können Moleküle mit geringer Molekularmasse, z.B. unter 2000 Da, oder auch selbst Makromoleküle sein. Dabei schließt die Zahl der signalgebenden oder signalvermittelnden Einheiten, die zu einer Marker-Komponenten zusammengefaßt sind, folgende Bereichen ein: 1 und 2, 2 und 5, 5 und 20, 20 und 50, 50 und 100, 100 und 500, 500 und 1000. 1000 und 10000, 10000 und 100000.

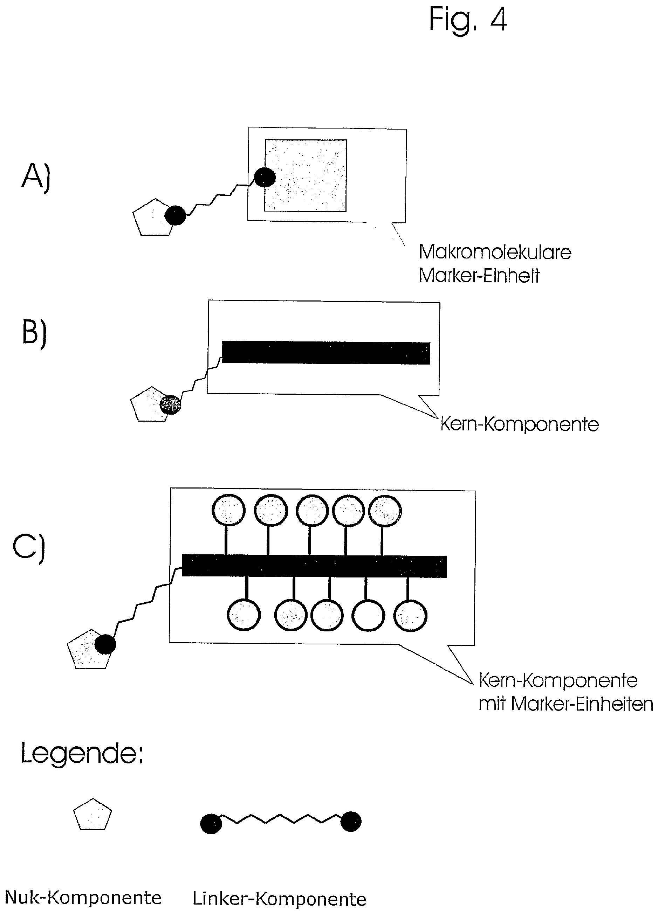

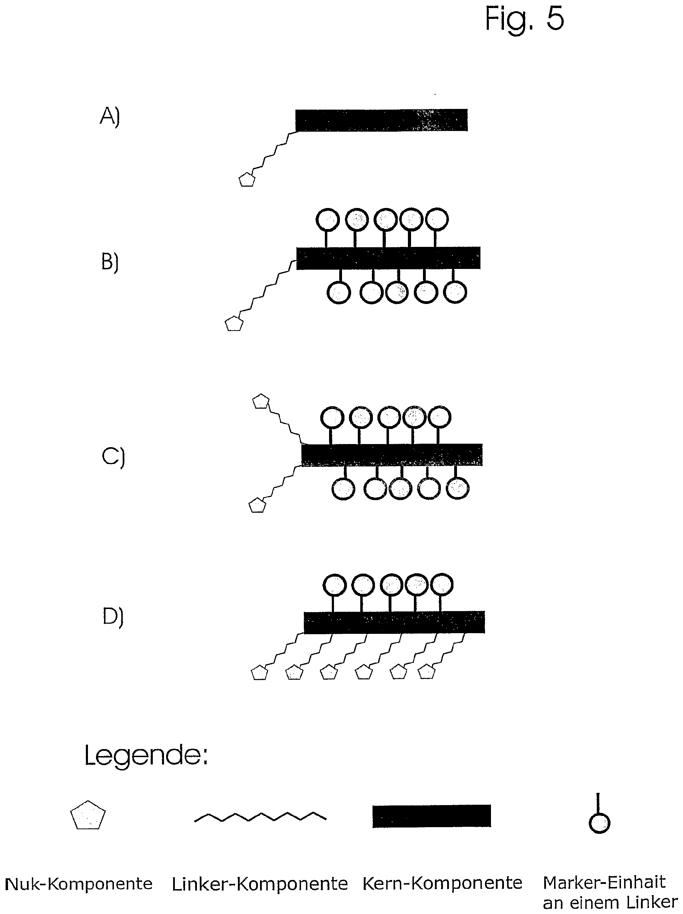

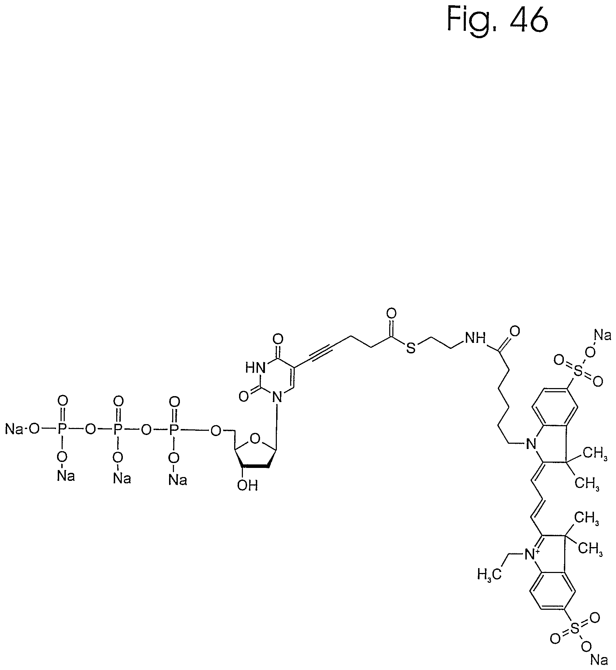

Falls mehrere Marker-Einheiten in einem Marker zusammengefasst sind, sind diese Einheiten in einer Ausführungsform an ein Gerüst gebunden, die Kern-Komponente des Markers (Fig. 4b,c). Diese Kern-Komponente verbindet die Einheiten untereinander. Die Kern-Komponente kann die Verbindung zu einem oder mehreren Nuk-Linker-Komponenten herstellen (Fig 5). Die Kern-Komponente schließt niedermolekulare oder makromolekulare Verbindungen ein.

### 1.3.3.3.2 Struktur der signalgebenden oder der signalvermittelnden Einheiten des Markers

Die strukturellen Marker-Einheiten schließen folgende Gruppen ein:

### 1.3.3.3.2.1 Strukturen mit niedriger Molmasse:

Biotin-Moleküle, Hapten-Moleküle (z.B. Digoxigenin), radioaktive Isotope (z.B. P ³², J ¹³¹), oder deren Verbindungen, seltene Erden , Farbstoffe, Fluoreszenzfarbstoffe (viele Farbstoffe sind kommerziell erhältlich z.B. von Molecular Probes, Inc.) mit gleichen oder unterschiedlichen spektralen Eigenschaften, Farbstoffe, die untereinander FRET eingehen.

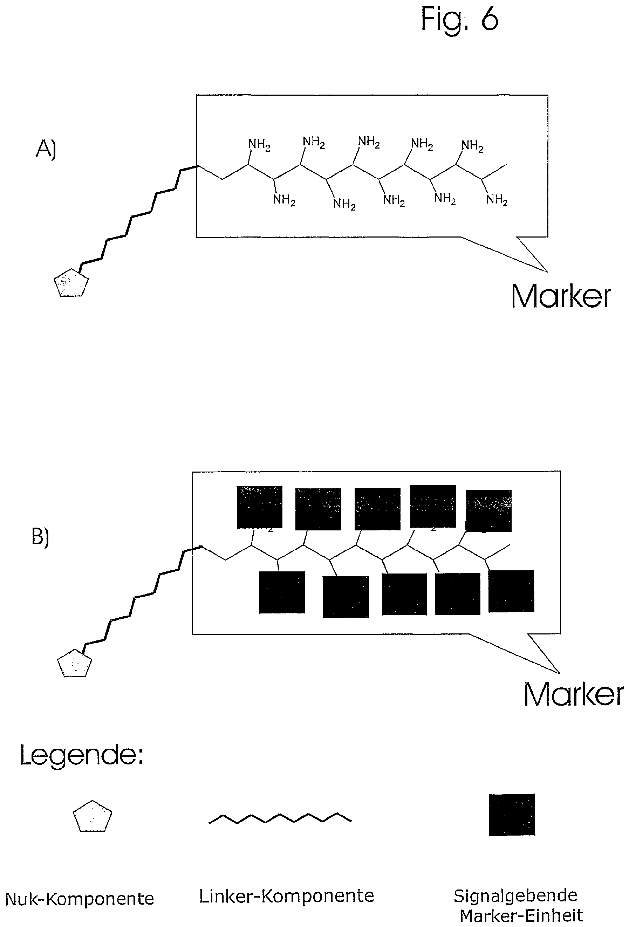

Auch chemisch reaktive Gruppen, wie beispielsweise Amino-, Carboxy-, Merkapto-, Aldehyd-, Jodacetat-, Acryl-, Dithio-, Thioester-Verbindungen können als signalvermittelnde strukturelle Einheiten dienen (Fig. 6a). Nach dem Einbau können diese reaktiven Gruppen mit signalgebenden Elementen, wie beispielsweise Farbstoffe mit entsprechenden reaktiven Gruppen (beispielsweise NHS-Ester, Merkapto-, AminoGruppen) modifiziert werden (Fig. 6b). Allgemeine Grundlagen für die Wahl eines passenden Paares von reaktiven Gruppen sind in "Chemistry of protein conjugation and crosslinking" Shan S. Wong 1993 dargestellt.

In einer speziellen Ausführungsform erfühlt die Kombination aus einer Nuk-Einheit, einer markomolekularen Linker-Komponente und einem Marker mit niedriger Molmasse bereits die Anforderungen der vorliegenden Erfindung. Solche Verbindungen sind ebenfalls Gegenstand dieser Erfindung. Sie können sowohl als Zwischenprodukte für die chemische Synthese von Nuk-Makromolekülen mit einem makromolekularen Marker, z.B. dUTP-PEG-Biotin, als auch selbständig in den enzymatischen Reaktionen verwendet werden, wie beispielsweise mit nur einem Farbstoff markierte Nukleotide.

Als Farbstoffe können verschiedene Fluoreszenzfarbstoffe auftreten, ihre Wahl ist nicht eingeschränkt, solange sie die enzymatische Reaktion nicht wesentlich beeinflußen. Beispiele für Farbstoffe sind Rhodamine (Rhodamin 110, Tetramethylrhodamin, erhältlich bei Fluka-Sigma), Cyanin-Farbstoffe (Cy2, Cy3, Cy3.5, Cy5, Cy5.5, Cy7 von Amersham Bioscience), Coumarine, Bodipy, Fluorescein, Alexa-Farbstoffe: z.B. Alexa 532, Alexa 548, Alexa 555 (Molecular Probes). Viele Farbstoffe sind kommerziell erhältlich, beispielsweise von Molecular Probes Europe, Leiden, Niederlande (im weiteren als Molecucular Probes bezeichnet) oder von Sigma-Aldrich-Fluka (Taufkirchen, Deutschland). Beispiele für die Synthese eines Nuk-Makromoleküls mit einem niedermolekularem Marker ist in Beispiel 19, 20, 23, 36, 37, 38 angegeben.

Nach dem Einbau des Nukleotid-Teils in die Nukleinsäurekette befindet sich der am Linker gekoppelte Marker erfindungsgemäß in einem großen Abstand vom Nukleinsäurestrang, was z.B. zu Verringerung der Einflüße der Nukleobasen auf die Fluoreszenzeigenschaften des Farbstoffes führt. Fluoreszenzausbeuten einzelner Farbstoffe werden dadurch weniger von der lokalen Zusammensetzung der Nukleinsäuresequenz beeinflußt, was zum gleichmäßigen Signalenintesnitäten führt. Die potenzielle intermolekulare Löschung der Fluoreszenz hat dabei einen wesentlich geringeren Einfluß auf die Fluoreszenzsignale von den dicht an einander eingebauten Nuk-Makromolekülen, verglichen mit dem Fall bei den konventionell modifizierten Nukleotien.

In einer Ausführungsform schließt ein Marker mehrere Marker-Einheiten ein. Dabei können die Marker-Einheiten untereinander gleiche oder verschiedene Eigenschaften haben. Beispielsweise können Fluoreszenzfarbstoffe mit unterschiedlichen Spektraleigenschaften eingesetzt werden. In einer Ausführungsform werden Fluoreszenzfarbstoffe eingesetzt, die FRET-Paare bilden.

### 1.3.3.3.2.2 Strukturen mit hoher Masse (Makromoleküle)

### 1.3.3.3.2.2.1 Nanokristalle

Nanokristalle, z.B. Quantum Dots können als Marker-Einheiten auftreten. Dabei können in einer Marker-Komponente Quantum Dots mit gleichen oder unterschiedlichen spektralen Eigenschaften verwendet werden, US Pat. 6.322.901, US Pat. 6.423.551, US Pat. 6.251.303, US Pat. 5.990.479).

### 1.3.3.3.2.2.2 Nano oder Mikro-Teilchen.

Nano oder Mikro-Teilchen können als Marker-Einheiten auftreten, wobei die größten Ausmaße dieser Teilchen von 1nm bis 2nm, von 2nm bis 5nm, von 5nm bis 10nm, von 10nm bis 20 nm, von 20nm bis 50nm, von 50nm bis 100nm, von 100 nm bis 200nm, von 200nm bis 500nm, von 500nm bis 1000nm, von 1000 nm bis 5000 nm betragen. Das Material der Teilchen können beispielsweise reine Metalle wie Gold, Silber, Aluminium (beispielsweise Teilchen mit surface plasmon resonance), - Protein-Au-Konjugate: J. Anal.Chem. 1998, V. 70,S. 5177, - Nukleinsäure-Au-Konjugaten: J. Am. Chem. Soc. 2001, V. 123, S.5164, J. Am. Chem. Soc. 2000, V. 122, S. 9071 , Biochem. Biophys. Res. Commun 2000, V. 274, S. 817, Anal. Chem. 2001, V. 73, S. 4450), - Latex (z.B. Latex-Nanopartikel, Anal. Chem. 2000,V. 72, S. 1979) - Kunststoff (Polystyrene), - paramagnetische Verbindungen / Gemische Zhi ZL et al. Anal Biochem. 2003 Jul 15;318(2):236-43, Dressman D et al. Proc Natl Acad Sci U S A. 2003 Jul 22;100(15):8817-22" - Metall-Teilchen, - magnetische Verbindungen / Gemische Jain KK. Expert Rev Mol Diagn. 2003 Mar;3(2):153-61, Patolsky F et al. Angew Chem Int Ed Engl. 2003 May 30;42(21):2372-2376, Zhao X et al. Anal Chem. 2003 Jul 15;75(14):3144-51, Xu H et al. J Biomed Mater Res. 2003 Sep 15;66A(4):870-9, JOSEPHSON Lee et al. US Pat. 2003092029, KLICHE KAY-OLIVER et al. WO0119405.

### 1.3.3.3.2.2.3 Proteinmoleküle,

Proteinmoleküle können als Marker-Einheiten auftreten, wobei sie folgende Gruppen schließen: Enzyme (z.B. Peroxidase, alkalische Phosphotase, Urease, beta-Galaktosidase), - fluoreszierenden Proteine (z.B. GFP), Antigen-bindende Proteine (z.B. Antikörper, Tetramere, Affibodies (Nord et. Al Nature Biotechnology, V. 15 (S.772-777) oder deren Bestandteile (z.B. Fab-Fragmente), Nukleinsäurebindende Proteine (z.B. Transcriptionsfaktor)

### 1.3.3.3.2.2.4 Nukleinsäureketten,

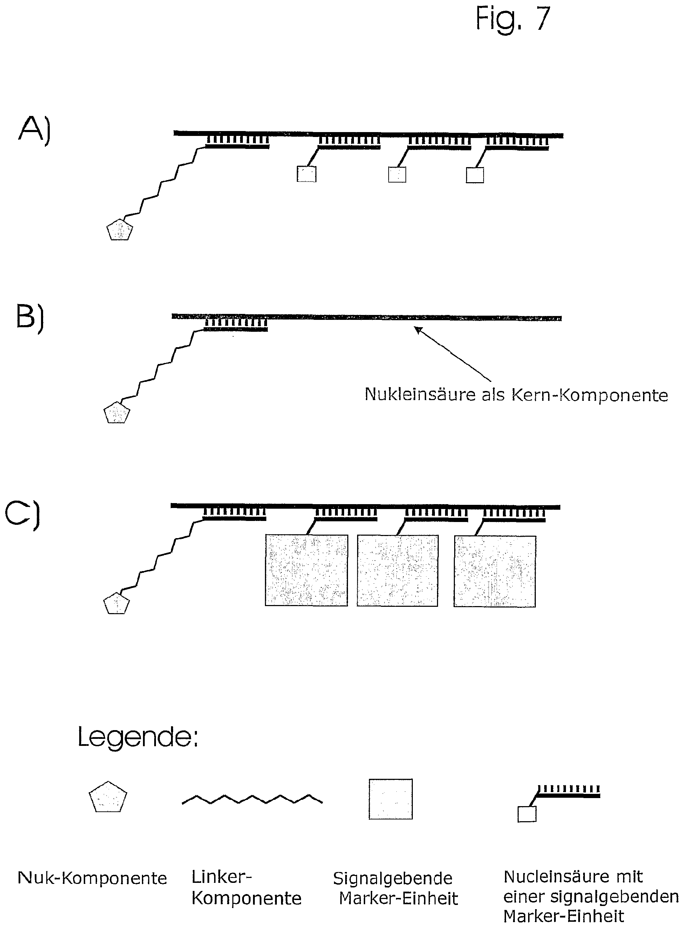

Die Nukleinsäureketten einschließlich der Gruppe, Oligonukleotide, modifizierte Oligonukleotide, können als Marker-Einheiten auftreten. Die Länge der Nukleinsäureketten liegt vorzugsweise in folgenden Bereichen von 10 bis 20, von 20 bis 50, von 50 bis 100, von 100 bis 200, von 200 bis 500, von 500 bis 1000, 1000 bis 5.000, von 5.000 bis 10.000, von 10.000 bis 100.000 Nukleotiden. Es können DNA, RNA, PNA-Moleküle verwendet werden. Nukleinsäureketten können zusäzliche Modifikationen tragen, wie beispielsweise freie Aminogruppen, Farbstoffe und andere signalgebende Moleküle, z.B. makromolekulare Stoffe, Enzyme oder Nanokristalle (Fig. 7a,c). Modifizierte Nukleinsäureketten sind kommerziell zugängig, z.B. MWG-Biotech. Weitere Beispiele für Makromoleküle oder Makromolekülkomplexe, die im Rahmen der vorliegenden Erfindung als Marker oder Marker-Einheiten in der Marker-Komponente für die Signalverstärkung eingesetzt werden können, sind in US Pat. 4882269, US Pat. 4687732, WO 8903849, US Pat. 6017707, US Pat. 6627469 beschrieben.

### 1.3.3.3.3 Die Kern-Komponente der Marker-Komponente

Die Kernkomponente hat die Funktion mehrere strukturelle Elemente der Nuk-Makromoleküle zu binden. Beispielsweise bindet die Kern-Komponente mehrere Marker-Einheiten zusammen. In einer weiteren Ausführungsform können Linker-Komponenten an die Kern-Komponente gebunden werden s. Fig. 5.

### 1.3.3.3.3.1 Bestandteile

In einer Ausführungsform besteht die Kern-Komponente aus einer oder mehreren niedermolekularen Verbindungen. Sie haben die Funktion, die Marker-Einheiten untereinander zu verbinden. Die Verbindung zwischen der Kern-Komponente und den Marker-Einheiten kann kovalent oder affin sein. Bei kovalenter Bindung können beispielsweise Verbindungen mit allgemeiner Struktur-Formel (F)ₘ-R-(H)ₙ als Ausgangsmateriel dienen, wo (F) und (H) - reaktive Gruppen darstellen, und R - eine Verbindungskomponente, wobei Zahl solcher Gruppen und ihre Anordnung stark variieren kann. Viele Beispiele sind dem Fachmann bekannt, z.B. Verbindungen aus der Gruppe Cross-Linker ("Chemistry of protein conjugation and crosslinking" Shan S. Wong 1993 CRC Press Inc.). Beispielsweise schließen (F) und (H) unabhängig von einander folgende Gruppen ein: NH2 (Amino), OH (Hydroxy), SH (Mercapto), COOH (Carboxy), CHO (Aldehyd), Acryl- oder Maleimid.

In einer Ausführungsform besteht die Kern-Komponente aus einem wasserlöslichen Polymer, dabei kann das Polymer aus gleichen oder unterschiedlichen Monomeren bestehen.

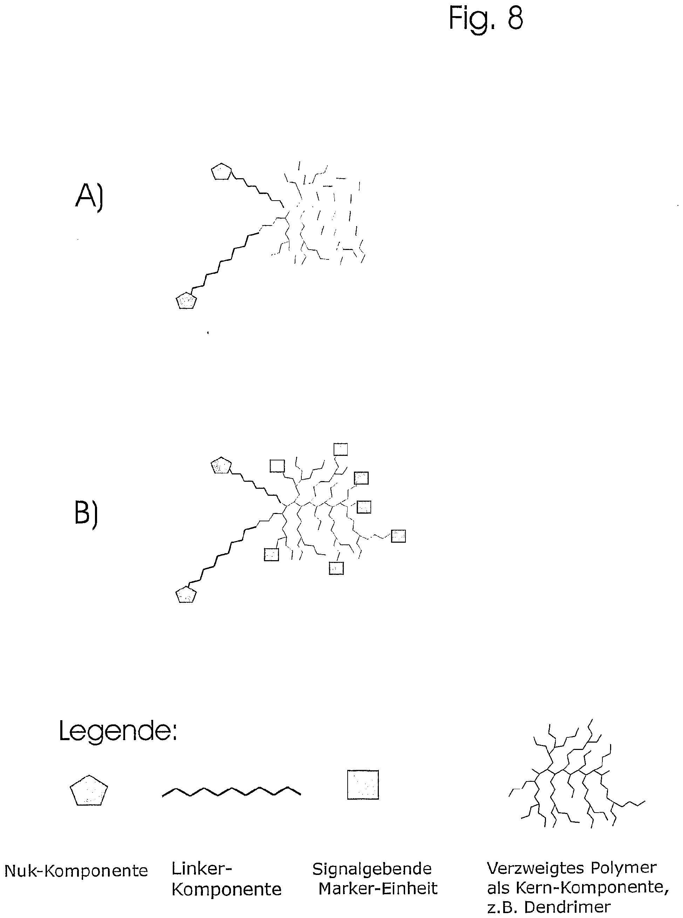

Beispiele für den Kernteil-Komponennte stellen Derivate folgender Polymere: Polyamide (z.B. Polypeptide), Poly-Acrylsäure, Poly-Acrylamide, Poly-Vinylalkohole, Nukleinsäuren, Proteinen. Diese Polymere können linear, globulär, z.B. Streptavidin oder Avidin, oder verzweigt sein, z.B. Dendrimere (Fig. 8a). Auch vernetzte lösliche Polymere, wie beispielswiese vernetzte Poly-Acrylamide (Crosslinker Bisacrylamid in Kombination mit Poly-Acrylamid), eingesetzt werden.

Die Kern-Komponennte hat vorzugsweise mehrere Kopplungsstellen, an die weitere Elemente gebunden werden können, z.B. strukturelle Marker-Einheiten oder Nuk-Linker-Komponente.

Beispielsweise Poly-Lysin-Moleküle haben multiple freie Aminogruppen, an die mehrere Farbstoffmoleküle, Biotinmoleküle, Haptenmoleküle oder Nukleinsäureketten gekoppelt werden können. Poly-Lysine haben unterschiedliche Molmasse, z.B. 1000-2000, 2000-10000, 10000-50000 Da können käuflich erworben werden.

Als weiteres Beispiel für die Kernkomponente dienen Nukleinsäurenstränge, wobei die Nukleinsäureketten eine Länge von 10 bis 20, von 20 bis 50, von 50 bis 100, von 100 bis 200, von 200 bis 500, von 500 bis 1000, 1000 bis 5000, von 5000 bis 10000 Nukleotiden haben. Diese Nukleinsäuren dienen als Bindungspartner für sequenzkomplementäre Markereinheiten (Fig. 7b).

In einer weiteren Ausführungsform besteht die Kern-Komponente aus einem Dendrimer, z.B. Polypropylenimine, Polyaminoamine. Beispiele für andere Dendrimere sind bekannt: Cientifica "Dendrimers", 2003, Technology white papers Nr. 6, Klajnert et al. Acta Biochimica Polonica, 2001, v. 48, S. 199, Manduchi et al. Physiol.

Genomics 2002, V.10, S.169, Sharma et al. Electrophoresis. 2003, V. 24, S. 2733, Morgan et al. Curr Opin Drug Discov Devel. 2002 Nov;5(6):966-73, Benters et al. Nucleic Acids Res. 2002 Jan 15;30(2):E10, Nilsen et al. J Theor Biol. 1997 Jul 21;187(2):273-84. Viele Dendrimere sind kommerziell erwerblich (Genisphere, www.genisphere.com, Chimera Biotech GmbH).

Weitere Kombinationen für die Kern-Komponennte aus den oben dargestellten Bestandteilen sind einem Fachmann naheliegend.

### 1.3.3.3.3.2 Kopplung der Marker-Einheiten

Marker-Einheiten können an die Kern-Komponente oder an die Linker-Komponente durch eine kovalente Bindung, beispielsweise über einen Cross-linker (Chemistry of protein conjugation and cross-linking, S. Wang,1993, ISBN 0-8493-5886-8, "Bioconjugation: protein coupling techniques for the biomedical sciences", M. Aslam, 1996, ISBN 0-333-58375-2), oder über eine affine Kopplung erfolgen, beispielsweise Biotin-Streptavidin-Verbindung oder Hybridisierung von Nukleinsäuresträngen oder Antigen-Antikörper-Wechselwirkung ("Bioconjugation: protein coupling techniques for the biomedical sciences", M. Aslam, 1996, ISBN 0-333-58375-2).

Die Kopplung von Marker-Einheiten an die Kern-Komponente erfolgt in einer Ausführungsform bereits während der Synthese der Nuk-Makromoleküle.

In einer anderen Ausführungsform erfolgt zunächst die chemische Synthese von Nuk-Makromoleküle, bei denen die Markerkomponente nur aus der Kern-Komponente besteht. Die Kopplung von Marker-Einheiten an die Kern-Komponente erfolgt erst nach dem Einbau von Nuk-Makromoleküle in die Nukleinsäurekette. Durch eine große Zahl der potenziellen Bindungsstellen am Kernteil ist die Wahrscheinlichkeit der Bindung der Marker-Einheiten an die Kern-Komponente und somit an die eingebaute Nuk-Komponente wesentlich größer im Vergleich zu konventionellen Nukleotid-Strukturen. Die Kopplungschemie im einzelnen hängt von der Struktur der Marker-Einheiten und der Struktur der Kern-Komponente ab.

**Kovalente Kopplung:** Die Verbindung zwischen den Marker-Einheiten und der Kern-Komponente kann in einer Ausführungsform widerstandsfähig sein (Beispiel 33), z.B. bei Temperaturen bis zu 100°C, für pH-Bereiche zwischen 3 und 12, und/oder resistent gegen hydrolytische Enzyme (z.B. Esterasen) sein. In einer anderen Ausführungsform der Erfindung ist die Verbindung zwischen der Nuk-Komponente und dem Linker unter milden Bedingungen spaltbar.

Beispiele für die Kopplung von Nukleinsäuren an Dendrimere (entspricht einer Kopplung von Marker-Einheiten an eine Kernkomponente) sind z.B. in Shchepinov et al. Nucleic Acids Res. 1999 Aug 1;27(15):3035-41, Goh et al. Chem Commun (Camb). 2002 Dec 21;(24):2954 dargestellt.

### 1.3.3.3.3.3 Kopplung zwischen Linker und Marker

Die Verbindung zwischen der Linker-Komponente und dem Marker hängt von der jeweiligen Struktur der Marker-Einheiten bzw. der Struktur der Kern-Komponente. In einer Ausführungsform ist die Linker-Komponente direkt an die signalgebende oder signalvermittelnde Marker-Einheit gebunden, Fig. 4a . Dabei kann der Marker aus nur einer oder mehreren Marker-Einheiten bestehen.

In einer weiteren Ausführungsform sind ein oder mehrere Linker-Komponenten an die Kern- Komponente des Markers gebunden, Fig. 5d. Dabei besteht der Marker aus mehreren Marker-Einheiten.

Die Bindung zwischen der Linker-Komponente und dem Marker kann sowohl kovalent als auch affine erfolgen. Viele Beispiele sind dem Fachmann bekannt, s. z.B. "Bioconjugation: protein coupling techniques for the biomedical sciences", M. Aslam, 1996, ISBN 0-333-58375-2. "Chemistry of protein conjugation and crosslinking" Shan S. Wong 1993 CRC Press Inc.).

**Kovalente Kopplung:** Die Verbindung zwischen der Linker-Komponente und dem Marker kann in einer Ausführungsform widerstandsfähig sein, z.B. bei Temperaturen bis zu 130°C, für pH-Bereiche zwischen 1 und 14, und/oder resistent gegen hydrolytische Enzyme (z.B. Proteasen, Esterasen)sein . In einer anderen Ausführungsform der Erfindung ist die Verbindung zwischen der Nuk-Komponente und dem Linker unter milden Bedingungen spaltbar.

### 1.3.3.3.4 Das Verhältnis der Nuk- Komponenten in einem Nuk-Makromolekül

Ein Nuk-Makromolekül kann durchschnittlich 1 bis 2, 2 bis 5, 5 bis 10, 10 bis 30, 30 bis 100, 100 bis 1000 Nuk-Komponenten einschließen.

In einer Ausführungsform haben alle Nuk-Makromoleküle eine gleiche Anzahl an Nuk-Komponenten pro ein Nuk-Makromolekül. Beispielsweise können pro ein Strepavidin-Molekül maximal 4 Biotin-Moleküle gebunden werden, bei sättigender Konzentration von Nuk-Linker- Komponenten, eine einheitliche Population an Nuk-Makromolekülen entsteht.

In einer anderen Ausführungsform haben die Nuk-Makromoleküle einer Population eine definierte durchschnittliche Anzahl von Nuk- Komponenten pro Nuk-Makromolekül, in der Population selbst findet allerdings eine Verteilung der tatsächlichen Besetzung der Nuk-Makromoleküle mit Nuk- Komponenten statt. Die Verteilungsangaben von Nuk- Komponenten pro Nuk-Makromolekül stellen in diesem Fall einen Mittelwert dar.

### 1.3.3.3.5 Das Verhältnis von Marker-Einheiten in einem Nuk-Makromolekül

Die Zahl der Marker-Einheiten in einem Nuk-Makromolekül schließt folgende Bereiche ein: 1 und 2, 2 und 5, 5 und 20, 20 und 50, 50 und 100, 100 und 500, 500 und 1000. 1000 und 10000, 10000 und 100000.
In einer Ausführungsform der Erfindung haben Nuk-Makromoleküle eine feste Anzal von signalgebenden Einheiten pro Marker.
In einer anderen Ausführunsform kann die Verteilung von Marker-Einheiten in einer Nuk-Makromolekül-Population schwanken und muß nicht notwendigerweise einen konstanten Wert für jedes einzelne Nuk-Makromolekül darstellen.

In einer Ausführungsform haben alle Nuk-Makromoleküle eine gleiche Anzahl der Marker-Einheiten pro ein Nuk-Makromolekül. Beispielsweise können pro ein Strepavidin-Molekül maximal 4 Biotin-Moleküle gebunden werden, Avidin-Biotin-Technology, Methods in Enzymology v.184, 1990..

In einer anderen Ausführungsform haben die Nuk-Makromoleküle einer Population eine definierte durchschnittliche Zahl der Marker-Einheiten pro Nuk-Makromolekül, in der Population selbst findet allerdings eine Verteilung der tatsächlichen Besetzung der Nuk-Makromoleküle mit Marker-Einheiten statt. Bei sättigenden Konzentrationen bei der Synthese von Marker-Komponenten findet eine zunehmend einheitlichere Besetzung der Nuk-Makromoleküle mit Marker-Einheiten statt.

So spielt beispielsweise in Bereichen, wo es um den qualitativen Nachweis geht, die exakte Zahl der Marker-Einheiten pro Nuk-Makromolekül eine untergeordnete Rolle. In solchen Fällen ist das Vorhandensein eines stabilen Signals an sich von Bedeutung.

Es sollte einem Fachmann naheliegend erscheinen, dass die angeführten Marker-Komponenten eine beträchtlich größere Molekül-Größe und -Masse haben, als die jeweilige Nuk-Komponente selbst. Weiterhin sollten andere Beispiele für makromolekulare Marker-Komponenten einem Fachmann naheliegend erscheinen.

### 1.3.3.3.6 Substrateigenschaften der Nuk-Makromoleküle

Die Nuk-Komponente gebunden an ein Nuk-Makromolekül kann als Substrat für unterschiedliche Enzyme dienen. Beispielsweise dient die Nuk-Komponente, in dieser Ausführungsform als Nukleosid-Triphosphat, als Substrat für eine Polymerase, so dass die Nuk-Komponente in einen wachsenden Strang durch eine Polymerase eingebaut werden kann und somit das gesamte Nuk-Makromolekül an den Strang kovalent gekoppelt wird.
Weitere Beispiele für Enzyme stellen Kinasen, Phosphorylasen, Transferasen dar.

Als Monomer-Teil einer Nukleinsäurekette können Nuk-Makromoleküle ebenfalls als Substrate für Enzyme auftreten, beispielsweise für die 3'- oder 5'-Exonukleasen oder Endonukleasen ("Molecular cloning" 1989, Ed. Maniatis, Cold Spring Harbor Laboratory), oder für die entsprechenden Teilaktivitäten von Polymerasen, wie beispielsweise in für real-time-PCR beschrieben (S. Meuer "Rapid cycle real time PCR", Springer 2004, ISBN 3-540-66736-9, T. Weissensteiner "PCR-Technology: current innovations" CRC Press 2004 ISBN 0-8493-1184-5).

Die Substrateigenschaften der Nuk-Komponente(n) bestimmt/bestimmen die Substrateigenschaften der Nuk-Makromoleküle. So kann die Nuk-Komponente als ein Terminator dienen, so dass nur ein einziges Nuk-Makromolekül eingebaut werden kann. In einer anderen Ausführungsform dient die Nuk-Komponente als ein reversibler Terminator, der die Durchführung einer schrittweise kontrollierten Verlängerungsreaktion erlaubt, wie z.B. in Ju et al. US Pat. 6664079, Tcherkassov WO 02088382 dargestellt ist.

Als Monomer-Teil einer Nukleinsäurekette können sie alleine für die enzymatischen Eigenschaften bestimmtend sein, wie z.B. für Exonuklease-Aktivität. In einer anderen Ausführungsform bestimmen die enzymatischen Eigenschaften nicht nur die Nuk-Komponenten der Nuk-Makromoleküle, sondern auch die benachbarten Nukleotide, wie im Falle der Endonukleasen.

### 1.3.3.3.7 Funktion der Marker

Die makromolekulare Marker-Komponente kann in einer Ausführungsform eine signalgebende Funktion haben. In einer anderen Ausführungsform hat sie eine signalvermittelnde Funktion. In einer weiteren Ausführungsform hat sie eine katalytische Funktion. In einer weiteren Ausführungsform hat sie eine affine Funktion. In einer weiteren Ausführungsform hat der Marker mehr als nur eine Funktion, sondern z.B. vereinigt sowohl signalgebende als auch eine signalvermitteldne Funktion. Weitere Kombinationen sind nahe liegend.

Bei der signalgebenden Funktion enthält die Marker-Komponente Bestandteile, die bereits während der chemischen Synthese an Nuk-Makromoleküle gebunden werden, s. Beispiel 33.

Bei der signalvermittelnden Funktion trägt die Marker-Komponente Bestandteile, die erst durch eine Reaktion mit signalgebenden Molekülen ihre Signaleigenschaften entfalten, s. Beispiel 32.
Beispielsweise können mehrere Biotin-Moleküle, z.B. 100 Biotin-Moleküle, den Marker-Teil bilden. Nach dem Einbau der Nuk-Makromoleküle erfolgt eine Nachweisreaktion mit modifizierten Streptavidin-Molekülen. In einem anderen Beispiel haben die Nukleinsäureketten die signalvermittelnde Funktion. Nach dem Einbau von Nuk-Makromoleküle, erfolgt eine Hybridisierung von einheitlichen Oligonukleotiden mit detektierbaren Einheiten, z.B. Fluoreszenzfarbstoffen (MWG-Biotech) an die Marker-Komponente. In einem weiteren Beispiel haben Amino- oder Merkapto-Gruppen, beispielsweise 50 Aminogruppen pro Marker, die signalvermittelnde Funktion. Nach dem Einbau der Nuk-Makromoleküle in die Nukleinsäurekette erfolgt eine chemische Modifikation mit reaktiven Komponenten, z.B. mit Farbstoffen, wie beispielsweise für eingebaute Allyl-Amino-dUTP beschrieben, Diehl et al. Nucleic Acid Research, 2002, V. 30, Nr. 16 e79.

In einer anderen Ausführungsform hat die makromolekulare Marker-Komponente eine katalytische Funktion (in Form eines Enzyms oder Ribozyms). Dabei können unterschiedliche Enzyme verwendet werden, z.B. Peroxidase oder alkalische Phosphatase. Dank der Kopplung an die Nuk-Komponente kann das jeweilige Enzym durch den Einbau von Nuk-Makromolekülen an den Nukleinsäurestrang kovalent gebunden werden.

In einer weiteren Ausführungsform hat die makromolekularer Marker-Komponente eine Affinitätsfunktionalität zu einem anderen Molekül. Beispiele für solche Marker bilden Streptavidin-Moleküle, Antikörper oder Nukleinsäureketten, Beispiel 30 oder 32.

**1.3.4. Niedermolekularer Marker -** zum Stand der Technik gehörende Markierung von Nukleotiden beispielsweise mit ein oder zwei Biotin-Molekülen, ein oder zwei Farbstoff-Molekülen, ein oder zwei Hapten-Moleküln (z.B. Digoxigenin).

**1.3.5. Konventionell modifiziertes Nukleotid** - ein Nukleotid mit einem Linker (durchschnittliche Länge zwischen 5 und 30 Atomen) und einem Marker. Üblicherweise trägt ein konventionell modifiziertes Nukleotid einen niedermolekularen Marker, z.B. ein Farbstoff- oder ein Biotinmolekül.
Zu Demonstration der Tatsache, dass eine einfache Kombination aus einem konventionell modifizierten Nukleotid mit einem makromolekularen Marker zur Aufhebung der Substrateigenschaften der Nukleotide führt, werden in dieser Anmeldung Nukleotide dargestellt, die zwar einen makromolekularen Marker aber einen kurzen Linker mit einer einer durchschnittlichen Länge von 5 bis 30 Atome tragen. Auch solche Nukleotide werden als konventionell modifizierte Nukleotide bezeichnet. Alleine die Kombination zwischen einem konventionell modifizierten Nukleotid und einem makromolekularen Marker reicht erfindungsgemäß nicht aus, um Anforderungen der Definition der Nuk-Makromoleküle zu erfüllen.

Im Gegensatz dazu, können Nuk-Makromoleküle durch eine Kombination aus einer oder mehreren Nuk-Komponenten, entsprechend einem oder mehreren langen Linkern und einem Marker definiert werden.

### 1.3.6. Enzyme (Polymerasen)

In einer Ausführungsform können die Nuk-Makromoleküle als Substrate für Enzyme eingesetzt werden. Polymerasen stellen in Anwendungen oft eingesetzte Enzyme, die Nukleotide als Substrate verwerden. Sie werden im weiteren beispielhaft und stellvertretend für andere Nukleotid-umsetzende Enzyme betrachtet. Eine der zentralen Fähigkeit der Polymerasen besteht in kovalenten Kopplung von Nukleotid-Monomeren zu einem Polymer. Dabei kann die Synthese sowohl matrizen-abhängig (wie z.B. DNA-oder RNA-Synthese mit DNA- oder RNA-abhängigen Polymerasen) als auch matrizenunabhängig, z.B. durch Terminale Transferasen ("J Sambrook "Molecular Cloning" 3. Ed. CSHL Press 2001).

Falls RNA als Substrat (z.B. mRNA) in die Sequenzierungsreaktion eingesetzt wird, können handelsübliche RNA-abhängige DNA-Polymerasen eingesetzt werden, z.B. AMV-Reverse Transcriptase (Sigma), M-MLV Reverse Transcriptase (Sigma), HIV-Reverse Transcriptase ohne RNAse-Aktivität. Für bestimmte Anwendungen, können Reverse Transcriptasen von RNAse-Aktivität weitgehend frei sein ("Molecular cloning" 1989, Ed. Maniatis, Cold Spring Harbor Laboratory), z.B. bei mRNA-Makrierung für Hybridisierungsexperimente.

Falls DNA als Substrat (z.B. cDNA) verwendet wird, eignen sich als Polymerasen prinzipiell alle DNA-abhängigen DNA-Polymerasen mit oder ohne 3'-5' Exonuklease-Aktivität (DNA-Replication" 1992 Ed. A.Kornberg, Freeman and company NY), z.B. modifizierte T7-Polymerase vom Typ "Sequenase Version 2" (Amersham Pharmacia Biotech), Klenow Fragment der DNA-Polymerase I mit oder ohne 3'-5' Exonukleaseaktivität (Amersham Pharmacia Biotech), Polymerase Beta verschiedenen Ursprungs (Animal Cell DNA Polymerases" 1983, Fry M., CRC Press Inc., kommerziell erhältlich bei Chimerx) thermostabile Polymerasen wie beispielsweise Taq-Polymerase (GibcoBRL), proHA-DNA-Polymerase (Eurogentec), Vent, Vent exo minus, Pfu, Thermosequenase, Pwo-Polymerase usw. (Promega).

Auch DNA-abhängige RNA-Polymerasen können verwendet werden, z.B. E.coli RNA-Polymerase, T7-RNA-Polymerase, SP6 RNA Polymerase.

Die Polymerasen können eine 3'- oder eine 5' -Exonuklease-Aktivität aufweisen und in bestimmten Anwendungen eingesetzt werden (z.B. bei real-time PCR).
In der Anmeldung werden DNA-abhängige DNA-Polymerasen als Beispiele für Polymerasen betrachtet.

**1.3.7. Spaltbare Verbindung -** Eine unter milden Bedingungen spaltbare Verbindung. Diese Verbindung kann einen Abschnitt im Linker darstellen und kann an einer oder an mehreren Stellen spaltbar sein. Es kann sich um eine chemisch spaltbare Verbindung, wie z.B. eine Disulfid-, eine Ester-, eine Acetal-, eine Thioesterverbindung (Short WO 9949082, Tcherkassov WO 02088382) handeln. Es kann auch eine photochemisch spaltbare Verbindung, wie in (Rothschild WO 9531429) dargestellt sein. Es kann auch eine enzymatisch spaltbare Verbindung (beispielsweise eine Peptid- oder Polypeptide-Bindung, Odedra WO 0192284), spaltbar durch Peptidasen, eine Poly- oder Oligosaccharid-Bindung, spaltbar durch Disaccharidasen) sein, wobei die Spaltung durch ein spezifisches Enzym zwischen bestimmten Monomeren der spaltbaren Stellen stattfinden kann.
Mehrere Beispiele für spaltbare Verbindungen sind bekannt. Die Kopplung einer solchen Verbindung ist beispielsweise beschrieben in (Tcherkassov 02088382, Metzker et al. Nucleic Acid Research 1994, V.22, S. 4259, Canard et al. Gene, 1994, V. 148, 1, , Kwiatkowski US Pat. 6255475, Kwiatkowski WO 01/25247, Parce WO 0050642.). Eine Spaltbare Verbindung kann ein Teil des Linkers sein oder die Kopplungsstelle des Linkers an das Nukleotid bilden, oder die Verbindung zwischen der Linker-Komponente und der Makrer-Komponente, oder die Verbindung zwischen den Marker-Einheiten und der Kern-Komponente.

**1.3.8** DNA - Deoxyribonukleinsäure verschiedenen Ursprungs und unterschiedlicher Länge (z.B. Oligonukleotide, Polynukleotide, Plasmide, genomische DNA, cDNA, ssDNA, dsDNA)

**1.3.9** RNA - Ribonukleinsäure

**1.3.10** dNTP - 2'-deoxi-Nucleosid-Triphosphate, Substrate für DNA-Polymerasen und Reverse-Transkriptasen, z.B. dATP, dGTP, dUTP, dTTP, dCTP.

**1.3.11** NTP - Ribonukleosid-Triphosphate, Substrate für RNA-Polymerasen, UTP, CTP, ATP, GTP.

**1.3.12** Abkürzung "NT" wird auch bei der Längenangabe einer Nukleinsäuresequenz verwendet, z.B. 1.000 NT. In diesem Fall steht "NT" für Nukleosid-Monophosphate.

Im Text wird bei Abkürzungen die Mehrzahl durch Verwendung des Suffixes "s" gebildet, "NT" steht zum Beispiel für "Nukleotid", "NTs" steht für mehrere Nukleotide.

**1.3.13** NSK - Nukleinsäurekette. DNA oder RNA

**1.3.14** Gesamtsequenz - Summe aller Sequenzen im Ansatz, sie kann ursprünglich aus einer oder mehreren NSKs bestehen. Dabei kann die Gesamtsequenz Teile oder Äquivalente einer anderen Sequenz oder von Sequenz-Populationen darstellen (z.B. mRNA, cDNA, Plasmid-DNA mit Insert, BAC, YAC) und aus einer oder unterschiedlichen Spezies stammen.

**1.3.15** NSKF - Nukleinsäurekettenfragment (DNA oder RNA), das einem Teil der Gesamtsequenz entspricht, NSKFs - Nukleinsäurekettenfragmente. Die Summe der NSKFs bildet ein Äquivalent zur Gesamtsequenz. Die NSKFs können beispielsweise Fragmente von DNA- oder RNA-Gesamtsequenz sein, die nach einem Fragmentierungsschritt entstehen.

**1.3.16** Primerbindungstelle (PBS) - Teil der Sequenz in der NSK oder NSKF, an den der Primer bindet.

**1.3.17** Referenzsequenz - eine bereits bekannte Sequenz, zu der die Abweichungen in der zu untersuchenden Sequenz bzw. in den zu untersuchenden Sequenzen (z.B. Gesamtsequenz) ermittelt werden. Als Referenzsequenzen können in Datenbanken zugängliche Sequenzen verwendet werden, wie z.B. aus der NCBI-Datenbank.

**1.3.18** Tm - Schmelztemperatur

**1.3.19** Sterisches Hindernis: Sterisch anspruchsvolle Gruppe, die durch ihre chemische Struktur die Eigenschaften der mit dieser Gruppe gekoppelten Nukleotide so verändert, dass diese durch eine Polymerase in einer Extensionsreaktion nicht nacheinander eingebaut werden können. Eine an die Base gekoppelte sterisch anspruchsvolle Gruppe kann zur Behinderung der weiteren Synthese führen. Biotin, Digoxigenin und Fluoreszenzfarbstoffe wie Fluoreszein, Tetramethylrhodamine, Cy3-Farbstoff stellen Beispiele einer solchen sterisch anspruchsvollen Gruppe dar (Zhu et al. Cytometry 1997, v.28, S.206, Zhu et al. NAR 1994, v.22, S.3418, Gebeyehu et al., NAR 1987, v.15, S.4513, Wiemann et al. Analytical Biochemistry 1996, v.234, S.166, Heer et al. BioTechniques 1994 v.16 S.54)

**1.3.20** PNA - Peptide Nucleic Acid

### 2. Detaillierte Beschreibung:

Die Erfindung beschreibt eine neue Klasse an modifizierten Nukleotiden. 1. Aspekt der Erfindung betrifft makromolekulare Verbindungen mit der Struktur:

(Nuk-Linker)ₙ-Marker

wobei:
- Nuk: - ein Nukleotid oder ein Nukleosid ist (Nuk-Komponente)
- Linker: - eine Linker-Komponente, die folgende Bestandteile einschließt:
a) Kopplungseinheit L - ein Teil des Linkers, das die Verbindung zwischen Nuk und dem Linkerrest darstellt
b) Polymer - ein Teil des Linkers, das ein wasserlösliches Polymer mit einer durchschnittlichen Länge zwischen 100 und 10.000 Atome ist
c) Kopplungseinheit T - ein Teil des Linkers, das die Verbindung zwischen dem Linkerrest und dem Marker darstellt
- Marker: - eine Marker-Komponente ist
- n: - eine ganze Zahl zwischen 1 und 100 ist

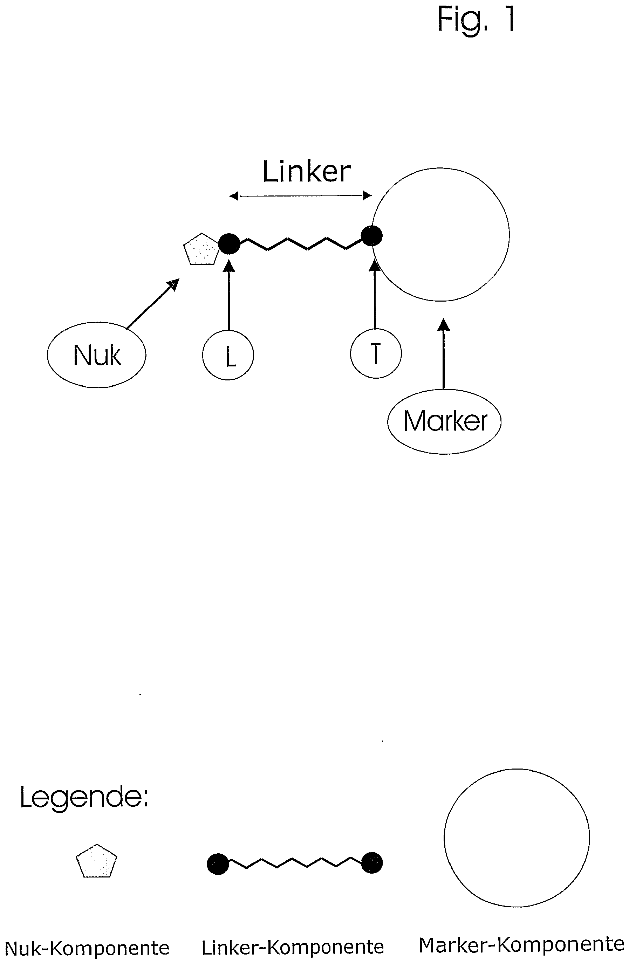

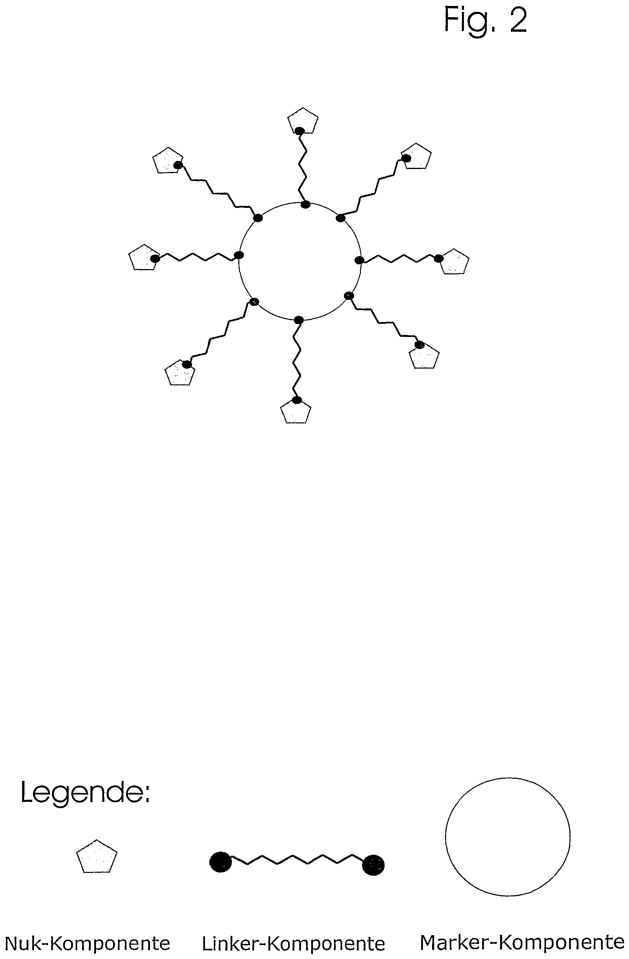

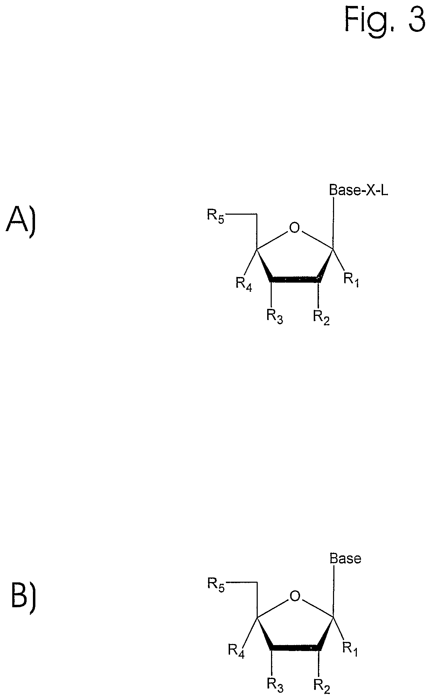

Ein weiterer Aspekt 2 der Erfindung betrifft makromolekulare Verbindungen nach Aspekt 1, wobei die Nuk-Komponente folgende Strukturen einschließt (Fig. 3A):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren Modifikationen, wobei L die Verbindung zwischen der Nuk-Komponente und der Linker-Komponente darstellt (Kopplungseinheit L) und X die Kopplungsstelle der Kopplungseinheit L an der Base ist
   R₁ - ist H
   R₂ - ist unabhängig gewählt aus der Gruppe H, OH, Halogen, NH2, SH oder geschützte OH-Gruppe
   R₃ - ist unabhängig gewählt aus der Gruppe H, OH, Halogen, PO₃, SH, N₃, NH₂, O-R₃₋₁, P(O)ₘ-R₃₋₁ (m ist 1 oder 2 ist), NH-R₃₋₁, S-R₃₋₁, Si-R₃₋₁ wobei R₃₋₁ eine chemisch, photochemisch oder enzymatisch spaltbare Gruppe ist, oder eine der folgenden Modifikationen einschließt: -CO-Y, -CH₂-O-Y, -CH₂-S-Y, -CH₂-N₃, -CO-O-Y, -CO-S-Y, -CO-NH-Y, -CH₂-CH=CH₂, wobei Y ein Alkyl ist, beispielsweise (CH₂)ₙ-CH₃ wobei n zwischen 0 und 4 liegt, oder ein substituiertes Alkyl ist, beispielsweise mit Halogen, Hydroxy, Amino, Carboxy-Gruppen).
   R₄ - ist H oder OH
   R₅ - ist unabhängig gewählt aus der Gruppe OH, oder eine geschützte OH-Gruppe, eine Monophosphat-Gruppe, oder eine Diphosphat-Gruppe, oder eine Triphosphat-Gruppe, oder eine Alpha-Thiotriphosphat-Gruppe ist.

Ein weiterer Aspekt 3 der Erfindung betrifft makromolekulare Verbindungen nach Aspekt 1, wobei die Nuk-Komponente folgende Strukturen einschließt (Fig. 3B):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen
   R₁ - ist H
   R₂ - ist unabhängig gewählt aus der Gruppe H, OH, Hal, NH₂, SH oder geschützte OH-Gruppe
   R₃ - ist unabhängig gewählt aus der Gruppe O- R₃₋₂-L, P(O)ₘ- R₃₋₂-L, wobei m 1 oder 2 ist, NH-R₃₋₂-L, S-R₃₋₂-L, Si-R₃₋₂-L oder, wobei R₃₋₂ die Kopplungsstelle des Linkers an das Nukleotid ist und L - die Kopplungseinheit des Linkers (L) ist.
   R₄ - ist H oder OH
   R₅ - ist unabhängig gewählt aus der Gruppe OH, oder eine geschützte OH-Gruppe, eine Monophosphat-Gruppe, oder eine Diphosphat-Gruppe, oder eine Triphosphat-Gruppe, oder eine Alpha-Thiotriphosphat-Gruppe ist.

Ein weiterer Aspekt 4 der Erfindung betrifft makromolekulare Verbindungen nach Aspekt 1, wobei die Nuk-Komponente folgende Strukturen einschließt (Fig. 3B):
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen
   R₁ - ist H
   R₂ - ist unabhängig gewählt aus der Gruppe H, OH, Hal, NH2, SH oder geschützte OH-Gruppe
   R₃ - ist unabhängig gewählt aus der Gruppe H, OH, Hal, PO₃, SH, NH₂, O- R₃₋₁, P(O)m- R₃₋₁, NH-R₃₋₁, S-R₃₋₁, Si-R₃₋₁ wobei R₃₋₁ eine chemisch, photochemisch oder enzymatisch spaltbare Gruppe ist und m 1 oder 2 ist.
   R₄ - ist H oder OH
   R₅ - ist unabhängig gewählt aus der Gruppe O- R₅₋₁-L, oder P-(O)₃- R₅₋₁-L (modifizierte Monophosphat-Gruppe), oder P-(O)₃-P-(O)₃-R₅₋₁-L (modifizierte Diphosphat-Gruppe)
   oder P-(O)₃-P-(O)₃₋P-(O)₃- R₅₋₁-L (modifizierte Triphosphat-Gruppe), wobei R₅₋₁ die Kopplungsstelle der Kopplungseinheit L an das Nukleotid ist und Kopplungseinheit L die Verbindung zwischen der Nuk-Komponente und der Linker-Komponente ist.

Ein weiterer Aspekt 5 der Erfindung betrifft makromolekulare Verbindungen nach Aspekten 1 bis 4, wobei Kopplungseinheit L folgende strukturelle Elemente einschließt:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇,
   R₆-(CH=CH-)ₙ-R₇, R₆-(A-CH=CH-)ₙ-R₇, R₆-(CH=CH-B-)ₙ-R₇, R₆-(CH=CH-CH₂-B-)ₙ-R₇, R₆-A-CH=CH-(CH₂-)ₙ-R₇, R₆-(-CH=CH-CH₂)ₙ-B-R₇,
   R₆-(C≡C-)ₙ-R₇, R₆-(A-C≡C-)ₙ-R₇, R₆-(A-C≡C-CH₂)ₙ-R₇, R₆-(C≡C-B-)ₙ-R₇, R₆-(C≡C-CH₂-B-)ₙ-R₇, R₆-A-C≡C-(CH₂-)ₙ-R₇, R₆-(-C≡C-CH₂)ₙ-B-R₇, R₆-(-C≡C-CH₂-CH₂)ₙ-B-R₇
wobei R₆ - die Nuk-Komponente ist, R₇ - der Linkerrest ist und A und B unabhängig folgende strukturelle Elemente einschließen: -NH-, -O-, -S-, -SS-, - CO-NH-, -NH-CO-, -CO-O-, -O-CO-, -CO-S-, -S-CO-, -P(O)₂-, -Si-, -(CH₂)ₙ-, eine photolabile Gruppe, wobei n - gleich 1 bis 5 ist

Ein weiterer Aspekt 6 der Erfindung betrifft makromolekulare Verbindungen nach Aspekten 1 bis 5, wobei die Linker-Komponente ein wasserlösliches Polymer einschließt.

Ein weiterer Aspekt 7 der Erfindung betrifft makromolekulare Verbindungen nach Aspekt 6, wobei die Linker-Komponente wasserlösliche Polymere unabhängig ausgewählt aus folgender Gruppe einschließt:
Polyethylen-glycol (PEG), Polysaccharide, Dextran, Polyamide, Polypeptide, Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acrylsäure, Poly-Acrylamid, Poly(Vinylalkohol).

Ein weiterer Aspekt 8 der Erfindung betrifft makromolekulare Verbindungen nach Aspekten 1 bis 7, wobei die Linker-Komponente eine durchschnittliche Länge zwischen 50 bis 100, 100 bis 200, 200 bis 500, 500 bis 1000, 1000 bis 2000, 2000 bis 10000, 10000 bis 100000 Atomen hat.

Ein weiterer Aspekt 9 der Erfindung betrifft makromolekulare Verbindungen nach Aspekten 1 bis 8, wobei eine Marker-Komponente eine signalgebende, signalvermittelnde, katalytische oder affine Funktion hat

Ein weiterer Aspekt 10 der Erfindung betrifft makromolekulare Verbindungen nach Aspekten 1 bis 9, wobei eine Marker-Komponente aus einer strukturellen Marker-Einheit besteht

Ein weiterer Aspekt 11 der Erfindung betrifft makromolekulare Verbindungen nach Aspekten 1 bis 9, wobei eine Marker-Komponente aus mehreren strukturellen Marker-Einheiten gebunden an eine Kern-Komponente besteht

Ein weiterer Aspekt 12 der Erfindung betrifft makromolekulare Verbindungen nach Aspekten 10 oder 11, wobei eine strukturelle Marker-Einheit unabhängig eines der folgenden strukturellen Elemente einschließt:
Biotin, Hapten, radioaktives Isotop, seltenes Erdenelement, Farbstoff, Fluoreszenzfarbstoff.

Ein weiterer Aspekt 13 der Erfindung betrifft makromolekulare Verbindungen nach Aspekten 10 oder 11 , wobei eine strukturelle Marker-Einheit unabhängig eines der folgenden Elemente einschließt:
Nanokristalle oder deren Modifikationen, Proteine oder deren Modifikationen, Nukleinsäureketten oder deren Modifikationen, Teilchen oder deren Modifikationen.

Ein weiterer Aspekt 14 der Erfindung betrifft makromolekulare Verbindungen nach Aspekt 13, wobei eine strukturelle Marker-Einheit eines der folgenden Proteine einschließt:
Enzyme oder deren Konjugate oder Modifikationen,
Antikörper oder deren Konjugate oder Modifikationen,
Streptavidin oder seine Konjugate oder Modifikationen,
Avidin oder seine Konjugate oder Modifikationen

Ein weiterer Aspekt 15 der Erfindung betrifft makromolekulare Verbindungen nach Aspekt 13, wobei eine strukturelle Marker-Einheit eine der folgenden Arten Nukleinsäureketten einschließt: DNA, RNA, PNA, wobei die Länge von Nukleinsäureketten zwischen 10 und 10.000 Nukleotiden oder deren Äquivalente liegt.

Ein weiterer Aspekt 16 der Erfindung betrifft makromolekulare Verbindungen nach Aspekten 11 bis 15, wobei die Kern-Komponente der Marker-Komponente unabhängig eines der folgenden Elemente einschließt: wasserlöslichen Polymer unabhängig ausgewählt aus der Gruppe von: Polyamide (z.B. Polypeptide), Poly-Acrylsäure und deren Derivate, Poly-Acrylamide und deren Derivate, Poly-Vinylalkohole und deren Derivate, Nukleinsäureketten und deren Derivate, Streptavidin oder Avidin und deren Derivate, Dendrimere, wobei einzelne Polymere linear oder verzweigt oder unter einander vernetzt sein können

Ein weiterer Aspekt 17 der Erfindung betrifft makromolekulare Verbindungen nach Aspekten 1 bis 9, 11 bis 16, wobei die Verbindung zwischen mehreren strukturellen Marker-Einheiten und der Kern-Komponte kovalent oder affine erfolgt.

Ein weiterer Aspekt 18 der Erfindung betrifft makromolekulare Verbindungen nach Aspekten 1 bis 10, wobei die Verbindung zwischen einer strukturellen Marker-Einheit und dem Linker kovalent oder affine erfolgt.

Ein weiterer Aspekt 19 der Erfindung betrifft makromolekulare Verbindungen nach Aspekten 1 bis 9, 11 bis 17, wobei die Verbindung zwischen der Kern-Komponente und dem Linker kovalent oder affine erfolgt

Ein weiterer Aspekt 20 der Erfindung betrifft makromolekulare Verbindungen nach Aspekten 1 bis 19, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt.

Ein weiterer Aspekt 21 der Erfindung betrifft makromolekulare Verbindungen nach Aspekten 1 bis 20, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt und die Linker-Komponente eine oder mehrere unter milden Bedingungen spaltbare Verbindungen beinhaltet.

Ein weiterer Aspekt 22 der Erfindung betrifft makromolekulare Verbindungen nach Aspekten 1 bis 21, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt und ein oder mehrere Teile des Nuk-Makromoleküls dermaßen modifiziert sind, dass nur eine Nuk-Komponente in einen wachsenden Strang eingebaut werden kann.

Ein weiterer Aspekt 23 der Erfindung betrifft makromolekulare Verbindungen nach Aspekten 1 bis 19, wobei mehrere Nuk-Komponenten jeweils über einen Linker an einer Marker-Komponenten gekoppelt sind, wobei die Länge der jeweiligen Linker- Komponente zwischen 50 bis 100, 100 bis 200, 200 bis 500, 500 bis 1000, 1000 bis 2000, 2000 bis 5000 Atome beträgt.

Ein weiterer Aspekt 24 der Erfindung betrifft makromolekulare Verbindungen nach Aspekten 1 bis 19, 23, wobei mehrere Nuk- Komponenten jeweils über einen Linker an einer Marker-Komponenten gekoppelt, wobei die Länge der jeweiligen Linker- Komponente zwischen 50 bis 100, 100 bis 200, 200 bis 500, 500 bis 1000, 1000 bis 2000, 2000 bis 5000 Atome beträgt und der jeweilige Linker eine oder mehrere unter milden Bedingungen spaltbare Verbindungen beinhaltet.

Ein weiterer Aspekt 25 der Erfindung betrifft makromolekulare Verbindungen nach Aspekten 1 bis 19, 23, 24, wobei mehrere Nuk- Komponenten jeweils über einen Linker an einer Marker- Komponenten gekoppelt, wobei die Länge der jeweiligen Linker- Komponente zwischen 50 bis 100, 100 bis 200, 200 bis 500, 500 bis 1000, 1000 bis 2000, 2000 bis 5000 Atome beträgt und ein oder mehrere Teile des Nuk-Makromoleküls dermaßen modifiziert sind, dass nur eine Nuk-Komponente in eine wachsende Nukleinsäurekette eingebaut werden kann.

Ein weiterer Aspekt 26 der Erfindung betrifft Oligonucleotide oder Polynucleotide die mindestens ein Nuk-Makromolekül nach Aspekten 1 bis 25 pro eine Nukleinsäurekette einschließen.

Ein weiterer Aspekt 27 der Erfindung betrifft Oligonucleotide oder Polynucleotide nach Aspekt 26, wobei Oligo- oder Polynucleotide RNA, DNA oder PNA sind, deren Länge zwischen 5 und 50.000 Nukleotide beträgt.

Ein weiterer Aspekt 28 der Erfindung betrifft ein Verfahren zur Modifizierung von Nukleinsäureketten, wobei für die Kopplung Nuk-Makromoleküle nach Aspekten 1 bis 25 verwendet werden

Ein weiterer Aspekt 29 der Erfindung betrifft ein Verfahren nach Aspekt 28, wobei die Modifizierung durch eine enzymatische Kopplung erfolgt und das Reaktionsgemisch folgende Komponenten einschließt:
- mindestens eine Art der Nuk-Makromoleküle oder deren Zwischenstufen nach Aspekten 1 bis 25, wobei jede Art der Nuk-Makromoleküle eine für sie charakteristische Markierung besitzt
- mindestens eine Population der Nukleinsäureketten,
- mindestens eine Enzymart zur Kopplung von Nuk-Makromoleküle an die Nukleinsäureketten,

Ein weiterer Aspekt 30 der Erfindung betrifft ein Verfahren nach Aspekt 28, wobei die Modifizierung durch eine enzymatische Kopplung erfolgt und das Reaktionsgemisch folgende Komponenten einschließt:
- mindestans eine Art der Nuk-Makromoleküle oder deren Zwischenstufen nach Aspekten 1-25, wobei jede Art der Nuk-Makromoleküle eine für sie charakteristische Markierung besitzt
- mindestens eine Population der Nukleinsäureketten,
- mindestens eine Enzymart zur Kopplung von Nuk-Makromoelküle an die Nukleeinsäureketten
- mindestens eine weitere Art von Nukleosidtriphosphaten

Ein weiterer Aspekt 31 der Erfindung betrifft ein Verfahren nach Aspekten 29, 30, wobei die Enzymart unabhängig eine der folgenden Gruppen einschließt: DNA-Polymerase, RNA-Polymerase, Terminale Transferase,

Ein weiterer Aspekt 32 der Erfindung betrifft ein Verfahren nach Aspekt 30, wobei die "weitere Art" der Nukleosidtriphosphaten unabhängig ausgewählt ist aus der Gruppe von: Ribo-Nukleosidtriphosphaten (ATP, GTP, UTP, CTP), von 2'-Deoxyribonukleosid-Triphosphaten (dATP, dUTP, dTTP, dCTP, dGTP), von 2',3' - Dideoxynukleosidtriphosphaten (ddATP, ddGTP, ddUTP, ddCTP, ddTTP).

Ein weiterer Aspekt 33 der Erfindung betrifft ein Verfahren nach Aspekt 32, wobei die "weitere Art" der Nukleosidtriphosphaten konventionell modifizierte Nukleotide mit einer Markierung sind, wobei diese Markierung unabhängig ausgewählt ist aus der Gruppe von: ein Fluoreszenzfarbstoff, ein Biotin, ein Hapten, ein radioakives Element.

Ein weiterer Aspekt 34 der Erfindung betrifft ein Verfahren nach Aspekten 28 bis 33, wobei mindestens zwei unterschiedliche Populationen an Nukleinsäureketten präsent sind

Ein weiterer Aspekt 35 der Erfindung betrifft ein Verfahren nach Aspekt 34, wobei mindestens eine der Populationen der Nukleinsäureketten eine Primer-Funktion hat und mindestens eine Pupulation der Nukleinsäureketten eine Matrizen-Funktion.

Ein weiterer Aspekt 36 der Erfindung betrifft ein Verfahren nach Aspekt 28, wobei die Modifizierung durch eine chemische Kopplung erfolgt, wobei die Kopplung der Nuk-Makromoleküle an Nukleinsäureketten durch Phosphoroamidit-Kopplung erfolgt.

Ein weiterer Aspekt 37 der Erfindung betrifft ein Verfahren nach Aspekten 28 bis 36, wobei im Markierungsverfahren Nuk-Makromoleküle eingesetzt werden, die die Kopplung nur einer einzigen Nuk-Komponente in den wachsenden Nukleinsäure-Strang zulassen und der mehrfache Einbau durch Modifikationen an der Nuk-Komponente, und/oder der Linker-Komponente und/oder der Marker-Komponente verhindert wird.

Ein weiterer Aspekt 38 der Erfindung betrifft ein Verfahren nach Aspekt 37, wobei die weitere Kopplung reversibel verhindert wird.

Ein weiterer Aspekt 39 der Erfindung betrifft ein Verfahren nach Aspekt 37, wobei die weitere Kopplung irreversibel verhindert wird.

Ein weiterer Aspekt 40 der Erfindung betrifft ein Verfahren nach Aspekten 28 bis 36, wobei im Markierungsverfahren Nuk-Makromoleküle eingesetzt werden, die eine Kopplung mehrer Nuk-Komponente in den wachsenden Nukleinsäurestrang zulassen

Ein weiterer Aspekt 41 der Erfindung betrifft ein Verfahren nach Aspekten 28-40, wobei die an der Reaktion teilnehmenden Nukleinsäureketten an eine feste Phase gekoppelt sind und adressierbare Positionen haben.

Ein weiterer Aspekt 42 der Erfindung betrifft ein Verfahren nach Aspekt 41, wobei die Nukleinsäureketten eine einheitliche Population darstellen

Ein weiterer Aspekt 43 der Erfindung betrifft ein Verfahren nach Aspekt 41, wobei die Nukleinsäureketten zwei oder mehrere unterschiedliche Populationen darstellen und jede der Populationen eine adressierbare Position auf der festen Phase hat

Ein weiterer Aspekt 44 der Erfindung betrifft ein Verfahren nach Aspekten 41,42, wobei die Kopplung von Nuk-Makromolekülen an einer Population einheitlicher, an der festen Phase fixierter Nukleinsäuremoleküle erfolgt, wobei die Marker-Komponente des Nuk-Makromoleküls nach der Kopplung am verlängerten Nukleinsäurestrang verbleibt und nicht abgetrennt wird.

Ein weiterer Aspekt 45 der Erfindung betrifft ein Verfahren nach Aspekten 41,42, wobei die Kopplung von Nuk-Makromolekülen an einer Population einheitlicher, an der festen Phase fixierter Nukleinsäuremoleküle erfolgt, wobei die Marker-Komponente oder ihre einzelnen Komponenten mit oder ohne Linker-Komponente des Nuk-Makromoleküls während der Kopplung oder nach der Kopplung von der in den wachsenden Nukleinsäurestrang eingebauten Nuk-Komponente abgetrennt wird.

Ein weiterer Aspekt 46 der Erfindung betrifft ein Verfahren nach Aspekten 41,43, wobei die Kopplung von Nuk-Makromolekülen in einem Reaktionsansatz parallel an zwei oder mehreren unterschiedlichen Populationen an der festen Phase fixierter Nukleinsäuremoleküle erfolgt, wobei diese Populationen jeweils unterschiedliche adressierbare Positionen an der festen Phase haben und die Marker-Komponente des Nuk-Makromoleküls nach der Kopplung am verlängerten Nukleinsäurestrang verbleibt und nicht abgetrennt wird.

Ein weiterer Aspekt 47 der Erfindung betrifft ein Verfahren nach Aspekten 41,43, wobei die Kopplung von Nuk-Makromolekülen in einem Reaktionsansatz parallel an zwei oder mehreren unterschiedlichen Populationen an der festen Phase fixierter Nukleinsäuremoleküle erfolgt, wobei diese Populationen unterschiedliche adressierbare Positionen an der festen Phase haben und die Marker-Komponente oder ihre einzelnen Komponenten mit oder ohne Linker-Komponente des Nuk-Makromoleküls während der Kopplung oder nach der Kopplung von der Nuk-Komponente abgetrennt wird.

Ein weiterer Aspekt 48 der Erfindung betrifft ein Verfahren nach Aspekten 41 bis 47, wobei die adressierbaren Positionen mit Nukleinsäuremolekülen auf der festen Phase als Spots auf einer planen Oberfläche verteilt sind, wobei pro ein Spot Nukleinsäuremoleküle einheitlich sind.

Ein weiterer Aspekt 49 der Erfindung betrifft ein Verfahren nach Aspekten 41 bis 47, wobei die adressierbaren Positionen mit Nukleinsäuremolekülen an den Kügelchen bzw. Partikel befestigt sind, wobei pro ein Kügelchen Nukleinsäuremoleküle einheitlich sind.

Ein weiterer Aspekt 50 der Erfindung betrifft ein Verfahren nach Aspekten 41 bis 47, wobei die adressierbaren Positionen mit Nukleinsäuremolekülen in einem Multigefäßsystem, wie Mikrotiterplatte oder Nanotiterplatte oder Pikotiterplatte, verteilt sind, wobei in einem Gefäß des Multigefäßsystems die Nukleinsäuremoleküle einheitlich sind.

Ein weiterer Aspekt 51 der Erfindung betrifft ein Verfahren nach Aspekten 28 bis 35 und 37 bis 50, das folgende Schritte einschließt:
a) Bereitstellung mindestens einer Population an einzelsträngigen Nukleinsäureketten
b) Hybridisierung von sequenzspezifischen Primern an diese Nukleinsäureketten, wobei extensionsfähige NSK-Primer-Komplexe entstehen
c) Inkubation von mindestens einer Art der Nuk-Makromoleküle nach Aspekten 1 bis 25 zusammen mit einer Art der Polymerase nach Aspekt 31 mit den in Schritten a und b bereitgestellten NSK-Primer-Komplexen unter Bedingungen, die den Einbau von komplementären Nuk-Makromolekülen zulassen, wobei jede Art der Nuk-Makromoleküle eine für sie charakteristische Markierung besitzt.
d) Entfernung der nicht eingebauten Nuk-Makromoleküle von den NSK-Primer-Komplexen
e) Detektion der Signale von in die NSK-Primer-Komplexe eingebauten Nuk-Makromolekülen
f) Entfernung der Linker-Komponente und der Marker-Komponente von den in die NSK-Primer-Komplexe eingebauten Nuk-Makromolekülen
g) Waschen der NSK-Primer-Komplexe
gegebenenfalls Wiederholung der Schritte (c) bis (g),

Ein weiterer Aspekt 52 der Erfindung betrifft ein Verfahren nach Aspekten 28-40, wobei die Nukleinsäureketten an eine feste Phase in zufälliger Anordnung gekoppelt sind.

Ein weiterer Aspekt 53 der Erfindung betrifft ein Verfahren nach Aspekten 28 bis 41, 52 zur parallelen Sequenzanalyse von Nukleinsäuresequenzen (Nukleinsäureketten, NSKs), bei dem man
Fragmente (NSKFs) einzelsträngiger NSKs mit einer Länge von etwa 50 bis 1000 Nukleotiden erzeugt, die überlappende Teilsequenzen einer Gesamtsequenz darstellen können, man
die NSKFs unter Verwendung eines einheitlichen oder mehrerer unterschiedlichen Primer in Form von NSKF-Primer-Komplexen auf einer Reaktionsoberfläche in einer zufälligen Anordnung bindet, wobei die Dichte der an die Oberfläche gebudenen NSKF-Primer-Komplexen eine optische Detektion der Signale von einzelnen eingebauten Nuk-Makromolekülen zuläßt, man
eine zyklische Aufbaureaktion des komplementären Stranges der NSKFs unter Verwendung einer oder mehrerer Polymerasen durchführt, indem man
a) zu den auf der Oberfläche gebundenen NSKF-Primer-Komplexen eine Lösung zugibt, die eine oder mehrere Polymerasen und ein bis vier Nuk-Makromoleküle enthält, die eine mit Fluoreszenzfarbstoffen markierte Marker-Komponente haben, wobei die bei gleichzeitiger Verwendung von mindestens zwei Nuk-Makromoleküle jeweils an die Marker-Komponente befindlichen Fluoreszenzfarbstoffe so gewählt sind, dass sich die verwendeten Nuk-Makromoleküle durch Messung unterschiedlicher Fluoreszenzsignale voneinander unterscheiden lassen, wobei die Nuk-Makromoleküle strukturell so modifiziert sind, dass die Polymerase nach Einbau eines solchen Nuk-Makromoleküls in einen wachsenden komplementären Strang nicht in der Lage ist, ein weiteres Nuk-Nakromolekül in denselben Strang einzubauen, wobei die Linker-Komponente mit der Marker-Komponente abspaltbar sind, man
b) die in Stufe a) erhaltene stationäre Phase unter Bedingungen inkubiert, die zur Verlängerung der komplementären Stränge geeignet sind, wobei die komplementären Stränge jeweils um ein Nuk-Makromolekül verlängert werden, man
c) die in Stufe b) erhaltene stationäre Phase unter Bedingungen wäscht, die zur Entfernung nicht in einen komplementären Strang eingebauter Nuk-Makromoleküle geeignet sind, man
d) die einzelnen, in komplementäre Stränge eingebauten Nuk-Makromoleküle durch Messen des für den jeweiligen Fluoreszenzfarbstoff charakteristischen Signals detektiert, wobei man gleichzeitig die relative Position der einzelnen Fluoreszenzsignale auf der Reaktionsoberfläche bestimmt, man
e) zur Erzeugung unmarkierter (NTs oder) NSKFs die Linker-Komponente und die Marker-Komponente von den am komplementären Strang angefügten Nuk-Komponenten abspaltet, man
f) die in Stufe e) erhaltene stationäre Phase unter Bedingungen wäscht, die zur Entfernung der Marker-Komponente geeignet sind, man
die Stufen a) bis f) gegebenenfalls mehrfach wiederholt,
wobei man die relative Position einzelner NSKF-Primer-Komplexe auf der Reaktionsoberfläche und die Sequenz dieser NSKFs durch spezifische Zuordnung der in Stufe d) in aufeinanderfolgenden Zyklen an den jeweiligen Positionen detektierten Fluoreszenzsignale zu den Nuk-Makromolekülen bestimmt.

Ein weiterer Aspekt 54 der Erfindung betrifft ein Verfahren nach Aspekt 53, dadurch gekennzeichnet, dass man die Stufen a) bis f) der zyklischen Aufbaureaktion mehrfach wiederholt, wobei man in jedem Zyklus nur jeweils eine Art der Nuk-Makromoleküle einsetzt.

Ein weiterer Aspekt 55 der Erfindung betrifft ein Verfahren nach Aspekt 53, dadurch gekennzeichnet, dass man die Stufen a) bis f) der zyklischen Aufbaureaktion mehrfach wiederholt, wobei man in jedem Zyklus jeweils zwei unterschiedlich markierte Arten der Nuk-Makromoleküle einsetzt.

Ein weiterer Aspekt 56 der Erfindung betrifft ein Verfahren nach Aspekt 53, dadurch gekennzeichnet, dass man die Stufen a) bis f) der zyklischen Aufbaureaktion mehrfach wiederholt, wobei man in jedem Zyklus jeweils vier unterschiedlich markierte Arten der Nuk-Makromoleküle einsetzt.

Ein weiterer Aspekt 57 der Erfindung betrifft ein Verfahren nach Aspekt 53, dadurch gekennzeichnet, dass die NSKs Varianten einer bekannten Referenzsequenz sind und man die Stufen a) bis f) der zyklischen Aufbaureaktion mehrfach wiederholt, wobei man in den Zyklen abwechselnd jeweils zwei unterschiedlich markierte Arten der Nuk-Makromoleküle und zwei unmarkierte NTs einsetzt und man die Gesamtsequenzen durch Vergleich mit der Referenzsequenz ermittelt.

Ein weiterer Aspekt 58 der Erfindung betrifft ein Verfahren nach den Aspekten 53 bis 57, dadurch gekennzeichnet, dass man in die NSKFs jeweils eine Primerbindungsstelle (PBS) einführt, wobei man bei doppelsträngigen NSKs an beiden komplementären Einzelsträngen jeweils eine PBS einführt und wobei die Primerbindungsstellen für alle NSKFs jeweils gleiche oder verschiedene Sequenzen aufweisen.

Ein weiterer Aspekt 59 der Erfindung betrifft ein Verfahren nach den Aspekten 53 bis 57, dadurch gekennzeichnet, dass man die NSKFs mit Primern in einer Lösung unter Bedingungen in Kontakt bringt, die zur Hybridisierung der Primer an die Primerbindungsstellen (PBSs) der NSKFs geeignet sind, wobei die Primer untereinander gleiche oder verschiedene Sequenzen aufweisen, und man die gebildeten NSKF-Primer-Komplexe anschließend auf der Reaktionsoberfläche bindet.

Ein weiterer Aspekt 60 der Erfindung betrifft ein Verfahren nach den Aspekten 53 bis 57, dadurch gekennzeichnet, dass man die NSKFs zunächst auf der Reaktionsoberfläche immobilisiert und erst anschließend mit Primern unter Bedingungen in Kontakt bringt, die zur Hybridisierung der Primer an die Primerbindungsstellen (PBSs) der NSKFs geeignet sind, wobei NSKF-Primer-Komplexe gebildet werden, wobei die Primer untereinander gleiche oder verschiedene Sequenzen aufweisen.

Ein weiterer Aspekt 61 der Erfindung betrifft ein Verfahren nach Aspekten 53 bis 60, bei dem an 10 bis 100.000 unterschiedlichen Sequenzenpopulationen die Einbaureaktion parallel durchgeführt

Ein weiterer Aspekt 62 der Erfindung betrifft ein Verfahren nach Aspekten 53 bis 60, bei dem an 100.000 bis 100.000.000 unterschiedlichen Sequenzenpopulationen die Einbaureaktion parallel durchgeführt.

Ein weiterer Aspekt 63 der Erfindung betrifft ein Verfahren nach Aspekten 28 bis 62, wobei die Sequenzen der Nukleinsäureketten ermittelt werden

Ein weiterer Aspekt 64 der Erfindung betrifft ein Verfahren nach Aspekten 28 bis 63, wobei die Marker-Komponente fluoreszent markiert ist

Ein weiterer Aspekt 65 der Erfindung betrifft ein Verfahren nach Aspekten 41 bis 64, wobei die feste Phase unabhängig aus der folgenden Gruppe ausgewählt ist: Silicon, Glas, Keramik, Kunststoffe, Gele oder deren Modifikationen

Im weiteren Aspekt 66 der Erfindung sind besonders bevorzugt makromolekulare Verbindungen nach Aspekt 1, wobei die Nuk-Komponente folgende Strukturen einschließt, Fig. 3A:
Wobei:
   Base - ist unabhängig gewählt aus der Gruppe von Adenin, oder 7-Deazaadenin, oder Guanin, oder 7-Deazaguanin, oder Thymin, oder Cytosin, oder Uracil, oder deren Modifikationen , wobei L die Verbindung zwischen der Nuk-Komponente und der Linker-Komponente darstellt (Kopplungseinheit L) und X die Kopplungsstelle der Kopplungseinheit L an der Base ist, wobei die Kopplungsstelle an Pyrimidinbasen an der 5-Position oder 4-Position am Pyrimidinring ist und an der 7-Position der Deazapurine.
   R₂ - ist unabhängig gewählt aus der Gruppe H, OH, oder geschützte OH-Gruppe
   R₃ - ist unabhängig gewählt aus der Gruppe H, OH, NH₂,
   R₄ - ist H oder OH
   R₅ - ist unabhängig gewählt aus der Gruppe OH, oder eine geschützte OH-Gruppe, oder Monophosphat-Gruppe, oder Diphosphat-Gruppe oder Triphosphat-Gruppe

Ein weiterer Aspekt 67 der Erfindung betrifft makromolekulare Verbindungen nach Aspekt 66, wobei die Kopplungseinheit L folgende strukturelle Elemente einschließt:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇, R₆-(CH=CH-)ₙ-R₇, R₆-(A-CH=CH-)ₙ-R₇, R₆-(CH=CH-B-)ₙ-R₇, R₆-A-CH=CH-(CH₂-)ₙ-R₇, R₆-(-CH=CH-CH₂)ₙ-B-R₇, R₆-(C≡C-)ₙ-R₇, R₆-(A-C≡C-)ₙ-R₇, R₆-(C≡C-B-)ₙ-R₇, R₆-A-C≡C-(CH₂-)ₙ-R₇, R₆-(-C≡C-CH₂)ₙ-B-R₇,
wobei R₆ - die Nuk-Komponente ist, R₇ - der Linkerrest ist und A und B folgende strukturelle Elemente einschließen: -NH-, -O-, -S-, -SS-, -CO-NH-, -NH-CO-, - CO-O-, -O-CO-, -CO-S-, -S-CO-, -P(O)₂-, -Si-, -(CH₂)ₙ-, eine photolabile Gruppe, wobei n - gleich 1 bis 5 ist

Ein weiterer Aspekt 68 der Erfindung betrifft makromolekulare Verbindungen nach Aspekt 66, wobei die Linker-Komponente ein wasserlösliches Polymer einschließt.

Ein weiterer Aspekt 69 der Erfindung betrifft makromolekulare Verbindungen nach Aspekt 68, wobei die Linker-Komponente wasserlösliche Polymere unabhängig ausgewählt aus folgender Gruppe einschließt:
Polyethylen-glycol (PEG), Polysaccharide, Dextran, Polyamide, Polypeptide, Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acrylsäure, Poly-Acrylamid, Poly(Vinylalkohol).

Ein weiterer Aspekt 70 der Erfindung betrifft makromolekulare Verbindungen nach Aspekten 66 bis 69, wobei eine Linker-Komponente eine durchschnittliche Länge zwischen 50 bis 100, 100 bis 200, 200 bis 500, 500 bis 1000, 1000 bis 2000, 2000 bis 10000, 10000 bis100000 Atome hat.

Ein weiterer Aspekt 71 der Erfindung betrifft makromolekulare Verbindungen nach Aspekten 66 bis 69, wobei eine Marker-Komponente eine signalgebende, signalvermittelnde, katalytische oder affine Funktion hat

Ein weiterer Aspekt 72 der Erfindung betrifft makromolekulare Verbindungen nach Aspekten 66 bis 69, wobei eine Marker-Komponente aus einer strukturellen Marker-Einheit besteht

Ein weiterer Aspekt 73 der Erfindung betrifft makromolekulare Verbindungen nach Aspekten 66 bis 69, wobei eine Marker-Komponente aus mehreren strukturellen Marker-Einheiten gebunden an eine Kern-Komponente besteht

Ein weiterer Aspekt 74 der Erfindung betrifft makromolekulare Verbindungen nach Aspekten 72 oder 73, wobei eine strukturelle Marker-Einheit unabhängig eines der folgenden strukturellen Elemente einschließt:
Biotin, Hapten, radioaktives Isotop, seltene Erdenatom, Farbstoff, Fluoreszenzfarbstoff.

Ein weiterer Aspekt 75 der Erfindung betrifft makromolekulare Verbindungen nach Aspekten 72 oder 73, wobei eine strukturelle Marker-Einheit unabhängig eines der folgenden Elemente einschließt:
Nanokristalle oder deren Modifikationen, Proteine oder deren Modifikationen, Nukleinsäureketten oder deren Modifikationen, Teilchen oder deren Modifikationen.

Ein weiterer Aspekt 76 der Erfindung betrifft makromolekulare Verbindungen nach Aspekt 75, wobei eine strukturelle Marker-Einheit eines der folgenden Proteine einschließt:
Enzyme oder deren Konjugate und Modifikationen,
Antikörper oder deren Konjugate und Modifikationen,
Streptavidin oder seine Konjugate und Modifikationen,
Avidin oder seine Konjugate oder Modifikationen

Ein weiterer Aspekt 77 der Erfindung betrifft makromolekulare Verbindungen nach Aspekt 75, wobei eine strukturelle Marker-Einheit eine der folgenden Arten Nukleinsäureketten einschließt: DNA, RNA, PNA, wobei die Länge von Nukleinsäureketten zwischen 10 und 10.000 Nukleotide liegt

Ein weiterer Aspekt 78 der Erfindung betrifft makromolekulare Verbindungen nach Aspekten 73 bis 77, wobei die Kern-komponente der Marker-Komponente unabhängig eines der folgenden Elemente einschließt: wasserlöslichen Polymer aus der Gruppe von: Polyamide (z.B. Polypeptide), Poly-Acrylsäure und deren Derivate, Poly-Acrylamide und deren Derivate, Poly-Vinylalkohole und deren Derivate, Nukleinsäuren und deren Derivate, Streptavidin oder Avidin und deren Derivate, Dendrimere, wobei diese Elemente linear oder verzweigt oder unter einander vernetzt sein können

Ein weiterer Aspekt 79 der Erfindung betrifft makromolekulare Verbindungen nach Aspekten 66 bis 71, 73 bis 78, wobei die Verbindung zwischen mehreren strukturellen Marker-Einheiten und der Kern-Komponte kovalent oder affine erfolgt.

Ein weiterer Aspekt 80 der Erfindung betrifft makromolekulare Verbindungen nach Aspekten 66 bis 73, wobei die Verbindung zwischen einer strukturellen Marker-Einheit und dem Linker kovalent oder affine erfolgt

Ein weiterer Aspekt 81 der Erfindung betrifft makromolekulare Verbindungen nach Aspekten 66 bis 71, 73 bis 78, wobei die Verbindung zwischen der Kern-Komponente und dem Linker kovalent oder affine erfolgt

Ein weiterer Aspekt 82 der Erfindung betrifft makromolekulare Verbindungen nach Aspekten 66 bis 81, wobei nur eine Nuk- Komponente mit einem gekoppelten Linker an die Marker-Komponente gekoppelt ist

Ein weiterer Aspekt 83 der Erfindung betrifft eine Makromolekulare Verbindung nach Aspekten 66 bis 82, wobei nur eine Nuk- Komponente mit einem gekoppelten Linker an die Marker-Komponente gekoppelt ist, wobei der Linker eine oder mehrere unter milden Bedingungen spaltbare Verbindungen beinhaltet.

Ein weiterer Aspekt 84 der Erfindung betrifft eine Makromolekulare Verbindung nach Aspekten 66 bis 82, wobei nur eine Nuk- Komponente mit einem gekoppelten Linker an die Marker-Komponente gekoppelt ist, wobei ein oder mehrere Teile des Nuk-Makromoleküls dermaßen modifiziert sind, dass nur eine Nuk-Komponente in einen wachsenden Strang eingebaut werden kann.

Ein weiterer Aspekt 85 der Erfindung betrifft eine Makromolekulare Verbindung nach Aspekten 66 bis 71, 73 bis 82, wobei mehrere Nuk- Komponenten jeweils über einen Linker an einer Marker-Komponenten gekoppelt

Ein weiterer Aspekt 86 der Erfindung betrifft eine Makromolekulare Verbindung nach Aspekten 66 bis 71, 73 bis 82, wobei mehrere Nuk- Komponenten jeweils über einen Linker an einer Marker-Komponenten gekoppelt, wobei der jeweilige Linker eine oder mehrere unter milden Bedingungen spaltbare Verbindungen beinhaltet.

Ein weiterer Aspekt 87 der Erfindung betrifft eine Makromolekulare Verbindung nach Aspekten 66 bis 71, 73 bis 82, wobei mehrere Nuk- Komponenten jeweils über einen Linker an einer Marker- Komponenten gekoppelt, wobei ein oder mehrere Teile des Nuk-Makromoleküls dermaßen modifiziert sind, dass nur eine Nuk-Komponente in eine wachsende Nukleinsäurekette eingebaut werden kann.

Ein weiterer Aspekt 88 der Erfindung betrifft Oligonucleotide oder Polynucleotide die mindestens ein Nuk-Makromolekül nach Aspekten 66 bis 87 pro eine Nukleinsäurekette einschließen.

Ein weiterer Aspekt 89 der Erfindung betrifft Oligonucleotide oder Polynucleotide nach Aspekt 88, wobei Oligo- oder Polynucleotide RNA, DNA oder PNA sind, deren Länge zwischen 5 und 50.000 Nukleotide beträgt.

### Gegenüberstellung von Eigenschaften konventionell modifizierter Nukleotide und Nuk-Makromoleküle:

### Substrateigenschaften der konventionell modifizierten Nukleotide

Der Einfluß unterschiedlicher Linkergrößen, der chemischen Linkerzusammensetzung und die unterschiedlicher Größe und Beschaffenheit von niedermolekularen Markern auf die Substrat-Eigenschaften der Nukleotide (G. Wright et al. Pharmac.Ther. 1990, V. 47, S. 447, Klevan US Patent 4828979, Lee. et al. Nucleic Acid Research 1992, V. 20, 2471, J. Brandis Nucleic Acid Research, 1999, V.27, 1912) macht deutlich, dass bereits geringfügige Veränderungen an der Nukleotid- , Linker- und Marker-Struktur zu einer starken Veränderung der Substrateigenschaften der modifizierten Nukleotide führen können.

Folgerichtig läßt sich nachweisen, dass die Kopplung eines deutlich größeren Moleküls (z.B. eines Proteins) an ein konventionell modifiziertes Nukleotid zur Aufhebung von dessen Substrateigenschaften führt, s. Beispiel 34B.

Aus diesem Grund konnten bis jetzt signalverstärkende Makromoleküle nur nach erfolgtem enzymatischem Einbau der Nukleotide in die Nukleinsäurekette mit dem Nukleotid gekoppelt werden, wie beispielsweise markiertes Streptavidin an biotinylierte Nukleotide.

Ähnliches lässt sich feststellen, wenn Nukleotid-Monomere in einer Nukleinsäurekette betrachtet werden: nur niedermolekulare Stoffe können beispielsweise an ein Oligonukleotid in der Nähe der 3' -OH-Posiition gekoppelt werden, falls dieses Oligonukleotid als Primer in weiteren Reaktionen auftreten sollte. Zu große Moleküle, z.B. Streptavidin, gekoppelt an ein Nukleotid-Monomer am 3'-Ende oder in der Nähe des 3' -Endes eines Oligonukleotids führen zum Verlust der Primereigenschaften. Ohne an eine bestimmte Theorie gebunden sein zu wollen, kann dies beispielsweise durch sterische Einflusse der Makromoleküle erklärt werden. Die räumlichen Verhältnisse im aktiven Zentrum vieler Enzyme sind sehr anspruchsvoll und der katatalytische Mechanismus vieler Enzyme schließt komplexe konformationelle Änderungen der Enzym-Struktur selber sowie die der Substrate (Nukleotid-Monomere). In vielen Fällen führen auch nur geringfügige chemische Modifikationen an Nukleotid-Monomeren, die als Monomere einer Polymer-Kette auftreten, zu einer veränderten enzymatischen Akzeptanz der Nukleinsäureketten. Ein Beispiel dafür ist die alpha-thiophosphonat-Modifikation der Nukleotid-Monomere, die zur Exonuklease-Resistenz der Nukleinsäurekette führt.

Auch wenn eine Markierung mit Fluoreszenzfarsbtoffen einen breiten Einsatz in der modernen Diagnostik und Forschung gefunden hat, besteht nach wie vor ein dringeder Bedarf nach Signalverstärkung der biologisch aktiven markierten Molekülen. Man bedient sich oft einer sekundären Signalverstärkung durch Enzymreaktionen.

Trotz der naheliegenden Überlegung, eine mehrfache Markierung in Form einer makromolekularen Marker-Komponente an Nukleotide zu koppeln, gelang es noch nicht, diesen Schritt durch einfache Kombination zwischen konventionellen Nukleotidstrukturen und einer makromolekularen Marker-Komponente unter Beibehaltung der Substrateigenschaft der Nukleotide zu realisieren.

Die Masse der konventionell modifizierten Nukleotide liegt im Wesentlichen im selben Bereich wie bei nicht modifizierten Nukleotiden, und ist relativ gering verglichen mit der Masse von Proteinen, wie z.B. Streptavidin oder Polymerasen. Die Steigerung der Masse des Nukleotids durch Einführung makromolekularer Komponenten kann zu Veränderung von biochemischen und physikalsichen Eigenschaften von Nukleotiden führen.

Die Überwindung dieses Nachteils des Standes der Technik gelang überraschenderweise durch die Einführung einer wesentlich längeren Linker-Komponente zwischen der Nuk- Komponente und der Marker- Komponente, als es vom Stand der Technik bekannt war.

Überraschenderweise behalten die erfindungsgemäßen Nuk-Makromoleküle ihre Substrataktivität trotz der massiven Veränderungen in ihren Eigenschaften bei und können von Enzymen verwendet als Substrate werden. So bauen beispielsweise Polymerasen die erfindungsgemäßen Nuk-Makromoleküle in den wachsenden Strang ein. Terminale deoxy-Nucleotidyl-Transferase (TdT) können Nuk-Makromoleküle an ein 3' -Ende einer Nukleinsäure koppeln (Beispiel 34B,C und Beispiel 35).

Einem Fachmann sollte es naheliegend erscheinen, dass die Masse der erfindungsgemäßen Nuk-Makromoleküle ein vielfaches der natürlichen Nukleotide bildet und einen großen Einfluß auf die Nuk- Komponente ausübt.

### Einsatzgebiete der Nuk-Makromoleküle:

Die Kopplung eines makromolekularen Markers an ein Substrat für Enzyme, an ein Nukleotid, bietet die Möglichkeit eines breiten Einsatzes dieser Nuk-Makromoleküle in unterschiedlichen Anwendungen in der Biotechnologie, Medizin und Wissenschaft.

Erfindungsgemäß können Nuk-Makromoleküle in Verfahren eingesetzt werden, bei denen Sie als Substrate für Enzyme dienen.

In einer Ausführungsform der Erfindung werden Nuk-Makromoleküle in den Verfahren zur Markierung von Nukleinsäuren eingesetzt. Dabei erfolgt eine Einbaureaktion von Nuk-Makromolekülen nach allgemeinen Regeln der enzymatischen Verlängerungsreaktion von Nukleinsäureketten, ("Molecular Cloning", J. Sambrook, 3. Ed. 2001).

Der große Vorteil eines makromolekularen Markes pro ein eingebautes Nukleotid liegt in einer wesentlich höheren Signalstärke im Vergleich zu herkömmlichen modifizierten Nukleotiden.

Ein weiterer Vorteil ist die große Distanz des Markers zum Nukleinsäurestrang, so dass nur geringfügige Wechselwirkung zwischen dem Marker und dem Nukleinsäurestrang zu erwarten sind. Dies hat einen Einfluß beispielsweise auf die Fluoreszenzeigenschaften der Marker: Die Fluoreszenz der Marker wird von den Nukleobasen (Purine und Pyrimidine) nicht gequencht. Falls mehrere Nuk-Makromoleküle in einer Nukleinsäurekette eingebaut sind, führt eine größere Distanz zwischen dem Marker und der Nukleotid-Komponenten zu einer deutlichen Verminderung der Interaktion zwischen Markern von benachbarten Nukleotiden.

Erfolgt die Markierung einer Nukleinsäurekette beispielsweise während der enzymatischen Synthese dieser Kette durch den Einbau von Nuk-Makromolekülen, so sind unter Umständen keine weiteren mehrstufigen Signalamplifikationsschritte mehr notwendig. Viele der bekannten Signalamplifikationsschritte, z.B. Biotin-Strepavidin-Antikörper, oder Digoxigenin-Antikörper I -Antikörper II laufen mit nur mäßigen Ausbeuten ab, wie z.B. Signalamplifikationen bei FISH-Analysen. Schwankende und schwache Ausbeuten in der Markierung verursachen ebenfalls Schwankungen und Schwäche im Signal, was zu Fehlinterpretationen führen kann. Durch den Einsatz von Nuk-Makromolekülen kann die Schäche der Markierung beseitigt werden. Durch den Einsatz von Nuk-Makromolekülen mit konstanten Signalmengen können auch die Schwankungen weitgehend ausgeglichen werden.
Die Markierung von Nukleinsäuren kann in unterschiedlichen Verfahren eingesetzt werden. Die konkreten Bedingungen der Nukleinsäurevorbereitung, die Reihenfolge der enzymatischen Schritte und Detektionsvorgänge hängen im einzelnen vom Verfahren ab, in dem die Markierung eingesetzt wird.

Insgesamt kann festgestellt werden, dass die erfindungsgemäßen Nuk-Makromoleküle eine überdurchschittliche Verbesserung der Markierungsstrategien der Nukleinsäureketten ermöglichen.

### Modifizierte Nukleinsäureketten

In einer Ausführung der Erfindung schließen Nukleinsäureketten Nuk-Komponenten der Nuk-Makromoleküle als Einheiten der Kette ein. Die Nuk-Makromoleküle werden als Monomere einer Polymerkette, der Nukleinsäurekette, betrachtet. Solche Nukleinsäureketten mit integriereten Nuk-Makromolekülen können als Sonden und als Reaktionspartner in verschiedenen Bereichen eingesetzt werden (z.B. Real-Time-PCR, Ligase-Ketten-Reaktion).

In einer Ausführungsform ist ein Nuk-Makromolekül an das 5'-Ende der Nukleinsäurekette integriert. In einer Ausführungsform ist ein Nuk-Makromolekül an das 3' -Ende der Nukleinsäurekette integriert. In einer weiteren Ausführungsform ist ein Nuk-Makromolekül im Inneren der Nukleinsäurekette integriert, wobei der Abstand vom am nächsten liegenden Kettenende in folgenden Bereichen liegt (Zahl der Monomeren der Kette bis zum nächsten Kettenende): 1 bis 2, 2 bis 4, 4 bis 8, 8 bis 15, 15 bis 30, 30 bis 100, 100 bis 500.

Ein Nuk-Makromolekül kann mehrere Nuk-Komponente einschließen. In einer Ausführungsform wird nur eine Nuk-Komponente eines Nuk-Makromoleküls in eine Nukleinsäurekette integriert, die anderen Nuk-Komponenten liegen in Monomer-Form vor. In einer anderen Ausführungsform können mehrere Nuk-Komponenten eines Nuk-Makromoleküls in unterschiedliche Nukleinsäureketten intergriert werden, wobei die Nukleinsäureketten unter einander identische oder auch unterschiedliche Sequenzen aufweisen können.

Besonders vorteilhaft ist der Einsatz mit Nuk-Makromolekülen modifizierter Nukleinsäureketten dann, wenn Nuk-Makromolekül als Teil einer Polymer-Kette an einer enzymatischen Reaktion / Umwandlung teilnimmt oder sich in unmittelbaren Nähe vom an der Reaktion teilnehmenden Nukleotid befindet. Durch den langen Linker der Nuk-Makromolekülen wird der Einfluß einer makromolekularen Marker-Komponente auf das Enzym stark reduziert, so dass die modifizierten Nukleotid-Komponenten an den enzymatischen Reaktionen (z.B. Primer-Funktion bei einer matrizen-abhängigen, polymerase-gesteuerten Reaktion, bei einer Ligase-abängigen Reaktion (z.B. Ligase-Ketten-Reaktion), 3' -Exonuklease oder 5' -Exonuklease-Aktivitäten verschiedener Enzyme, Endonuklease-Spaltung,) teilnehmen können, bzw. die Reaktion an benachbarten Nukleotiden nicht beeinträchtigen (J. Wilhelm "Entwicklung Real-Time-PCR-basierter Methoden für die moderne DNA-Analytik" Dissertation, 2003, Gießen, S. Meuer "Rapid cycle real time PCR", Springer 2004, ISBN 3-540-66736-9, T. Weissensteiner "PCR-Technology: current innovations" CRC Press 2004 ISBN 0-8493-1184-5). Der Abstand zwischen der Position des Nuk-Makromoleküls in der Nukleinsäurekette und dem Nukleotid derselben Nukleinsäurekette, das als Substrat an der enzymatischen Reaktion teilnimmt, liegt beispielsweise in folgenden Bereichen (Zahl von Nukleotiden): 0 bis 3, 3 bis 6, 6 bis 10, 10 bis 20, 20 bis 40. Zahl 0 bedeutet, dass das Nuk-Makromolekül direkt an das an der Reaktion teilnehmende Nukleotid gekoppelt ist.

Im weiteren folgen einige Beispiele zum Einsatz der Nuk-Makromoleküle.

### Verfahren in der Flüssigphase

In einer Ausführungsform der Markierungsverfahren befinden sich die zu markierenden Nukleinsäureketten in der Flüssigphase, s. Beispiel 34, 35.

Viele andere Verfahren, z.B. PCR, Transkription ("Molecular Cloning", J. Sambrook, 3. Ed. 2001) können mit erfindungsgemäßen Nuk-Makromolekülen durchgeführt werden. Dabei werden Nuk-Makromoleküle ähnlich zu konventionell mit Farbstoff modifizierten Nukleotiden in die Reaktion zugegeben. Allgemeine Regeln der Verwendung konventionell modifizierter Nukletide, wie beispielsweise dCTP-Cy3 (Amersham Bioscience) oder dUTP-TMR (NEN) sind ausführlich in Literatur beschrieben ("Molecular Cloning", J. Sambrook, 3. Ed. 2001).

Beispielsweise kann für die Kopplung eines einzelnen komplementären Nukleotids an den Primer eine Art der Nuk-Makromoleküle, z.B. dATP oder dCTP, verwendet werden. Bei den meisten Reaktionen werden allerdings Gemische aus modifizierte und nicht modifizierten Nukleotiden eingesetzt (H. Yu et al. Nucleic Acid Research 1994, v. 22 3226-, "Molecular Cloning", J. Sambrook, 3. Ed. 2001). Beispielsweise können nicht markierte und markierte Nukleotide für eine Markierung mit einem Nuk-Makromolekül, bei dem dUTP als Nuk-Komponente auftritt, in folgenden Verhältnissen gemischt werden:

| | |
|---|---|
| dATP : dCTP : dGTP : dTTP : dUTP-Nuk-Makromolekül | = 1:1:1:0,9:0,1 |
| oder dATP : dCTP : dGTP : dTTP : dUTP-Nuk-Makromolekül | = 1:1:1:0,7:0,3 |
| oder dATP : dCTP : dGTP : dTTP : dUTP-Nuk-Makromolekül | = 1:1:1:0,95:0,05 |

die genaue Mischverhältnisse können für einzelne Markierungsreaktionen optimiert werden.

Auch mehrere Arten der Nuk-Makromoleküle können in einer Reaktion eingesetzt werden. Beispielsweise können Nuk-Makromoleküle unterschiedliche Markierung tragen. In einer Ausführungsform werden die Marker der Nuk-Makromoleküle so gewählt, dass sie ein FRET-Paar bilden (Faner, R et al. Hum Immunol 2004, v.65, 826-38, Lazowski, K. W et al. Antisense Nucleic Acid Drug Dev 2000, v.10, 97-103, Talavera, E. M., Appl Spectrosc 2003, v.57, 208-15, Tsourkas, A. et al. Anal Chem, 2003, v. 75, 3697-703, Singh, K. K., et al. Methods Mol Biol 2004, v.252, 33-48, Wang, L., Spectrochim Acta A Mol Biomol Spectrosc, 2004, v.60, 2741-50). Nach dem Einbau solcher Nuk-Makromoleküle in die wachsende Nukleinsäurekette verringert sich die durchschnittliche Distanz zwischen den Fluorophoren, so dass es bei der Anregung des Donors zu FRET an den Akzeptor kommt. Da die Nuk-Makromoleküle eine deutlich stärkere Markierung tragen als die konventionell modifizierten Nukleotide, kann die Signalintensität eines FRET-Signal deutlich größer sein. Die genauen Bedingung der Reaktion können im Hinblick auf die Wahl der Fluorophore, ihre Kopplung im Nuk-Makromolekül, die Konzentration der Nuk-Makromoleküle und Verhältnis zwischen den Nuk-Makromolekülen und nicht markierten Nukleotiden optimiert werden. Als generelle Regel gilt dabei, dass die durchschnittliche Distanz zwischen den Fluorophoren eines FRET-Paars sollte unter 10 nm liegen.

### Festphasenverfahren

In einer weiteren Ausführungsform der Markierungsverfahren sind die zu markierenden Nukleinsäureketten oder deren komplementären Stränge an einer festen Phase fixiert. Viele Verfahren zur Markierung von immobilisierten Nukleinsäureketten mit konventionell modifizierten Nukleotiden sind bekannt (Suomalainen A et al. Methods Mol Biol. 2003, Pirrung MC et al. Bioorg Med Chem Lett. 2001 Sep 17;11(18):2437-40). Beispiele für die feste Phase stellen Mikrokügelchen (Spherotech Inc, Streptavidin-polystyre Particle, 2.17µ). Im Beispiel 34C wird eine Einbaureaktion von Nuk-Makromolekülen an fester Phase beschrieben. Ein weiteres Beispiel für die feste Phase stellen planen Oberflächen, an die Nukleinsäuren gebunden sind.

Die Nuk-Makromoleküle eignen sich für Analysenverfahren mit einer Einbaureaktion an den an einer festen Phase gekoppelten Nukleinsäuren, sie können ähnlich zu konventionell modifizierten Nukleotiden in vielen Verfahren eingesetzt werden, wie beispielsweise Minisequencing (Suomalainen A et al. Methods Mol Biol. 2003;226:361-6. Liljedahl U et al. Pharmacogenetics. 2003 Ja;13(1):7-17, Olsson C et al. Methods Mol Biol. 2003;212:167-76), Primer-Extension (Pirrung MC et al. Bioorg Med Chem Lett. 2001 Sep 17;11(18):2437-40, Cai H, et al. Genomics. 2000 Jun 1;66(2):135-43., Kurg A et al. Genet Test. 2000;4(1):1-7., Pastinen T et al. Genome Res. 1997 Jun;7(6):606-14). US pat. 6287766, US pat. 2003148284, US pat. 2003082613, EP 1256632, WO0194639, WO 2004/076692, Ju et al. US Pat. 6664079. Fest-Phasen-PCR (WO 9626291, WO 9409156, US Pat. 6221635), Sequenzierung durch die Synthese (Ju et al US Pat. 6664079), Single-Molecular-Sequencing (Tcherkassov WO 02088382, Seeger WO 0018956, Kartalov WO 02072892). In vielen Fällen erfolgt dabei eine Synthese der komplementären Stränge an den fixierten Primer-Matrizen-Komplexen.

Viele Analyse-Verfahren bei denen Nukleinsäureketten fixiert sind, benötigen routinemäßig Vervielfältigungsschritte. In solchen Verfahren können Nuk-Makromoleküle einen besonders großen Vorteil bringen, da die Signal-Intensität überdurchschnittlich gesteigert wird. Die Zahl der signalgebenden Marker-Einheiten von Nuk-Makromolekülen kann eine noch während der Synthese festgelegte Größe aufweisen, so dass die Intensität des Signal von den eingebauten Nuk-Makromolekülen quantifiziert werden kann.

Bei solchen Verfahren können Nuk-Makromoleküle eingesetzt werden, die fluoreszente, radioaktive oder Enzyme als Marker-Einheiten einschließen.

Sehr vorteilhaft bei solchen Verfahren ist die Verwendung von Nuk-Makromolekülen mit Fluoreszenzsignalen, da die Fluoreszenz eine hohe Sensitivität aufweist.

In einer Ausführung solcher Verfahren erfolgt die Detektion der Fluoreszenz-Signale von den Markern der eingebauten Nuk-Makromoleküle. Dabei können auch FRET-Paare zwischen einzelnen Nuk-Makromolekülen verwendet werden. In einer weiteren Ausführung können bei der Markierung an einer festen Phase fixierten Nukleinsäureketten markierte Primer eingesetzt werden, wobei die Primer in einer Ausführungsform einen oder mehrere Nuk-Makromoleküle einschließen und in einer weiteren Ausführungsform eine konventionelle Markierung einschlißen.
In diesen beiden Ausführungsformen kann die Markieung am Primer als ein Teil eines FRET-Paars auftreten. Der an Primer gekoppelte Partner eines FRET-Paars kann dabei als Donor als auch als Akzeptor während der Detektion auftreten. Die eingebauten Nuk-Makromoleküle mit einem entsprechenden Partner des FRET-Paars können dabei einen abspaltbaren oder auch einen nicht abspaltbaren Marker tragen.
Durch den Einsatz des FRET zwischen den eingebauten Nuk-Makromolekülen oder der Primer-Markierung und den eingebauten Nuk-Makromolekülen kann eine große Steigerung der Signalspezifität erzielt werden. Die Detektion kann dabei in einer Ausführungsform während des Einbaus in einer anderen Ausführugnsform als separater Schritt des Verfahrend erfolgen.

In einer Ausführungsform tragen Nuk-Makromoleküle mit Nuk-Komponente einer Art (beispielsweise dTTP) vorzugsweise eine für sie spezifischen Marker-Komponente, so dass beispielsweise 4 Arten der Nuk-Makromoleküle (entsprechend dem dTTP, dCTP, dATP und dGTP) gleichzeitig eingesetzt und unterschieden werden können. Andere Markierungsschema sind in bekannt s. z.B. Tcherkassov WO 02088382. Je nach Verfahren werden auch nicht markierte, z.B. natürliche Nukleotide in das Reaktionsgemisch zusammen mit den Nuk-Makromolekülen zugegeben.

In einer Ausführungsform der Markierungsverfahrens werden Nuk-Makromoleküle eingesetzt, die den Einbau nur einer einzelnen Nuk-Komponente in den wachsenden Nukleinsäure-Strang erlauben und der mehrfache Einbau durch Modifikationen an der Nuk-Komponente, und/oder der Linker-Komponente und/oder der Marker-Komponente verhindert wird. Der weitere Einbau kann sowohl reversibel als auch irreversibel verhindert werden. Ein irreversibler Stopp kann beispielsweise durch den Einsatz von Nuk-Makromolekülen erreicht werden, die als Nuk-Komponente ein Dideoxi-Nukleosidtriphosphat einschließen. Ein reversibler Stops kann in einem weiteren Verfahrensschritt aufgehoben werden, so dass weitere Einbaureaktionen stattfinden können. Beispiele für eine reversible Blockade der Reaktion sind in Anmeldungen (Metzker et al. Nucleic acid Research 1994, V.22, S. 4259, Canard et al. Gene, 1994, V. 148, 1, Kwiatkowski US Pat. 6255475, Kwiatkowski WO 01/25247, Parce WO 0050642, Tcherkassov WO 02088382, Ju et al. US Pat. 6664079, Milton et al. WO 2004018497, Milton et al. WO 2004018493, Balasubramanian et al. WO 03048387) beschrieben.

In einer anderen Ausführungsform verhindern die bereits eingebauten Nuk-Makromoleküle den weiteren Einbau von Nukleotiden nicht. Falls ein Gemisch aus modifizierten und nicht modifizierten Nukleotiden eingesetzt wird, können nach dem Einbau von einem Nuk-Makromolekül auch weitere Nuk-Makromoleküle in den wachsenden Strang nacheinander eingebaut werden.

Die feste Phase kann beispielsweise eine plane Oberfläche oder Kügelchen (Beads) oder eine Art von Multigefäßarrays (z.B. Microtiterplatte, Nanotiterplatte) darstellen. Dabei können Nukleinsäuren mit verschiedenen Methoden an die feste Phase gekoppelt werden (McGall et al. US Patent 5412087, Nikiforov et al. US Patent 5610287, Barrett et al. US Patent 5482867, Mirzabekov et al. US Patent 5981734, "Microarray biochip technology" 2000 M.Schena Eaton Publishing, "DNA Microarrays" 1999 M. Schena Oxford University Press, Rasmussen et al. Analytical Biochemistry v.198, S.138, Allemand et al. Biophysical Journal 1997, v.73, S.2064, Trabesinger et al. Analytical Chemistry 1999, v.71, S.279, Osborne et al. Analytical Chemistry 2000, v.72, S.3678, Timofeev et al. Nucleic Acid Research (NAR) 1996, v.24 S.3142, Ghosh et al. NAR 1987 v.15 S.5353, Gingeras et al. NAR 1987 v.15 S.5373, Maskos et al. NAR 1992 v.20 S.1679). Es sind Verfahren zur Vervielfältigung der Nukleinsäureketten ausgehend von einzelnen Molekülen beschrieben. Solche Verfahren können zur Ausbildung ortsspezifisch gebundener Populationen der Nukleinsäureketten mit identischer Sequenz eingesetzt werden, wobei sowohl plane Oberflächen als auch Beads verwendet werden können. Vorzugsweise sind die zu analysierenden Nukleinsäurestränge mit einem Primer versehen, so dass es ein Primer-Matrizen-Komplex ausgebildet ist und daran eine enzymatische Synthese eines komplementären Stranges stattfinden kann ("Molecular Cloning", Maniatis, 3. Ed. 2001).

### Primer-Extension:

In einer Ausführungsform des Verfahrens erfolgt die Einbaureaktion von Nuk-Makromolekülen an einer einzigen Population einheitlicher, an der festen Phase fixierter Nukleinsäuremoleküle, wobei die Marker-Komponente des Nuk-Makromoleküls nach dem Einbau am verlängerten Primer verbleibt und nicht abgetrennt wird.

Solche Verfahren schließen im wesentlichen folgende Schritte ein:
1) Bereitstellung einer festen Phase mit fixierten Primer-Matrizen-Komplexen
2) Inkubation der bereitgestellten festen Phase mit einer Reaktionslösung, die eine oder mehrere Arten der Polymerasen, eine oder mehrere Arten der Nuk-Makromoleküle enthält, wobei die Nuk-Makromoleküle durch ihre Markierung eindeutig identifiziert werden können.
3) Enfernung der Reaktionslösung und waschen der festen Phase
4) Detektion der Signale von eingebauten Nuk-Makromolekülen und die Zuordnung der Art der eingebauten Nuk-Makromoleküle aufgrund ihrer Signaleigenschaften.

### Sequenzierung:

In einer weiteren Ausführungsform des Verfahrens erfolgt die Einbaureaktion von Nuk-Makromolekülen an einer einzigen Population einheitlicher, an der festen Phase fixierter Nukleinsäuremoleküle, wobei die Marker-Komponente oder ihre einzelnen Komponenten mit oder ohne Linker-Komponente des Nuk-Makromoleküls während der Einbaureaktion oder nach der Einbaureaktion von der Nuk-Komponente abgetrennt wird.

Solche Verfahren schließen im wesentlichen folgende Schritte ein:
1) Bereitstellung einer festen Phase mit fixierten Primer-Matrizen-Komplexen
2) Inkubation der bereitgestellten festen Phase mit einer Reaktionslösung, die eine oder mehrere Arten der Polymerasen, eine oder mehrere Arten der Nuk-Makromoleküle enthält, wobei die Nuk-Makromoleküle durch ihre Markierung eindeutig identifiziert werden können.
3) Enfernung der Reaktionslösung und waschen der festen Phase
4) Detektion der Signale von eingebauten Nuk-Makromolekülen und die Zuordnung der Art der eingebauten Nuk-Makromoleküle aufgrund ihrer Signaleigenschaften.
5) Entfernung der Marker-Komponente von den eingebauten Nuk-Makromolekülen
6) Wiederholung der Schritte 2 bis 5
Die Wiederholung kann beispielsweise 1 bis 2, 2 bis 5, 5 bis 10, 10 bis 20, 20 bis 30 mal durchgeführt werden.

In einer weiteren Ausführungsform des Verfahrens erfolgt der Einbau von Nuk-Makromolekülen in einer enzymatischen Reaktion parallel an zwei oder mehreren unterschiedlichen Populationen einheitlicher, an der festen Phase fixierter Nukleinsäuremoleküle, wobei diese Populationen adressierbare Positionen an der festen Phase haben, wobei die Marker-Komponente des Nuk-Makromoleküls nach dem Einbau am verlängerten Primer verbleibt und nicht abgetrennt wird.
Die adressierbaren Positionen können beispielsweise die Form von Spots haben, im Falle einer planen Oberfläche. Bei der Verwendung von Kügelchen als feste Phase werden unterschiedliche Populationen von Nukleinsäuren an unterschiedlichen Kügelchen fixiert. Bei der Verwendung von Multigefäßarrays (z.B. Mikrotiter-Platte, oder Nanotiter-Platte) sind einzelne Nukleinsäurepopulationen in einzelnen Gefäßen getrennt fixiert.

Solche Verfahren schließen im wesentlichen folgende Schritte ein:
1) Bereitstellung einer festen Phase mit fixierten Primer-Matrizen-Komplexen
2) Inkubation der bereitgestellten festen Phase mit einer Reaktionslösung, die eine oder mehrere Arten der Polymerasen, eine oder mehrere Arten der Nuk-Makromoleküle enthält, wobei die Nuk-Makromoleküle durch ihre Markierung eindeutig identifiziert werden können.
3) Enfernung der Reaktionslösung und waschen der festen Phase
4) Detektion der Signale von eingebauten Nuk-Makromolekülen und die Zuordnung der Art der eingebauten Nuk-Makromoleküle aufgrund ihrer Signaleigenschaften.

In einer weiteren Ausführungsform des Verfahrens erfolgt der Einbau von Nuk-Makromolekülen in einer enzymatischen Reaktion parallel an zwei oder mehreren unterschiedlichen Populationen einheitlicher, an der festen Phase fixierter Nukleinsäuremoleküle, wobei diese Populationen adressierbare Positionen an der festen Phase haben. Die einheitlichen Nukleinsäurepopulationen können durch unterschidliche Verfahren an die Oberfläche fixiert werden (s.o.). Die Marker-Komponente oder ihre einzelnen Komponenten mit oder ohne Linker-Komponentedes Nuk-Makromoleküls wird bei dieser Ausführungsform während der Einbaureaktion oder nach der Einbaureaktion vom der eingebauten Nuk-Komponente abgetrennt. Für solche Verfahren eignen sich Nuk-Makromoleküle mit spaltbaren Verbindungen im Linker. Die adressierbaren Positionen können beispielsweise die Form von Spots haben, im Falle einer planen Oberfläche. Bei der Verwendung von Kügelchen als feste Phase werden unterschiedliche Populationen von Nukleinsäuren an unterschiedlichen Kügelchen fixiert. Bei der Verwendung von Multigefäßarrays sind einzelne Nukleinsäurepopulationen in einzelnen Gefäßen getrennt fixiert.

Solche Verfahren schließen im wesentlichen folgende Schritte ein:
1) Bereitstellung einer festen Phase mit fixierten Primer-Matrizen-Komplexen
2) Inkubation der bereitgestellten festen Phase mit einer Reaktionslösung, die eine oder mehrere Arten der Polymerasen, eine oder mehrere Arten der Nuk-Makromoleküle enthält, wobei die Nuk-Makromoleküle durch ihre Markierung eindeutig identifiziert werden können.
3) Enfernung der Reaktionslösung und waschen der festen Phase
4) Detektion der Signale von eingebauten Nuk-Makromolekülen und die Zuordnung der Art der eingebauten Nuk-Makromoleküle aufgrund ihrer Signaleigenschaften.
5) Entfernung der Marker-Komponente von den eingebauten Nuk-Makromolekülen
6) Wiederholung der Schritte 2 bis 5
Die Wiederholung kann beispielsweise 1 bis 2, 2 bis 5, 5 bis 10, 10 bis 20, 20 bis 30, 30 bis 50, 50 bis 100, 100 bis 200 mal durchgeführt werden.

Ein einer Ausführungsform eines solchen Verfahrens werden im Schritt (2) Nuk-Makromoleküle zusammen mit den anderen modifizierten Nukleotiden eingesetzt. Dabei erfolgt die Synthese des komplementären Stranges schrittweise: pro ein Syntheseschritt werden komplementäre Stränge maximal um ein Nukleotid verlängert. Die Kontrolle der enzymatischen Reaktion erfolgt dabei durch reversible Terminatoren. Vorzugsweise werden die an 3' -OH-Gruppe modifizierten Terminatoren eingesetzt, die keine weiteren Modifikationen an der Base tragen. Nach der Abspaltung der Modifikation vom eingebauten Nukleotid kann weitere Synthese an diesen Strängen stattfinden. Die Struktur der in dieser Ausführungsform eingesetzten Nuk-Makromoleküle kann unterschiedlich sein. Vorzugsweise sind es terminierende Nuk-Makromoleküle, d.h. nach ihren Einbau kann kein weiteres Nukleotid durch eine Polymerase eingebaut werden. Der Linker ist vorzugsweise an die Base der Nuk-Komponente gekoppelt und schließt eine spaltbare Verbindung ein. Das Zusammenmischen der Nuk-Makromoleküle mit terminierenden Eigenschaften und der reversiblen Terminatoren erlaubt an einer Population der Nukleinsäureketten die Markierung und die reversible Termination zu trennen. Da die Nuk-Makromoleküle in dieser Ausführungsform des Verfahrens terminierend wirken, steht in jedem weiteren Einbauschritt eine geringerere Anzahl an Strängen für weitere Sequenzierungsreaktion zur Verfügung. Damit eine Einbaureaktion in weiteren Schritten stattfinden kann, muß ein Teil der Stränge in jedem Schritt reversibel blockiert werden. Der Anteil der mit Nuk-Makromolekülen modifizierten Nukleinsäureketten kann sehr gering sein, da die Signalstärke der Nuk-Makromoleküle sehr groß sein kann. Das Verhältnis zwischen den Nuk-Makromolekülen und reversiblen Terminatoren in einem Reaktionsschritt kann beispielsweise in folgenden Bereichen liegen: zwischen 100 :1 und 1:10, zwischen 10 :1 und 1:1, 1 :1 und 1:10, zwischen 1:10 und 1:100, zwischen 1:100 und 1:1000 (Konzentration von Nuk-Makromoleküle : Konzentration von reversiblen Terminatoren). Dieses Verhältnis kann während der gesamten Sequenzierunsreaktion konstant bleiben oder varisieren. Da Polymerasen die Nuk-Makromoleküle und die reversible Terminatoren unterschiedlich gut akzeptieren können, können mehrere Polymerase-Arten während des Einbauschritts eingesetzt werden.
Die Entfernung der Signale nach der Detektion führt zu einem besseren Signal-Rauschen-Verhältnis bei den nächsten Detektionen und ist typisch für die Verfahren der Sequenzierung durch die Synthese. Die Aufhebung der reversiblen Terminierung kann in einem eigenen Schritt erfolgen oder beispielsweise mit dem Schritt der Entfernung der Markierung zusammenfallen.
Der Vorteil des Einsatzes der Nuk-Makromoleküle besteht darin, dass die Markierung eines geringen Anteils der Gesamtpopulation zur Detektion des Einbauereignisses ausreicht. Dies erlaubt mit wesentlich geringeren Mengen des Ausgangsmaterials die Sequezierungsreaktion durchzuführen. Die Verwendung von reversiblen Terminatoren mit einer Schutzgruppe an der 3' -Position und einer Base ohne Modifikation führt dazu, dass das nach der Entferung der blockierenden Gruppe in der Nukleinsäurekette verbleibende Nukleotid keine weiteren Modifikationen trägt und somit als natürliches Substrat von Polymerasen gut akzeptiert wird.

In einer weiteren Ausführungsform des Verfahren, werden folgende Schritte durchgeführt:
a) Bereitstellung mindestens einer Population an einzelsträngigen Nukleinsäureketten
b) Hybridisierung von sequenzspezifischen Primern an diese Nukleinsäureketten, wobei extensionsfähige NSK-Primer-Komplexe entstehen
c) Inkubation von mindestens einer Art der Nuk-Makromoleküle nach Aspekten 1 bis 25 zusammen mit einer Art der Polymerase nach Aspekt 31 mit den in Schritten a und b bereitgestellten NSK-Primer-Komplexen unter Bedingungen, die den Einbau von komplementären Nuk-Makromolekülen zulassen, wobei jede Art der Nuk-Makromoleküle eine für sie charakteristische Markierung besitzt.
d) Entfernung der nicht eingebauten Nuk-Makromoleküle von den NSK-Primer-Komplexen
e) Detektion der Signale von in die NSK-Primer-Komplexe eingebauten Nuk-Makromolekülen
f) Entfernung der Linker-Komponente und der Marker-Komponente von den in die NSK-Primer-Komplexe eingebauten Nuk-Makromolekülen
g) Waschen der NSK-Primer-Komplexe
gegebenenfalls Wiederholung der Schritte (c) bis (g),

In einer weiteren Ausführungsform des Verfahrens erfolgt die Einbaureaktion von NT-Makromolekülen gleichzeitig an einer Population unterschiedlicher, an der festen Phase fixierter Nukleinsäuremoleküle, wobei diese Nukleinsäuremoleküle in einer zufälligen Anordnung an die feste Phase gebunden sind (Tcherkassov WO 02088382). Bei diesem Verfahren werden Sequenzen von einzelnen Nukleinsäurekettenmolekülen ermittelt. Die an der enzymatischen Reaktion teilnehmende Primer-Nukleinsäurekomplexe sind in einer Dichte fixiert, die die Detektion der Signale von einzelnen Nuk-Makromolekülen ermöglicht, die an ein einzelnes Nukleinsäure-Molekül gekoppelt sind, wobei die Dichte der fixierten Primer oder Nukleinsäuren wesentlich höher liegen kann. Beispielsweise beträgt die Dichte der an der Einbaureaktion teilnehmenden Primer-Nukleinsäurekomplexe von 1 Komplex auf 10µm² bis 1 Komplex auf 100µm², von 1 Komplex auf 100µm² bis 1 Komplex auf 1000µm², von 1 Komplex auf 1000µm² bis 1 Komplex auf 10.000µm².
Beispiele der Fixierung der Nukleinsäuren an die feste Phase mit einer Auflösung, die Analysen an einzelnen Molekülen erlaubt sind in WO0157248, US Pat. 2003064398, US Pat.2003013101 und WO 02088382 dargestellt. Eine passende Detektionsapparatur ist in der Anmeldung WO 03031947 beschrieben.

Die Zahl der parallel zu analysierenden einzelnen Nukleinsäuremoleküle liegt beispielsweise zwischen 1000 und 100.000, 10.000 bis 1.000.000, 100.000 bis 10.000.000 Moleküle. Wobei die Marker-Komponente oder ihre einzelnen Komponenten mit oder ohne Linker- Komponente des Nuk-Makromoleküls während der Einbaureaktion oder nach der Einbaureaktion von der Nuk- Komponente abgetrennt wird..
Dieses Verfahren zur parallelen Sequenzanalyse von Nukleinsäuresequenzen (Nukleinsäureketten, NSKs) schließt folgende Schritte ein, man:
- Fragmente (NSKFs) einzelsträngiger NSKs mit einer Länge von etwa 50 bis 1000 Nukleotiden erzeugt, die überlappende Teilsequenzen einer Gesamtsequenz darstellen können, man
- die NSKFs unter Verwendung eines einheitlichen oder mehrerer unterschiedlichen Primer in Form von NSKF-Primer-Komplexen auf einer Reaktionsoberfläche in einer zufälligen Anordnung bindet, wobei die Dichte der an die Oberfläche gebudenen NSKF-Primer-Komplexen eine optische Detektion der Signale von einzelnen eingebauten Nuk-Makromolekülen zuläßt, man
- eine zyklische Aufbaureaktion des komplementären Stranges der NSKFs unter Verwendung einer oder mehrerer Polymerasen durchführt, indem man
   a) zu den auf der Oberfläche gebundenen NSKF-Primer-Komplexen eine Lösung zugibt, die eine oder mehrere Polymerasen und ein bis vier Nuk-Makromoleküle enthält, die eine mit Fluoreszenzfarbstoffen markierte Marker-Komponente haben, wobei die bei gleichzeitiger Verwendung von mindestens zwei Nuk-Makromoleküle jeweils an die Marker-Komponente befindlichen Fluoreszenzfarbstoffe so gewählt sind, dass sich die verwendeten Nuk-Makromoleküle durch Messung unterschiedlicher Fluoreszenzsignale voneinander unterscheiden lassen, wobei die Nuk-Makromoleküle strukturell so modifiziert sind, dass die Polymerase nach Einbau eines solchen Nuk-Makromoleküls in einen wachsenden komplementären Strang nicht in der Lage ist, ein weiteres Nuk-Nakromolekül in denselben Strang einzubauen, wobei die Linker-Komponente mit der Marker-Komponente abspaltbar sind, man
   b) die in Stufe a) erhaltene stationäre Phase unter Bedingungen inkubiert, die zur Verlängerung der komplementären Stränge geeignet sind, wobei die komplementären Stränge jeweils um ein Nuk-Makromolekül verlängert werden, man
   c) die in Stufe b) erhaltene stationäre Phase unter Bedingungen wäscht, die zur Entfernung nicht in einen komplementären Strang eingebauter Nuk-Makromoleküle geeignet sind, man
   d) die einzelnen, in komplementäre Stränge eingebauten Nuk-Makromoleküle durch Messen des für den jeweiligen Fluoreszenzfarbstoff charakteristischen Signals detektiert, wobei man gleichzeitig die relative Position der einzelnen Fluoreszenzsignale auf der Reaktionsoberfläche bestimmt, man
   e) zur Erzeugung unmarkierter (NTs oder) NSKFs die Linker-Komponente und die Marker-Komponente von den am komplementären Strang angefügten Nuk-Komponenten abspaltet, man
   f) die in Stufe e) erhaltene stationäre Phase unter Bedingungen wäscht, die zur Entfernung der Marker-Komponente geeignet sind, man
   die Stufen a) bis f) gegebenenfalls mehrfach wiederholt,
   wobei man die relative Position einzelner NSKF-Primer-Komplexe auf der Reaktionsoberfläche und die Sequenz dieser NSKFs durch spezifische Zuordnung der in Stufe d) in aufeinanderfolgenden Zyklen an den jeweiligen Positionen detektierten Fluoreszenzsignale zu den Nuk-Makromolekülen bestimmt.

### Anwendung von Nukleinsäuereketten, die Nuk-Makromoleküle einschließen.

Nach dem Einbau eines Nuk-Makromolekül an das 3'-Enden der Nukleinsäure behalten die nun ihrerseits modifizierten Nukleinsäureketten überraschenderweise die Fähigkeit zur Kopplung weiterer Nukleotide an die 3' -Hydroxylgruppe durch Polymerasen, s. Beispiel 34C. Dies bedeutet, dass nicht nur die Nuk-Komponente in den Nuk-Makromolekülen sondern auch die mit diesen Nuk-Makromolekülen modifizierten Nukleinsäureketten für die Enzyme zugängig bleiben können und in unterschiedlichen Bereichen der Biotechnologie eine Anwendung finden. Nicht nur am 3' -Enden mit Nuk-Makromolekülen modifizierte Nukleinsäureketten, sondern Nukleinsäureketten, die am 5'-Ende oder im inneren Bereich des Nukleinsäurepolymers eines oder mehrere Nuk-Makromoleküle als Monomere einschließen, behalten ihre Substrateigenschaften für Polymerasen, Exonukleasen und Ligasen. Beispiele für die Anwendungen von mit Nuk-Makromolekülen modifizierten Oligonucleotiden sind dem Fachmann bekannt, z.B. in den Primer-Extension-Reaktionen, Real Time PCR oder Ligase-Reaktionen.

### Wahl der Enzyme:

Nukleotide spielen als Monomere eine zentrale Rolle in unterschiedlichen Stoffwechselvorgängen, beispielsweise bei der Speicherung und Übertragung der genetischen Information in der Zelle ("Genes V" B. Lewin, 1994). Auch sind Nukleotide als Energieträger der Zelle bekannt (ATP, UTP), oder Signalvemittler (Messenger, GTP) der intrazellulären Signalvermittlung ("Biochemie und Pathobiochemie", G. Löffler, 2003). Aus diesem Grund werden Nukleotide und ihre Analoga als Therapeutika und Diagnostika eingesetzt.
Gekoppelt in Nukleinsäurepolymere (Nukleinsäureketten) stellen die Nukleotid-Monomere die Basis für die Informationsspeicherung in den lebenden Organismen dar.

Nuk-Makromoleküle haben das Potenzial, in unterschiedlichen Bereichen der Biotechnologie eine Anwendung zu finden.

Die Möglichkeit einer Kopplung der Nukleotide an ein Makromolekül unter Beibehaltung der Substrateigenschaften der Nukleotide eröffnet viele Wege auch für eine gezielte Adressierung der modifizierten Nukleotiden innerhalb eines Organismus oder einer Zelle, so dass Nuk-Makromoleküle ein neues grundsätzliches Model für Nukleotid-Prodrugs darstellen.

Als Enzyme können beispielsweise unterschiedliche Sorten von Polymerasen verwendet werden ("DNA Replication", Kornberg, 2. Ed. 1992), im einzelnen DNA-abhängige DNA-Polymerasen, RNA-abhängige DNA-Polymerasen, DNA-abhängige RNA-Polymerasen und RNA-abhängige RNA-Polymerasen. Dabei können sowohl thermostabile als auch thermolabile Polymerasen verwendet werden, wie beispielsweise Klenow-Polymerase oder Taq-Polymerase. Andere Beispiele für mögliche Polymerasen findet ein Fachmann in der hier zitierten Literatur. Ein weiteres Beispiel für Enzyme stellen Transferasen, wie Terminale Deoxynucleotidyltransferase ("Molecular Cloning", Maniatis, 3. Ed. 2001), dar. Auch andere Enzyme und Proteine (beispielsweise Kinasen, Membranrezeptoren) die Nukleotide als Substrate), Energiequelle, Cofaktoren oder als Messenger-Substanzen akzeptieren können verwendet werden.

Enzyme unterscheiden sich in ihrer Fähigkeit, modifizierte Nukleotide als Substrate zu akzeptieren. Einem Fachmann sollte es naheliegend erscheinen, dass verschiedene funktionelle Tests eingesetzt werden müssen, um bestimmte Eigenschaften von Nukleotiden zu untersuchen und anzuwenden. Beispiele für unterschiedliche Testabläufe für die Markierung von Nukleinsäuren sind in H. Held et al. Nucleic Acid Research 2002, V. 30, S. 3857, M. Metzger et al. Nucleic Acid Research 1994, V. 22, 4259, M. Herrlein et al. Helvetica Chimica Acta 1994, V.77, S. 586 , B. Canard et al. PNAS 1995, V. 92, S. 10859 , Canard US Pat. 5798210, J. Hovinen et al. J. Chem. Soc. Perkin 1994, 1994, 211 und auch in anderen hier zitierten Patenten und Publikationen angegeben.

Die passenden Kombinationen zwischen Polymerasen und modifizierten Nukleotiden können für den jeweiligen Zweck entsprechend gewählt werden. Beispiele für den Einbau von Nuk-Makromolekülen in die Primer sind in Beispiel 34 angegeben. Die angegebenen Beispiele dienen nicht zur Einschränkung der Verwendungsmöglichkeit von Nuk-Makromolekülen, sondern sollten dem Fachmann den Unterschied in Eigenschaften der Nuk-Makromoleküle zu den konventionellen modifizierten NT darstellen.

Nuk-Makromoleküle oder deren Zwischenprodukte können auch in der konventionellen chemischen Oligonucleotidsynthese verwendet werden, beispielsweise in einer Festphasensynthese (Giegrich, "Neue photolabile Schutzgruppen für die lichtgesteuerte Oligonucleotidsynthese", 1997, Beier, "Neue Strategien zum Aufbau von RNA- und DNA-Oligonucleotiden", 1996), dabei trägt die Nuk-Komponente der Nuk-Makromoleküle entsprechende Modifikationen, die eine chemische Kopplung an die Nukleinsäurekette erlaubt, wie beispielsweise in Herrlein, "Synthese von modifizierten Nukleosiden, Nukleotiden und Oligonukleotiden", 1993, Gugler, "Aufbau und Anwendung von Systemen zur vereinfachten chemo-enzymatischen Synthese von Oligonukletid-Hybridisierungssonden", 1993, Schmidt, "Neue Methoden zur Synthese und Isolierung langkettiger Oligonucleotide", 1991, Bühler, "Neue photolabile Schutzgruppen für die Oligonucleotidsynthese", 2000, Bretzger, "Wege zur präparativen Oligonucleotidsynthese" 1991, Stengele, "Automatisierte Oligonucleotidsynthese unter Verwendung [beta]-eliminierbare Schutzgruppen", 1991).

Ein weiterer Aspekt der vorliegenden Erfindung ist eine Methode zur schnellen Reinigung der von markierten Nukleotiden direkt vor ihrem Einsatz in die Markierungsreaktion.
Verfahren zur Sequenzierung einzelner Moleküle (z.B. Balasubramanian WO 03048387, Tcherkassov WO 02088382, Kartalov WO02072892) benötigen markierte Nukleotide in einem sehr sauberen Zustand, weil Verunreinigungen einer markierten Nukleotidpräparation mit nicht markierten Nukleotiden einen Sequenzfehler verursachen können. Aus diesem Grund ist es wichtig, dass die markierten Nukleotide möglichst frei von nicht markierten Nukleotiden sind. Viele Nukleotidmodifikationen, die in solchen Verfahren eingesetzt werden, haben eine oder mehrere unter milden Bedingungen spaltbare Gruppe (Balasubramanian WO 03048387, Tcherkassov WO 02088382)

Während der Lagerung können solche Nukeotide zu einem Teil zerfallen und stellen die Quelle für Nukleotidanaloga ohne Markierung dar, die beim Einbau in die Nukleinsäure zu einem Sequenzfehler führen würden.

Dieses Problem führt dazu, dass eine Reinigung direkt vor dem Einsatz markierter Nukletide durchgeführt werden muß. Eine Standardreinigung für modifizierten Nukleotide ist beispielsweise eine HPLC-Reinigung mit Wasser-Methanol-Gradient. Nach einer solchen Reinigung muß die Fraktion mit modifizierten Nukleotiden aufgearbeitet werden, beispielsweise durch Lyophilisation. Eine solche Reinigung stellt eine aufwendige Prozedur dar.

Nuk-Makromoleküle können erfindungsgemäß direkt vor dem Gebrauch durch eine Ultrafiltration von geringsten Verunreinigungen befreit werden.
Es werden Filter mit einer Porengröße ausgewählt, die für Nukleotide ohne Marker-Anteile frei passierbar sind. Die mit einem makromolekularen Marker modifizierten Nukleotide können aber durch den Filter nicht durchgehen. Durch eine solche Reinigung gelingt es in kurzer Zeit Nuk-Makromoleküle in einem sehr sauberen Zustand zu erhalten.

Es können auch Zwischenprodukte von Nuk-Makromolekülen und Nuk-Makromoleküle mit einem niedermolekularen Marker auf diese Weise gereinigt werden, beispielsweise Nukleotide, die in Beispielen 36 und 38 beschrieben sind.

Ein weiterer Aspekt der Erfindung ist die Verwendung von modifizierten Klenow-Fragment Exo-minus der DNA-Polymerase zusammen mit den Nuk-Makromolekülen in den enzymatischen Reaktionen, wobei die SH-Gruppe des Cysteins des Klenow-Fragments Exo-minus der DNA-Polymerase chemisch modifiziert ist.
Diese Modifizierung ist vorzugsweise eine kovalente Modifizierung. Beispiele für eine solche Modifizierung stellt eine Alkylierung an der SH-Gruppe dar, z.B. mit alpha-Halogen-Acetyl-Derivate, wie beispielsweise Iodacetamid und seinen Derivaten, Iodacetat und seinen Derivaten, oder mit N-Maleimid-Derivaten, weitere selektive SH-Gruppen Reagenten sind in "Chemistry of protein conjugation and crosslinking" Shan S. Wong 1993 CRC Press Inc. beschrieben. Diese Modifizierung kann auch durch einen Fluoreszenzfarbstoff erfolgen. Aktivierte Fluoreszenzfarbstoffe, die selektiv mit SH-Gruppen reagieren, sind kommerziell erhältlich, z.B. von Molecular Probes Inc.
In einer bevorzugten Ausführungsform der Erfindung erfolgt eine selektive Modifikation des Klenow-Fragment Exo-minus der DNA-Polymerase an SH-Gruppe des Cysteins. Beispiel für die Herstellung eines solchen Klenow-Fragments Exo-minus der DNA-Polymerase ist unter Beispiel 43 angegeben.
In einer anderen Ausführungsform können auch andere Modifikationen an der DNA-Polymerase vorgenommen werden, wie beispielsweise Kopplungen an Amino-Gruppen der DNA-Polymerase.
Das an dem Cysten modifizierte Klenow-Fragment Exo-minus der DNA-Polymerase kann in einer Ausführungsform an stelle eines nicht modifizierten Klenow-Fragment Exo-minus der DNA-Polymerase zusammen mit Nuk-Makromolekülen in der enzymatischen Einbau-Reaktion eingesetzt werden.

### Allgemeine Hinweise für die Synthesen von Nuk-Makromolekülen

Die erfindungsgemäßen Nuk-Makromoleküle können auf unterschiedliche Art und Weise synthetisiert werden. Die Reihenfolge der Kopplungsschritte kann variieren. Beispielsweise kann zunächst eine Linker-Komponente an die Nuk-Komponente gekoppelt werden, anschließend wird die Marker-Komponente gekoppelt. Andererseits können ein oder mehrere Linker an die Marker-Komponente gekoppelt werden und anschließend der / die Nuk-Komponenten.

Die Kopplung zwischen einzelnen Komponenten der Nuk-Makromoleküle kann kovalent oder affin erfolgen. Wobei sowohl chemische als auch enzymatische Kopplung zur Verknüpfung einzelner Komponenten eingesetzt werden kann. Kopplungen an Amino-und Thiolgruppen dienen als Beispiele für kovalente Kopplungen (D. Jameson et al. Methods in Enzymology 1997, V. 278, S. 363-, "The chemistry of the amino group" S. Patai, 1968, "The chemistry of the thiol group" S. Patai, 1974). Biotin-Streptavidin-Bindung oder Hybridisierung zwischen komplementären Nukleinsäuresträngen oder Antigen-Antikörper-Wechselwirkungen stellen Beispiele für affine Kopplungen dar.

Die makromolekularen Marker bieten oft eine Vielfalt an Kopplungsmöglichkeiten. Ein makromolekularer Marker kann mehrere Kopplungsstellen für den Linker haben, beispielsweise mehrere Bindungsstellen für Biotin, wie es bei Streptavidin der Fall ist. Ein makromolekularer Marker kann auch mehrere Amino- bzw. Thiol-Gruppen aufweisen. Die Kern-Komponente des Markers kann mit unterschiedlicher Zahl von signalgebenden oder signalvermittelnden Einheiten modifiziert werden. Die genauen Verhältnisse zwischen Marker-Einheiten können variieren. Beispiele für die Modifikation von Polymeren mit Farbstoffen sind bekannt (Huff et al. US Pat. 5661040, D. Brigati US Pat. 4687732). Falls Nukleinsäuren als makromolekulare Marker eingesetzt werden, so können diese unterschiedliche Abschnitte zur Kopplung anderer Makromoleküle besitzen. An einen makromolekularen Marker können andere Makromoleküle gebunden werden, z.B. Enzyme.
Ein Nuk-Makromolekül kann makromolekulare Marker mit unterschiedlichen Detektionseigenschaften tragen, beispielsweise kann ein Nuk-Makromolekül sowohl mehrere Farbstoffmoleküle als auch Stellen zur affinen Bindung (z.B. durch Hybridisierung) weiterer Makromoleküle tragen.

Die Kopplung zwischen der Nuk-Komponenten und der Linker-Komponenten erfolgt vorzugsweise kovalent. Viele Beispiele für eine kovalente Kopplung an Nukleotide oder deren Analoga sind bekannt (Jameson et al. Method in Enzymology, 1997, v. 278, S. 363-, Held et al. Nucleic acid research, 2002, v. 30 3857-, Short US Pat. 6579704, Odedra WO 0192284). Dabei kann die Kopplung beispielsweise an Phosphat, Amino-, Hydroxy- oder Mercaptogruppen erfolgen.
Oft kann die Linker-Komponente in mehreren Schritten aufgebaut werden. Beispielsweise wird im ersten Schritt ein kurzer Linker mit einer reaktiven Gruppe an das Nukleotid oder Nucleosid angekoppelt, z.B. Propargylamin-Linker an Pyrimidine Hobbs et al. US Patent 5.047.519 oder andere Linker z.B. Klevan US Pat. 4,828,979, Seela US pat. 6211158, US pat. 4804748, EP 0286028, Hanna M. Method in Enzymology 1996 v.274, S.403, Zhu et al. NAR 1994 v.22 S.3418, Jameson et al. Method in Enzymology, 1997, v. 278, S. 363-, Held et al. Nucleic acid research, 2002, v. 30 3857-, Held et al. Nucleosides, nucleotides & nnucleic acids, 2003, v. 22, S. 391, Short US Pat. 6579704, Odedra WO 0192284, Herrlein et al. Helvetica Chimica Acta, 1994, V. 77, S. 586, Canard US. Pat. 5798210, Kwiatkowski US Pat. 6255475, Kwiatkowski WO 01/25247, Parce WO 0050642, Faulstich et al. DE 4418691, Phosphoroamidite (Glen Research Laboratories, http://www.glenres.com/, Trilink Biotechnologies, S. Agrawal "Protocols for oligonucleotide conjugation", Humana Press 1994, M.Gait "Oligonucleotide synthesis: a practical approach" IRL Press, 1990), Dissertation "Synthese basenmodifizierter Nukleosidtriphosphate und ihre enzymatische Polymerisation zu funktionalierter DNA", Oliver Thum, Bonn 2002. Einige Verbindungen kann man käuflich erwerben, z.B. bei Trilink Biotechnologies, Eurogentec, Jena Bioscience.
Diese kurzen Linker dienen als Kopplungseinheiten L oder deren Teile, und sind ein Bestandteil der Linker-Komponente im fertigen Nuk-Makromolekül.
Im zweiten Schritt kann die Kopplung des Nukleotides oder Nucleosides mit einem kurzen Linker an das Linker-Polymer erfolgen. Polymere mit reaktiven funktionellen Gruppen können käuflich erworben werden (Fluka).
Nach der Kopplung des Nukleotids an das Polymer kann nun die Marker-Komponente als letzter Schritt gekoppelt werden.

Oft ist es vorteilhaft an ein Nukleosid einen kurzen Linker zu koppeln, dann, falls erforderlich, dieses modifizierte Nucleosid in ein Nucleosid-Triphosphat umzuwandeln (Synthesen von Triphosphaten können beispielsweise in folgenden Literaturstellen gefunden werden: Held et al. Nucleosides, nucleotides & nnucleic acids, 2003, v. 22, S. 391, Faulstich et al. DE 4418691, T. Kovacs, L. Ötvös, Tetrahedron Letters, Vol 29, 4525-4588 (1988) oder Dissertation "Synthese basenmodifizierter Nukleosidtriphosphate und ihre enzymatische Polymerisation zu funktionalierter DNA", Oliver Thum, Bonn 2002).
Weitere Modifikationen können am Nukleosid-Triphosphat-Analog durchgeführt werden.

Vorstufen für modifizierte Nukleoside können sind beispielsweise bei Trilink Biotechnologies (San Diego, CA, USA) oder bei Chembiotech (Münster, Deutschland) kommerziel erhältlich.

Die Kopplung zwischen der Linker-Komponente und der Marker-Komponente kann beispielsweise zwischen reaktiven Gruppen an der Linker-Komponente und der Marker-Komponente erfolgen. Reagenzien für solche Kopplungen sind in "Chemistry of protein conjugation and cross-linking", S. Wang, 1993, ISBN 0-8493-5886-8, ausführlich dargestellt. Die Verfahren zur Handhabung und zur Kopplung von mehreren Makromolekülen sind für unterschiedliche Typen von Makromolekülen auch in oben genannten Patenten dargestellt. Weitere Beispiele für Kopplungen an die und zwischen den Makromolekülen sind für Proteine in "Bioconjugation: protein coupling techniques for the biomedical sciences", M. Aslam, 1996, ISBN 0-333-58375-2; "Reactive dyes in protein an enzyme technology", D. Clonis, 1987, ISBN 0-333-34500-2; "Biophysical labeling methods in molecular biology" G. Likhtenshtein, 1993, 1993, ISBN 0-521-43132-8; "Techniques in protein modification" R. Lundblad, 1995, ISBN 0-8493-2606-0; "Chemical reagents for protein modification" R. Lundblad, 1991, ISBN 0-8493-5097-2; für Nukleinsäuren in "Molecular-Cloning", J. Sambrook, band 1-3, 2001, ISBN 0-87969-576-5, für andere Polymerarten "Makromoleküle, Chemische Struktur und Synthesen", Band 1, 4, H. Elias, 1999, ISBN 3-527-29872-X beschrieben.

Da die Marker-Komponente meistens viele Kopplungsstellen aufweist, können weitere Modifikationen an kompletten Nuk-Makromolekülen vorgenommen werden. Beispielsweise können überschüssige Amino-Gruppen abgeblockt werden oder durch weiterführende Modifikationen verändert werden.

Je nach Einsatzgebiet und Reaktionsbedingungen, unter denen Nuk-Makromoleküle verwendet werden, können unterschiedliche chemische Bindungsarten zwischen einzelnen Teilen der Makromoleküle von Vorteil sein. So eignen sich beispielsweise bei Verfahren mit Schritten mit höheren Temperaturen, wie z.B. bei Hybridisierung oder PCR, Nuk-Makromoleküle, die kovalente, thermostabile Bindungen zwischen einzelnen Teilen haben.

### Nachfolgend sollen beispielhaft einige Möglichkeiten zur Synthese von

Nuk-Makromolekülen dargestellt werden. Diese dienen nicht zur Einschränkung der möglichen Synthesewege und nicht zur Einschränkung der möglichen Nuk-Makromolekülstrukturen.
In folgenden Beispielen werden Nuk-Makromoleküle mit Polyethylenglycol (PEG) als Linker-Komponente angegeben. Beispiele für die Kopplungen von PEG an andere Moleküle sind in "Poly(ethylene glycol) : chemistry and biological applications", 1997 beschrieben. Im einzelnen können sehr unterschiedliche reaktive Gruppen zur Kopplung eingesetzt werden: N-succinimidyl carbonate (US Pat.. 5,281,698, US Pat. 5,468,478), Amine (Buckmann et al. Makromol. Chem. V.182, S.1379 (1981), Zalipsky et al. Eur. Polym. J. V.19, S. 1177 (1983)), succinimidyl propionate und succinimidyl butanoate (Olson et al. in Poly(ethylene glycol) Chemistry & Biological Applications, 170-181, Harris & Zalipsky Eds., ACS, Washington, D.C., 1997; U.S. Pat. No. 5,672,662), Succinimidyl succinate (Abuchowski et al. Cancer Biochem. Biophys. v. 7, S.175 (1984), Joppich et al., Makromol. Chem. 1v. 80, S.1381 (1979), Benzotriazole carbonate (U.S. Pat. No. 5,650,234), Glycidylether (Pitha et al. Eur. J. Biochem. v. 94, S.11 (1979), Elling et al., Biotech. Appl. Biochem. v.13, S. 354 (1991), Oxycarbonylimidazole (Beauchamp, et al., Anal. Biochem. v.131, S. 25 (1983), Tondelli et al. J. Controlled Release v.1, S.251 (1985)), p-nitrophenyl carbonate (Veronese, et al., Appl. Biochem. Biotech., v.11, S.141 (1985); and Sartore et al., Appl. Biochem. Biotech., v.27, S.45 (1991)), Aldehyde (Harris et al. J. Polym. Sci. Chem. Ed. v.22, S. 341 (1984), US. Pat. 5824784, US. Pat. 5252714), Maleimide (Goodson et al. Bio/Technology v.8, S. 343 (1990), Romani et al. in Chemistry of Peptides and Proteins v.2, S.29 (1984)), and Kogan, Synthetic Comm. v.22, S.2417 (1992)), Orthopyridyl-disulfide (Woghiren, et al. Bioconj. Chem. v. 4, S. 314 (1993)), Acrylol (Sawhney et al., Macromolecules, v. 26, S.581 (1993)), Vinylsulfone (U.S. Pat. No. 5,900,461). Weitere Beispiele für Kopplungen von PEG an andere Moleküle sind in Roberts et al. Adv. Drug Deliv. Reviews v. 54, S. 459 (2002), US Pat. 2003124086, US Pat. 2003143185, WO 03037385, US Pat. 6541543, US Pat. 2003158333, WO 0126692 zu finden.

Andere ähnliche Polymere können in ähnlicher Art gekoppelt werden. Beispiele für solche Polymere stellen andere Poly(Alkylenglykole), Copolymere aus Ethylenglykol und Propyleneglykol, Poly(olefiniische Alkohole), Poly(Vinylpyrrolidone), Poly(Hydroxyalkylmethacrylamide), Poly(Hydroxyalkylmethacrylate), Poly(saccharide), Poly(x-Hydroxy-Säuren), Poly(Acrylsäure), Poly(Vinylalkohol).

Die Aufreinigung der Nuk-Komponenten der Nuk-Makromoleküle erfolgt mit konventionellen Mitteln der Nukleotidchemie: beispielsweise mit Kieselgel-Chromatographie in einem Wasser-Ethanol-Gemisch, IonenaustauschChromatographie in einem Salz-Gradienten und Reverse-Phase-Chromatographie in einem Wasser-Methanol-Gradienten. Für die Nukleotid-Aufreinigung optimierte Chromatographie-Säulen werden z.B. von der Firma Sigma-Aldrich angeboten.
Die Aufreinigung von makromolekularen Linker-Komponenten und Marker-Komponenten kann durch Ultrafiltration, Gel-Elektrophorese, Gelfiltration und Dialyse erfolgen, siehe "Bioconjugation: protein coupling techniques for the biomedical sciences", M. Aslam, 1996, ISBN 0-333-58375-2.

Die Masse der Nuk-Makromoleküle unterscheidet sich wesentlich von der Masse der Nukleotide. Aus diesem Grund ist es vorteilhaft, bei den Endreinigungsschritten die Ultrafiltration einzusetzen. Da für die Nuk-Makromoleküle nur eine durchschnittliche Masse bereichnet wird, eignet sich Ultrafiltration auch als analytische Methode zu Trennung von Syntheseprodukten.

Zur Charakterisierung der Nuk-Makromoleküle können unterschiedliche Methoden der makromolekularen Chemie angewendet werden, z.B. UV-Vis-Spektroskopie, Fluoreszenzmessung, Massenspektroskopie, Fraktionierung, Größenausschlußchromatographie, Ultrazentrifugation und elektrophoretische Techniken, wie IEF, denaturierende und nicht denaturierende Gel-Elektrophorese ("Makromoleküle, Chemische Struktur und Synthesen", Band 1, 4, H. Elias, 1999, ISBN 3-527-29872-X, "Bioconjugation: protein coupling techniques for the biomedical sciences", M. Aslam, 1996, ISBN 0-333-58375-2).
Die Messung von freien SH-Gruppen in einer Substanz erfolgte mit Ellmans Reagenz (5,5' -Dithiobis (2-Nitrobenzolsäure), Riddles et al. Method in Enzym. 1983, V.91, S. 49.

### Modifizierte Nukleinsäureketten

In einer Ausführung der Erfindung schließen Nukleinsäureketten Nuk-Komponenten der Nuk-Makromoleküle als Einheiten der Kette ein.

### Synthese modifizierter Nukleinsäureketten

Als Monomer einer Polymer-Kette können Nuk-Makromoleküle in unterschiedlicher Art und Weise in die Kette eingebaut bzw. integriert werden. Generell kann man zwischen den chemischen und enzymatischen Schritten unterscheiden. Im weiteren werden einige Beispiele der Synthese-Strategien vorgestellt.

### Chemischer Einbau:

Beim chemischen Einbau kann ein komplettes Nuk-Makromolekül (d.h. Nul-Linker-Marker) oder auch seine Bestandteile, z.B. nur Nuk-Komponente, oder Nuk-Linker-Komponente, in die Reaktion eingesetzt werden. Beispielsweise wird zunächst die Nuk-Komponente in die Nukleinsäurekette nach Regeln der Oligonukleotid-Synthese (MWG-Biotech, TriLink Biotechnologies, Glen Research Laboratories, , S. Agrawal "Protocols for oligonucleotide conjugation", Humana Press 1994, M.Gait "Oligonucleotide synthesis: a practical approach" IRL Press, 1990) eingebaut, wobei ein Monomer der Kette eine zur Modifikation geeignete, geschützte reaktive Gruppe, beispielsweise eine geschützte Amino- oder Merkapto-Gruppe trägt. Nach der Entfernung der Schutzgruppe kann die Linker-Komponente und die Marker-Komponente an das Oligonukleotid gekoppelt werden.

Die Reinigung kann beispielsweise durch die Ultrafiltration oder durch Elektrophrese erfolgen.

### Enzymatischer Einbau:

Ähnlich zum chemischen Einbau kann auch beim enzymatischen Einbau ein komplettes Nuk-Makromolekül (d.h. Nul-Linker-Marker) oder auch seine Bestandteile, z.B. nur Nuk-Komponente, oder Nuk-Linker-Komponente, in die Reaktion eingesetzt werden. Beispielsweise werden Nuk-Komponenten, die eine zur Kopplung einer Linker-Komponente geeignete reaktive Gruppe tragen, als Triphosphate in einer matrizenabhängigen polymerase-gesteuerten Reaktion in die Nukleinsäurekette eingebaut. Anschließen kann eine Linker- und Marker-Kopplung erfolgen.
In einer anderen Ausführungsform kann ein komplettes Nuk-Makromolekül in die Nukleinsäurekette eingebaut werden (s. Beispiel 34).

Modifizierte Nukleinsäureketten schließen Ribonucleinsäuren und auch Desoxy-Ribonukleinsäuren ein.

Der Anteil der Nuk-Makromoleküle und der nicht modifizierten Monomeren der Nukleinsäurekette liegt vorzugsweise im Verhältnis zwischen 1:5 und 1:20, 1:20 und 1:100, 1:50 und 1:1000, 1:500 und 1:10000. Auch mehrere Nuk-Makromoleküle können pro eine Nukleinsäurekette eingebaut sein. In einer Ausführungsform schließt eine Nukleinsäurekette nur ein Nuk-Makromolekül ein, in einer weiteren Ausführungsform liegt die Zahl der Nuk-Makromoleküle pro eine Nukleinsäurekette in folgenden Bereichen: 2 bis 20, 5 bis 50, 10 bis 100.

In einer Ausführungsform ist ein Nuk-Makromolekül an das 5'-Ende der Nukleinsäurekette integriert.
In einer Ausführungsform ist ein Nuk-Makromolekül an das 3'-Ende der Nukleinsäurekette integriert.
In einer Ausführungsform ist ein Nuk-Makromolekül im Inneren der Nukleinsäurekette integriert, wobei der Abstand vom am nächsten liegenden Kettenende in folgenden Bereichen liegt (Zahl der Monomeren der Kette bis zum nächsten Kettenende): 1 bis 2, 2 bis 4, 4 bis 8, 8 bis 15, 15 bis 30, 30 bis 100, 100 bis 500.

### 3. Synthesen von modifizierten Nukleotiden

### Trennungsmethoden:

### Dünnschichtchromatographie, DC:

Analytisch: "DC-Alufolien 20 x 20 cm Kieselgel 60 F 254" (VWR), beschichtet mit Fluoreszenzindikator. Visualisierung erfolgt mittels UV-Licht. Laufmittel Ethanol-Wasser-Gemisch (70:30) (Laufmittel, LM 1) oder Ethanol-Wasser-Gemisch (90:10) (LM 2).

### Präparativ: Kieselgel-Glasplatten mit Sammelschicht (VWR). LM 1 oder LM 2.

### Umkehrphasen-Chromatographie (RP-Chromatographie), RP-18:

C-18 Material (Fluka), Säulenvolumen 10ml, Wasser-Methanol-Gradient. Fraktionen je 1ml wurden gesammelt und mit UV-Vis-Spektrometer analysiert. Fraktionen mit ähnlichen Spektren wurden vereinigt und lyophilisiert.

HPLC-Säulen mit ähnlichem Material können verwendet werden (Sigma).

### Ionenaustausch-Chromatographie

DEAE-Zellulose (VWR), Gradient NH₄HCO₃ 20mmol/l - 1mol/l, Fraktionen wurden unter UV/Vis -Kontrolle gesammelt und auf Grundlage gleicher Spektren vereinigt.

Die Affinitätsisolierung von Nuk-Makromolekülen kann beispielsweise eingesetzt werden, wenn Nuk-Makromoleküle Oligonukleotide Bestandteil der Marker-Komponente sind. Durch die Hybridisierung an eine komplementäre, auf einer festen Phase fixierte Nukleinsäure können sie selektiv isoliert werden.

Die Bestimmung der Ausbeuten für farbstoffmarkierte Produkte erfolgte an UV-Vis-Spektrometer.

Die Vollständigkeit der Kopplung an Strepavidin wurde durch eine Kontrolltitration mit einem Biotin-Farbstoff (Biotin-4-Fluoreszein, Sigma) 100 µmol/l in 50 mmol/l Borat, pH 8, 5 min bei RT durchgeführt. Bei einer kompletten Besetzung von Biotin-Bindungsstellen an Strepavidin während der Synthese erfolgt keine Markierung von Streptavidin. Bei einer nicht ausreichenden Reaktion erfolgt eine Bindung an SA. Analyse mit UV-Vis.

### Material:

Diamono PEG 10.000 (Diamino-Polyethylenglycol 10.000, Sigma), dUTP-AA (dUTP-Allylamine, Jena-Bioscience), TTP (Thymidin-Triphosphat, kann auch als dTTP gekennzeichnet werden, Sigma), 3' -Amino-TTP (3' -Amino-3' -deoxy-Thymidin-Triphosphat, Trilink Biotechnologies), PDTP (3-(2-Pyridinyl-dithio)-propionsäure, Fluka), 7-(3-Phthalimido-1-propynyl)-2'-desoxy-7-deazaguanosin und 7-(3-Phthalimido-1-propynyl)-2'-desoxy-7-deazaadenosin (Chembiotech), PDTP-NHS (3-(2-Pyridinyl-dithio)-propionsäure-N-hydroxysuccinimidyl-ester, Sigma), Cy3-NHS (Farbstoff, Amerscham Bioscience), Cy3- NHS (Cy3- N- hydroxysuccinimidyl-ester, Amerscham Bioscience), MEA (Mercaptoethylamine, Sigma), DTT (1,4-Dithio-DL-threit, Sigma), CA (Cystamine, Sigma), TCEP - (Tris-(2-carboxyethyl)phosphine, Sigma), DTBP (3,3'-Dithio-Bis-Propionsäure, Fluka), Biotin-NHS (Biotin-N-hydroxysuccinimidyl-ester, Sigma). J-Ac (Iodoacetat, Sigma), Iodacetamid (Sigma), TEAE (Tris-(2-Aminoethyl)amine, Sigma), Maleimido-ES-NHS (Maleimido-Essigsäure-N-hydroxysuccinimidyl-ester, Sigma), EDA (Ethylendiamin, Sigma), CDI (1,1' -Carbonyldiimidazol, Sigma), PAS 100 kDa (Polyacrylsäure, 100 kDa, Aldrich), NHS-PEG-Maleimid, 3.400 Da, Biotin-PEG-NHS, 5.000 Da, Fmoc-PEG-NHS, 3.400 Da, mPEG-SPA 5.000 Da, mPEG-SPA 20.000 Da (Nektar), Diamin-PEG, 6.000 Da (Fluka), 3' -Biotin-dT31 ein Oligonucleotid mit einer Sequenz aus 31 Thymidinmonophosphaten, mit einem Biotin-Molekül am 3'-Ende (MWG-Biotech), 3'-SH-Oligo-dT30 ein Oligonucleotid mit einer Sequenz aus 30 Thymidinmonophosphaten, mit einer Merkaptogruppe am 3'-Ende (MWG-Biotech), 3'-Amino-Oligo-dT31-5'-Cy3 ein Oligonucleotid mit einer Sequenz aus 31 Thymidinmonophosphaten, mit einer über einen Linker aus 6 Atomen am 3'-Ende gekoppelten Amino-Gruppe und einem Cy3-Farbstoff, gekoppelt am 5'-Enden (MWG-Biotech,), SA (Streptavidin, Roche,), SA-Cy2 (mit Cy2-Farbstoff modifiziertes Streptavidin, Amersham Bioscience). QDot (Qdot 605 Streptavidin Conjugat, Quantum Dot). Poly-Lysin 1000 - 2000 (Poly-L-lysin-hydrobromid 1000 -2000 Da, Fluka), Poly-Lysin 10.000 - 20.000 (Poly-L-lysin-hydrobromid 10.000 -20.000 Da, Fluka).

### Lieferanten und Firmenverzeichnis:

- Aldrich: - s. Sigma
- Amersham: - Amerscham Bioscience, Freiburg, Deutschland
- Chembiotech: - Chembiotech, Münster, Deutschland
- Fluka: - s. Sigma
- Jena-Bioscience: - Jena-Bioscience, Jena, Deutschland
- Molecular Probes: - Molecular Probes Europe, Leiden, Niederlande
- MWG: - MWG-Biotech, Ebersberg bei München, Deutschland,
- Nektar: - Nektar Molecular engineering, früher *Shearwater Corporation, Huntsville, AL, USA*
- Quantum Dot: - Quantum Dot Hayward, CA, USA
- Roche: - Roche, Mannheim, Deutschland
- Sigma: - Sigma-Aldrich-Fluka, Taufkirchen, Deutschland,
- Trilink: - Trilink Biotechnologies Inc. San Diego, CA, USA,

Lösungsmittel wurden, wenn nötig, absolutiert verwendet (Fluka) oder nach Standardverfahren getrocknet. Bei Lösungsmittelgemischen beziehen sich die angegebenen Mischungsverhältnisse auf die eingesetzten Volumina (v/v).

### Synthese einzelner Komponenten:

### Beispiel 1:

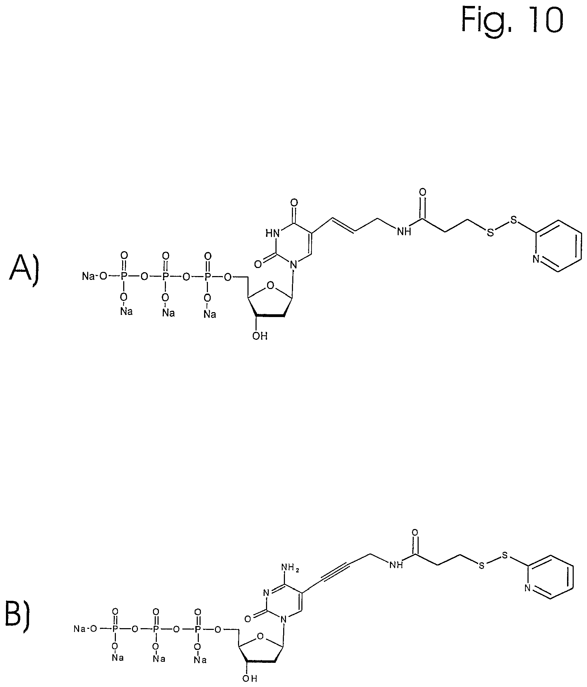

### dUTP-AA-PDTP, Fig 10A.

20 mg dUTP-AA wurden in 1ml Wasser gelöst und der pH-Wert mit NaOH auf 8.5 eingestellt. Zur dUTP-AA - Lösung wurde PDTP-NHS 60mg in 0.5 ml Methanol, tropfenweise unter Rühren zugegeben. Reaktion wurde 2 h bei 40°C durchgeführt. DC-Kontrolle: dUTP-AA-PDTP (in LM 1 Rf 0.45).
Die Trennung von überschüssigem PDTP-NHS und PDTP erfolgte auf präparativen Kieselgel-Platten, LM 2. Das Produkt, dUTP-AA-PDTP, und dUTP-AA bleiben auf der Startlinie. Die Nukleotide wurden von der Platte mit Wasser eluiert und eingeengt. Dieses dUTP-Analog trägt nun eine Disulfid-Bindung, die in einer Thiolaustauschreaktion mit anderen Thiolen reagieren kann und eine unter milden Bedingungen spaltbare Verbindung darstellt.

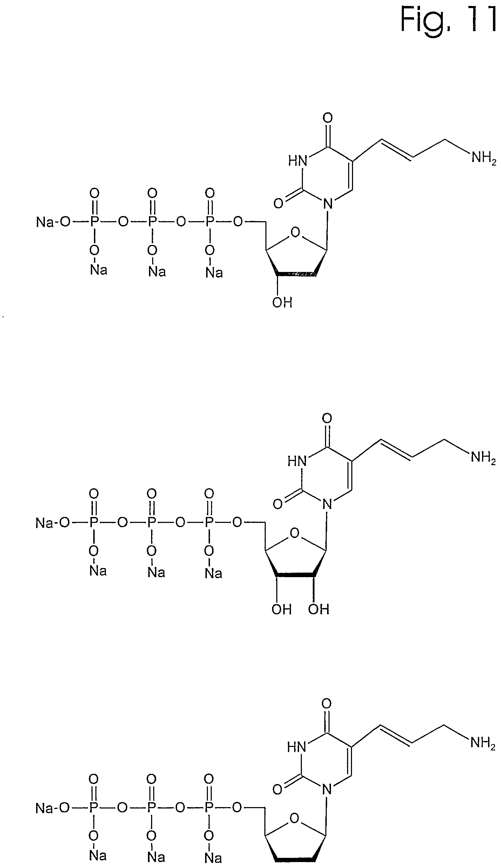

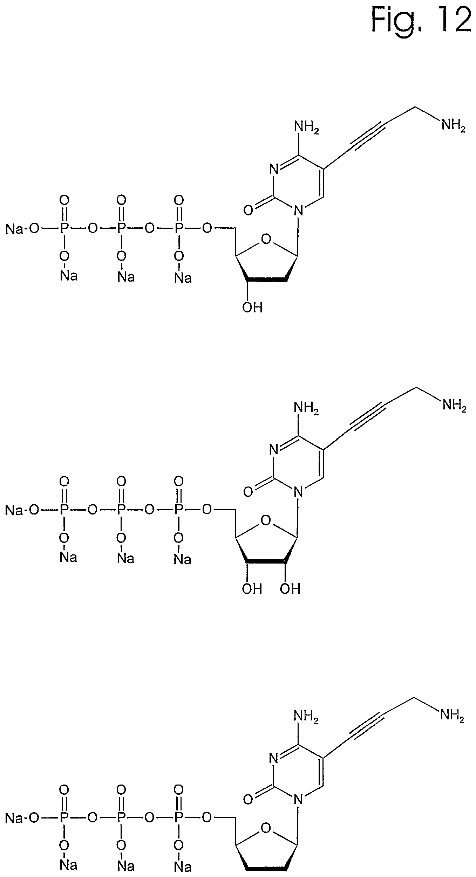

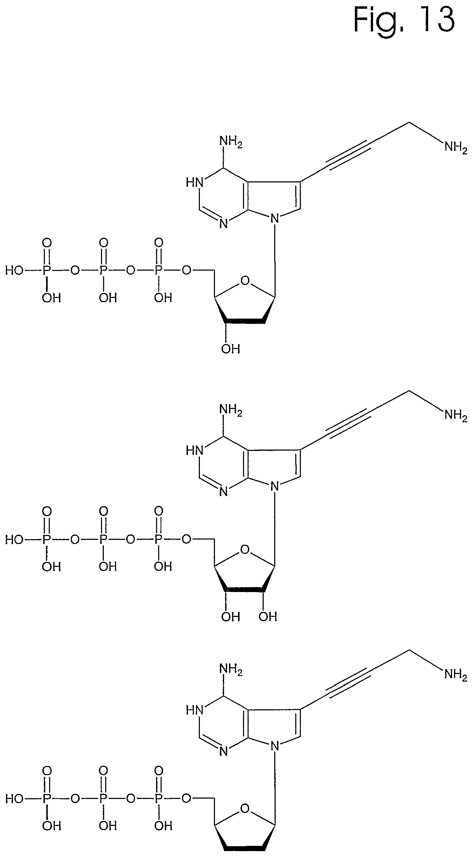

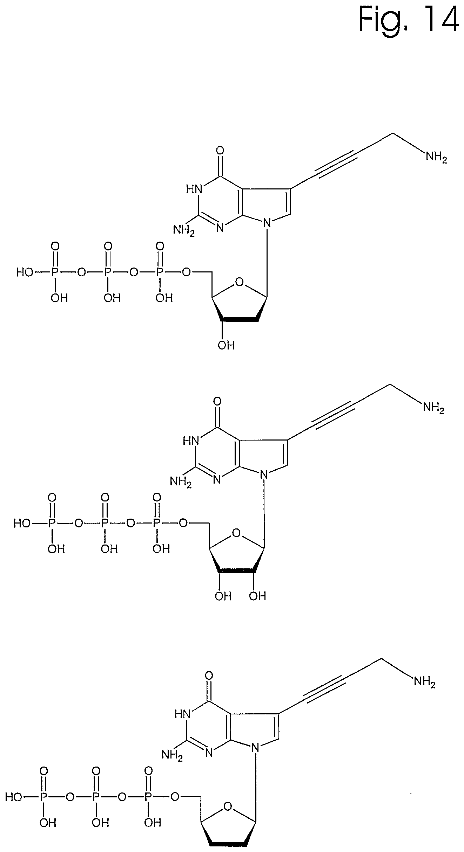

Dieses Beispiel zeigt eine generelle Möglichkeit, an Nukleotiden weitere Modifikationen vorzunehnem. Weitere basenmodifzierte Nukleotidanaloga, wie z.B. 7-Deaza-Aminopropargyl-Desoxy-Guanosin-Triphosphat und 7-Deaza-Aminopropargyl Desoxy-Adenosin-triphosphate, 5-Amino-Propargyl-Desoxy-Uridin-Triphosphate, 5-Amino-Allyl-Desoxy-Uridin-Triphosphate, 5-Amino-Propargyl-Desoxy-Cytidin-Triphosphate können wie oben angeführt ebenfalls modifiziert werden. Es können sowohl Ribonukleotide, 2' -Deoxyribonukleotide als auch 2',3'-Deoxyribonukletide verwendet werden, Fig. 11 bis 14.

### Beispiel 2:

### dCTP-PA-PDTP, Fig. 10B

Die Synthese wurde wie für dUTP-AA-PDTP, Beispiel 1, beschrieben durchgeführt.

### Beispiel 3:

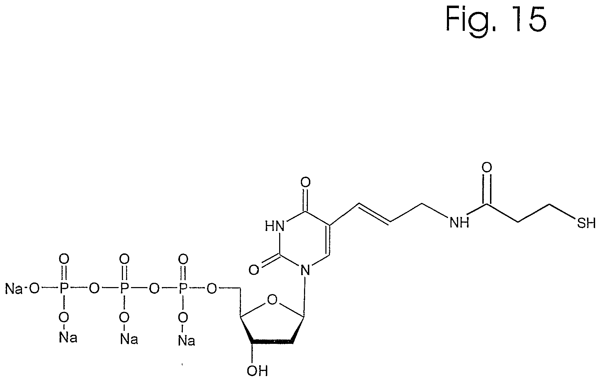

### dUTP-AA-Propionat-SH, Fig. 15

Zu 200µl 40mmol/l Lösung von dUTP-AA-PDTP wurde 1ml TCEP-Lösung 250mmol/l, pH 8, eingestellt mit NaOH, zugegeben und 10 min bei RT gerührt.

Die Trennung der Nukleotide von anderen Reagenzien erfolgte auf präparativen Kieselgel-Platten, LM 2. Produkt, dUTP-AA-Propionat-SH, bleibt auf der Startlinie. Die Nukleotide wurden von der Platte mit Wasser eluiert und eingeengt.

Dieses dUTP-Analog trägt eine reaktive SH-Gruppe, die leicht modifiziert werden kann, beispielsweise unter Ausbildung von Disulfid-Bindung.

### Beispiel 4:

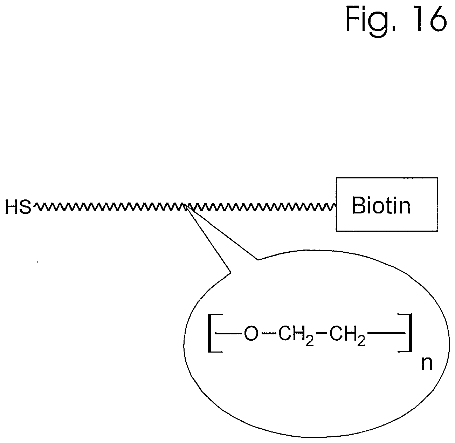

### Biotin-PEG-Ethyl-SH, Fig.16

Zu 200 µl wässriger CA-Lösung (100mmol/l), pH 8.5, eingestellt mit NaOH, wurden 10 mg Biotin-PEG-NHS zugegeben und bei 40°C 18 Stunden gerührt. Anschließend wurde 200 µl TCEP-Lösung (0.5 mol/l), pH 8.0, zugegeben und weitere 10 min bei RT gerührt. Das Produkt wurde durch Ultrafiltration auf 3.000 MWCO (Molecular weight cut off) von anderen Reagenzien getrennt. Ausbeute 35%.

Das Produkt trägt eine reaktive SH-Gruppe, die leicht modifiziert werden kann, beispielsweise unter Ausbildung von Disulfid-Bindung.

### Beispiel 5:

### Bis-Dithio-(Ethyl-PEG-Biotin),

Zu 1 ml wässriger CA-Lösung (2 mmol/l), pH 8.5 mit NaOH eingestellt, wurden 100mg Biotin-PEG-NHS zugegeben und bei RT 18 Stunden gerührt. Das Produkt wurde durch Ultrafiltration auf 10.000 MWCO von anderen Reagenzien getrennt und lyophilisiert. Ausbeute 13%.

Das Produkt besitzt eine Disulfid-Verbindung, die an Thiolaustauschreaktionen teilnehmen kann.

### Beispiel 6:

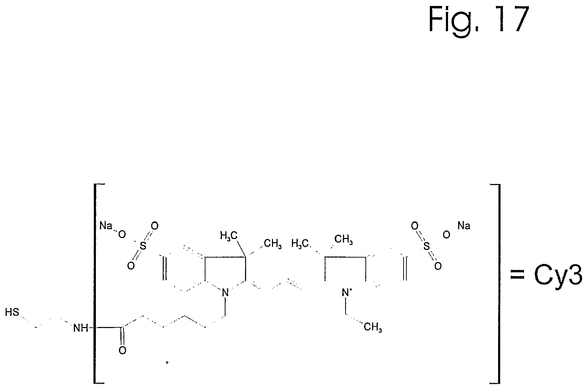

### MEA-Cy3, Fig. 17

Zu 1ml wässriger CA-Lösung, 200 mmol/l, pH 8.5 eingestellt mit NaOH, wurde Cy3-NHS zugegeben, bis die Konzentration des Cy3-Farbstoffs 10 mmol/l betrug. Die Reaktion wurde 10 min bei RT gerührt. Anschließend wurde 1 ml TCEP-Lösung (0.5 mol/l), pH 8.0 eingestellt mit NaOH, zugegeben und weitere 10 min bei RT gerührt. Das Produkt wurde auf RP-18 getrennt (Wasser-Methanol-Gradient) und auf 0.5ml eingeengt, Ausbeute 93%, UV-Vis.

Das Produkt trägt eine reaktive SH-Gruppe, die leicht modifiziert werden kann, beispielsweise unter Ausbildung von Disulfid-Bindung.

### Beispiel 7:

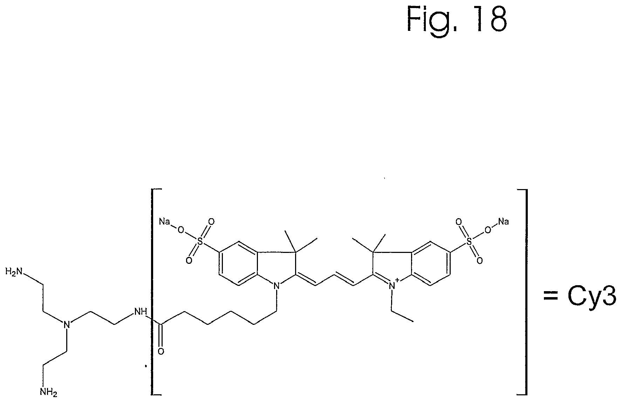

### Cy3-TEAE, Fig. 18

Zu 1ml wässriger TEAE-Lösung, 300 mmol/l, pH 8.5 mit NaOH eingestellt, wurde Cy3-NHS zugegeben, bis die Konzentration des Cy3-Farbstoffs 5mmol/l betrug. Die Reaktion wurde 10 min bei RT gerührt. Das Produkt wurde auf RP-18 getrennt und auf 0.5ml eingeengt, Ausbeute 82%, UV-Vis.

Das Produkt hat zwei Aminogruppen, die leicht mit anderen Reagentien modifiziert werden können und neue Funktionalitäten an den Farbstoff koppeln können.

### Beispiel 8:

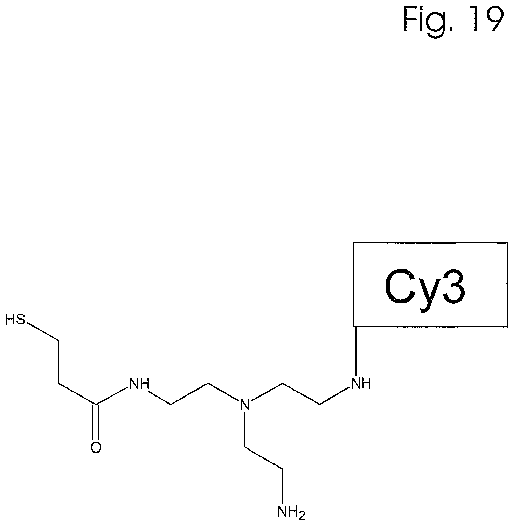

### Cy3-TEAE-Propionat-SH, Fig. 19

Zu 300µl wässriger Cy3-TEAE, 2mmol/l, pH 7.5, wurden langsam 30µl einer frisch bereiteten methanolischen Lösung von PDTP-NHS, 30mmol/l, zugegeben. Der Ablauf der Reaktion wurde durch DC, LM 1, kontrolliert. Als Reaktionsprodukte erscheinen auf DC Substanzen (LM 1) mit Rf. 0.55 (Cy3-TEAE-PDTP) und 0.95 (Cy3-TEAE-(PDTP)2). Nach Ablauf von 1 h wurde die Reaktion gestoppt und die Produkte auf DC mit LM 1 getrennt. Die Substanz mit Rf. 0.55 wurde isoliert, eingeengt und in 200µl Wasser aufgelöst. Zu dieser Lösung wurde 0.1ml TCEP-Lösung (0.5 mol/l), pH 8.0, zugegeben und weitere 10 min bei RT gerührt. Das Produkt, Cy3-TEAE-Propionat-SH, wurde auf RP-18 getrennt (Wasser-Methanol-Gradient) und auf 0.5ml eingeengt, Ausbeute 26%, UV-Vis.

Das Produkt trägt einerseits eine reaktive SH-Gruppe, die leicht modifiziert werden kann, beispielsweise unter Ausbildung von Disulfid-Bindung, andererseits eine Amino-Gruppe, die mit anderen Reagentien reagieren kann.

### Beispiel 9:

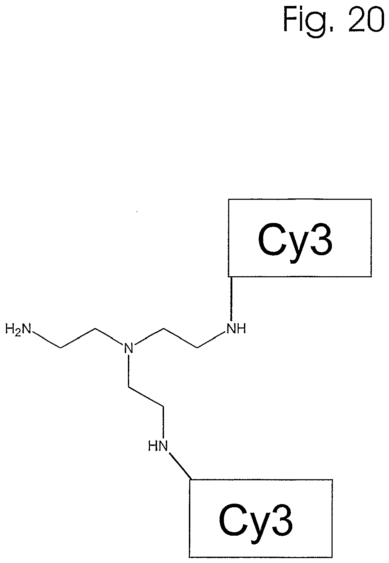

### TEAE-(Cy3)2, Fig. 20

Zu 1ml wässriger TEAE-Lösung, 2 mmol/l, wurde Cy3-NHS zugegeben, bis die Konzentration des Cy3-Farbstoffs 4mmol/l betrug. Die Reaktion wurde 10 min bei RT gerührt. Das Produkt (Rf. 0.45) wurde auf präparat. Kieselgel DC mit LM 1 von anderen Reaktionsprodukten getrennt, mit 50mM Borat-Puffer, pH 9, eluiert, danach auf RP-18 gesammelt und anschließend mit 50% Et-OH eluiert und auf 0.5ml eingeengt, Ausbeute 22%, UV-Vis.

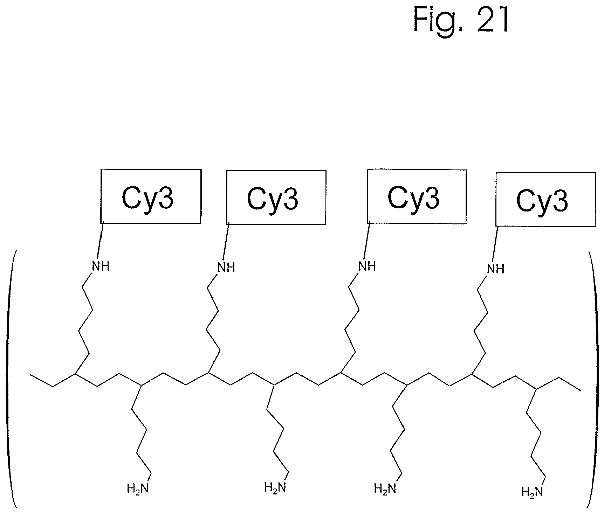

### Beispiel 10, Fig. 21

### Poly-Lysin-(Cy3)n, n = 10-15, Poly-Lysin 10.000-20.000

Zu 1ml wässriger Poly-Lysin-Lösung, 1 mmol/l, wurde Cy3-NHS zugegeben, bis die Konzentration des Cy3-Farbstoffs 18mmol/l betrug. Die Reaktion wurde 40 min bei RT gerührt. Die Trennung von modifiziertem Poly-lysin von Farbstoffresten erfolgte durch Ultrafiltration, 3000 MWCO. Bestimmung der durchschnittlichen Zahl Cy3-Moleküle erfolgte über UV-Vis-Spektrometer.

Poly-Lysin stellt ein Beispiel für ein Kern-Komponente dar, an die mehrere Marker-Einheiten, beispielsweise Farbsoffe gekoppelt werden können. In diesem Experiment wurde die Verteilung von Cy3-Molekülen in Poly-Lysin-Population rechnerisch ermittelt, aus den bekannten Größenunterschieden der Poly-Lysin-Moleküle und der mittleren ermittelten Zahl von Cy3-Moleküle.

### Beispiel 11,

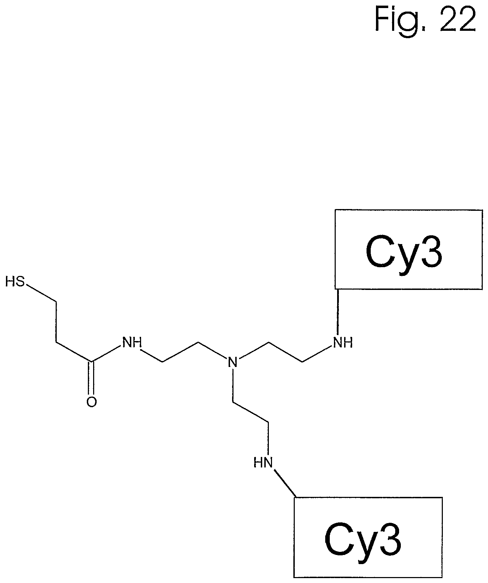

### TEAE-(Cy3)2-PDTP und TEAE-(Cy3)2-Propionat-SH, Fig 22:

Zu 200µl TEAE-(Cy3)2, 1mmol/l wurden 10mg, PDTP-NHS zugegeben und 1 Stunde bei RT gerührt. Der Reaktionsverlauf wurde durch DC, LM 1, kontrolliert. Nach 1 h wurde eine fast quantitative Umsetzung von TEAE-(Cy3)2 (Rf. 0.45) zu TEAE-(Cy3)2-PDTP, (Rf. 0.85) festgestellt. Der Ansatz wurde in zwei gleiche Teile geteilt.
Das Produkt, TEAE-(Cy3)2-PDTP, aus dem ersten Teil wurde auf RP-18 getrennt und lyophilisiert. Ausbeute 82%, UV-Vis.
Das Produkt trägt eine Disulfidbindung, die leicht an einer Thiolaustauschreaktion teilnehmen kann und somit an andere Verbindungen gekoppelt werden kann.

Zum zweiten Teil wurde 0.1ml TCEP-Lösung (0.5 mol/l), pH 8.0, zum Ansatz zugegeben und noch 10 min bei RT gerührt. Das Produkt, TEAE-(Cy3)2-Propionat-SH, wurde auf RP-18 getrennt, Ausbeute 68%, UV-Vis.
Das Produkt trägt eine reaktive SH-Gruppe, die leicht modifiziert werden kann, beispielsweise unter Ausbildung von Disulfid-Bindung.

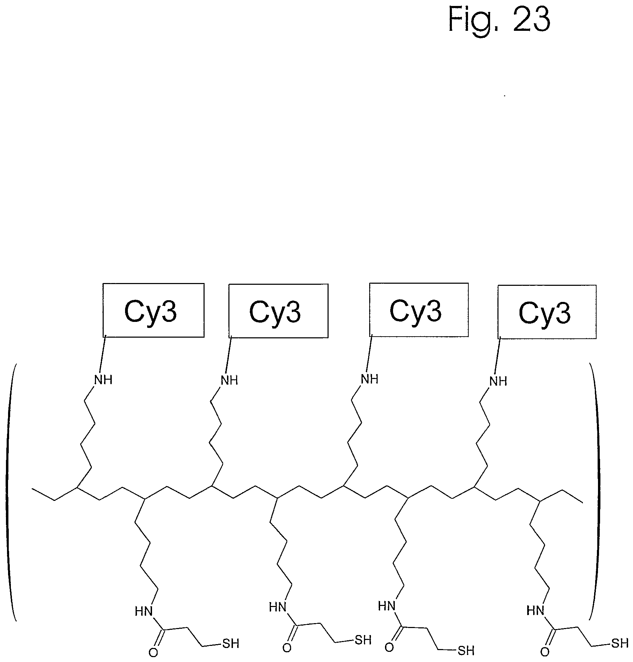

### Beispiel 12, Fig 23

(HS-Propionat)m-Poly-Lysin-(Cy3)n, (n = 10-15, m 3-9, Poly-Lysin 10.000-20.000) Zu 200µl Poly-Lysin-(Cy3)n, 1mmol/l wurden 10mg, PDTP-NHS zugegeben und 1 Stunde bei RT gerührt. Das Produkt, (PDTP)m-Poly-Lysin-(Cy3)n, wurde durch Ultrafiltration von den PDTP-Resten abgetrennt und anschließend in 100µl Wasser gelöst. Danach wurde 0.1ml TCEP-Lösung (0.5 mol/l), pH 8.0, zum Ansatz zugegeben und weitere 10 min bei RT gerührt. Das Produkt, (HS-Propionat)m-Poly-Lysin-(Cy3)n, wurde mit 3.000 MWCO von den niedermolekularen Bestandteilen getrennt.

Das Produkt trägt mehrere reaktive SH-Gruppen, die leicht modifiziert werden können, beispielsweise unter Ausbildung von Disulfid-Bindung.

### Beispiel 13:

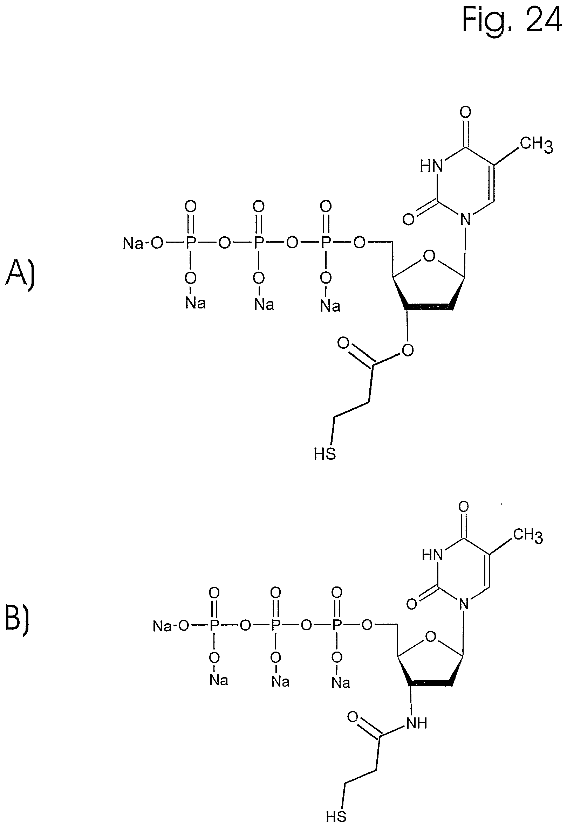

### TTP-3' -O-Propionat-SH, Fig 24A,

Die Synthese von 3' -modifizierten Nukleotiden erfolgt nach Gottikh et al. Tetrahedron, 1970, v. 26, 4419-, Schäfer et al. Method in Enzymology, 1986, v. 126, S. 682-.

DTBP, 210 mg, wurden in 1ml DMF gelöst. Zu dieser Lösung wurde CDI 320mg zugegeben und 1 h bei RT gerührt. Anschließend wurden 10µl Methanol zugegeben und nach weiteren 10 min wurden 100µl dieser Lösung, 1mol/l, zu 300µl wässriger 100mmol/l-Lösung von TTP, pH 8.5 eingestellt mit NaOH, zugegeben und ca. 4 h bei RT kräftig gerührt. Die Nukleotide wurden durch Präzipitation mit Ethanol isoliert und dann in 300µl Wasser gelöst. Anschließend wurde 200µl TCEP-Lösung (0.5 mol/l), pH 8.0, zugegeben und nach 10 min bei RT erfolgt die wiederholte Präzipitation der Nukleotide. Die Trennung der modifizierten Nukleotide von den nicht modifizierten ist auf dieser Stufe nicht notwendig. Ausbeute 13%, UV-Vis.

Das Produkt trägt eine reaktive SH-Gruppe, die leicht modifiziert werden kann, beispielsweise unter Ausbildung von Disulfid-Bindung.

### Beispiel 14:

### TTP-3' -Amino-PDTP, Fig 24B

Die Synthese erfolgte wie für dUTP-AA, Beispiel 1, beschrieben: Als Edukte wurden 3' -Amino-3' -Deoxy-TTP, 100µl, 10mmol/l Lösung, pH 8 und PDTP-NHS eingesetzt. Ausbeute 19%, UV-Vis.

Das Produkt trägt eine Disulfidbindung, die leicht an einer Thiolaustauschreaktion teilnehmen kann und somit an andere Verbindungen gekoppelt werden kann.

Auch andere, am 3'-Ende mit einer Bindungsstelle, z.B. einem kurzen Linker, modifizierte Nukleotide können eingesetzt werden. Synthesen sind dargestellt beispielsweise in Metzker et al. Nucleic acid Research 1994, V.22, S. 4259, Canard et al. Gene, 1994, V. 148, 1, Hovinen et al. J. Chem. Soc. Perk. Trans. 1994V.1, S. 211, Herrlein et al. Helvetica Chimica Acta, 1994, V. 77, S. 586, Jameson et al. Method in Enzymology, 1997, V. 278, S. 363, Canard US. Pat. 5798210, Kwiatkowski US Pat. 6255475, Kwiatkowski WO 01/25247, Parce WO 0050642, Faulstich DE 4418691.

Weitere Beispiele für basenmodifizierte Nukleotide, die als Nuk-Komponente verwendet werden können, sind in Balasubramanian WO 03048387 beschrieben. Noch weitere Beispiele für Nukleotid-Analoga sind in "Nucleotide Analogs" Scheit, 1980, ISBN 0-471-04854-2, "Nucleoside and Nucleic Acid Chemistry", Kisakürek 2000, "Anti-HIV Nucleosides" Mitsuya, 1997, "Nucleoside Analogs in cancer therapy", Cheson, 1997 angegeben. Ein Fachmann sollte erkennen, dass auch andere Nukleoside und Nukleotide verwendet werden können.

### Beispiele für Kopplung von Linker-Komponenten und Marker-Komponenten an Nuk-Komponenten

### Beispiel 15:

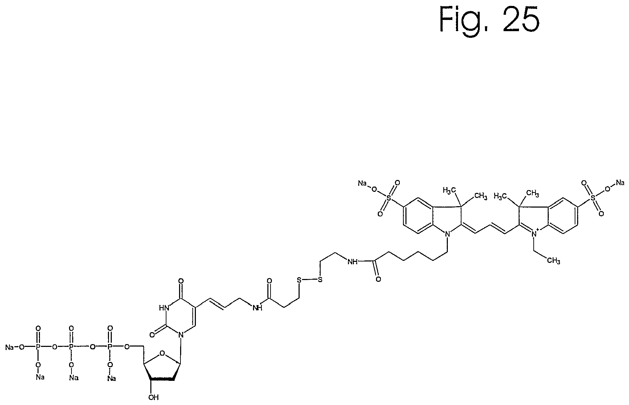

### dUTP-AA-SS-MEA-Cy3, Fig. 25

Zu 100µl, 10mmol/l MEA-Cy3 in 50mM Borat, pH 9.5, wurde 50µl 30mmol/l dUTP-AA-PDTP in 50mM Borat, pH 9.5 zugegeben. Nach 1 Stunde wurde das dUTP-AA-SS-MEA-(Cy3) durch Dünnschicht-Chromatographie, LM 1, Rf. 0.6, von MEA-Cy3, Rf. 0.9, getrennt. Anschließend wurde dUTP-AA-SS-MEA-(Cy3) auf RP-18 von dUTP-AA-PDTP getrennt. Ausbeute 67%, UV-Vis.

Es wurde eine Verbindung erhalten, die eine Nukleotid-Funktionalität und eine niedermolekulare Marker-Funktionalität trägt.
Das Produkt ist definitionsgemäß ein konventionell modifiziertes Nukleotid: die Linker-Länge ist kleiner als 30 Atome, die Marker-Komponente ist niedermolekular. Es kann stellvertretend für konventionell modifizierte Nukleotide mit nur einem niedermolekularen Marker auftreten.
Diese Verbindung wird von Polymerasen (z.B. Klenow-Exo minus Polymerase) als Substrat akzeptiert, s. Beispiel 34A.

### Beispiel 16:

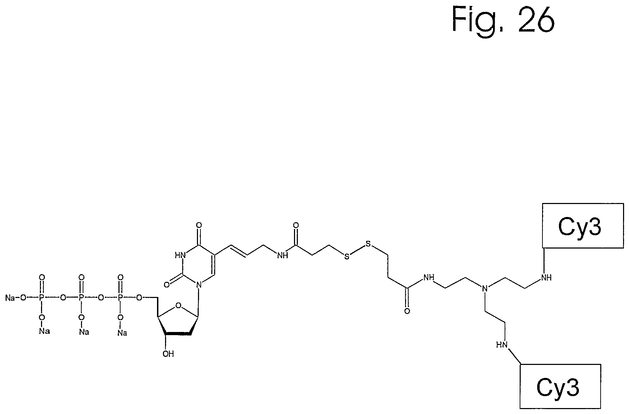

### dUTP-AA-SS-TEAE-(Cy3)2, Fig. 26

dUTP-AA-SS-TEAE-(Cy3)2 wurde ähnlich wie dUTP-AA-SS-MEA-(Cy3), Beispiel 15, synthetisiert, dabei wurde TEAE-(Cy3)2-Propionat-SH anstatt von MEA-Cy3 verwendet. Ausbeuten 43%, UV-Vis.

Es wurde eine Verbindung erhalten, die eine Nukleotid-Funktionalität und zwei niedermolekulare Marker-Funktionalitäten trägt.
Das Produkt ist definitionsgemäß ein konventionell modifiziertes Nukleotid: die Linker-Länge ist kleiner als 30 Atome, die Marker-Komponente ist niedermolekular. Es kann stellvertretend für konventionell modifizierte Nukleotide mit mehreren niedermolekularen Markern auftreten.

Diese Verbindung wird von Polymerasen (z.B. Klenow-Exo minus Polymerase) als Substrat nicht akzeptiert. Die Modifikation des Nukleotids führt zum Verlust der Substrateigenschaften.

### Beispiel 17:

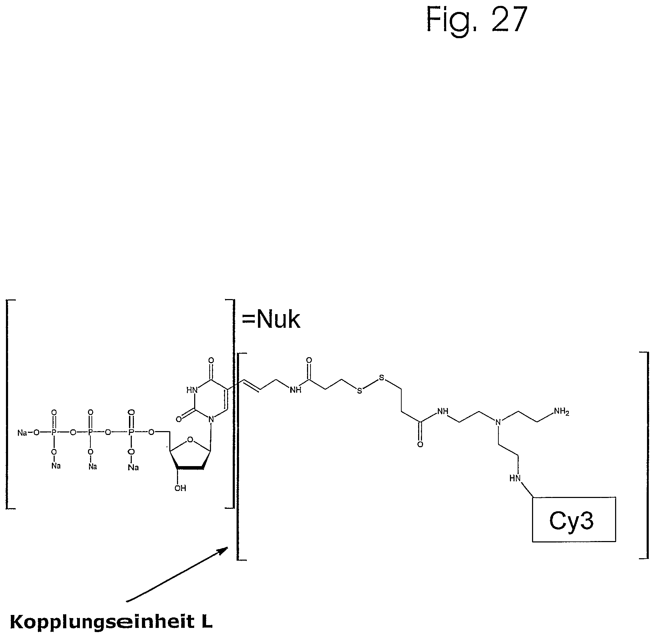

### dUTP-AA-SS-Propionat-TEAE-Cy3, Fig. 27

dUTP-AA-SS-Propionat-TEAE-Cy3 wurde ähnlich wie dUTP-AA-SS-MEA-Cy3, Beispiel 15, synthetisiert, dabei wurde Cy3-TEAE-Propionat-SH anstatt von MEA-Cy3 verwendet. Ausbeute 37%, UV-Vis.

Diese Verbindung trägt eine Nukleotid-Funktionalität und eine niedermolekulare Marker-Funktionalität. Der Linker an der Base hat eine freie Aminogruppe, die modifiziert werden kann.
Das Produkt ist definitionsgemäß ein konventionell modifiziertes Nukleotid: die Linker-Länge ist kleiner als 30 Atome, die Marker-Komponente ist niedermolekular. Das Nukleotid wird von Polymerasen als Substrat akzeptiert.

### Beispiel 18:

### (dUTP-AA-SS-Propionat)m-Poly-Lysin-(Cy3)n

### Edukte:

### dUTP-AA-PDTP

### (HS-Propionat)m-Poly-Lysin-(Cy3)n, n = 10-15, m 3-9, Poly-Lysin 10.000-20.000

Zu 50µl einer Lösung mit dUTP-AA-PDTP, 20mmol/l, in 50mM Borat-Puffer, pH 9.0, wurde 20µl (HS-Propionat)m-Poly-Lysin-(Cy3)n, ca. 1mmol/l, in Wasser, gegeben und 18 h bei RT gerührt. Das Produkt wurde durch Ultrafiltration, 30.000 MWCO, gereinigt.

Es wurde eine Verbindung erhalten, die eine Nukleotid-Funktionalität und eine makromolekulare Marker-Funktionalitäten trägt. Das Produkt der Reaktion ist definitionsgemäß ein konventionell modifiziertes Nukleotid: die Linker-Länge ist unter 30 Atome, die Marker-Komponente ist makromolekular.

Diese Verbindung wird von Polymerasen (z.B. Klenow-Exo minus Polymerase und Terminaler Transferase) als Substrat nicht akzeptiert. Die Modifikation des Nukleotids führt zum Verlust der Substrateigenschaften.

### Beispiel 19:

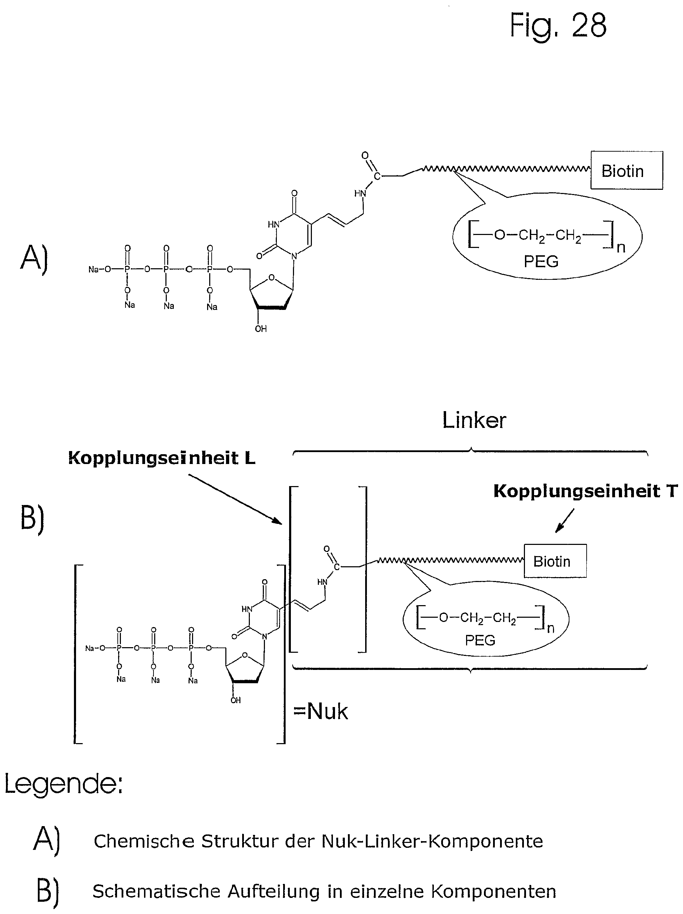

### dUTP-AA-PEG-Biotin, Fig. 28

Zu 100µl wässriger Lösung dUTP-AA, 50mmol/l, pH 8.0, wurden 10mg Biotin-PEG-NHS gegeben und bei 40°C 18 Stunden gerührt. Anschließend wurde das nicht umgesetzte Nukleotid durch Ultrafiltration, 3.000 MWCO, abgetrennt und das Produkt der Reaktion, das dUTP-AA-PEG-Biotin, mehrmals mit Wasser gewaschen.
Diese Verbindung trägt eine Nukleotid-Funktionalität und einen makromolekularen Linker. Biotin dient als Kopplungseinheit T. An diese Kopplungseinheit T können makromolekulare Strukturen gekoppelt werden, z.B. Streptavidin, ohne dass die Substrateigenschaften dieser Analoga verloren gehen. Dieses Nuk-Makromolekül dient als Substrat für Polymerasen.

Biotin kann auch als eine niedermolekulare Marker-Einheit mit signalvermittelnden Funktion betrachtet werden, die an den langen Linker gekoppelt ist.

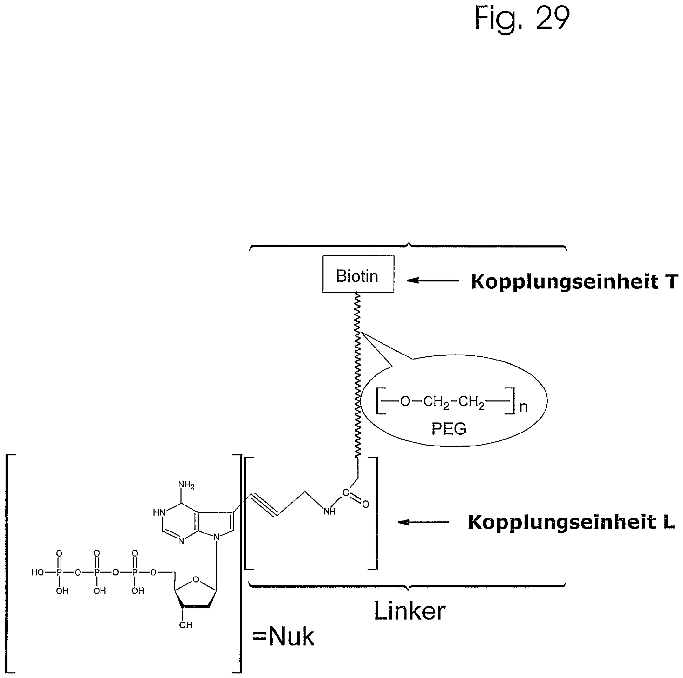

Das Produkt der Reaktion ist definitionsgemäß eine Zwischenstufe eines Nuk-Makromoleküls: die Linker-Länge ist deutlich größer als 30 Atome und an die Kopplungseinheit T können weitere Makromoleküle gekoppelt werden.
Dieses Beispiel zeigt eine generelle Möglichkeit, an Nukleotiden weitere Modifikationen vorzunehnem. Weitere basenmodifzierte Nukleotidanaloga, wie z.B. 5-PropargylaminodCTP, 7-Deaza-Aminopropargyl-dGTP, 5-Amino-Propargyl-dUTP und 7-Deaza-Aminopropargyl-dATP (Fig. 29) können wie oben angeführt ebenfalls modifiziert werden. Es können sowohl Ribonukleotide, 2' -Deoxyribonukleotide als auch 2',3'-Deoxyribonukletide verwendet werden, Fig. 11 bis 14.

In einer ähnlichen Weise können auch andere Polymere, z.B. PEG-Derivate, als Linker gekoppelt werden. Ein Beispiel dafür stellt dATP-PA-PEG-NH₂.
Zu 100pl wässriger Lösung 7-Deaza-7-Aminopropargyl-dATP (hergestellt aus 7-(3-Phthalimido-1-propynyl)-2' -desoxy-7-deazaadenosin von Jena Bioscience), 50mmol/l, pH 8.0, wurden 10mg Fmoc-PEG-NHS (Fmoc-geschützter NH₂-PEG-NHS) gegeben und bei 40°C 18 Stunden gerührt. Anschließend wurde der pH-Wert auf 11 erhöht und die Reaktionsmischung weitere 2 Stdunden bei RT gerührt. Anschließend wurde das nicht umgesetzte Nukleotid durch Ultrafiltration, 3.000 MWCO, abgetrennt und das Produkt der Reaktion, das dATP-PA-PEG-NH₂, mehrmals mit Wasser gewaschen.
Diese Verbindung trägt eine Nukleotid-Funktionalität und einen makromolekularen Linker. NH2-Gruppe dient als Kopplungseinheit T für die Marker-Komponente. An diese Kopplungseinheit T können makromolekulare Strukturen gekoppelt werden, z.B. Polyacryl-säurederivate, ohne dass die Substrateigenschaften dieser Nukleotid-Analoga verloren gehen. Dieses Nuk-Makromolekül dient als Substrat für Polymerasen.

### Beispiel 20:

### TTP-3'-Amino-PEG-Biotin,

Die Synthese erfolgte ähnlich wie für dUTP-AA-PEG-Biotin, Beispiel 19. Als Edukte wurden 3' -Amino-3' -Deoxy-TTP und Biotin-PEG-NHS eingesetzt. Diese Verbindung trägt eine Nukleotid-Funktionalität und einen makromolekularen Linker und eine niedermolekulare Marker-Einheit (Biotin), die eine signalvermittelnde Fuktion hat. An das Biotin können makromolekulare signaltragende Streptavidin-Moleküle gekoppelt werden.

Das Produkt der Reaktion ist definitionsgemäß eine Zwischenstufe eines Nuk-Makromolekül: die Linker-Länge ist deutlich größer als 30 Atome, die Marker-Komponente ist niedermolekular.

Auch andere Nukleotid-Analoga mit einer Amino-Gruppe in der 3' -Position können in ähnlicher Weise gekoppelt werden.

### Beispiel 21:

### dCTP-PA-PEG-Maleimid,

Die Synthese erfolgte wie für dUTP-AA-PEG-Biotin, Beispiel 19, beschrieben. Als Edukte wurden dCTP-PA und Maleimid-PEG-NHS eingesetzt.
Diese Verbindung hat eine Nukleotid-Funktionalität und einen makromolekularen Linker. Kopplungseinheit T an diesem Linker ist die Maleimid-Gruppe. An Maleimid-Funktionalität können makromolekulare signaltragende Moleküle gekoppelt werden, die eine oder mehrere SH-Gruppen tragen.

Maleimid-Gruppe kann auch als eine niedermolekulare Marker-Einheit mit signalvermittelnden Funktion betrachtet werden, die an den langen Linker gekoppelt ist.

Das Produkt der Reaktion ist definitionsgemäß eine Zwischenstufe eines Nuk-Makromoleküls: die Linker-Länge ist deutlich größer als 30 Atome, die Marker-Komponente ist niedermolekular. An diese Marker-Komponente können makromolekulare Strukturen gekoppelt werden, ohne dass die Substrateigenschaften dieser Analoga verloren gehen.

Dieses Beispiel zeigt eine generelle Möglichkeit, an Nukleotiden weitere Modifikationen vorzunehnem. Weitere basenmodifzierte Nukleotidanaloga, wie z.B. 5-AllylaminodUTP, 5-Amino-Propargyl-dUTP, 7-Deaza-Aminopropargyl-dGTP und 7-Deaza-Aminopropargyl-dATP können wie oben angeführt ebenfalls modifiziert werden. Es können sowohl Ribonukleotide, 2' -Deoxyribonukleotide als auch 2',3'-Deoxyribonukletide verwendet werden, Fig. 11 bis 14.

### Beispiel 22:

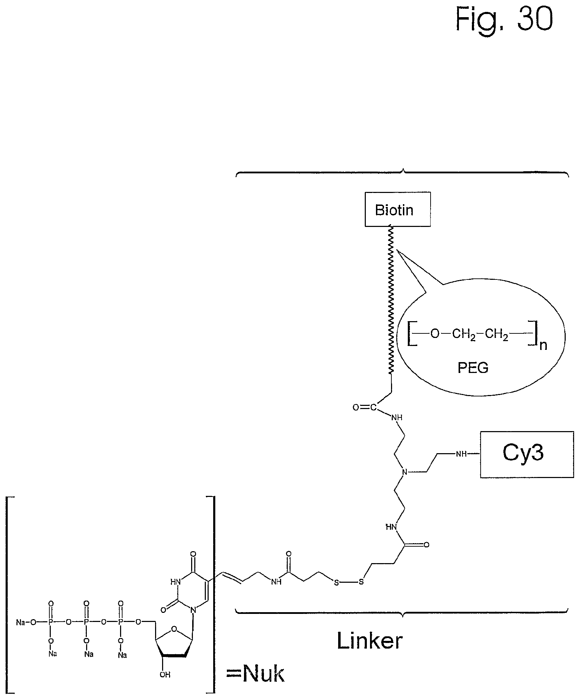

### dUTP-AA-SS-Propionat-TEAE-(Cy3)-PEG-Biotin, Fig. 30

Die Synthese erfolgte ähnlich wie für dUTP-AA-PEG-Biotin, Beispiel 19 beschrieben. Als Edukte wurden dUTP-AA-SS-Propionat-TEAE-Cy3 und Biotin-PEG-NHS eingesetzt. Die Trennung vom nicht abreagierten dUTP-Analog erfolgte durch Ultrafiltration, 3.000 MWCO.

Diese Verbindung trägt eine Nukleotid-Funktionalität, einen Farbstoff, einen makromolekularen Linker und eine niedermolekulare Marker-Funktionalität (Biotin), die eine signalvermittelnde Fuktion hat. Biotin kann auch als eine Kopplungseinheit T des Linkers betrachtet werden.

Das Produkt der Reaktion ist definitionsgemäß eine Zwischenstufe eines Nuk-Makromoleküls: die Linker-Länge ist deutlich größer als 30 Atome, Biotin dient als Kopplungseinheit T. An diese Kopplungseinheit T können makromolekulare Strukturen gekoppelt werden, ohne dass die Substrateigenschaften dieser Analoga verloren gehen. Dieses Nuk-Makromolekül dient als Substrat für Polymerasen.

Der Farbstoff dient als sterisch anspruchsvolle Gruppe, die den enzymatischen Einbau von nur einem Nuk-Makromolekül in den wachsenden Strang durch die Polymrase zuläßt. Die Eigenschaften solcher Nukleotide sind detailliert in Tcherkassov WO 02088382 beschrieben.

### Beispiel 23:

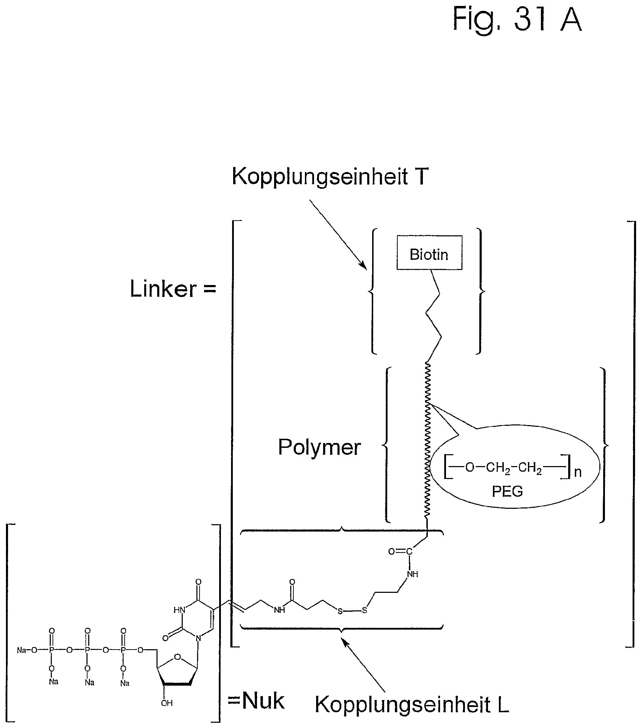

### dUTP-AA-SS-PEG-Biotin, Fig. 31 A,

Zu 100µl, 10mmol/l Biotin-PEG-Ethyl-SH in 50mM Borat, pH 9.5, wurde 50µl 30mmol/l dUTP-AA-PDTP in 50mM Borat, pH 9.5 zugegeben. Reaktion wurde 18 Stunden bei RT gerührt. Die Trennung erfolgt ähnlich wie für die Synthese von dUTP-AA-PEG-Biotin, Beispiel 19 beschrieben.

Diese Verbindung trägt eine Nukleotid-Funktionalität und einen makromolekularen Linker. Biotin dient als Kopplungseinheit T. An diese Kopplungseinheit T können makromolekulare Strukturen gekoppelt werden, z.B. Streptavidin, ohne dass die Substrateigenschaften dieser Analoga verloren gehen. Dieses Nuk-Makromolekül dient als Substrat für Polymerasen. Über Streptavidin können auch weitere makromoleküle, beispielsweise Enzyme oder Nukleinsäurenge koppelt werden.

Das Produkt der Reaktion ist definitionsgemäß eine Zwischenstufe eines Nuk-Makromolekül: die Linker-Länge ist deutlich größer als 30 Atome.

Biotin kann auch als signalvermittelnde niedermolekulare Marker-Einheit bezeichnet werden.

Die Linker-Komponente kann zusammen mit der Marker-Komponente von der Nuk-Komponente unter milden Bedingungen abgespalten werden. Dies kann beispielsweise von Vorteil sein, wenn eine Sequenzierung durch Synthese (Balasubramanian WO 03048387, Tcherkassov WO 02088382, Quake WO0132930, Kartalov WO02072892) durchgeführt wird, wobei nach jedem Detektionsschritt eine Entfernung der Marker notwendig ist.

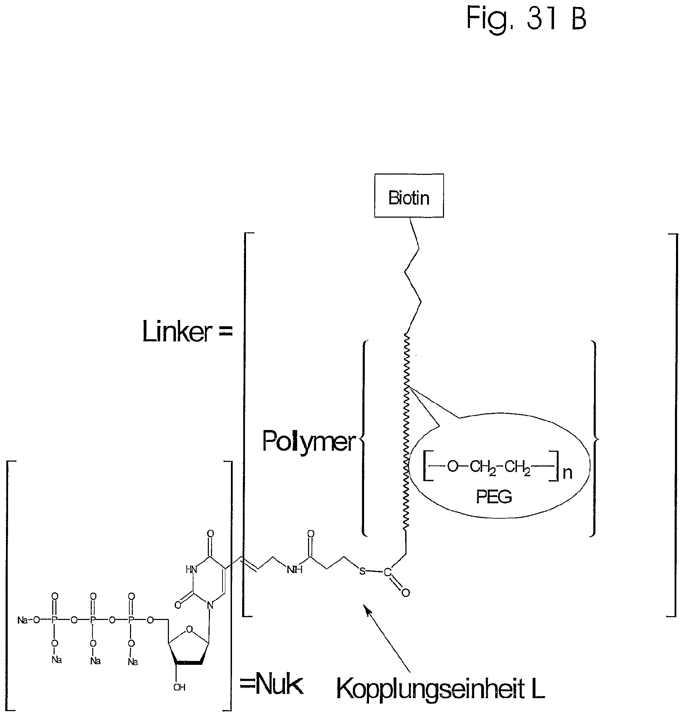

Ein weiteres Beispiel für die Nuk-Makromoleküle mit einer unter milden Bedingungen spaltbaren Gruppe: dUTP-AA-Propionat-S-CO-PEG-Biotin (Fig. 31 B).
Für diese Reaktion wurde das dUTP-AA-PDTP zunächst auf RP-HPLC in Wasser-Methanol-Gradient gereinigt.
Zu 10µl einer 50 mmol/l Lösung von dUTP-AA-PDTP in Wasser wurden 20 µl einer 100 mmol/l Lösung von TCEP, pH 8, zugegeben. Die Reaktion wurde bei RT 10 min durchgeführt. Das Produkt der Reaktion, das dUTP-AA-Propioat-SH, wurde von anderen Reagenzien auf präparativen Kieselgel-Platten, LM 2, getrennt. Das Produkt, dUTP-AA-Propionat-SH, bleibt auf der Startlinie. Es wurde von der Platte mit Wasser eluiert, eingeengt und in 50 µl 50 mmol/l Borat-Puffer, pH 8, aufgelöst.
Zu dieser Lösung wurden 50 µl einer frisch angesetzten 2 % wässrigen Lösung von Biotin-PEG-NHS zugegeben. Die Reaktion erfolgte be RT 30 min lang. Anschließend wurde das Produkt der Reaktion, das dUTP-AA-Propionat-S-CO-PEG-Biotin, durch Ultrafiltration mit MWCO 3000 von niedermolekularen Reaktionskomponenten abgetrennt, fünf mal mit 0,5 ml Wasser gewaschen und in einem Volumen von 50 µl aufgelöst.

Das auf diese Weise erhaltene das dUTP-AA-Propionat-S-CO-PEG-Biotin kann von DNA-Polymerasen, beispielsweise Klenow-Fragment Exo minus oder Taq-Polymerase, in den wachsenden Strang der Nukleinsäuren eingebaut werden.

An das Biotin können über das Straptavidin weitere Marker-Komponenten gekoppelt werden.

Das das dUTP-AA-Propionat-S-CO-PEG-Biotin enthält eine unter milden Bedingungen spaltbare Gruppe, so dass der Linker mit dem Farbstoff von dem Nukleotid abgespalten werden kann. Dies ist beispielsweise in Verfahren der Sequenzierung durch die Synthese vom besonderen Interesse (Balasubramanian WO 03048387, Tcherkassov WO 02088382, Quake WO0132930, Kartalov WO02072892).

Dieses Beispiel zeigt eine generelle Möglichkeit, an Nukleotiden weitere Modifikationen vorzunehnem. Weitere basenmodifzierte Nukleotidanaloga, wie z.B. 5-PropargylaminodCTP, 5-Amino-Propargyl-dUTP, 7-Deaza-Aminopropargyl-dGTP und 7-Deaza-Aminopropargyl-dATP können wie oben angeführt ebenfalls modifiziert werden. Es können sowohl Ribonukleotide, 2' -Deoxyribonukleotide als auch 2',3'-Deoxyribonukletide verwendet werden, Fig. 11 bis 14.

### Beispiel 24:

### TTP-O-Propionat-SS-PEG-Biotin,

Zur 100µl Lösung von TTP-O-Propionat-SH, 10mmol/l, in 50mM Borat-Puffer, pH 9.5, wurden 100µl einer Lösung von Bis-Dithio-(Ethyl-PEG-Biotin), 0.5mmol/l, in Wasser gegebenund 24 Stunden bei RT gerührt. Die Aufreinigung erfolgte durch Ultrafiltration mit 3.000 MWCO ähnlich wie in Beispiel 19.

Beispiele für die Kopplung von Aminogruppen an die Phosphatreste der Nukleotide sind in D. Jameson et al. Methods in Enzymology 1997, V. 278, 363 angegeben. An diese Amino-Gruppe kann eine Linker-Komponente gekoppelt werden. Beispiele für Kopplungen von Linkern an Phosphat-Gruppen von Nukleotiden sind in US Patent 5.981.507 dargestellt. An einen solchen Linker können weitere makromolekulare Linker gekoppelt werden, die einen niedermolekularen Marker oder eine niedermolekulare Kopplungseinheit oder einen markomolekularen Marker tragen.
In einer Ausführungform wird markomolekularer Linker an die Phosphat-Gruppen angefügt, die an der 5'-Position der Ribose gekoppelt sind. Die Kopplung erfolgt vorzugsweise an der Gamma-Phosphat-Gruppe der Nukleotide, wobei sowohl Ribonukleotide als auch 2' -Deoxyribonukleotide als auch 2',3'-Dideoxynukleotide eingesetzt werden können.
In einer anderen Ausführungsform erolgt die Kopplung des makromolekularen Linker an die 3'-Phosphat-Gruppe eines 5'-Nukleosid-Triphosphat. Synthese eines solchen Derivaten ist in WO 91/06678 dargestellt.

### Kopplungen an Marker-Komponenten

### Beispiel 25, :

### (dUTP-16-Biotin)4-SA,

Zu 200µl einer Lösung von Biotin-16-dUTP 200µmol/l in 50mM Tris-HCl, pH 8.0, wurden 200µl einer Streptavidin-Lösung, 1mg/ml, in 50mM Tris-HCl, pH 8.0, zugegeben. Nach 1 Stunde bei RT wurde wurde das (dUTP-16-Biotin)4-SA vom nicht umgesezten Biotin-16-dUTP durch Ultrafiltration, 50.000 MWCO, abgetrennt.

Es wurde eine Verbindung erhalten, die eine Nukleotid-Funktionalität und eine makromolekulare Marker-Funktionalitäten trägt.
Das Produkt der Reaktion ist definitionsgemäß ein konventionell modifiziertes Nukleotid: die Linker-Länge ist kleiner als 30 Atome, die Marker-Komponente ist makromolekular. Es kann stellvertretend für konventionell modifizierte Nukleotide mit einem makromolekularen Markern betrachtet werden.

Diese Verbindung wird von Polymerasen (z.B. Klenow-Exo minus Polymerase und Terminaler Transferase) als Substrat nicht akzeptiert. Die Modifikation führt zum Verlust der Substrateigenschaften, s. Beispiel 34B.
Eigenschaften der Biotin-Streptavidin-Bindung sind z.B.in Gonzalez et al. Journal Biolog. Chem. 1997, v. 272, S. 11288 beschrieben.

### Beispiel 26:

### (dUTP-16-Biotin)4-SA-Cy2,

Die Kopplung von dUTP-16-Biotin an SA-Cy2 wurde wie für (dUTP-16-Biotin)4-SA beschrieben durchgeführt.

Die Verbindung dient als Äquivalent zu Verbindung aus Beispiel 25, wobei zur Visualisierung Streptavidin fluoreszent markiert ist.

### Beispiel 27:

### dCTP-PA-SS-Oligo-dT30,

Die Synthese erfolgte wie bei dUTP-AA-SS-MEA-Cy3 beschrieben. Zu dCTP-PA-PDTP, 100µl, 20mmol/l, wurde Oligo-dT30-3' -SH (MWG-Biotech) zugegeben, Endkonzentration 200µmol/l, und 18 Std bei RT, pH 9, gerührt. Die Aufreinigung erfolgte durch Ultrafiltration mit 3.000 MWCO.

Das Produkt der Reaktion ist definitionsgemäß ein konventionell modifiziertes Nukleotid-Analog: die Linker-Länge ist kleiner als 30 Atome, die Marker-Komponente ist makromolekular.
Diese Verbindung wird von Polymerasen (z.B. Klenow-Exo minus Polymerase und Terminaler Transferase) als Substrat nicht akzeptiert. Die Modifikation des Nukleotidteils führt zu Aufhebung der Substrateigenschaften.

### Beispiel 28:

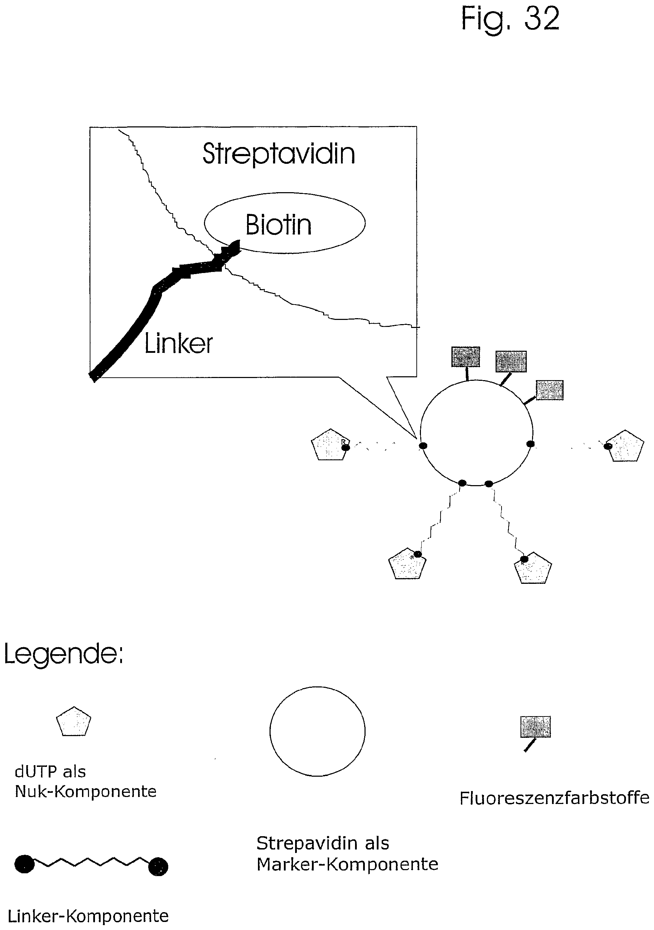

### (dUTP-AA-PEG-Biotin)4-SA-Cy2 und (dUTP-AA-PEG-Biotin)4-SA, Fig. 32

Die Kopplung von dUTP-AA-PEG-Biotin an SA-Cy2 bzw. an SA wurde wie für (dUTP-16-Biotin)4-SA beschrieben durchgeführt. Zu 200µl 1µg/µl Streptavidin wurden 10µl einer Lösung von dUTP-AA-PEG-Biotin, ca. 1mmol/l, gegeben, und bei RT 1 h gerührt. Danach wurde das Produkt durch Ultrafiltration, 50.000 MWCO, von dem nicht gekoppelten dUTP-AA-PEG-Biotin abgetrennt und das Produkt 2 mal mit Wasser gewaschen.

Ein Teil des gewonnenen (dUTP-AA-PEG-Biotin)4-SA-Cy2 wurde mit Cy3-NHS modifiziert: 50µl (dUTP-AA-PEG-Biotin)4-SA-Cy2 wurden in 50mmol/l Borat, pH 8.5, aufgelöst, bis zur Konzentration von 1.4µg/µl, und anschließend mit Cy3-NHS versetzt. Endkonzentration von Cy3 betrug 10mmol/l. Die Reaktion wurde 1 Std. bei RT durchgeführt. Das Produkt, (dUTP-AA-PEG-Biotin)4-SA-Cy2/Cy3,wurde durch Ultrafiltration mit 30.000 MWCO getrennt.
Dadurch wurde ein Nuk-Makromolekül hergestellt, dass sehr wenige freie Amino-Gruppen am Marker-Teil aufweist.

Es wurde eine Verbindung erhalten, die eine Nukleotid-Funktionalität, einen langen makromolekularen Linker und eine makromolekulare Marker-Komponente trägt.

Das Produkt der Reaktion ist definitionsgemäß ein Nuk-Makromolekül: die Linker-Länge ist deutlich größer als 30 Atome, die Marker-Komponente ist makromolekular. Es kann stellvertretend für Nuk-Makromoleküle betrachtet werden.

Diese Verbindung wird von Polymerasen (z.B. Klenow-Exo minus Polymerase und Terminaler Transferase) als Substrat akzeptiert s. Beispiel 34,35.

Auch andere Verbindungen, die einen langen Linker haben und ein Biotin-Molekül tragen, s.Beispiele 20, 22, 23, 24, können ähnlich in der Synthese eingesetzt werden.

### Beispiel 29:

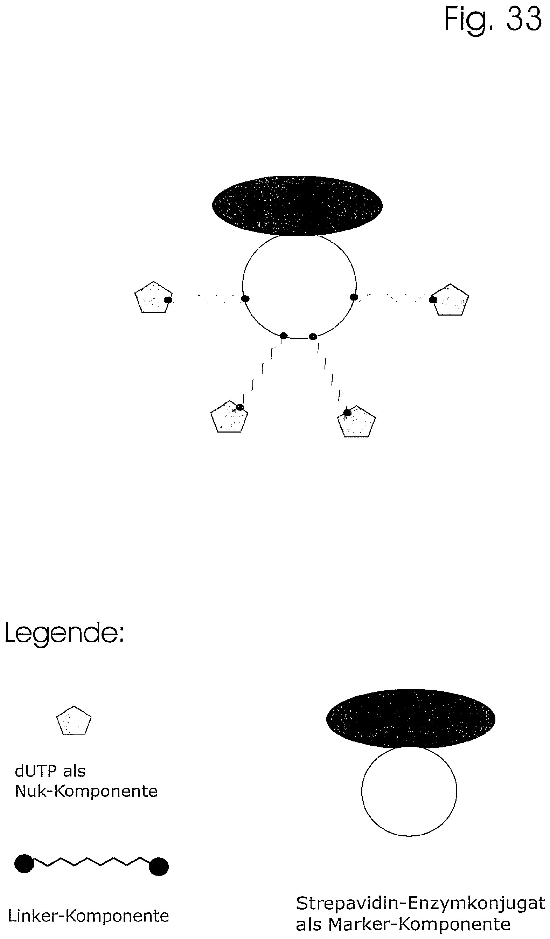

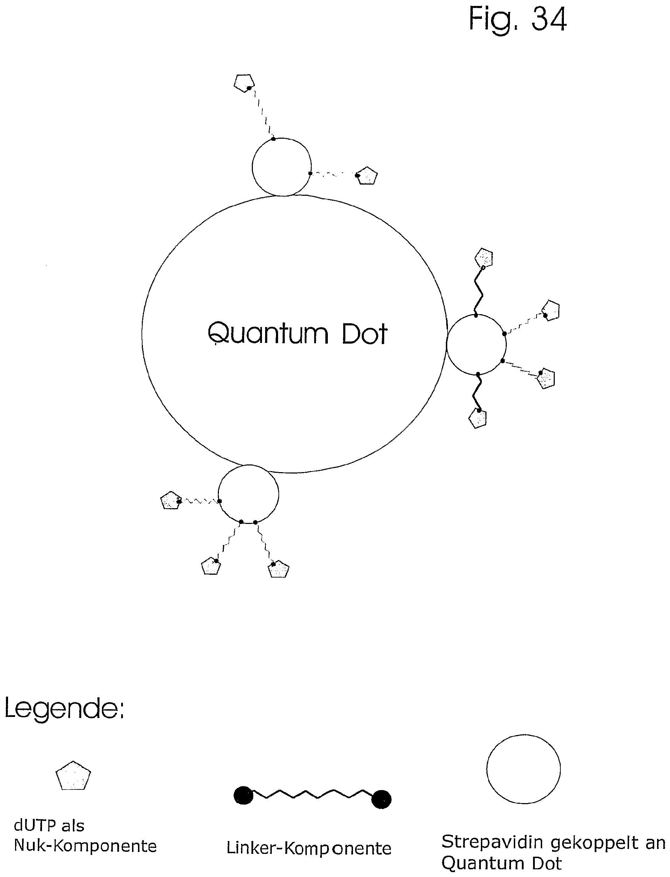

### (dUTP-AA-PEG-Biotin)4-SA-Alkalische Phosphatase, Fig.33, und (dUTP-AA-PEG-Biotin)4-SA-QDot, Fig. 34,

Die Kopplung von dUTP-AA-PEG-Biotin an SA-AP bzw. QDot wurde wie für (dUTP-16-Biotin)4-SA beschrieben durchgeführt.

Im Falle von QDot werden Nuk-Linker-Teile an der Oberfläche der QDots angeordnet. Es wurden Verbindung erhalten, die eine Nukleotid-Funktionalität, einen langen makromolekularen Linker und eine makromolekulare Marker-Komponente mit einem Enzym bzw. Q-Dots trägt.

Das Produkt der Reaktion ist definitionsgemäß ein Nuk-Makromolekül: die Linker-Länge ist deutlich größer als 30 Atome, die Marker-Komponente ist makromolekular. Diese Verbindung wird von Polymerasen (z.B. Klenow-Exo minus Polymerase und Terminaler Transferase) als Substrat akzeptiert.

Auch andere Verbindungen, die einen langen Linker haben und ein Biotin-Molekül tragen, s.Beispiele 20, 22, 23, 24, können ähnlich in der Synthese eingesetzt werden.

### Beispiel 30:

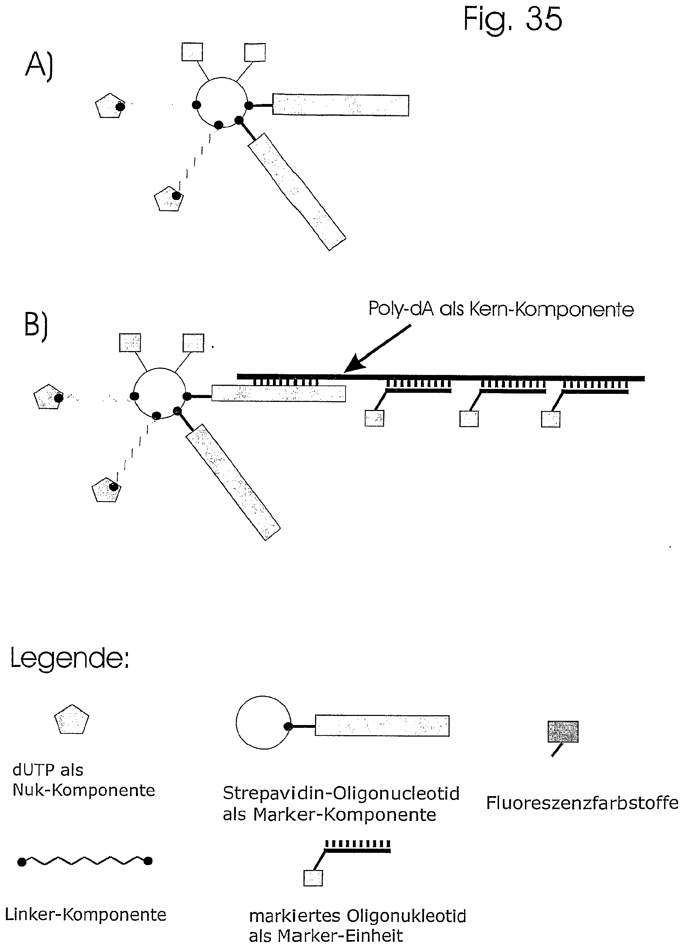

### (dUTP-AA-PEG-Biotin)2-(dT31-TEG-Biotin)2- SA-Cy2, , Fig. 35A

Die Kopplung von dUTP-AA-PEG-Biotin an SA-Cy2 wurde wie für (dUTP-16-Biotin)4-SA beschrieben durchgeführt:
Zu 100µl einer Streptavidin-Cy2-Lösung, 20µmol/l (1.2mg/ml), in Tris-HCl, 50mmol/l, pH 8, wurden 80µl 80µmol/l dT31-3' -TEG-Biotin (MWG Biotech) zugegeben und 10 min bei RT inkubiert. (TEG ist ein kurzer Linker zwischen Biotin und dT31). Danach wurden 100µl einer Lösung dUTP-AA-PEG-Biotin 50µmol/l in 50mM Tris-HCl, pH 8.0, zugegeben. Nach 10 min bei RT wurde (dUTP-AA-PEG-Biotin)2-(dT31-TEG-Biotin)2-SA-Cy2 durch Ultrafiltration, 50.000 MWCO, gereinigt.

Die Substanz trägt eine Nukleosid-Triphosphat-Funktionalität und eine makromolekulare Marker-Funktionalitäten (Oligo-dT31). Das Oligo-dT31 besteht aus Nukleosidmonophosphaten, die allerdings an der enzymatischen Reaktion nicht teilnehmen und nur eine signalvermittelnde Funktion haben. An ein solches Oligonukleotid können komplementäre Nukleinsäuren hybridisiert werden, die eine signalgebende Funktion haben (Fig. 35B). (allgemeine Regeln zur Hybridisierung von Nukleinsäuren sind dem Fachmann bekannt, Anderson "Nucleic Acid Hybridization", 1999).

Das Produkt der Reaktion ist definitionsgemäß ein Nuk-Makromolekül: die Linker-Länge ist deutlich größer als 30 Atome, die Marker-Komponente ist makromolekular. Diese Verbindung wird von Polymerasen (z.B. Klenow-Exo minus Polymerase und Terminaler Transferase) als Substrat akzeptiert.

Dieses Derivat kann beispielsweise an Poly-dA oder poly-A (z.B. mit durchschnittlicher Länge 260NT, Amersham Bioscience) durch Hybridisierung gekoppelt werden. Sowohl ein einziges als auch mehrere (dUTP-AA-PEG-Biotin)2-(dT31-Biotin)2- SA-Cy2 Moleküle können an ein Poly-dA-Molekül gekoppelt werden, s.auch Fig. 5. Das Verhältnis wird durch die Konzentrationsverhältnisse bestimmt. Auch andere Oligonukleotide, beispielsweise markiert mit Farbstoffen, können zusammen mit(dUTP-AA-PEG-Biotin)2-(dT31-Biotin)2- SA-Cy2 an denselben Strang der Poly-dA oder poly-A gekoppelt werden, wobei die Verhältnisse zwischen einzelnen Molekülen variabel sind. Dadurch ist es möglich, ein polyfunktionales Nuk-Makromolekül herzustellen. Der große Vorteil eines solchen Nuk-Makromoleküls besteht in einer leicht spaltbaren makromolekularen Markierung: die an die poly-dA bzw. poly-A Stränge hybridisierten markierten Oligonukleotide können durch Denaturierung abgelöst werden. Durch die Wahl der Länge der markierten Oligonukleotide, kann die Tm dieser Oligonukleotide für die jeweiligen Anforderungen der reversiblen Markierung angepasst werden. Die Regeln zur Tm-Berechnung sind dem Fachmann bekannt ("Molecular-Cloning", J. Sambrook, band 1-3, 2001). Beispielsweise können dT₂₅-Oligonukleotide, markeirt mit einem Cy3-Molekül an Poly-dA gekoppelt werden. Durch den Einsatz von RNA, z.B. poly-A, zur Bindung mehrer (dUTP-AA-PEG-Biotin)2-(dT31-Biotin)2-SA Moleküle, kann die Spaltung durch eine RNase erfolgen.

Da Streptavidin 4 Bindungsstellen für Biotin hat, entsteht ein Gemisch von Nuk-Makromolekülen, bei dem 4 Bindungsstellen unterschiedlich besetzt sind. Dieses Gemisch kann mit unterschiedlichen Mitteln getrennt werden. Eine der Möglichkeit besteht in Isolierung von Nuk-Makromolekülen, die mindestens ein Oligo-dT31 tragen, beispielsweise durch eine Absorbtion an einem Anionaustauscher (z.B. einer DEAE-Cellulose-Säule). Auf Gel-Elektrophorese eignet sich zur Trennung einzelner Derivaten.

Auch längere Nukleinsäureketten, die ein Biotin-Molekül tragen, beispielsweise poly-dA-Biotin, hergestellt beispielsweise durch eine terminale Kopplung von ddUTP-18-Biotin durch eine TdT-abhängige Reaktion ("Molecular-Cloning", J. Sambrook, Band 1-3, 2001), können in ähnlicher Weise an das Streptavidin gekoppelt werden, so dass Moleküle mit einer durchschnittlichen Zusammensetzung von (dUTP-AA-PEG-Biotin)_{N}-(Nukleinsäureketten-Biotin)_{M}-SA entstehen. Sowohl einzelsträngige als auch doppelsträngige Nukleinsäureketten können gekoppelt werden. Die Länge der gekoppelten Nukleinsäureketten kann zwischen 10 und 100, 100 und 1000 Nukleotiden liegen.

Die hybridisierten Oligonukleotide, die einen Farbstoff tragen, können an den Poly-dA Strang auch kovalent durch Cross-Linker gebunden werden.

Auch andere Verbindungen, die einen langen Linker haben und ein Biotin-Molekül tragen, s.Beispiele 20, 22, 23, 24, können ähnlich in der Synthese eingesetzt werden.

### Beispiel 31:

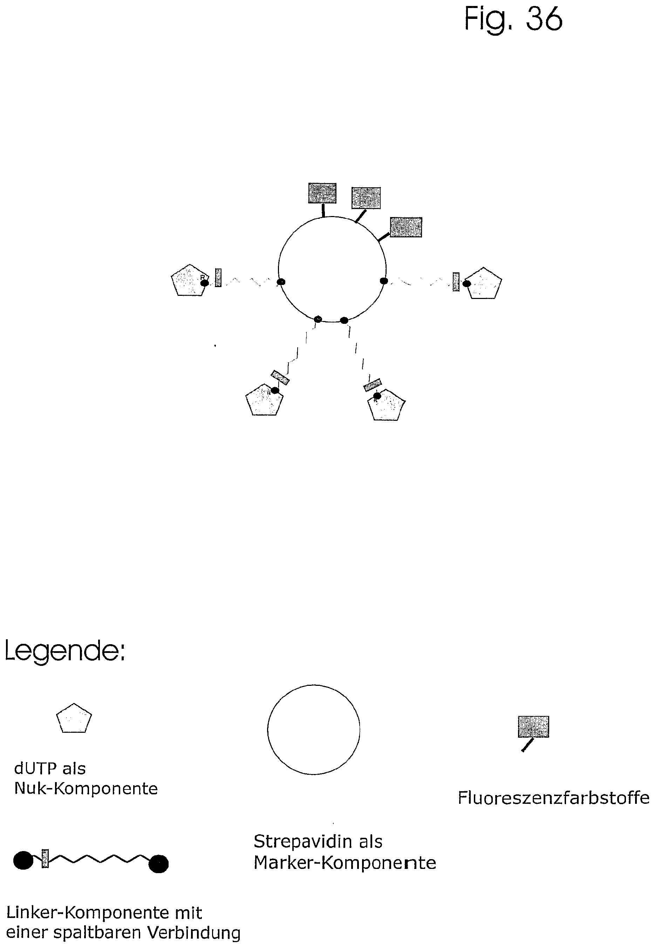

### (dUTP-AA-SS-PEG-Biotin)4-SA und (dUTP-AA-SS-PEG-Biotin)4-SA-Cy2, Fig. 36

Die Kopplung von dUTP-AA-SS-PEG-Biotin an SA bzw. an SA-Cy2 wurde wie für (dUTP-AA-PEG-Biotin)4-SA beschrieben durchgeführt. Als Edukte wurden Streptavidin und dUTP-AA-SS-PEG-Biotin eingesetzt.

Es wurde eine Verbindung erhalten, die eine Nukleotid-Funktionalität, einen langen makromolekularen Linker und eine makromolekulare Marker-Komponente trägt. Die Linker-Komponente und die Marker-Komponente können von der Nuk-Komponente unter milden Bedingungen abgespalten werden.

Das Produkt der Reaktion ist definitionsgemäß ein Nuk-Makromolekül: die Linker-Länge ist deutlich größer als 30 Atome, die Marker-Komponente ist makromolekular. Es kann stellvertretend für Nuk-Makromoleküle betrachtet werden, die eine unter milden Bedingungen spaltbare Verbindung in der Linker-Komponente tragen.

Diese Verbindung wird von Polymerasen (z.B. Klenow-Exo minus Polymerase und Terminaler Transferase) als Substrat akzeptiert s. Beispiel 34.

### Beispiel 32:

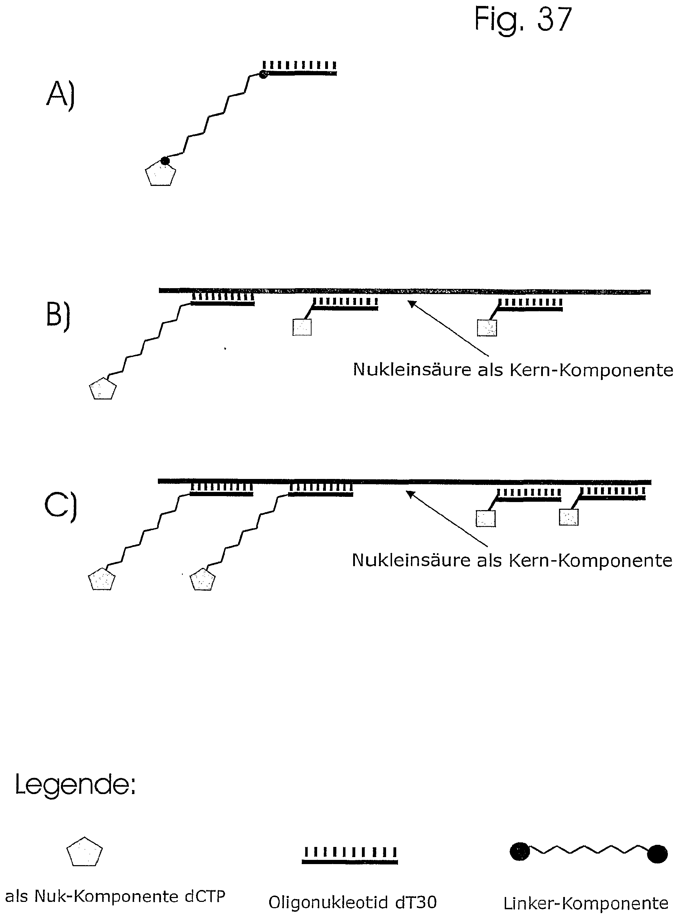

### dCTP-PA-PEG-Maleimid-S-Oligo-dT30, Fig. 37A

Zu 100µl einer Lösung mit dCTP-PA-PEG-Maleimid, 5mmol/l, in 50mM Borat-Puffer, pH 9.5, wurde 100µl einer Lösung von 3'-SH-Oligo-dT30, 200µmol/l, in Wasser zugegeben und 48 Std. bei RT gerührt. Das Produkt wurde mittels präparativer Gel-Elektrophorese, 12% Polyacrylamid-Gel, gereinigt.

Die Substanz trägt eine Nukleosid-Triphosphat-Funktionalität und eine makromolekulare Marker-Funktionalitäten (Oligo-dT30). Das Oligo-dT30 besteht aus Nukleotiden, die allerdings an der enzymatischen Reaktion nicht teilnehmen und nur eine signalvermittelnde Funktion haben. An ein solches Oligonukleotid können komplementäre Nukleinsäuren hybridisiert werden, die eine signalgebende Funktion haben (Fig. 37B). (allgemeine Regeln zur Hybridisierung von Nukleinsäuren sind dem Fachmann bekannt, Anderson "Nucleic Acid Hybridization", 1999.)

Das Nukleotid ist definitionsgemäß ein Nuk-Makromolekül: die Linker-Länge ist deutlich größer als 30 Atome, die Marker-Komponente ist makromolekular.
Diese Verbindung wird von Polymerasen (z.B. Klenow-Exo minus Polymerase und Terminaler Transferase) als Substrat akzeptiert.

Dieses Beispiel zeigt eine generelle Möglichkeit, an Nukleotiden weitere Modifikationen vorzunehnem. Weitere basenmodifzierte Nukleotidanaloga, wie z.B. 5-AllylaminodUTP, 5-Amino-Propargyl-dUTP, 7-Deaza-Aminopropargyl-dGTP und 7-Deaza-Aminopropargyl-dATP können wie oben angeführt ebenfalls modifiziert werden. Es können sowohl Ribonukleotide, 2' -Deoxyribonukleotide als auch 2' ,3' - Deoxyribonukletide verwendet werden, Fig. 11 bis 14.

Durch Zugabe von Poly-dA oder Poly-A können mehrere dCTP-PA-PEG-Maleimid-S-Oligo-dT30, z.B. 10-20, zu einem Nuk-Makromolekül gekoppelt werden. Dabei entsteht ein Nuk-Makromolekül mit linear angeordneten Nuk-Linker-Komponenten (Fig. 37 C).

### Beispiel 33 :

### (dCTP-PA-PEG-Maleimid-S)n-Poly-Lysin-(Cy3)m,

### Edukte: dCTP-PA-PEG-Maleimid

(HS-Propionat)m-Poly-Lysin-(Cy3)n, n = 10-15, m 3-9, Poly-Lysin 10.000-20.000 Zu 100µl einer Lösung mit dCTP-PA-PEG-Maleimid, 5mmol/l, in 50mM Borat-Puffer, pH 9.5, wurde 20µl einer Lösung von (HS-Propionat)m-Poly-Lysin-(Cy3)n, ca. 1mmol/l, in Wasser, gegeben und 18 Std. bei 40°C gerührt. Das Produkt wurde durch Ultrafiltration, 30.000 MWCO, gereinigt.

Es wurde eine Verbindung erhalten, die eine Nukleotid-Funktionalität, einen langen makromolekularen Linker und eine makromolekulare Marker-Komponente trägt. Pro Nuk-Makromolekül sind mehrere Nuk-Komponenten gekoppelt. Das Produkt der Reaktion ist definitionsgemäß ein Nuk-Makromolekül: die Linker-Länge deutlich größer als 30 Atome, die Marker-Komponente ist makromolekular.
Diese Verbindung wird von Polymerasen (z.B. Klenow-Exo minus Polymerase und Terminaler Transferase) als Substrat akzeptiert.

Weitere Kombinationen von Nuk-Komponenten, Linker-Komponenten und Marker-Komponenten sind dem Fachmann naheliegend.

**Vergleich von Substrateigenschaften einiger Vertreter von Nuk-Makromolekülen mit konventionell modifizierten Nukleotiden.**

Substrateigenschaften der Nuk-Makromoleküle gegenüber Polymerasen und Terminale deoxy-Nucleotidyl-Transferase (TdT) wurden mit den Eigenschaften der konventionell modifizierten Nukleotide in einer Markierungsreaktion verglichen. Allgemeine Prinzipien von Markierungsreaktionen findet man in "Molecular-Cloning", J. Sambrook, Band 1-3, 2001, ISBN 0-87969-576-5.

### Beispiel 34: Substrateigenschaften von Nuk-Makromolekülen oder konventionell modifizierten Nukleotiden gegenüber Polymerasen

Dieses Beispiel soll nicht zur Einschränkung der möglichen Markierungsreaktionen dienen, sondern lediglich Unterschiede in den Substrateigenschaften verdeutlichen.

In den Reaktionen wurden sowohl selbst synthetisierte als auch kommerziell erhältliche modifizierte Nukleotide dUTP-Cy3 (Amersham) und dUTP-16-Biotin (Roche) verwendet. Nicht modifizierte dNTP (dATP, dGTP, dTTP, dCTP) wurden bei der Firma Roth erworben.

Als Matrizen dienten sowohl kurze Oligonucleotide als auch poly-dA. Primer und Oligonukleotide wurden von MWG-Biotech synthetisiert.

Reaktionen wurden in 20mmol/l Tris-Puffer, pH 8.5, 5mmol/l MgCl₂, 10% Glycerin, durchgeführt. Die Konzentrationen der Primer betrugen 1µmol/l, die der Oligonukleotide 1µmol/l und die Konzentration der poly-dA, 0.1µg/µl (für die Konzentrationsverhältnisse an der festen Phase s.u.). Als Polymerase wurde Klenow Exo minus 1Unit/100µl (Amersham) verwendet. Die Konzentrationen von Nukleotiden betrugen 20µmol/l für konventionell modifizierte Nukleotide und 5 µmol/l für Nuk-Makromoleküle. Nicht modifizierte Nukleotide wurden in Konzentrationen von 50µmol/l eingesetzt.

Zunächst wurden Primer an die jeweilige Matrize hybridisiert: Das Reaktionsgemisch ohne Polymerase wurde auf 75°C erhitzt und über 5 min auf 37°C abgekühlt. Danach wurde die Polymerase zugegeben. Alle Reaktionen wurden über 1 h bei 37°C durchgeführt. Die Reaktionen wurden durch Zugabe von EDTA (Endkonzentration von 10mmol/l) gestoppt.

Zu einigen Ansätzen wurde nach dem Stop der Reaktion Streptavidin, bis zu Endkonzentration von 1mg/ml, zugegeben und der Ansatz weitere 10 min bei 37°C inkubiert. Dadurch können bereits eingebaute Nukleotide, die ein Biotin tragen, mit Streptavidin reagieren und somit Streptavidin und Oligonukleotid verbinden. Diese Experimente eignen sich als Kontrolle für die Laufeigenschaften von modifizierten Primern.

Zu entsprechend gekennzeichneten Ansätzen wurde Mercaptoethanol (bis 20mmol/l Endkonzentration) zugegeben und der jeweilige Ansatz wurde 10 min bei 37°C inkubiert. Mercaptoethanol wurde bei einigen Anstzen während, bei anderen Ansätzen auch nach der Reaktion zugegeben.

Die Analyse der Reaktion erfolgte mittels denaturierender Gel-Elektrophorese, 20% Polyacrylamid-Gel, 50mmol/l Tris-HCl, pH 8.7, wie in "Gel electrophoresis of nucleic Acids", Ed. D. Rickwood, 1990, dargestellt. Zur Denaturierung der Proben wurde anstatt von 7M Urea eine erhöhte Temperatur bei der Gelelektrophorese eingesetzt (60°C). Die Elektrophorese wurde in Biorad-Gelkammern (Protean 3) durchgeführt, 200V, ca. 1h. Die Visualisierung erfolgte auf einer UV-Vis Gel-Dokumentationsanlage (Biorad).

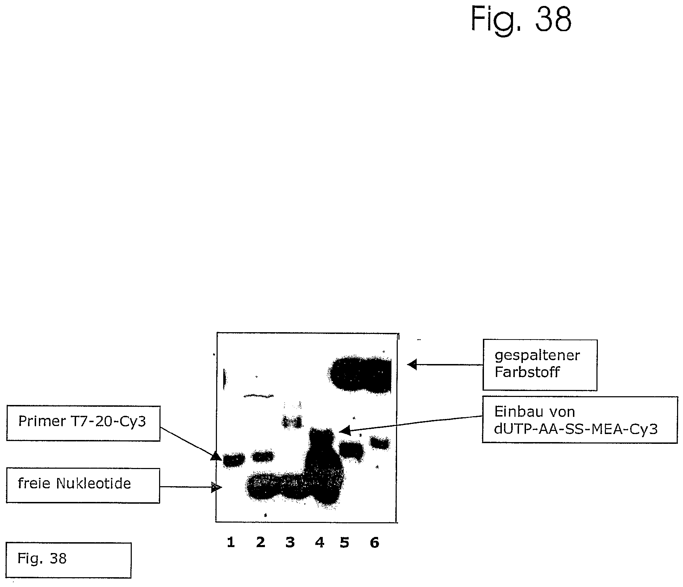

### Beispiel 34A, Fig. 38

### Darstellung des Einbaus und Spaltung von einem konventionell modifizierten Nukleotid (dUTP-AA-SS-MEA-Cy3)

### Sequenzen:

### Primer:

Primer-T7-20-5' -Cy3: 5'- Cy3-TAATACGACTCACTATAGGG-3'

### Matrize:

### Oligonukleotid

Oligo 1: 5' -AGTTTTAGTTTTACCCTATAGTGAGTCGTATTA-3'
Die Primer-Bindungsstelle ist unterstrichen

### Legende zu Figur 38:

### Spuren 1-6:

1) nur PrimerT7-20-Cy3 + Oligo 1
2) PrimerT7-20-Cy3 + Oligo 1+ dCTP-Cy3 + dATP + dGTP+ Polymerase
3) PrimerT7-20-Cy3 + Oligo 1+ dCTP-Cy3 + dATP + dGTP + dTTP+ Polymerase
4) PrimerT7-20-Cy3 + Oligo 1+ dUTP-AA-SS-MEA-Cy3 + Polymerase
5) nach 1 Std. wurde zu einem Aliquot von Ansatz 4 Mercaptoethanol zugegeben und weitere 10 min inkubiert, es erfolgt eine Abspaltung der Markierung
6) nach 10 min wurde zum Aliquot von Ansatz 5 dATP, dGTP zugegeben und 30 min bei 37°C inkubiert

Wie man sieht, wird dUTP-AA-SS-MEA-Cy3 von der Polymerase eingebaut (Spur 4). Der Farbstoff kann vom Primer abgespalten werden (Spur 5) (man sieht eine Verschiebung der Bande, die durch die geringere Größe von Oigonukleotid zu begründen ist). Anschließend können weitere Nukleotide eingebaut werden (Spur 6).

Ein ähnlich durchgeführter Reaktionsansatz mit dUTP-AA-SS-TEAE-(Cy3)2 führte nicht zum Einbau des Nukleotid-Analogs in den Primer.

Dieses Beispiel zeigt, dass sogar geringfügige Veränderungen in der Analog-Struktur, wie z.B. Verdopplung der Zahl der Farbstoffe, die an ein Nukleotid gekoppelt sind, zu Veränderung in Substrateigenschaften der Nukleotide führen können.

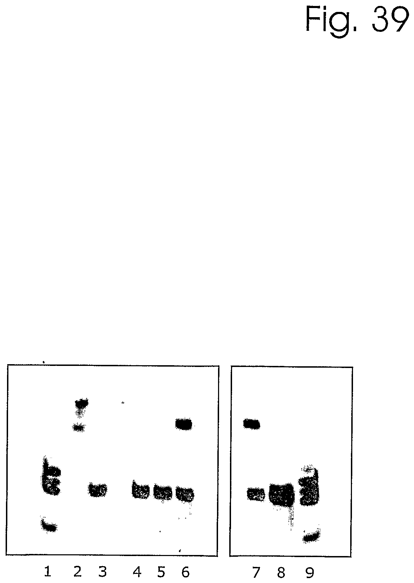

### Beispiel 34B, Fig. 39

### Vergleich von Substrateigenschaften eines konventionell modifizierten Nukleotid mit einem makromolekularen Marker und einem Nuk-Makromolekül

### Sequenzen:

### Primer:

Primer-dT₃₅-5'-Cy3 (dT35-Cy3): 5'Cy3-TTTTTTTTTTTTTTTTTTTTTTTTTTTTTTTTTTT-3'

### Matrize:

Poly-dA (Amersham), durchschnittlich 270 Nukleotide lang.
Nukleotide: (dUTP-AA-SS-PEG-Biotin)4-SA, (dUTP-16-Biotin)4-SA, dUTP-16-Biotin

### Legende zu Figur 39:

### Spuren 1-9:

1) Leiter: T-7-20-Cy3, dT35-Cy3, dT40-Cy3, dT50-Cy3
2) (dUTP-AA-SS-PEG-Biotin)4-SA + dT35-Cy3 + Poly-dA+ Polymerase
3) (dUTP-AA-SS-PEG-Biotin)4-SA + dT35-Cy3 + Poly-dA
4) (dUTP-16-Biotin)4-SA + dT35-Cy3 + Poly-dA+ Polymerase
5) (dUTP-16-Biotin)4-SA + dT35-Cy3 + Poly-dA
6) (dUTP-16-Biotin)4-SA-Cy3 + dT35-Cy3 + Poly-dA

### Im Kontroll-Ansatz, Spuren 7-9:

7) dUTP-16-Biotin + dT35-Cy3 + Poly-dA + Polymerase, Inkubation 1 std. 37°C danach + EDTA bis 10mmol/l Endkonzeentration, danach + Streptavidin 10 min 37°C
8) dUTP-16-Biotin + dT35-Cy3 + Poly-dA+ Polymerase, Inkubation 1 std. 37°C danach + EDTA bis 10mmol/l Endkonzeentration,
9) Leiter: T-7-20-Cy3, dT35-Cy3, dT40-Cy3, dT50-Cy3

Ein Nuk-Makromolekül, (dUTP-AA-SS-PEG-Biotin)4-SA, wird in den Primer eingebaut (Spur 2). Der markierte Primer hat nach dem Einbau eines Nuk-Makromoleküls eine stark veränderte elektrophoretische Beweglichkeit. Bloße Anwesenheit von Nuk-Makromolekülen hat keinen Einfluß auf den Primer (Spur 3).

Ein konventionell modifiziertes Nukleotid, (dUTP-16-Biotin)4-SA, mit einem makromolekularen Marker wird nicht in den Primer eingebaut (Spur 4). Trotz der Anwesenheit der Polymerase in Reaktion 4 (Spur 4) lassen sich keine Unterschiede zwischen Spur 4 und Spur 5 feststellen.

Spur 6 zeigt die Position des konventionell modifizierten Nukleotids mit einem makromolekularen Marker, (dUTP-16-Biotin)4-SA-Cy3, die obere Bande, und die Position des markierten Primers (die untere Bande).

Spur 7 zeigt das Ergebnis des Einbaus von dUTP-16-Biotin mit einer nachfolgenden Reaktion mit dem Streptavidin: die mit Biotin makrierten Primer reagieren mit Streptavidin und verändern ihre Laufeigenschaften. Die nicht modifizierten Primer behalten ihre elektrophoretischen Eigenschaften bei.

Spur 8 zeigt das Ergebnis der Einbaureaktion eines konventionell modifizierten Nukleotides, dUTP-16-Biotin. Man sieht eine verbreitete Primer-Bande, die durch den Einbau von dUTP-16-Biotin in den Primer zustande kommt. Die Verlängerung von Primer ist eingeschränkt, da dUTP-16-Biotin nicht unbegrenzt nacheinander eingebaut werden kann, durchschnittlich wird ca. 3 dUTP-Analoga eingebaut, so dass die Länge von Primer durchschnittlich auf 38 NT ansteigt. Erwartungsgemäß führt der Einbau von konventionell modifizierten Nukleotiden mit einem niedermolekularen Marker nicht zu einer starken Veränderung in der elektrophoretischen Mobilität der Primer.

In diesem Experiment wurden Eigenschaften der Nuk-Makromoleküle, (dUTP-AA-SS-PEG-Biotin)4-SA, mit denen der Konventionell modifizierten Nukleotide verglichen. Man sieht deutlich, dass die Kopplung eines makromolekularen Markers an ein kommerziell erworbenen dUTP-16-Biotin zum vollständigen Verlust der Substrateigenschaften der Nukleotide führt. Spuren 7 und 8 zeigen allerdings, dass die Polymerase sehr wohl in der Lage ist, dUTP-16-Biotin ohne makromolekularen Marker in den Primer einzubauen. Die Koppung von Streptavidin an das Biotin nach der Einbaureaktion führt zu genannten Veränderungen in Primer-Eigenaschaften.

Im Gegensatz dazu können Nuk-Makromoleküle (dUTP-AA-SS-PEG-Biotin)4-SA in den Primer ohne Schwierigkeiten durch die Polymerasen eingebaut werden. Das Auftreten von mehreren Banden im Gel führen die Erfinder auf einen mehrfachen Einbau von Nuk-Makromolekülen in den Primer (3 Banden) zurück.

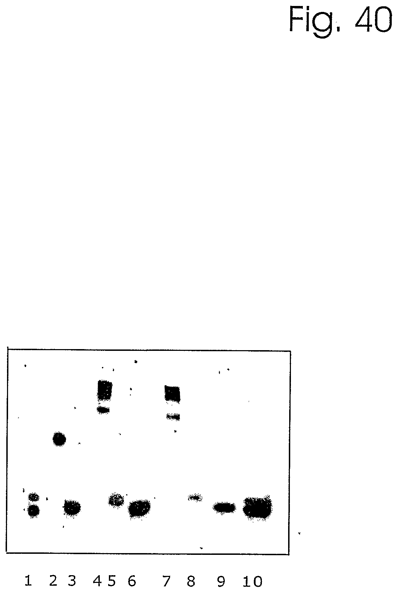

### Beispiel 34C, Fig. 40:

### Vergleich von Substrateigenschaften von Nuk-Makromolekülen, Einbau-Reaktion in der Lösung und an einer festen Phase

### Sequenzen:

### Primer: (dT35-Cy3)

Primer-dT-35-5'-Cy3: 5'Cy3-TTTTTTTTTTTTTTTTTTTTTTTTTTTTTTTTTTT-3'

### Matrize:

Poly-dA (Amersham), durchschnittlich 270 Nukleotide lang.
Oligo-dA50-3' -TEG-Biotin (MWG-Biotech)
Nukleotide: (dUTP-AA-SS-PEG-Biotin)4-SA, (dU-AA-PEG-Biotin)4-SA
Streptavidin-polystyre Particle, 2.17µ, Spherotech Inc,

### Vorbereitung von Streptavidin-polystyre Particle (feste Phase).

Drei Ansätze wurden in gleicher Weise vorbereitet.

Je 0.5 ml der Lösung mit Kügelchen (im Hersteller-Puffer) wurde kurz zentrifugiert und Kügelchen-Pellet wurde in 100µl Einbaupuffer (20 mmol/l Tris, pH 8.5, 5mmol/l MgCl2) resuspendiert. Anschließend wurden 100µl Oligo-dA50-3'-TEG-Biotin Konz. 50µmol/l zugegeben und 1 h bei RT gerührt. In dieser Zeit binden die Oligo-dA-Moleküle an die Kügelchen. Anschließend wurden Kügelchen kurz abzentrifugiert und drei mal im Einbaupuffer gewaschen. Endvolumen der festen Phase betrugt je 100µl.

Diese Menge an Oligo-dA50-feste-Phase kann Primer-dT-35-Cy3 in 2µmol/l hybridisieren.

Die Hybridisierung von Primer-dT-35-Cy3 erfolgte bei 40°C 10 min, mit anschließender Abkühlung auf RT innerhalb von 10 min. Alle anderen Schritte wurden gleich bei allen Ansätzen durchgeführt.

### Legende zu Figur 40:

### Spuren 1-10:

1) Leiter: dT35-Cy3, dT40-Cy3,

### Reaktionen in der Flüssigphase:

2) (dUTP-AA-PEG-Biotin)4-SA + dT35-Cy3 + Poly-dA+ Polymerase
3) (dUTP-AA-PEG-Biotin)4-SA + dT35-Cy3 + Poly-dA
4) (dUTP-AA-SS-PEG-Biotin)4-SA + dT35-Cy3 + Poly-dA+ Polymerase, Inkubation 1 h bei 37°C, dann + EDTA
5) (dUTP-AA-SS-PEG-Biotin)4-SA + dT35-Cy3 + Poly-dA+ Polymerase, Inkubation 1 h bei 37°C, dann + EDTA, anschließend + Mercaptoethanol bis zu 200mmol/l (Endkonzentration), für 30 min.
6) (dUTP-AA-SS-PEG-Biotin)4-SA + dT35-Cy3 + Poly-dA + EDTA

### Reaktionen an der Festphase:

7) (dUTP-AA-SS-PEG-Biotin)4-SA + dT35-Cy3 + Oligo-dA50-feste-Phase+ Polymerase, Inkubation 1 h bei 37°C, dann + EDTA
8) (dUTP-AA-SS-PEG-Biotin)4-SA + dT35-Cy3 + Oligo-dA50-feste-Phase + Polymerase, Inkubation 1 h bei 37°C, dann + EDTA, anschließend + Mercaptoethanol bis zu 200mmol/l (Endkonzentration), für 30 min.
9) (dUTP-AA-SS-PEG-Biotin)4-SA + dT35-Cy3 + Oligo-dA50-feste-Phase, vor Elektrophorese + EDTA
10) Leiter: dT35-Cy3, dT40-Cy3,

Man sieht deutlich das Ergebnis der Einbaureaktion von Nuk-Makromolekülen in Spuren 2,4,5,7,8. Sowohl in der Lösung, als auch an der festen Phase läuft die enzymatische Markierungsreaktion gut ab.

Nach Zugabe von Mercaptoethanol zu mit EDTA gestoppten Reaktionen erfolgt die Abspaltung von Linker-Komponenten mit dem gebundenen Streptavidin von den Primern. Dies fühhrt zu Wiederherstellung der elektrophoretischen Eigenschaften der Primer. Die Verschiebung der Primer-banden in Spuren 5 und 8 ist durch multiplen Einbau von Nuk-Makromolekülen in die Primer zu begründen. Tatsächlich, liegen die Primer-Banden nach der Spaltung in Höhe von dT40-Cy3 (s. Leiter). Dies bedeutet, dass bis zu 5 Nuk-Makromolekülen während der Reaktion in die Primer eingebaut wurden.

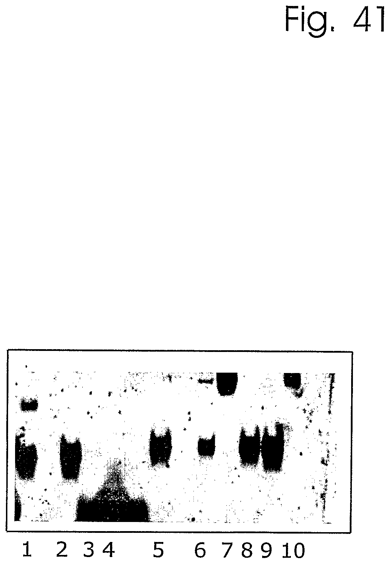

### Beispiel 35, Fig. 41:

### Substrateingenschaften der Nuk-Makromoleküle und konventionell modifizierter Nukleotide gegenüber Terminaler Transferase (TdT).

Die Reaktion wurde nach Angaben des Kit-Herstellers (Roche) durchgeführt: Auf jeweils 50µl Volumen wurden zugegeben: 10µl 5x Reaktionspuffer, 1µl TdT (25Units), 5µl 25mmol/l CoCl₂. Die Primer- und Nukleotid-Konzentrationen waren wie bei den Polymerase-Reaktionen. Die Reaktion wurde für 2 h bei 37°C durchgeführt.
Primer: Primer-dT₃₅-5' -Cy3 (dT35-Cy3), Primer-dT₃₅ (dT35)

Legende zu Figur 41:
1) (dUTP-AA-PEG-Biotin)4-SA + dT35-Cy3 + TdT
2) (dUTP-AA-PEG-Biotin)4-SA + dT35-Cy3
3) dUTP-Cy3 (Amersham)+ dT35 + TdT
4) dUTP-Cy3 (Amersham)+ dT35
5) dUTP-16-Biotin (Roche) + dT35-Cy3 + TdT;
6) Ansatz 5; nach dem Stop + Streptavidin
7) Streptavidin -Cy2
8) (dUTP-16-Biotin)4-SA + dT35-Cy3 + TdT
9) (dUTP-16-Biotin)4-SA + dT35-Cy3
10) (dUTP-16-Biotin)4-SA-Cy2

In der Spur 1 sieht man deutlich 2 Banden, die Bande in der Mitte entspricht dem dT35-Cy3, die obere Bande entspricht dem Reaktionsprodukt: Nuk-Makromolekül wurde in dT35 durch TdT eingebaut. Spur 2 dient als Negativkontrolle. In Spur 3 sieht man das Ergebnis der Markierung des dT35 mit dem konventionellen Nukleotid dUTP-Cy3, Spur 4 ist die Nagativkontrolle. In Spur 5 ist das Ergebnis der Kopplung von dUTP-16-Biotin an die dT35-Cy3 schlecht zu erkennen. In Spur 6 sieht man allerdings eine schache Bande im oberen Bereich, die dem Ergebnis der Reaktion des mit dUTP-16-Biotin modifizierten Primers mit Streptavidin entspricht. Spur 7 zeigt die Position des modifizierten Streptavidins. In Spur 8 und 9 sieht man nur eine Bande in der Mitte des Gels, die dem dT35-Cy3 entspricht, im oberen Gel-bereich ist keine Bande zu sehen, was eindeutig darauf deutet, dass konventionell modifizierten Nukleotide mit einem makromolekularen Marker von TdT nicht eingebaut werden. Spur 10 zeigt die Position des (dUTP-16-Biotin)4-SA-Cy2 im Gel.

### Beispiel 36:

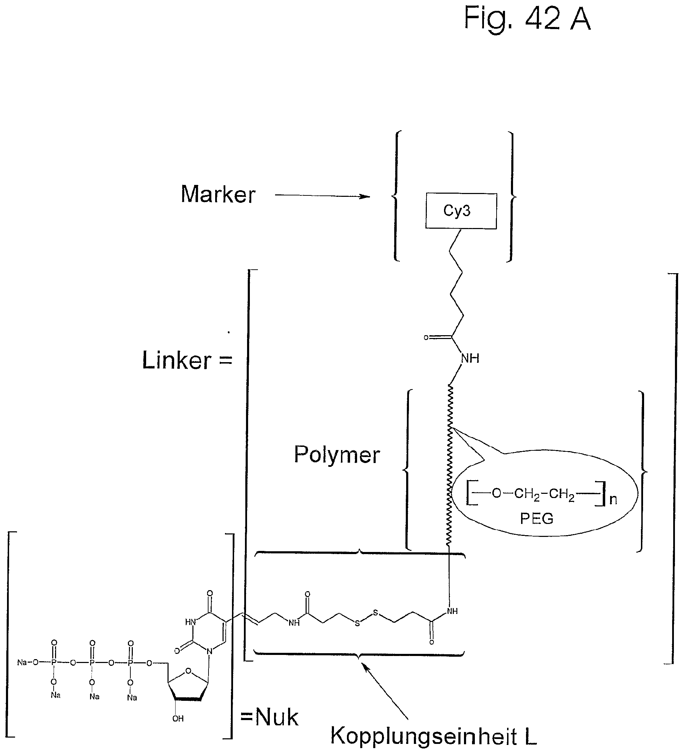

### dUTP-AA-SS-PEG-Cy3, Fig. 42 A

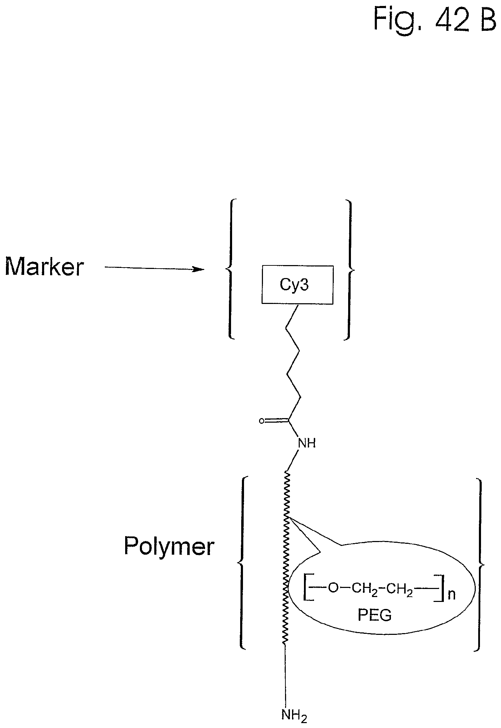

Zunächst wurde SH-PEG-Cy3 hergestellt. Zu 200 µl einer 10 mmol/l Lösung von Diamin-PEG (6kDa, Fluka) in 50 mmol/l Borat-Puffer, pH 8, wurde Cy3-NHS (Amersham-Bioscience) bis zu Endkonzentration von 15 mmol/l zugegeben. Die Reaktion wurde bei RT 30 min lang durchgeführt. Anschließend wurde NH₂-PEG-Cy3 (Fig. 42 B) von Farbstoffresten durch Ultrafiltration mit MWCO 3000 abgetrennt, 3 mal mit 1 ml 50 mmol/l Borat-Puffer, pH 8, gewaschen und in einem Volumen von 200 µl 50 mmol/l Borat-Puffer, pH 8, aufgelöst.
Zu dieser Lösung wurde PDTP-NHS bis zu Endkonzentration von 50 mmol/l zugegeben. Die Reaktion wurde bei RT 30 min lang durchgeführt. Anschließend wurden 50 µl einer 1 mol/l Lösung von NH₄HCO₃, pH 8, zugegeben und das Reaktionsgemisch wurde weitere 60 min inkubiert. Zur Reduktion der Disulfidbindungen wurden 100 µl einer 1 mol/l TCEP-Lösung, pH 8, zum Reaktionsgemisch zugegeben. Nach 5 min bei RT wurde das Produkt der Reaktion, das SH-PEG-Cy3, von den niedermolekularen Komponenten durch Ultrafiltration mit MWCO 3000 abgetrennt, 5 mal mit 1 ml 50 mmol/l Tris-HCl-Puffer, pH 7, gewaschen und in einem Volumen von 200 µl 50 mmol/l Tris-HCl, pH 7, aufgelöst. Unmittelbar vor Kopplung des Nukleotid-Teils wurde der pH-Wert auf 9.0 mit 1 mol/l NaOH eingestellt.

### Kopplung des SH-PEG-Cy3 an den Nukleotid-Teil:

Zu 100µl 20 mmol/l dUTP-AA-PDTP in 50 mmol/l Borat-Puffer, pH 9, wurden 100 µl der Lösung mit SH-PEG-Cy3 in 50 mmol/l Tris-HCl, pH 9.0, zugegeben. Reaktion wurde bei RT über 30 min durchgeführt. Das Produkt der Reaktion wurde durch Ultrafiltration mit MWCO 3000 von niedermolekularen Komponenten abgetrennt, 5 mal mit 1 ml 50 mmol/l Tris-HCl-Puffer, pH 7, gewaschen und in einem Volumen von 200 µl 50 mmol/l Tris-HCl, pH 7, aufgelöst.

Das auf diese Weise erhaltene dUTP-AA-SS-PEG-Cy3 kann von DNA-Polymerasen, beispielsweise Klenow-Fragment Exo-minus oder Taq-Polymerase, in den wachsenden Strang der Nukleinsäuren eingebaut werden.

Das dUTP-AA-SS-PEG-Cy3 enthält eine unter milden Bedingungen spaltbare Gruppe, so dass der Linker mit dem Farbstoff von dem Nukleotid abgespalten werden kann. Dies ist beispielsweise in Verfahren der Sequenzierung durch die Synthese vom besonderen Interesse (Balasubramanian WO 03048387, Tcherkassov WO 02088382, Quake WO0132930, Kartalov WO02072892).

Dieses Beispiel zeigt eine generelle Möglichkeit, an Nukleotiden weitere Modifikationen vorzunehnem. Weitere basenmodifzierte Nukleotidanaloga, wie z.B. 5-PropargylaminodCTP, 5-Propargylamino-dUTP, 7-Deaza-Aminopropargyl-dGTP und 7-Deaza-Aminopropargyl-dATP können wie oben angeführt ebenfalls modifiziert werden. Es können sowohl Ribonukleotide, 2' -Deoxyribonukleotide als auch 2' ,3' - Deoxyribonukletide verwendet werden, Fig. 11 bis 14.

### Beispiel 37:

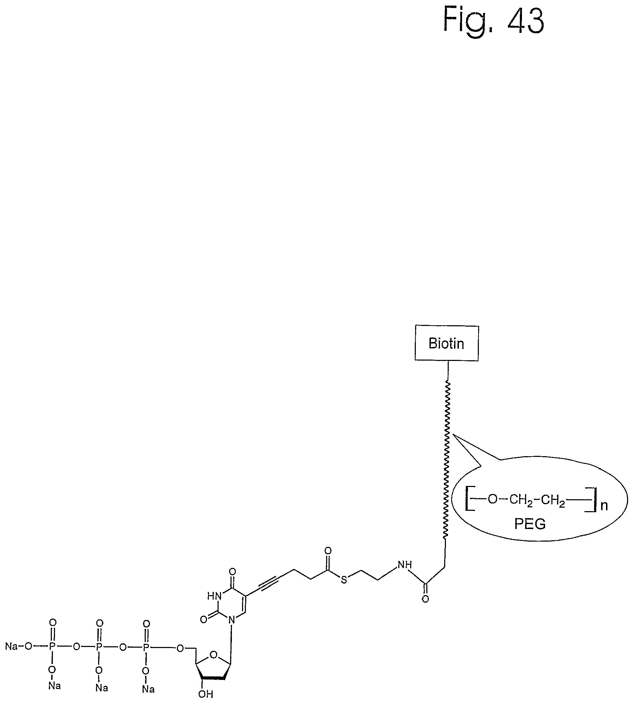

### dUTP-R-CO-S-PEG-Biotin Fig. 43

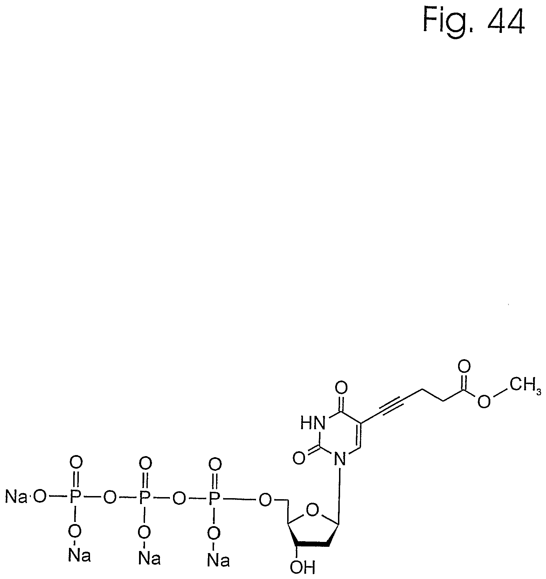

Das dUTP-R-COOCH₃ (Fig. 44) wurde analog zur Vorschrift (Heike A. Held, Abhijit Roychowdhury, and Steven A. Benner , Nucleosides, Nucleotides & Nucleic Acids, Vol 22,391-404 (2003)) synthetisiert. Die Triphosphatsynthese erfolgte nach T. Kovacs, L. Ötvös, Tetrahedron Letters, Vol 29, 4525-4588 (1988)).
500 mg (1.41 mmol) 5-Iod-2'-Deoxyuridin wurden bei Raumtemperatur in 10 ml wasserfreiem DMF unter Stickstoffatmosphäre suspendiert und 10 min. gerührt. Nun gibt man 160 mg (0.14 mmol) Tetrakis(triphenylphosphin)-palladium(0) zu. Nach weiteren 10 min gibt man nacheinander 400 ul Triethylamin, 480 mg (4.28 mmol) Pent-1-insäuremethylester und 55 mg (0.29 mmol) Kupfer-(I)-iodid zu. Nach 15 h wird die Reaktionsmischung am Rotationsverdampfer eingeengt und das erhaltene rote Öl chromatographisch an Kieselgel gereinigt (Dichlormethan : Methanol = 20 : 1). Man erhält 400 mg (84%) Produkt. Nach Triphosphorylierung wurde dUTP-R-COOCH₃.erhalten.

In ähnlicher Weise können auch andere Basen wie Cytosin, Adenosin und Guanosin-Derivaten mit dem Pent-1-insäuremethylester modifiziert werden (Dissertation "Synthese basenmodifizierter Nukleosidtriphosphate und ihre enzymatische Polymerisation zu funktionalierter DNA", Oliver Thum, Bonn 2002).

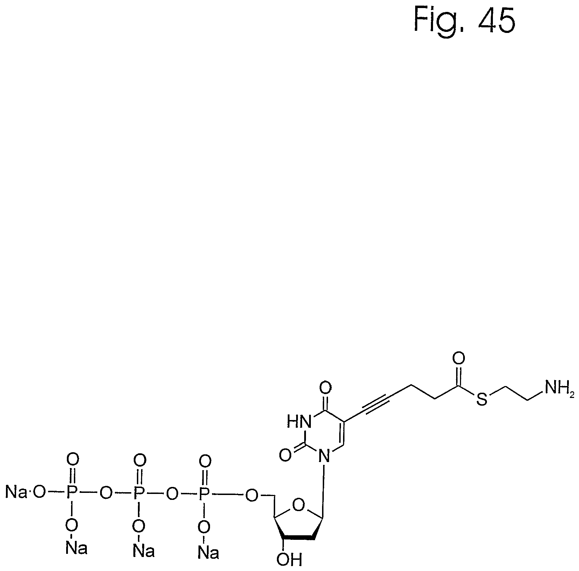

Weitere Modifikation erfolgt am dUTP-R-COOCH₃:
Zu 100 µl einer 50 mmol/l Lösung der dUTP-R-COOCH₃ in 50 mmol/l Borat-Puffer, pH 9, wird 100 µl einer 1 mol/l Lösung des Merkaptoethanolamins, pH 9, zugegeben und bei 40°C 3 h gerührt. Das Produkt der Reaktion,
das dUTP-R-CO-S-CH₂-CH₂-NH₂ (Fig. 45), wird vom überschüssigen Merkaptoethanolamin auf DEAE-Cellulose im Borat-Puffer 10 mmol/l abgetrennt und mit 0,3 mol/l NaCl von der Säule eluiert.

Dieses Nukleotid hat eine unter milden Bedingungen spaltbare Thioester-Gruppe und kann an der Aminogruppe am Linker modifiziert werden.

An diese Amino-Gruppe können weitere Modifikationen vorgenommen werden. Beispielsweise kann an sie ein Farbstoff gekoppelt werden:

### Synthese von dUTP-R-CO-S-CH₂-CH₂-NH-R-Cy3 (Fig. 46)

Zu 100 µl einer 10 mmol/l Lösung des dUTP-R-CO-S-CH₂-CH₂-NH₂ in 50 mmol/l Borat-Puffer, pH 9, wurde Cy3-NHS bis zu Konzentration von 15 mmol/l zugegeben. Die Reaktion erfolgte bei RT über 30 min. Anschließend wurde das mit Cy3-modifiziertes Nukleotid auf Kieselgel-Platte und RP-18 Säule gereinigt, ähnlich wie in Beispiel 15 dargestellt.

Ein solches Nukleotid kann als reversibler Terminator in einem Verfahren zur Sequenzierung von Nukleinsäuren eingesetzt werden (Tcherkassov WO 02088382). Die Spaltung der Thioester-Verbindung kann beispielsweise durch Zugabe von 100 mmol/l Merkaptoethanol in 50 mmol/l Borat-Puffer, pH 9, erfolgen.

An die Aminogruppe des dUTP-R-CO-S-CH₂-CH₂-NH₂ kann auch ein langer Linker mit einem niedermolekularen Marker gekoppelt werden, ähnlich wie im Beispiel 19. Das erhaltene dUTP-R-CO-S-CH₂-CH₂-NH-PEG-Biotin kann als Zwischenprodukt für ein Nuk-Makromolekül dienen.

### Beispiel 38:

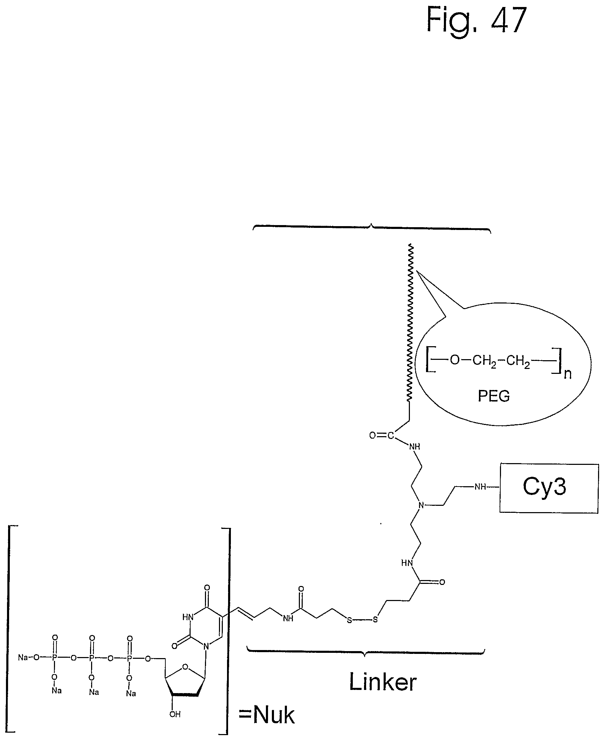

### dUTP-AA-SS-Propionat-TEAE-Cy3-PEG (Fig. 47),

Dieses Derivat kann aus dUTP-AA-SS-Propionat-TEAE-Cy3 (s. Beispiel 17) durch Modifikation der Aminogruppe im Linker mit einem mPEG-SPA, z.B. 5.000 Da, erhalten werden. Die Modifikationsbedingungen sind ähnlich wie im Beispiel 19. Dieses Molekül besitzt einen langen Linker und kann durch die erfindungsgemäße Methode zur schnellen Reinigung der modifizierten Nukleotide vor ihrem Einsatz in den Markierungsreaktionen eingesetzt werden. Für eine Trennung von markierten von unmarkierten Nukleotiden kann in diesem Beispiel ein Filter mit MWCO von 3000 verwendet werden.

### Beispiel 39:

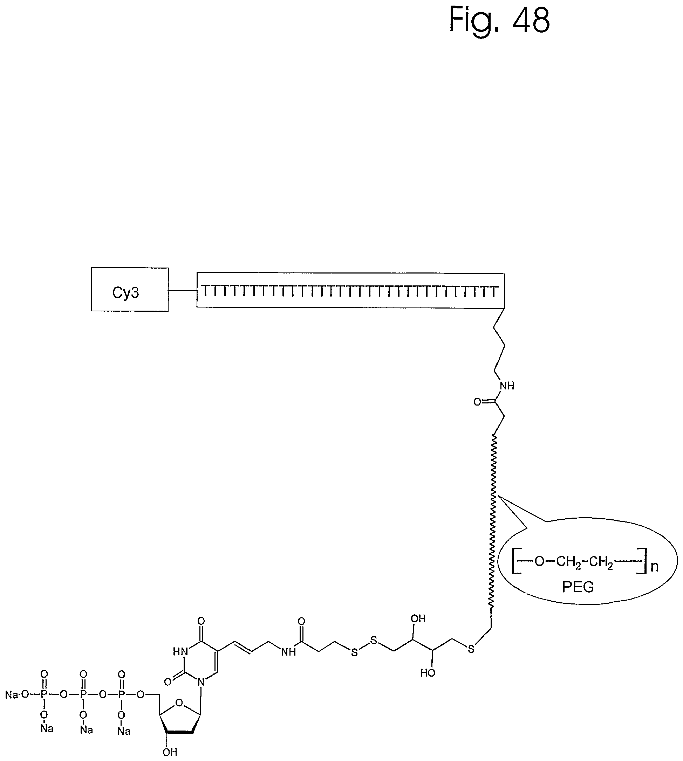

### Synthese von dUTP-AA-SS-R-PEG-Oligo-dT31-Cy3 (Fig. 48)

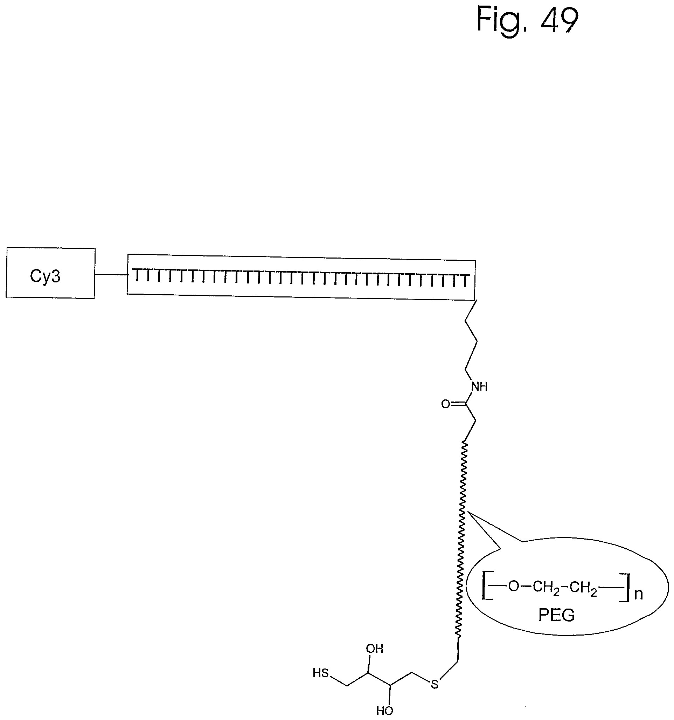

### Hestellung von SH-R-PEG-Oligo-dT₃₁-Cy3 (Fig. 49):

Zu 200 µl einer 100 µmol/l Lösung von 3'-Amino-Oligo-dT₃₁-Cy3 im 50 mmol/l Borat-Puffer, pH 9, wurde NHS-PEG-Maleimid zugegeben, bis die Konzetration von 20% (w/v) erreicht wurde. Das Gemisch wurde 2 h bei 40°C kräftig gerührt. Die Trennung des Maleimid-PEG-Oligo-dT₃₁-Cy3 vom Überschuß an PEG-Derivat erfolgte aug DEAE-Cellulose-Säulenchromatographie: Das Reaktionsgemisch wurde in 10 mmol/l Borat, pH 9, auf die Säule aufgetragen, und mit 20 Säulenvolumina 50 mmol/l Borat, pH 9, gewaschen. Elution von Maleimid-PEG-Oligo-dT₃₁-Cy3 von der Säule erfolgte mit 1 M NaCl in 50 mmol/l Borat, pH 9. Das Eluat wurde durch Ultrafiltration (MWCO 3000) zunächst eingeengt und das Produkt wurde in 20 mmol/l Borat-Puffer, pH 9.0, umgepuffert. Das Maleimid-PEG-Oligo-dT₃₁-Cy3 wurde vom Oligo-dT₃₁-Cy3 auf präparativem 15% Polyacrylgel durch die Elektrophorese getrennt, aus dem Gel isoliert und in 50 mmol/l Borat-Puffer, pH 9.0, aufgelöst. Ausbeute: 55%.
Zu Lösung mit Maleimid-PEG-Oligo-dT₃₁-Cy3 wurde DTT bis zu Konzentration 0,5 mol/l zugegeben und das Gemisch wurde 16 h bei RT gerührt. Durch Reaktion von DTT mit Maleimid entsteht SH-R-PEG-Oligo-dT₃₁-Cy3. Diese Substanz wurde auf DEAE-Säule vom DTT-Überschuß abgetrennt: Das Reaktionsgemisch wurde in 50 mmol/l Na-Acetat, pH 6.0, auf die Säule aufgetragen, und mit 20 Säulenvolumina 50 mmol/l Na-Acetat, pH 6.0 gewaschen. Elution von SH-R-PEG-Oligo-dT₃₁-Cy3 von der Säule erfolgte mit 1 mol/l NaCl in 50 mmol/l Na-Acetat, pH 6.0. Das Eluat wurde mit Ultrafiltration (MWCO 3000) zunächst eingeengt und das Produkt, SH-R-PEG-Oligo-dT₃₁-Cy3, wurde in 50 mmol/l Borat-Puffer, pH 9.0 umgepuffert. Die Konzentration von SH-R-PEG-Oligo-dT₃₁-Cy3 betrug 100 µmol/l.

Zu dieser Lösung wurden 50 Äquivalente an dUTP-AA-PDTP (synthetisiert wie im Beispiel 1) zugegeben. Nach 3 h bei RT erfolgte Trennung auf DEAE-Cellulose: Das Gemisch wurde in 50 mmol/l Na-Acetat-Puffer, pH 6.0, auf die Säule aufgetragen, und mit 20 Säulenvolumina 50 mmol/l Na-Acetat, pH 6.0, gewaschen. Elution von dUTP-AA-PDTP erfolgte mit 0,3 mol/l NaCl in 50 mmol/l Na-Acetat, pH 6.0, Elution von dUTP-AA-SS-R-PEG-Oligo-dT31-Cy3 erfolgte mit 0,8 mol/l NaCl in 50 mmol/l Na-Acetat, pH 6.0. Das Eluat wurde mit Ultrafiltration (MWCO 3000) zunächst eingeengt und das Produkt wurde in 50 mmol/l Na-Acetat, pH 6.0, umgepuffert.

Die Substanz trägt eine Nukleosid-Triphosphat-Funktionalität und eine makromolekulare Marker-Funktionalitäten (Oligo-dT31). Das Oligo-dT31 besteht aus Nukleosid-Monophosphaten, die allerdings an der enzymatischen Reaktion nicht teilnehmen und nur eine signalvermittelnde Funktion haben. An ein solches Oligonukleotid können komplementäre Nukleinsäuren hybridisiert werden, die eine signalgebende Funktion haben (Fig. 37B). (allgemeine Regeln zur Hybridisierung von Nukleinsäuren sind dem Fachmann bekannt, Anderson "Nucleic Acid Hybridization", 1999.).

Das Nukleotid ist definitionsgemäß ein Nuk-Makromolekül: die Linker-Länge ist deutlich größer als 30 Atome, die Marker-Komponente ist makromolekular.

Diese Verbindung wird von Polymerasen (z.B. Klenow-Exo minus Polymerase und Terminaler Transferase) als Substrat akzeptiert.

In ähnlicher Weise können auch andere Oligonukleotide an das dUTP-Derivat gekoppelt werden. In einer Ausführungsform der Erfindung können andere Homopolymer-Oligonukleotide, wie z.B. Poly-dC, poly-dG oder poly-dU oder poly-dA. In einer anderen Ausführungsform können auch Oligonukleotide mit spezifischen Sequenzen eingesetzt werden. Durch die spezifische Sequenzen von Oligonukleotiden können Nukleinsäureketten sequenzspezifisch hybridisiert werden. Zur Synthese von Nuk-Makromolekülen können auch Oligonukleotide eingesetzt werden, die Haarnadel-Strukturen (Stemloops) beinhalten.

In diesem Beispiel trägt das Oligonukleotid eine am 3'-Ende über einen Linker gekoppelte Amino-Gruppe, die als Kopplungsstelle für Maleimid-PEG-NHS auftritt, und den Cy3-Fluoreszenzfarbstoff. Auch andere Kopplungsgruppen, wie SH-, Carboxy -, Aldehyd-Gruppen können eingesetzt werden.
Die Position der Kopplungsgruppe kann an einem der Enden der Oligonukleotide sein, oder auch mitten in der Sequenz liegen. Solche Oligonukleotide können von der Firma MWG-Biotech, Deutschland, synthetisiert werden.
Als Modifikationen können Oligonukleotide Fluoreszenzfarbstoffe oder andere Reporter-Gruppen, wie Biotin, Digaxygenin tragen. Auch mehrere Modifikationen pro ein Oligonukleotid sind möglich. Beispielsweise können FRET-Paare oder Fluoreszenzfarbstoff-Quanchermolekül-Paar in ein Oligonukleotid eingefügt werden.

Dieses Beispiel zeigt eine generelle Möglichkeit, an Nukleotiden weitere Modifikationen vorzunehnem. Weitere basenmodifzierte Nukleotidanaloga, wie z.B. 5-PropargylaminodCTP, 5-Amino-Propargyl-dUTP, 7-Deaza-Aminopropargyl-dGTP und 7-Deaza-Aminopropargyl-dATP können wie oben angeführt ebenfalls modifiziert werden. Es können sowohl Ribonukleotide, 2'-Deoxyribonukleotide als auch 2',3'-Deoxyribonukletide verwendet werden, Fig. 11 bis 14. Auch andere basenmodifizierte Nukleotide können in ähnlicher Weise eingesetzt werden.

Durch Zugabe von Poly-dA oder Poly-A können mehrere dUTP-AA-SS-R-PEG-Oligo-dT31-Cy3, z.B. 10-20, zu einem Nuk-Makromolekül gekoppelt werden. Dabei entsteht ein Nuk-Makromolekül mit linear angeordneten Nuk-Linker-Komponenten (Fig. 37 C). Durch Zugabe von anderen modifizierten Oligonukleotiden, die an Poly-dA oder Poly-A binden können, können auch weitere Modifikationen eingeführt werden, z.B. Fluoreszenzmarkierung.

### Beispiel 40:

### Synthese von (dATP-PA-PEG)ₙ-PAS-(Cy3)ₘ

Die Herstellung von dATP-PA-PEG-NH₂ wurde im Beispiel 19 beschrieben.
EDA-Cy3 wurde wie folgt synthetisiert: Zu 1ml wässriger EDA-Lösung, 400 mmol/l, pH 8.5 eingestellt mit HCl, wurden 0,1 mg Cy3-NHS zugegeben. Die Reaktion wurde 30 min bei RT gerührt. Das Produkt wurde auf RP-18 getrennt (Wasser-Methanol-Gradient) und auf 0.2 ml eingeengt.

PAS 100 kDa (35% wässrige Lösung) wurde durch mehrmaliges Einrotieren mit DMF in eine wasserfreie DMF-Lösung überführt. Anschließend wurde zu 200 µl einer 0,1 mmol/l PAS (2 mg in 200 µl) in DMF 3 mg CDI zugegeben. Reaktion erfolgte und 30 min bei RT. Anschließend wurden zu dieser Lösung gleichzeitig 0,2 ml 0,7 mmol/l Lösung EDA-Cy3 und 0,2 ml 0,5 mmol/l dATP-PA-PEG-NH₂ zugegeben. Die Reaktion wurde 1 Std. bei RT durchgeführt. Anschließend wurde Produkt (dATP-PA-PEG)ₙ-PAS-(Cy3)ₘ durch Ultrafiltration mit 100 kDa MWCO von EDA-Cy3 und dATP-PA-PEG-NH₂ getrennt.
Die durchschnittliche Zahl der Cy3-derivaten beträgt 5 pro ein PAS-Molekül, die durchschnittliche Zahl der Nuk-Einheiten (dATP) pro ein PAS-Molekül beträgt 2. Auf diese Weise wurde ein Nuk-Makromolekül synthetisiert, das mehrere Makrer-Einheiten und mehrere Nuk-Einheiten einschließt.

(dATP-PA-PEG)ₙ-PAS-(Cy3)ₘ dient als Substrat für die DNA-Polymerasen und kann in den Markierungsreaktionen eingesetzt werden.

### Beispiel 41:

### Nuk-Makromoleküle als Monomer-Bestandteile eines Oligonukleotides

Beispiele der enzymatischen Einbaus von Nuk-Makromolekülen in die Nukleinsäure s. Beispiel 34 und 35.

Oben wurden Kopplungen eines langen Linkers und eines Markers an Nukleotid-Monomere beschrieben, die eine reaktive Gruppe tragen. In analoger Weise kann auch ein Nukleotid-Monomer, das als Teil eines Polymeres auftritt, z.B. einer Nukleinsäurekette, und eine reaktive Gruppe trägt, ebenfalls modifiziert werden. Bevorzugt wirde bei der Modifizierung ein langer, linearer, nicht verzweigter Linker, wie PEG. Nukleotid-Monomere mit einer reaktiven Gruppe, beispielsweise einer Amino- oder Merkapto-Gruppe, können mit den Mitteln der konventionellen OligonukleotidSynthese in eine Nukleinsäurekette gekoppelt werden. Viele Modifikationen können bei einer Auftragssynthse, beispielsweise durch MWG-Biotech, in ein Oligonukleotid eingeführt werden.

### Synthese eines modifizierten Oligonukleotids:

Ein Oligonukleotid mit 31 dT-Monomeren und einer am 5'-Ende gekoppelten Amino-Gruppe wurde von der MWG-Biotech synthetisiert (5'-Amino-dT31). An ein solches Oligonukleotid kann beispielsweise ein Fmoc-PEG-NHS Linker gekoppelt werden:

Zu 100µl wässriger Lösung 5'-Amino-dT31, 0,5 mmol/l, pH 8.0, wurden 1mg Fmoc-PEG-NHS (Fmoc-geschützter NH₂-PEG-NHS) gegeben und bei 30°C 8 Stunden gerührt. Anschließend wurde der pH-Wert auf 11 erhöht und die Reaktionsmischung weitere 2 Stdunden bei RT gerührt. Anschließend wurde das modifizierte Oligonukleotid durch Elektrophorese in einem 15% Polyacrylgel vom nicht modifizierten getrennt und isoliert. Das Produkt der Reaktion ist NH2-PEG-dT31 wurde in 50 µl 50 mmol Hydrogencarbonat-Puffer, pH 8,0, aufgelöst (Konzentration 0,3 mmol/l). An die endständige Aminogruppe kann ein makromolekularer Marker gekoppelt werden. Durch eine ähnliche Reaktion wie im Beispiel 40 kann ein mit einem makromolekularen Marker modifizieriertes Oligonukleotid synthetisiert werden:
EDA-Cy3 wurde wie im Beispiel 40 synthetisiert. PAS 100 kDa (35% wässrige Lösung) wurde durch mehrmaliges Einrotieren mit DMF in eine wasserfreie DMF-Lösung überführt. Anschließend wurde zu 50 µl einer 0,1 mmol/l PAS in DMF 1 mg CDI (als konzentrierte Lösung in DMF) zugegeben. Reaktion erfolgte und 30 min bei RT.
Anschließend wurden zu dieser Lösung gleichzeitig 50 µl 1,5 mmol/l Lösung EDA-Cy3 und 50 µl 0,3 mmol/l NH₂-PEG-dT31 zugegeben. Die Reaktion wurde 1 Std. bei RT durchgeführt. Anschließend wurde Produkt (dT31-PEG)ₙ-PAS-(Cy3)ₘ durch Ultrafiltration mit 100 kDa MWCO von EDA-Cy3 und NH₂-PEG-dT31 getrennt.
Die durchschnittliche Zahl der Cy3-derivaten beträgt 7 pro ein PAS-Molekül, die durchschnittliche Zahl der gekoppelten Oligonukleotide pro ein PAS-Molekül beträgt 1. Auf diese Weise wurde ein mit einem polymer mofidiziertes Oligonukleotid synthetisiert, das ein Nuk-Makromolekül einschließt.

### Beispiel 42

### Synthese eines Nuk-Makromoleküls mit einem Linker am Phosphat.

Ein Linker kann auch an Phosphat-Gruppen eines Nukleotid gekoppelt werden. Eine Kopplung einer reaktiven Gruppe, beispielsweise einer Amino-Gruppe an die endständige Phosphat-Gruppe ist bereits bekannt (Jameson et al. Method in Enzymology, 1997, v. 278, S. 363-, A. Draganescu et al. J. Biol. Chem. 2000 v.275, 4555-). Analog zu Synthesen in anderen Beispielen kann die Linker-Komponente an das Nukleotid gekoppelt werden.

### Beispiel 43

Herstellung eines modifizierten Klenow-Fragments Exo minus der DNA-Polymerase I der E. coli (im weiteren Bezeichnet als Klenow-Fragments Exo minus).

In einer Ausführungsform der Modifikation werden zu 70 µl einer Puffer-Lösung mit Klenow-Fragment Exo-minus der DNA-Polymerase (750 Units-Vial von Amersham Bioscience, aufgelöst im Hersteller-Puffer: 50 mmol/l Kalium-Phosphat-Puffer, pH7, 1,0 mmol/l DTT, 50% Glycerol) 100 µl einer Puffer-Lösung (200 mmol/l Tris-HCl-Puffer, pH 10.0, 60% Glycerin) zugegeben, der pH-Wert der Lösung mit Polymerase beträgt nun 9.0. Anschließend werden 30 µl einer 1 mol/l wässrigen Iod-Acetamid-Lösung zugegeben. Die Reaktion wird 30 min bei RT durchgeführt. Auf diese Weise erfolgt eine selektive Modifizierung der Polymerase an der SH-Gruppe des Cysteins.

In einer anderen Ausführungform der Modifikation erfolgt zunächst eine Zugabe von 10 µl 50 mmol/l TCEP-NaOH-Lösung, pH 8, zu 70 µl einer Puffer-Lösung mit Klenow-Fragment Exo-minus der DNA-Polymerase (750 Units-Vial von Amersham Bioscience aufgelöst im Hersteller-Puffer, s.o.). Nach 10 min bei RT erfolgt dann die Zugabe von 100 µl einer Puffer-Lösung (200 mmol/l Tris-HCl-Puffer, pH 10.0, 60% Glycerin) zur Lösung mit Polymerase. Anschließend werden 30 µl einer 1 mol/l wässrigen IodAcetamid-Lösung zugegeben. Die Reaktion erfolgt 30 min bei RT. Auf diese erfolgt eine selektive Modifizierung der Polymerase an der SH-Gruppe.

Die Reinigung der modifizierten Polymerase kann beispielsweise über Ultrafiltration oder über Ionenaustauscher oder Dialyse erfolgen.

Die Aufbewahrung der modifizierten Polymerase erfolgt beispielsweise in einem glycerinhaltigen Puffer. Als Puffer eignen sich beispielsweise Tris-HCl, Borat, Phosphat-Puffer. Der pH-Wert dieser Puffer liegt beispielsweise zwischen 5 und 10. Die Konzentration des Glycerins kann beispielsweise zwischen 10 und 70% liegen.
Auch andere Reagenzien, wie bespielsweise PEG oder Salze wie NaCl, NH₄Cl können zur Polymerase-Lösung zugegeben werden. Vorzugsweise wird kein Reduzierungsmittel, wie beispielsweise DTT, zur Lösung mit Polymerase zugegeben. In einer Ausführungform enthält der Aufbewahrungspuffer zusätzlich ein Reagenz, das mit SH-Gruppen selektiv reagieren kann, beispielsweise IodAcetamid in Konzentration zwischen 1 mmol/l und 500 mmol/l.

Eine auf diese Weise modifizierte Polymerase kann anstelle von Klenow-Fragment Exo-minus der DNA-Polymerase in Reaktionen mit Nuk-Makromolekülen eingesetzt werden.
Organization Applicant
   Street: Maria Goeppert Str 1, ICL
   City: Luebeck
   State:
   Country: Germany
   PostalCode: 23562
<110> OrganizationName : Genovoxx GmbH
   Individual Applicant
   Street: Moislinger Muehlenweg 21
   City: Luebeck
   State :
   Country : Germany
   PostalCode: 23560
   EmailAddress: cherkasov@genovoxx.com
   <110> LastName: Cherkasov
   <110> FirstName: Dmitry
Application Project
   <120> Title: Makromolekulare Nukleotidverbindungen und Methoden zu deren Anwendung
   <130> AppFileReference: WO 2005 044836
   <140> CurrentAppNumber: PCT/EP 2004 / 012556
   <141> CurrentFilingDate: 2006-05-31
Sequence
   <210> 1
   <211> Length: 20
   SequenceName: Primer T 7-20
   SequenceDescription:
   <212> Type : DNA
   <213> OrganismName: Artificial
   <223> Primer
   <223> 5'-Cy3
   <400> PreSequenceString:
   taatacgact cactataggg 20
Sequence
   <210> 2
   <211> Length: 33 SequenceName: oligonucleotide 1 SequenceDescription:
   <212> Type: DNA
   <213> OrganismName: Artificial
   <223> Oligonucleotide
   <400> PreSequenceString:
   agttttagtt ttaccctata gtgagtcgta tta 33
Sequence
   <210> 3
   <211> Length: 31
   SequenceName: oligonucleotide dT31
   sequenceDescription:
   <212> Type : DNA
   <213> OrganismName: Artificial
   <223> Oligonucleotide
   <223> 3' Amino-goup; 5'Cy3
<400> PreSequenceString:
   tttttttttt tttttttttt tttttttttt t 31
Sequence
   <210> 4
   <211> Length: 35
   SequenceName: Oligonucleotide dT35
   SequenceDescription:
   <212> Type : DNA
   <213> OrganismName: Artificial
   <223> Oligonucleotide
   <223> 5' Cy3
   <400> PreSequenceString:
   tttttttttt tttttttttt tttttttttt ttttt 35
Sequence
   <210> 5
   <211> Length: 40
   SequenceName: Oligonucleotide dT40
   SequenceDescription:
   <212> Type : DNA
   <213> OrganismName: Artificial
   <223> Oligonucleotide
   <223> 5' Cy3
   <400> PreSequenceString:
   tttttttttt tttttttttt tttttttttt tttttttttt 40
Sequence
   <210> 6
   <211> Length: 50
   SequenceName: Oligonucleotide dA50
   SequenceDescription:
   <212> Type: DNA
   <213> OrganismName: Artificial
   <223> Oligonucleotide
   <223> 3' Biotin
   <400> PreSequenceString:
   aaaaaaaaaa aaaaaaaaaa aaaaaaaaaa aaaaaaaaaa aaaaaaaaaa 50
Sequence
   <210> 7
   <211> Length: 50
   SequenceName: Oligonucleotide dT50
   SequenceDescription:
   <212> Type: DNA
   <213> OrganismName: Artificial
   <223> Oligonucleotide
   <223> 5' Cy3
   <400> PreSequenceString:
   tttttttttt tttttttttt tttttttttt tttttttttt tttttttttt 50
Sequence
   <210> 8
   <211> Length: 270
   SequenceName: poly d_{A}
SequenceDescription:
   <212> Type: DNA
   <213> OrganismName: Artificial
   <223> Oligonucleotide
   <223> calculated overage length of the poly dA is 270 nucleotides
   <400> PreSequenceString:

aaaaaaaaaa aaaaaaaaaa aaaaaaaaaa aaaaaaaaaa aaaaaaaaaa 270
aaaaaaaaaa aaaaaaaaaa aaaaaaaaaa aaaaaaaaaa aaaaaaaaaa
aaaaaaaaaa aaaaaaaaaa aaaaaaaaaa aaaaaaaaaa aaaaaaaaaa
aaaaaaaaaa aaaaaaaaaa aaaaaaaaaa aaaaaaaaaa aaaaaaaaaa
aaaaaaaaaa aaaaaaaaaa aaaaaaaaaa aaaaaaaaaa aaaaaaaaaa
aaaaaaaaaa aaaaaaaaaa

## Patentansprüche

1. Makromolekulare Verbindungen mit der Struktur:
(Nuk-Linker)ₙ-Marker
wobei:
Nuk - ein Nukleotid oder ein Nukleosid ist (Nuk-Komponente)
Linker - eine Linker-Komponente, die folgende Bestandteile einschließt:
a) Kopplungseinheit L - ein Teil des Linkers, das die Verbindung zwischen Nuk und dem Linkerrest darstellt
b) ein Polymer- ein Teil des Linkers, das ein wasserlösliches Polymer mit einer durchschnittlichen Länge zwischen 50 und 20.000 Atome (Kettenatome) ist und dieses Polymer gleiche oder unterschiedliche Monomere unabhängig ausgewählt aus folgender Gruppe von Polymeren einschließt:
Polyethylen-glycol (PEG), Polysaccharide, Dextran, Polyamide, Polypeptide, Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acylsäure, Poly-Acrylamid, Poly(Vinylalkohol).
c) Kopplungseinheit T - ein Teil des Linkers, das die Verbindung zwischen dem Linkerrest und dem Marker darstellt
Marker - eine makromolekulare Marker-Komponente ist, welche mindestens ein Makromolekül mit einer Masse von mindestens 2000 Da einschließt, oder eine Vielzahl von Marker-Einheiten mit molekularer Masse unter 2000 Da einschließt, oder eine Kombination aus einer Vielzahl von Marker-Einheiten mit molekularer Masse unter 2000 Da und mindestens einem Makromolekül mit einer molekularen Massen von mindestens 2000 Da einschließt.
n - eine ganze Zahl zwischen 1 und 100 ist

2. Makromolekulare Verbindungen nach Anspruch 1, wobei die Nuk-Komponente folgende Strukturen einschließt: 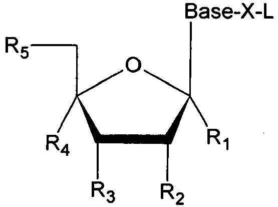 Wobei:
Base - ist unabhängig gewählt aus der Gruppe von Adenin, oder 7-Deazaadenin, oder Guanin, oder 7-Deazaguanin, oder Thymin, oder Cytosin, oder Uracil, oder deren Modifikationen , wobei X die Kopplungsstelle des Linkers an der Base ist und L - die Kopplungseinheit des Linkers (L).
R₁ - ist H
R₂ - ist unabhängig gewählt aus der Gruppe H, OH, Halogen, NH₂, SH oder geschützte OH-Gruppe
R₃ - ist unabhängig gewählt aus der Gruppe H, OH, Halogen, PO₃, SH, N₃, NH₂, O-CH₃, O-CH₂-O-CH₃, O-CH₂-CH=CH₂, O-R₃₋₁, P(O)ₘ-R₃₋₁ (m ist gleich 1 oder 2), NH-R₃₋₁, S-R₃₋₁, Si-R₃₋₁ wobei R₃₋₁ eine chemisch, photochemisch oder enzymatisch spaltbare Gruppe ist.
R₄ - ist H oder OH
R₅ - ist unabhängig gewählt aus der Gruppe OH, oder eine geschützte OH-Gruppe, oder eine Monophosphat-Gruppe, oder eine Diphosphat-Gruppe, oder eine Triphosphat-Gruppe, oder eine Alpha-Thiotriphosphat-Gruppe ist.

3. Makromolekulare Verbindungen nach Anspruch 1, wobei die Nuk-Komponente folgende Strukturen einschließt: 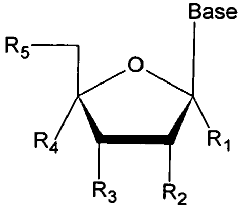 Wobei:
Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen
R₁ - ist H
R₂ - ist unabhängig gewählt aus der Gruppe H, OH, Halogen, NH₂, SH oder geschützte OH-Gruppe
R₃ - ist unabhängig gewählt aus der Gruppe O- R₃₋₂-L, P(O)ₘ- R₃₋₂-L, wobei m 1 oder 2 ist, NH-R₃₋₂-L, S-R₃₋₂-L, Si-R₃₋₂-L oder, wobei R₃₋₂ die Kopplungsstelle des Linkers an das Nukleotid ist und L - die Kopplungseinheit des Linkers (L) ist.
R₄ - ist H oder OH
R₅ - ist unabhängig gewählt aus der Gruppe OH, oder eine geschützte OH-Gruppe, oder eine Monophosphat-Gruppe, oder eine Diphosphat-Gruppe, oder eine Triphosphat-Gruppe, oder eine Alpha-Thiotriphosphat-Gruppe ist.

4. Makromolekulare Verbindungen nach Anspruch 1, wobei die Nuk-Komponente folgende Strukturen einschließt: 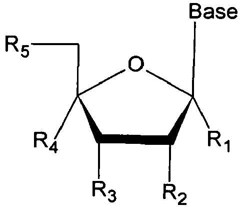 Wobei:
Base - ist unabhängig gewählt aus der Gruppe von Adenin, oder 7-Deazaadenin, oder Guanin, oder 7-Deazaguanin, oder Thymin, oder Cytosin, oder Uracil, oder deren zu enzymatischen Reaktionen fähigen Modifikationen
R₁ - ist
R₂ - ist unabhängig gewählt aus der Gruppe H, OH, Hal, NH2, SH oder geschützte OH-Gruppe
R₃ - ist unabhängig gewählt aus der Gruppe H, OH, Hal, PO₃, SH, NH₂, O- R₃₋₁, P(O)m- R₃₋₁ (m ist gleich 1 oder 2), NH-R₃₋₁, S-R₃₋₁, Si-R₃₋₁ wobei R₃₋₁ eine chemisch, photochemisch oder enzymatisch spaltbare Gruppe ist.
R₄ - ist H oder OH
R₅ - ist unabhängig gewählt aus der Gruppe O- R₅₋₁-L, oder P-(O)₃- R₅₋₁-L (modifizierte Monophosphat-Gruppe), oder P-(O)₃-P-(O)₃-R₅₋₁-L (modifizierte Diphosphat-Gruppe) oder P-(O)₃-P-(O)₃-P-(O)₃- R₅₋₁-L (modifizierte Triphosphat-Gruppe), wobei R₅₋₁ die Kopplungsstelle des Linkers an das Nukleotid ist und L - die Kopplungseinheit des Linkers (L) ist.

5. Makromolekulare Verbindungen nach Ansprüchen 1 bis 4, wobei die Kopplungseinheit des Linkers (L) folgende strukturelle Elemente einschließt:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇,
R₆-(CH=CH-)ₙ-R₇, R₆-(A-CH=CH-)ₙ-R₇, R₆-(CH=CH-B-)ₙ-R₇, R₆-A-CH=CH-(CH₂-)ₙ-R₇, R₆-(-CH=CH-CH₂)ₙ-B-R₇,
R₆-(C≡C-)n-R₇, R₆-(A-C≡C-)ₙ-R₇, R₆-(C≡C-B-)ₙ-R₇,
R₆-A-C≡C-(CH₂-)ₙ-R₇, R₆-(-C≡C-CH₂)ₙ-B-R₇, R₆-(-C≡C-CH₂-CH₂-)ₙ-B-R₇,
wobei R₆ - die Nuk-Komponente ist, R₇ - der Linkerrest ist und A und B folgende strukturelle Elemente einschließen: -NH-, -O-, -S-, -SS-, -CO-NH-, -NH-CO-, - CO-O-, -O-CO-, -CO-S-, -S-CO-, -P(O)₂-, -Si-, -(CH₂)ₙ-, eine photolabile Gruppe, wobei n - gleich 1 bis 5 ist

6. Makromolekulare Verbindungen nach Ansprüchen 1 bis 5, wobei die Linker-Komponente ein wasserlösliches Polymer einschließt und dieses Polymer aus mehreren Abschnitten besteht und Monomere eines Abschnitts gleich sind.

7. Makromolekulare Verbindungen nach Anspruch 6, wobei die Linker-Komponente in mindestens einem Abschnitt wasserlösliche Polymere unabhängig ausgewählt aus folgender Gruppe einschließt:
Polyethylen-glycol (PEG), Polysaccharide, Dextran, Polyamide, Polypeptide, Polyphosphate, Polyacetate, Poly(alkyleneglycole), Kopolymere aus Ethylenglycol und Propylenglycol, Poly(olefinische Alkohole), Poly(Vinylpyrrolidone), Poly(Hydroxyalkylmethacrylamide), Poly(Hydroxyalkylmethacrylate), Poly(x-Hydroxy-Säuren), Poly-Acrylsäure, Poly-Acrylamid, Poly(Vinylalkohol).

8. Makromolekulare Verbindungen nach Ansprüchen 1 bis 7, wobei ein Linker-Komponente eine durchschnittliche Länge zwischen 50 bis 100, 100 bis 200, 200 bis 500, 500 bis 1000, 1000 bis 2000, 2000 bis 10000, 10000 und 50000 Atome (Kettenatome) hat.

9. Makromolekulare Verbindungen nach Ansprüchen 1 bis 8, wobei eine Marker-Komponente eine signalgebende, signalvermittelnde, katalytische oder affine Funktion hat

10. Makromolekulare Verbindungen nach Ansprüchen 1 bis 9, wobei eine Marker-Komponente aus einer strukturellen Marker-Einheit besteht

11. Makromolekulare Verbindungen nach Ansprüchen 1 bis 9, wobei eine Marker-Komponente aus mehreren strukturellen Marker-Einheiten gebunden an eine Kern-Komponente besteht

12. Makromolekulare Verbindungen nach Ansprüchen 10 oder 11, wobei eine strukturelle Marker-Einheit unabhängig eines der folgenden strukturellen Elemente einschließt:
Biotin, Hapten, radioaktives Isotop, seltene Erdenatom, Farbstoff, Fluoreszenzfarbstoff.

13. Makromolekulare Verbindungen nach Ansprüchen 10 oder 11 , wobei eine strukturelle Marker-Einheit unabhängig eines der folgenden Elemente einschließt:
Nanokristalle oder deren Modifikationen, Proteine oder deren Modifikationen, Nukleinsäureketten oder deren Modifikationen, Teilchen oder deren Modifikationen.

14. Makromolekulare Verbindungen nach Anspruch 13, wobei eine strukturelle Marker-Einheit eines der folgenden Proteine einschließt:
Enzyme oder deren Konjugate oder Modifikationen,
Antikörper oder deren Konjugate oder Modifikationen,
Streptavidin oder seine Konjugate oder Modifikationen,
Avidin oder seine Konjugate oder Modifikationen

15. Makromolekulare Verbindungen nach Anspruch 13, wobei eine strukturelle Marker-Einheit eine der folgenden Arten Nukleinsäureketten einschließt: DNA, RNA, PNA, wobei die Länge von Nukleinsäureketten zwischen 10 und 10.000 Nukleotide liegt

16. Makromolekulare Verbindungen nach Ansprüchen 11 bis 15, wobei die Kern-komponente der Marker-Komponente unabhängig eines der folgenden Elemente einschließt: wasserlöslichen Polymer aus der Gruppe von: Polyamide (z.B. Polypeptide), Poly-Acrylsäure und deren Derivate, Poly-Acrylamide und deren Derivate, Poly-Vinylalkohole und deren Derivate, Nukleinsäuren und deren Derivate, Streptavidin oder Avidin und deren Derivate, Dendrimere, wobei diese Elemente linear oder verzweigt oder unter einander vernetzt sein können

17. Makromolekulare Verbindungen nach Ansprüchen 1 bis 9, 11 bis 16, wobei die Verbindung zwischen mehreren strukturellen Marker-Einheiten und der Kern-Komponte kovalent oder affine erfolgt.

18. Makromolekulare Verbindungen nach Ansprüchen 1 bis 10, wobei die Verbindung zwischen einer strukturellen Marker-Einheit und dem Linker kovalent oder affine erfolgt

19. Makromolekulare Verbindungen nach Ansprüchen 1 bis 9, 11 bis 17, wobei die Verbindung zwischen der Kern-Komponente und dem Linker kovalent oder affine erfolgt

20. Makromolekulare Verbindungen nach Ansprüchen 1 bis 19, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt.

21. Makromolekulare Verbindungen nach Ansprüchen 1 bis 20, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt und die Linker-Komponente eine oder mehrere unter milden Bedingungen spaltbare Verbindungen beinhaltet.

22. Makromolekulare Verbindungen nach Ansprüchen 1 bis 21, wobei nur eine Nuk-Komponente mit einer gekoppelten Linker-Komponente an die Marker-Komponente gebunden ist, wobei die Linkerlänge zwischen 50 bis 100, 100 bis 200, 200 bis 500, 500 bis 1000, 1000 bis 2000, 2000 bis 5000 Atome beträgt und ein oder mehrere Teile des Nuk-Makromoleküls dermaßen modifiziert sind, dass nur eine Nuk-Komponente in einen wachsenden Strang eingebaut werden kann.

23. Makromolekulare Verbindungen nach Ansprüchen 1 bis 19 wobei mehrere Nuk-Komponenten jeweils über einen Linker an einer Marker-Komponenten gekoppelt sind, wobei die Länge der jeweiligen Linker- Komponente zwischen 50 bis 100, 100 bis 200, 200 bis 500, 500 bis 1000, 1000 bis 2000, 2000 bis 5000 Atome beträgt.

24. Makromolekulare Verbindungen nach Ansprüchen 1 bis 19, 23, wobei mehrere Nuk-Komponenten jeweils über einen Linker an einer Marker-Komponenten gekoppelt, wobei die Länge der jeweiligen Linker- Komponente zwischen 50 bis 100, 100 bis 200, 200 bis 500, 500 bis 1000, 1000 bis 2000, 2000 bis 5000 Atome beträgt und der jeweilige Linker eine oder mehrere unter milden Bedingungen spaltbare Verbindungen beinhaltet.

25. Makromolekulare Verbindungen nach Ansprüchen 1 bis 19, 23, 24, wobei mehrere Nuk-Komponenten jeweils über einen Linker an einer Marker-Komponenten gekoppelt, wobei die Länge der jeweiligen Linker- Komponente zwischen 50 bis 100, 100 bis 200, 200 bis 500, 500 bis 1000, 1000 bis 2000, 2000 bis 5000 Atome beträgt und ein oder mehrere Teile des Nuk-Makromoleküls dermaßen modifiziert sind, dass nur eine Nuk-Komponente in eine wachsende Nukleinsäurekette eingebaut werden kann.

26. Makromolekulare Verbindungen nach Anspruch 2 bis 4, wobei R5 eine Triphosphat- oder eine Alpha-Thiotriphosphat-oder eine Tetraphosphat-Gruppe ist.

27. Makromolekulare Verbindungen nach Anspruch 2 bis 4, wobei R5 eine Monophosphat- oder eine Diphosphat-Gruppe ist.

28. Verfahren zur Modifizierung von Nukleinsäureketten, wobei für die Kopplung Nuk-Makromoleküle nach Ansprüchen 1 bis 27 verwendet werden

29. Verfahren nach Anspruch 28, wobei die Modifizierung durch eine enzymatische Kopplung erfolgt und das Reaktionsgemisch folgende Komponenten einschließt:
- mindestens eine Art der Nuk-Makromoleküle oder deren Zwischenstufen nach Ansprüchen 1 bis 25, wobei jede Art der Nuk-Makromoleküle eine für sie charakteristische Markierung besitzt
- mindestens eine Population der Nukleinsäureketten,
- mindestens eine Enzymart zur Kopplung von Nuk-Makromoleküle an die Nukleinsäureketten,

30. Verfahren nach Anspruch 28, wobei die Modifizierung durch eine enzymatische Kopplung erfolgt und das Reaktionsgemisch folgende Komponenten einschließt:
- mindestens eine Art der Nuk-Makromoleküle oder deren Zwischenstufen nach Ansprüchen 1-25, wobei jede Art der Nuk-Makromoleküle eine für sie charakteristische Markierung besitzt
- mindestens eine Population der Nukleinsäureketten,
- mindestens eine Enzymart zur Kopplung von Nuk-Makromoelküle an die Nukleeinsäureketten
- mindestens eine weitere Art von Nukleosidtriphosphaten

31. Verfahren nach Ansprüchen 29, 30, wobei die Enzymart unabhängig eine der folgenden Gruppen einschließt: DNA-Polymerase, RNA-Polymerase, Terminale Transferase,

32. Verfahren nach Anspruch 30, wobei die "weitere Art" der Nukleosidtriphosphaten unabhängig ausgewählt ist aus der Gruppe von: Ribo-Nukleosidtriphosphaten (ATP, GTP, UTP, CTP), von 2'-Deoxyribonukleosid-Triphosphaten (dATP, dUTP, dTTP, dCTP, dGTP), von 2',3'-Dideoxynukleosidtriphosphaten (ddATP, ddGTP, ddUTP, ddCTP, ddTTP).

33. Verfahren nach Anspruch 32, wobei die "weitere Art" der Nukleosidtriphosphaten konventionell modifizierte Nukleotide mit einer Markierung sind, wobei diese Markierung unabhängig ausgewählt ist aus der Gruppe von: ein Fluoreszenzfarbstoff, ein Biotin, ein Hapten, ein radioakives Element.

34. Verfahren nach Ansprüchen 28 bis 33, wobei mindestens zwei unterschiedliche Populationen an Nukleinsäureketten präsent sind

35. Verfahren nach Anspruch 34, wobei mindestens eine der Populationen der Nukleinsäureketten eine Primer-Funktion hat und mindestens eine Pupulation der Nukleinsäureketten eine Matrizen-Funktion.

36. Verfahren nach Anspruch 28, wobei die Modifizierung durch eine chemische Kopplung erfolgt, wobei die Kopplung der Nuk-Makromoleküle an Nukleinsäureketten durch Phosphoroamidit-Kopplung erfolgt.

37. Verfahren nach Ansprüchen 28 bis 36, wobei im Markierungsverfahren Nuk-Makromoleküle eingesetzt werden, die die Kopplung nur einer einzigen Nuk-Komponente in den wachsenden Nukleinsäure-Strang zulassen und der mehrfache Einbau durch Modifikationen an der Nuk-Komponente, und/oder der Linker-Komponente und/oder der Marker-Komponente verhindert wird.

38. Verfahren nach Anspruch 37, wobei die weitere Kopplung reversibel verhindert wird.

39. Verfahren nach Anspruch 37, wobei die weitere Kopplung irreversibel verhindert wird.

40. Verfahren nach Ansprüchen 28 bis 36, wobei im Markierungsverfahren Nuk-Makromoleküle eingesetzt werden, die eine Kopplung mehrer Nuk-Komponente in den wachsenden Nukleinsäurestrang zulassen

41. Verfahren nach Ansprüchen 28-40, wobei die an der Reaktion teilnehmenden Nukleinsäureketten an eine feste Phase gekoppelt sind und adressierbare Positionen haben.

42. Verfahren nach Anspruch 41, wobei die Nukleinsäureketten eine einheitliche Population darstellen

43. Verfahren nach Anspruch 41, wobei die Nukleinsäureketten zwei oder mehrere unterschiedliche Populationen darstellen und jede der Populationen eine adressierbare Position auf der festen Phase hat

44. Verfahren nach Ansprüchen 41,42, wobei die Kopplung von Nuk-Makromolekülen an einer Population einheitlicher, an der festen Phase fixierter Nukleinsäuremoleküle erfolgt, wobei die Marker-Komponente des Nuk-Makromoleküls nach der Kopplung am verlängerten Nukleinsäurestrang verbleibt und nicht abgetrennt wird.

45. Verfahren nach Ansprüchen 41,42, wobei die Kopplung von Nuk-Makromolekülen an einer Population einheitlicher, an der festen Phase fixierter Nukleinsäuremoleküle erfolgt, wobei die Marker-Komponente oder ihre einzelnen Komponenten mit oder ohne Linker-Komponente des Nuk-Makromoleküls während der Kopplung oder nach der Kopplung von der in den wachsenden Nukleinsäurestrang eingebauten Nuk-Komponente abgetrennt wird.

46. Verfahren nach Ansprüchen 41,43, wobei die Kopplung von Nuk-Makromolekülen in einem Reaktionsansatz parallel an zwei oder mehreren unterschiedlichen Populationen an der festen Phase fixierter Nukleinsäuremoleküle erfolgt, wobei diese Populationen jeweils unterschiedliche adressierbare Positionen an der festen Phase haben und die Marker-Komponente des Nuk-Makromoleküls nach der Kopplung am verlängerten Nukleinsäurestrang verbleibt und nicht abgetrennt wird.

47. Verfahren nach Ansprüchen 41,43, wobei die Kopplung von Nuk-Makromolekülen in einem Reaktionsansatz parallel an zwei oder mehreren unterschiedlichen Populationen an der festen Phase fixierter Nukleinsäuremoleküle erfolgt, wobei diese Populationen unterschiedliche adressierbare Positionen an der festen Phase haben und die Marker-Komponente oder ihre einzelnen Komponenten mit oder ohne Linker-Komponente des Nuk-Makromoleküls während der Kopplung oder nach der Kopplung von der Nuk-Komponente abgetrennt wird.

48. Verfahren nach Ansprüchen 41 bis 47, wobei die adressierbaren Positionen mit Nukleinsäuremolekülen auf der festen Phase als Spots auf einer planen Oberfläche verteilt sind, wobei pro ein Spot Nukleinsäuremoleküle einheitlich sind.

49. Verfahren nach Ansprüchen 41 bis 47, wobei die adressierbaren Positionen mit Nukleinsäuremolekülen an den Kügelchen bzw. Partikel befestigt sind, wobei pro ein Kügelchen Nukleinsäuremoleküle einheitlich sind.

50. Verfahren nach Ansprüchen 41 bis 47, wobei die adressierbaren Positionen mit Nukleinsäuremolekülen in einem Multigefäßsystem, wie Mikrotiterplatte oder Nanotiterplatte oder Pikotiterplatte, verteilt sind, wobei in einem Gefäß des Multigefäßsystems die Nukleinsäuremoleküle einheitlich sind.

51. Verfahren nach Ansprüchen 28 bis 35 und 37 bis 50, das folgende Schritte einschließt:
a) Bereitstellung mindestens einer Population an einzelsträngigen Nukleinsäureketten (NSK)
b) Hybridisierung von Primern an diese Nukleinsäureketten, wobei extensionsfähige NSK-Primer-Komplexe entstehen
c) Inkubation von mindestens einer Art der Nuk-Makromoleküle nach Ansprüchen 1 bis 25 zusammen mit einer Art der Polymerase nach Anspruch 31 mit den in Schritten a und b bereitgestellten NSK-Primer-Komplexen unter Bedingungen, die den Einbau von komplementären Nuk-Makromolekülen zulassen, wobei jede Art der Nuk-Makromoleküle eine für sie charakteristische Markierung besitzt.
d) Entfernung der nicht eingebauten Nuk-Makromoleküle von den NSK-Primer-Komplexen
e) Detektion der Signale von in die NSK-Primer-Komplexe eingebauten Nuk-Makromolekülen
f) Entfernung der Linker-Komponente und der Marker-Komponente von den in die NSK-Primer-Komplexe eingebauten Nuk-Makromolekülen
g) Waschen der NSK-Primer-Komplexe
gegebenenfalls Wiederholung der Schritte (c) bis (g).

52. Verfahren nach Ansprüchen 28-40, wobei die Nukleinsäureketten an eine feste Phase in zufälliger Anordnung gekoppelt sind.

53. Verfahren nach Ansprüchen 28 bis 41, 52 zur parallelen Sequenzanalyse von Nukleinsäuresequenzen (Nukleinsäureketten, NSKs), bei dem man
Fragmente (NSKFs) einzelsträngiger NSKs mit einer Länge von etwa 50 bis 1000 Nukleotiden erzeugt, die überlappende Teilsequenzen einer Gesamtsequenz darstellen können, man
die NSKFs unter Verwendung eines einheitlichen oder mehrerer unterschiedlichen Primer in Form von NSKF-Primer-Komplexen auf einer Reaktionsoberfläche in einer zufälligen Anordnung bindet, man
eine zyklische Aufbaureaktion des komplementären Stranges der NSKFs unter Verwendung einer oder mehrerer Polymerasen durchführt, indem man
a) zu den auf der Oberfläche gebundenen NSKF-Primer-Komplexen eine Lösung zugibt, die eine oder mehrere Polymerasen und ein bis vier Nuk-Makromoleküle enthält, die eine mit Fluoreszenzfarbstoffen markierte Marker-Komponente haben, wobei die bei gleichzeitiger Verwendung von mindestens zwei Nuk-Makromoleküle jeweils an die Marker-Komponente befindlichen Fluoreszenzfarbstoffe so gewählt sind, dass sich die verwendeten Nuk-Makromoleküle durch Messung unterschiedlicher Fluoreszenzsignale voneinander unterscheiden lassen, wobei die Nuk-Makromoleküle strukturell so modifiziert sind, dass die Polymerase nach Einbau eines solchen Nuk-Makromoleküls in einen wachsenden komplementären Strang nicht in der Lage ist, ein weiteres Nuk-Nakromolekül in denselben Strang einzubauen, wobei die Linker-Komponente mit der Marker-Komponente abspaltbar sind, man
b) die in Stufe a) erhaltene stationäre Phase unter Bedingungen inkubiert, die zur Verlängerung der komplementären Stränge geeignet sind, wobei die komplementären Stränge jeweils um ein Nuk-Makromolekül verlängert werden, man
c) die in Stufe b) erhaltene stationäre Phase unter Bedingungen wäscht, die zur Entfernung nicht in einen komplementären Strang eingebauter Nuk-Makromoleküle geeignet sind, man
d) die einzelnen, in komplementäre Stränge eingebauten Nuk-Makromoleküle durch Messen des für den jeweiligen Fluoreszenzfarbstoff charakteristischen Signals detektiert, wobei man gleichzeitig die relative Position der einzelnen Fluoreszenzsignale auf der Reaktionsoberfläche bestimmt, man
e) zur Erzeugung unmarkierter (NTs oder) NSKFs die Linker-Komponente und die Marker-Komponente von den am komplementären Strang angefügten Nuk-Komponenten abspaltet, man
f) die in Stufe e) erhaltene stationäre Phase unter Bedingungen wäscht, die zur Entfernung der Marker-Komponente geeignet sind, man
die Stufen a) bis f) gegebenenfalls mehrfach wiederholt,
wobei man die relative Position einzelner NSKF-Primer-Komplexe auf der Reaktionsoberfläche und die Sequenz dieser NSKFs durch spezifische Zuordnung der in Stufe d) in aufeinanderfolgenden Zyklen an den jeweiligen Positionen detektierten Fluoreszenzsignale zu den Nuk-Makromolekülen bestimmt.

54. Verfahren nach Anspruch 53, **dadurch gekennzeichnet, dass** man die Stufen a) bis f) der zyklischen Aufbaureaktion mehrfach wiederholt, wobei man in jedem Zyklus nur jeweils eine Art der Nuk-Makromoleküle einsetzt.

55. Verfahren nach Anspruch 53, **dadurch gekennzeichnet, dass** man die Stufen a) bis f) der zyklischen Aufbaureaktion mehrfach wiederholt, wobei man in jedem Zyklus jeweils zwei unterschiedlich markierte Arten der Nuk-Makromoleküle einsetzt.

56. Verfahren nach Anspruch 53, **dadurch gekennzeichnet, dass** man die Stufen a) bis f) der zyklischen Aufbaureaktion mehrfach wiederholt, wobei man in jedem Zyklus jeweils vier unterschiedlich markierte Arten der Nuk-Makromoleküle einsetzt.

57. Verfahren nach Anspruch 53, **dadurch gekennzeichnet, dass** die NSKs Varianten einer bekannten Referenzsequenz sind und man die Stufen a) bis f) der zyklischen Aufbaureaktion mehrfach wiederholt, wobei man in den Zyklen abwechselnd jeweils zwei unterschiedlich markierte Arten der Nuk-Makromoleküle und zwei unmarkierte NTs einsetzt und man die Gesamtsequenzen durch Vergleich mit der Referenzsequenz ermittelt.

58. Verfahren nach den Ansprüchen 53 bis 57, **dadurch gekennzeichnet, dass** man in die NSKFs jeweils eine Primerbindungsstelle (PBS) einführt, wobei man bei doppelsträngigen NSKs an beiden komplementären Einzelsträngen jeweils eine PBS einführt und wobei die Primerbindungsstellen für alle NSKFs jeweils gleiche oder verschiedene Sequenzen aufweisen.

59. Verfahren nach den Ansprüchen 53 bis 57, **dadurch gekennzeichnet, dass** man die NSKFs mit Primern in einer Lösung unter Bedingungen in Kontakt bringt, die zur Hybridisierung der Primer an die Primerbindungsstellen (PBSs) der NSKFs geeignet sind, wobei die Primer untereinander gleiche oder verschiedene Sequenzen aufweisen, und man die gebildeten NSKF-Primer-Komplexe anschließend auf der Reaktionsoberfläche bindet.

60. Verfahren nach den Ansprüchen 53 bis 57, **dadurch gekennzeichnet, dass** man die NSKFs zunächst auf der Reaktionsoberfläche immobilisiert und erst anschließend mit Primern unter Bedingungen in Kontakt bringt, die zur Hybridisierung der Primer an die Primerbindungsstellen (PBSs) der NSKFs geeignet sind, wobei NSKF-Primer-Komplexe gebildet werden, wobei die Primer untereinander gleiche oder verschiedene Sequenzen aufweisen.

61. Verfahren nach Ansprüchen 53 bis 60, bei dem an 10 bis 100.000 unterschiedlichen Sequenzenpopulationen die Einbaureaktion parallel durchgeführt

62. Verfahren nach Ansprüchen 53 bis 60, bei dem an 100.000 bis 100.000.000 unterschiedlichen Sequenzenpopulationen die Einbaureaktion parallel durchgeführt.

63. Verfahren nach Ansprüchen 28 bis 62, wobei die Sequenzen der Nukleinsäureketten ermittelt werden

64. Verfahren nach Ansprüchen 28 bis 63, wobei die Marker-Komponente fluoreszent markiert ist

65. Verfahren nach Ansprüchen 41 bis 64, wobei die feste Phase unabhängig aus der folgenden Gruppe ausgewählt ist: Silicon, Glas, Keramik, Kunststoffe, Gele oder deren Modifikationen

66. Ein Kit, das die makromolekularen Verbindungen nach Ansprüchen 1 bis 25 einschließt und optional weitere Komponenten aus folgender Gruppe einschließt: mindestens eine Art von Polymerasen, und mindesten eine weitere Art von Nukleosid-Triphosphaten oder 2'-Deoxy-Nukleosid-Triphosphaten.

67. Verfahren, bei denen makromolekulare Verbindungen nach Anspruch 1 bis 25 in enzymatischen Reaktionen eingesetzt werden, wobei Enzyme folgende Gruppen einschließen: Polymerasen, Ligasen, Nukleasen (Endo oder Exonukleasen).

68. Verfahren, bei denen makromolekulare Verbindungen nach Anspruch 26 und 27 in enzymatischen Reaktionen eingesetzt werden, wobei Enzyme folgende Gruppen einschließen: Polymerasen, Ligasen, Nukleasen (Endo oder Exonukleasen).

## Claims

1. Macromolecular compounds having the structure:
(Nuc-Linker)n-Marker
wherein:
nuc is a nucleotide or nucleoside (nuc-component);
linker is a linker component which comprises the following parts:
a) coupling unit L is a part of the linker, which provides linkage between the nuc and the rest of the linker
b) a polymer is a part of the linker, said polymer is water soluble-polymer with an average length between 50 and 20000 atoms (chain atoms) and said polymer comprises identical or different monomers independently selected from the group of polymers:
polyethylene glycol (PEG), polysaccharides, dextran, polyamides, polypeptides, polyphosphates, polyacetates, polyalkyleneglycoles, copolymers from ethyleneglycol and propyleneglycol, polyolefinic alcohols, polyvinylpyrrolidones, poly(hydroxyalkylmethacrylamides), polyhydroxyalkylmethacrylates, poly(x-hydroxy) acids, polyacrylic acid, polyacrylamide, and polyvinylalcohol
c) coupling unit T is a part of the linker which provides linkage between the marker and the rest of the linker;
marker is a macromolecular marker component comprising at least one macromolecule with molecular weight of at least 2000 Da, or a plurality of low molecular weight marker units with molecular weight below 2000 Da or a combination of a plurality of low molecular weight marker unit with a molecular weight below 2000 Da and at least one macromolecule with a molecular weight of at least 2000 Da;
(n) is a positive integer between 1 and 100

2. The macromolecular compounds according to claim 1, wherein the nuc-component comprises the following structure: 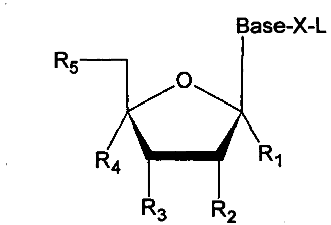 wherein:
base is selected independently from the group of adenine, or 7-deazaadenine, or guanine, or 7-deazaquanine, or thymine, or cytosine, or uracil, or their modifications, X is the coupling position of the linker to the base and L is the coupling unit of the linker (L)
R₁-is H,
R₂-is selected independently from the group of H, OH, halogen, NH₂, SH or protected OH group,
R₃-is selected independently from the group of H, OH, halogen, PO₃, SH, N₃, NH₂, O-CH₃, O-CH₂₋O-CH₃, O-CH₂-CH=CH₂, O-R₃₋₁, P(O)ₘ-R₃₋₁ ((m) is 1 or 2), NH-R₃₋₁, S-R₃₋₁, Si-R₃₋₁ wherein R₃₋₁ is a chemically, photochemically or enzymatically cleavable group,
R₄-is H or OH, and
R₅-is selected independently from the group of OH, or a protected OH group, or a monophosphate group, or a diphosphate group, or a triphosphate group, or is an alpha thiotriphosphate group.

3. The macromolecular compounds according to claim 1, wherein the nuc-component comprises the following structure: 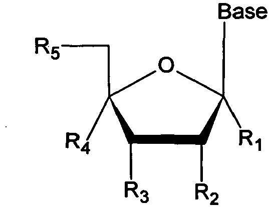 wherein:
base is selected independently from the group of adenine, or 7-deazaadenine, or guanine, or 7-deazaguanine, or thymine, or cytosine, or uracil, or their modifications capable of enzymatic reactions,
R₁-is H,
R₂-is selected independently from the group of H, OH, halogen, NH₂, SH or protected OH group,
R₃-is selected independently from the group of O-R₃₋₂-L, P(O)ₘ-R₃₋₂-L and (m) is 1 or 2, NH-R₃₋₂-L, S-R₃₋₂-L, Si-R₃₋₂-L, wherein R₃₋₂ is the coupling position of the linker to the nucleotide and L is the coupling unit of the linker (L),
R₄-is H or OH, and
R₅-is selected independently from the group of OH, or a protected OH group, or a monophosphate group, or a diphosphate group, or a triphosphate group, or is an alpha-thiotriphosphate group.

4. The macromolecular compounds according to claim 1, wherein the nuc-component comprises the following structure: 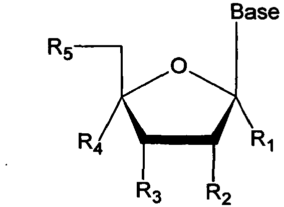 wherein:
base is selected independently from the group of adenine, or 7-deazaadenine, or guanine, or 7-deazaguanine, or thymine, or cytosine, or uracil, or their modifications capable of enzymatic reactions,
R₁-is H,
R₂-is selected independently from the group of H, OH, halogen, NH₂, SH or protected OH group,
R₃-is selected independently from the group of H, OH, halogen, PO₃, SH, NH₂, O-R₃₋₁, P(O)ₘ-R₃₋₁((m) is 1 or 2), NH-R₃₋₁, S-R₃₋₁, Si-R₃₋₁ wherein R₃₋₁ is a chemically, photochemically or enzymatically cleavable group,
R₄-is H or OH, and
R₅-is selected independently from the group of O-R₅₋₁-L, or P—(O)₃—R₅₋₁-L (modified monophosphate group), or P-(O)₃-P-(O)₃-R₅₋₁-L (modified diphosphate group) or P-(O)₃-P-(O)₃-P-(O)₃-R₅₋₁-L (modified triphosphate group), wherein R₅₋₁ is the coupling position of the linker to the nucleotide and L is the coupling unit of the linker (L).

5. The macromolecular compounds according to claims 1 to 4, wherein the coupling unit (L) of the linker comprises the following structural elements:
R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇,
R₆-(CH=CH-)ₙ-R₇, R₆-(A-CH=CH-)ₙ-R₇, R₆-(CH=CH-B-)ₙ-R₇, R₆-A-CH=CH-(CH₂-)ₙ-R₇, R₆-(-CH=CH-CH₂)ₙ-B-R₇,
R₆-(C≡C-)ₙ-R₇, R₆-(A-C≡C-)ₙ-R₇, R₆-(C≡C-B-)ₙ-R₇,
R₆-A-C≡C-(CH₂-)ₙ-R₇, R₆-(-C≡C-CH₂)ₙ-B-R₇, R₆-(-C≡C-CH₂-CH₂-)ₙ-B-R₇,
wherein R₆ is the nuc-component, R₇ is the rest of the linker, and A and B each comprise: -NH-, -O-, -S-, -SS-, -CO-NH-, -NH-CO-, -CO-O-, -O-CO-, -CO-S-, -S-CO-, -P(O)₂-, -Si-, or -(CH₂)ₙ-, wherein (n) ranges from 1 to 5, a photolabile group.

6. The macromolecular compounds according to claims 1 to 5, wherein the linker component comprises a water soluble-polymer and said polymer consists of several segments and monomers of one segment are identical.

7. The macromolecular compounds according to claim 6, wherein the linker component comprises in at least one segment water soluble-polymers independently selected from the group:
polyethylene glycol (PEG), polysaccharides, dextran, polyamides, polypeptides, polyphosphates, polyacetates, polyalkyleneglycoles, copolymers from ethyleneglycol and propyleneglycol, polyolefinic alcohols, polyvinylpyrrolidones, poly(hydroxyalkylmethacrylamides), polyhydroxyalkylmethacrylates, poly(x-hydroxy) acids, polyacrylic acid, polyacrylamide, and polyvinylalcohol.

8. The macromolecular compounds according to claims 1 to 7, wherein the average length of one linker component ranges between 50 to 100, 100 to 200, 200 to 500, 500 to 1000, 1000 to 2000, 2000 to 10000, 10000 to 50000 atoms (chain atoms).

9. The macromolecular compounds according to claims 1 to 8, wherein the marker component has one of the following functions: signal-giving function, signal-transmitting function, catalytic function or affine function.

10. The macromolecular compounds according to claims 1 to 9, wherein the marker component consists of one structural marker unit.

11. The macromolecular compounds according to claims 1 to 9, wherein the marker component consists of a plurality of structural marker units bound to one core component.

12. The macromolecular compounds according to claim 10 or 11, wherein one structural marker component independently comprises one of the following elements: biotin, hapten, radioactive isotope, rare-earth atom, dye, or fluorescent dye.

13. The macromolecular compounds according to claims 10 or 11, wherein the structural marker unit independently comprises one of the following elements: nanocrystals or their modifications, proteins or their modifications, nucleic acid chains or their modifications, particles or their modifications.

14. The macromolecular compounds according to claim 13, wherein the structural marker unit comprises one of the following proteins:
enzymes or conjugates or modifications thereof,
antibodies or conjugates or modifications thereof,
streptavidin or its conjugates or modifications thereof, and
avidin or its conjugates or modifications thereof.

15. The macromolecular compounds according to claim 13, wherein a structural marker unit comprises a nucleic acid chain selected from: DNA, RNA, and PNA, wherein the length of the nucleic acid chain ranges from 10 to 10,000 nucleotides.

16. The macromolecular compounds according to claims 11 to 15, wherein the core component of the marker component independently comprises one of the following elements: a water-soluble polyamide, polyacrylic acid or a derivative thereof, polyacrylamide or a derivative thereof, polyvinylalcohol or a derivative thereof, nucleic acid or a derivative thereof, streptavidin or avidin or a derivative thereof, and dendrimere, wherein the element is linear or branched, or is crosslinked to other element.

17. The macromolecular compounds according to claims 1 to 9, 11 to 16, wherein the linkage between several structural marker units and the core component is covalent or affine.

18. The macromolecular compounds according to claims 1 to 10, wherein the linkage between one structural marker unit and the linker is covalent or affine.

19. The macromolecular compounds according to claims 1 to 9, 11 to 17, wherein the linkage between the core component and the linker is covalent or affine.

20. The macromolecular compounds according to claims 1 to 19, wherein only one nuc-component with one linker component is linked to the marker component, wherein the linker length ranges between 50 to 100, 100 to 200, 200 to 500, 500 to 1000, 1000 to 2000, 2000 to 5000 atoms.

21. The macromolecular compounds according to claims 1 to 20, wherein only one nuc-component with one linker component is linked to the marker component, wherein the linker length ranges between 50 to 100, 100 to 200, 200 to 500, 500 to 1000, 1000 to 2000, or 2000 to 5000 atoms and the linker component comprises one or several bonds that are cleavable under mild conditions.

22. The macromolecular compounds according to claims 1 to 21, wherein only one nuc-component with one linker component is linked to the marker component, wherein the linker length ranges between 50 to 100, 100 to 200, 200 to 500, 500 to 1000, 1000 to 2000, or 2000 to 5000 atoms and one or several parts of the nuc-macromolecule are modified in such a way, that only one nuc-component can be incorporated into a growing nucleic acid chain.

23. The macromolecular compounds according to claims 1 to 19, wherein several nuc-component are linked to the marker component, each via one linker component, wherein the length of a particular linker component ranges between 50 to 100, 100 to 200, 200 to 500, 500 to 1000, 1000 to 2000, 2000 to 5000 atoms.

24. The macromolecular compounds according to claims 1 to 19, wherein several nuc-component are linked to the marker component, each via one linker component, wherein the length of each linker component ranges between 50 to 100, 100 to 200, 200 to 500, 500 to 1000, 1000 to 2000, 2000 to 5000 atoms and each linker component comprises one or several bonds that are cleavable under mild conditions.

25. The macromolecular compounds according to claims 1 to 19, wherein several nuc-component are linked to the marker component, each via one linker component, wherein the length of a particular linker component ranges between 50 to 100, 100 to 200, 200 to 500, 500 to 1000, 1000 to 2000, 2000 to 5000 atoms and one or several parts of the nuc-macromolecule are modified in such a way, that only one nuc-component can be incorporated into a growing nucleic acid chain.

26. The macromolecular compounds according to claims 2 to 4, wherein R5 is a triphosphate-moiety or alpha-thiotriphosphate moiety or a tetraphosphate moiety.

27. The macromolecular compounds according to claims 2 to 4, wherein R5 is a monophosphate-moiety or diphosphate moiety.

28. A method of modification of nucleic acid chains, wherein nuc-macromolecules according to claims 1 to 27 are used for coupling.

29. The method according to claim 28, wherein the modification is accomplished by an enzymatic coupling and wherein the reaction solution comprises the following components:
- at least one type of nuc-macromolecules or their intermediates according to claims 1 to 25, wherein each type of nuc-macromolecules has a characteristic label
- at least one population of nucleic acid chains
- at least one type of enzyme for coupling the nuc-macromolecules to nucleic acid chains.

30. The method according to claim 28, wherein the modification is accomplished by an enzymatic coupling and wherein the reaction solution comprises the following components:
- at least one type of nuc-macromolecules or their intermediates according to claims 1 to 25, wherein each type of nuc-macromolecules has a characteristic label
- at least one population of nucleic acid chains
- at least one type of enzyme for coupling the nuc-macromolecules to nucleic acid chains.
- at least one additional type of nucleoside triphosphate.

31. The method according to claims 29, 30, wherein the type of enzyme comprises independently one of the following groups: DNA-polymerases, RNA-polymerases, and terminal transferases.

32. The method according to claim 30, wherein the additional type of nucleoside triphosphate is independently selected from the group of: ribonucleoside tri-phosphates (ATP, GTP, UTP, CTP), 2'-deoxyribonucleoside triphosphates (dATP, dUTP, dTTP, dCTP, dGTP), 2',3'-dideoxynucleoside triphosphates (ddATP, ddGTP, ddUTP, ddCTP, ddTTP).

33. The method according to claim 32, wherein the additional type of nucleoside triphosphate is a conventionally labelled nucleotide modified with a label, selected from the group of fluorescent dye, biotin, hapten and a radioactive element.

34. The method according to claims 28 to 33, wherein at least two different populations of nucleic acid chains are present.

35. The method according to claim 34, wherein at least one of the populations of the nucleic acid chains has a primer function and at least one population of the nucleic acid chains has a template function.

36. The method according to claim 28, wherein the modification is accomplished by an chemical coupling and wherein the coupling of nuc-macromolecules to nucleic acid chains is conducted via phosphoramidite coupling.

37. The method according to claims 28 to 36, wherein nuc-macromolecules which allow for the coupling of only a single nuc-component into the growing nucleic acid chains are used in a labelling method and multiple incorporations of nuc-components are prevented by modifications of the nuc-component and/or the linker component and/or the marker component.

38. The method according to claim 37, wherein the next incorporation is prevented reversibly.

39. The method according to claim 37, wherein the next incorporation is prevented irreversibly.

40. The method according to claims 28 to 36, wherein nuc-macromolecules are used in a labelling method which allow for incorporation of multiple nuc-components into the growing nucleic acid chains.

41. The method according to claims 28 to 40, wherein nucleic acid chains participating in the reaction are attached to a solid phase and have addressable positions.

42. The method according to claim 41, wherein the said nucleic acid chains compose a uniform population of nucleic acid chains.

43. The method according to claim 41, wherein the said population of nucleic acid chains compose two or more different populations of nucleic acid chains and the solid phase has an addressable position for each of said populations of nucleic acid chains.

44. The method according to claims 41 or 42, wherein the coupling of nuc-macromolecules is conducted on the uniform population of nucleic acid molecules attached to the solid phase and the marker component of the nuc-macromolecule remains on the extended nucleic acid strand after the coupling and is not cleaved off.

45. The method according to claims 41 or 42, wherein the coupling of nuc-macromolecules is conducted on the uniform population of nucleic acid molecules attached to the solid phase and wherein the marker component or its individual parts with or without linker component of the nuc-macromolecule is cleaved-off from the nuc-macromolecule that is incorporated into the growing nucleic acid chains, wherein the step of the cleaving-off occurs during or after the coupling of the nuc-macromolecule into the nucleic acid chains.

46. The method according to claims 41 or 43, wherein the coupling of nuc-macromolecules is conducted during reaction parallel on two or multiple different population of nucleic acid molecules attached to the solid phase and the solid phase has an addressable position for each of said populations of nucleic acid chainsand the marker component of the nuc-macromolecule remains on the extended nucleic acid strand after the coupling and is not cleaved off.

47. The method according to claims 41 or 43, wherein the coupling of nuc-macromolecules is conducted during reaction parallel on two or multiple different population of nucleic acid molecules attached to the solid phase and the solid phase has an addressable position for each of said populations of nucleic acid chains and wherein the marker component or its individual parts with or without linker component of the nuc-macromolecule is cleaved-off from the nuc-macromolecule, wherein the step of the cleaving-off occurs during or after the coupling of the nuc-macromolecule.

48. The method according to claims 41 to 47, wherein the addressable positions having nucleic acid molecules on the solid phase are distributed as spots on a plane surface, and nucleic acid molecules are uniform on each spot.

49. The method according to the claims 41 to 47, wherein the sold phase is a bead or particle having an addressable position for a single uniform population of nucleic acid chains.

50. The method according to claims 41 to 47, wherein the addressable positions having nucleic acid molecules are distributed in a multivessel array (e.g. mikrotiter plate or nanotiter plate or picotiter plate) and each vessel of the multivessel array comprises uniform population of nucleic acid chains.

51. The method according to claims 28 to 35 and 37 to 50, which comprises the following steps:
a) Providing of at least one population of single-stranded nucleic acid chains (NAC),
b) Hybridizing of primers to these nucleic acid chains, whereas extendable NAC primer complexes are formed,
c) Incubation of at least one type of the nuc-macromolecule according to claims 1 to 25 together with one type of polymerase according to claim 31 with provided NAC primer complexes in steps (a) and (b) under conditions which allow for incorporation of complementary nuc-macromolecules, wherein each kind of the nuc-macromolecule having a distinctive label,
d) Removal of the non-incorporated nuc-macromolecules from the NAC primer complexes,
e) Detection of the signals from the nuc-macromolecules which are incorporated in the NAC primer complexes,
f) Removal of the linker component and the marker component from the nuc-macromolecules which are incorporated in the NAC primer complexes,
g) Wash the NAC primer complexes,
if necessary, repetition of the steps (c) to (g).

52. The method according to claims 28 to 40, wherein the nucleic acid chains are attached to a solid phase in a random arrangement.

53. The method according to claims 28 to 41, 52 for the parallel sequence analysis of nucleic acid sequences (nucleic acid chains, NACs), wherein
• fragments (NACFs) of single-stranded NACs with a length of approximately 50 to 1000 nucleotides that may represent overlapping partial sequences of the whole sequence are produced,
• the NACFs are attached to a reaction surface in a random arrangement using a uniform or several different primers in the form of NACF primer complexes,
• a cyclical synthesis reaction of the complementary strand of the NACFs is performed using one or more polymerases, wherein
a) adding a solution to the NACF primer complexes attached to the surface, wherein the solution contains one or more polymerases and one to four nuc-macromolecules which have a marker component labeled with fluorescent dyes, wherein the fluorescent dyes of the marker component are chosen in such a manner that the nuc-macromolecules used can be distinguished from one another by measurement of different fluorescent signals when at least two nuc-macromolecules are used simultaneously, the nuc-macromolecules being structurally modified in such a manner that the polymerase is not capable of incorporating another nuc-macromolecule into the same strand after such a nuc-macromolecule has been incorporated in a growing complementary strand, the linker component and marker component being cleavable,
b) incubating the stationary phase obtained in step a) under conditions suitable for extending the complementary strands, wherein the complementary strands each being extended by one nuc-macromolecule,
c) washing the stationary phase obtained in step b) under conditions suitable for removing nuc-macromolecules that are not incorporated into a complementary strand,
d) detecting the single nuc-macromolecules incorporated in complementary strands by measuring the characteristic signal of the respective fluorescent dye, wherein the relative position of the individual fluorescent signals on the reaction surface being determined at the same time,
e) cleaving-off the linker component and marker component of the nuc-components coupled to the complementary strand in order to produce unlabeled (nucleotides or) NACFs,
f) washing the stationary phase obtained in step e) under conditions suitable for the removal of the marker component,
repeating steps a) to f), several times if necessary,
wherein the relative position of individual NACF primer complexes on the reaction surface and the sequence of these NACFs being determined by specific assignment of the fluorescent signals, which were detected in the respective positions in step d) during successive cycles, to the nuc-macromolecules.

54. The method according to claim 53, **characterized in that** steps a) to f) of the cyclical synthesis reaction are repeated several times, wherein only one type of nuc-macromolecule being used in each cycle.

55. The method according to claim 53 **characterized in that** steps a) to f) of the cyclical synthesis reaction are repeated several times, wherein two types of differently labeled nuc-macromolecules being used in each cycle.

56. The method according to claim 53 **characterized in that** steps a) to f) of the cyclical synthesis reaction are repeated several times, wherein four types of differently labeled nuc-macromolecules being used in each cycle.

57. The method according to claim 53 **characterized in that** the NACs are variants of a known reference sequence and steps a) to f) of the cyclical synthesis reaction are repeated several times, wherein two differently labeled types of nuc-macromolecules and two unlabeled NTs being used alternately in the cycles and the whole sequences being determined by comparison with the reference sequence.

58. The method according to claims 53 to 57 **characterized in that** a primer binding site (PBS) is introduced in each of the NACFs, whererin individual PBS being introduced at both complementary single strands in the case of double-stranded NACs and the primer binding sites displaying identical or different sequences for all NACFs.

59. The method according to claims 53 to 57 **characterized in that** the NACFs are brought into contact with primers in a solution under conditions suitable for the hybridization of the primers to the primer binding sites (PBSs) of the NACFs, the primers exhibiting identical or different sequences to one another, and the NACF primer complexes formed then being attached to the reaction surface.

60. The method according to claims 53 to 57 **characterized in that** the NACFs are first immobilized on the reaction surface and only then brought into contact with primers under conditions suitable for the hybridization of the primers to the primer binding sites (PBSs) of the NACFs, whererin NACF primer complexes being formed and the primers exhibiting identical or different sequences to one another.

61. The method according to claims 53 to 60, wherein the incorporation reaction is being performed simultaneously on 10 to 100,000 different sequence populations.

62. The method according to claims 53 to 60, wherein the incorporation reaction is being performed simultaneously on 100,000 to 100,000,000 different sequence populations.

63. The method according to claims 28 to 62, wherein sequences of the nucleic acid chains are determined.

64. The method according to claims 28 to 63, wherein the marker component is fluorescently labeled.

65. The method according to claims 41 to 64, wherein the solid phase is independently selected from the following group: silicone, glass, ceramics, plastics, gels or their modifications.

66. A kit comprising macromolecular compounds according to claims 1 to 25 and optionally comprising further components from the following group: at least one type of polymerases, at least one type of nucleoside-triphosphates or 2'-deoxy-nucleoside triphosphates.

67. The method, wherein macromolecular compounds according to claims 1 to 25 are used in enzymatic reactions, wherein used enzymes comprise the following groups: polymerases, ligases, nucleases (endo- or exonucleases).

68. The method, wherein macromolecular compounds according to claims 26 and 27 are used in enzymatic reactions, wherein used enzymes comprise the following groups: polymerases, ligases, nucleases (endo- or exonucleases).

## Revendications

1. Composés macromoléculaires présentant la structure :
(Nuk-segment de liaison)ₙ-marqueur
dans laquelle :
Nuk - est un nucléotide ou un nucléoside (composant Nuk)
segment de liaison - désigne un composant de segment de liaison qui comprend les composants suivants :
a) unité de couplage L - une partie du segment de liaison qui représente la liaison entre Nuk et le radical de segment de liaison
b) un polymère - une partie du segment de liaison qui est un polymère hydrosoluble présentant une longueur moyenne entre 50 et 20 000 atomes (atomes de chaîne) et ce polymère comprend des monomères identiques ou différents choisis indépendamment parmi des groupes de polymères suivants :
polyéthylène glycol (PEG), polysaccharides, dextrane, polyamides, polypeptides, polyphosphates, polyacétates, poly(alkylène glycols), copolymères d'éthylène glycol et de propylène glycol, alcools poly(oléfiniques), poly(vinylpyrrolidones), poly(hydroxyalkylméthacrylamides), poly(hydroxyalkylméthacrylates), poly(x-hydroxy-acides), acide poly-acrylique, poly-acrylamide, alcool poly(vinylique),
c) unité de couplage T - une partie du segment de liaison qui représente la liaison entre le radical du segment de liaison et le marqueur,
marqueur - est un composant de marqueur macromoléculaire qui comprend au moins une macromolécule présentant une masse d'au moins 2000 Da ou une multiplicité d'unités de marqueur présentant une masse moléculaire inférieure à 2000 Da, ou une combinaison d'une multiplicité d'unités de marqueur présentant une masse moléculaire inférieure à 2000 Da et au moins une macromolécule présentant une masse moléculaire d'au moins 2000 Da,
n - un nombre entier entre 1 et 100.

2. Composés macromoléculaires selon la revendication 1, dans lesquels le composant Nuk comprend les structures suivantes : 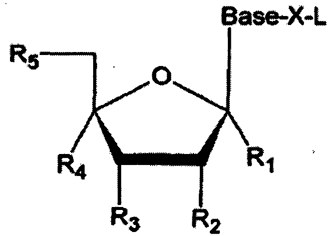 dans lesquelles :
base - est choisie indépendamment dans le groupe comprenant l'adénine ou la 7-désaza-adénine ou la guanine, ou la 7-désazaguanine, ou la thymine, ou la cytosine, ou l'uracile, ou leurs modifications, dans lesquelles X est le site de couplage du segment de liaison à la base et
L - est l'unité de couplage du segment de liaison (L),
R₁ - est H
R₂ - est choisi indépendamment dans le groupe H, OH, halogéno, NH₂, SH ou un groupe OH protégé
R₃ - est choisi indépendamment dans le groupe H, OH, halogéno, PO₃, SH, N₃, NH₂, O-CH₃, O-CH₂-O-CH₃, O-CH₂-CH=CH₂, O-R₃₋₁, P(O)ₘ-R₃₋₁ (m est égal à 1 ou 2), NH-R₃₋₁, S-R₃₋₁, Si-R₃₋₁, dans lesquelles R₃₋₁ est un groupe clivable de façon chimique, photochimique ou enzymatique,
R₄ - est H ou OH
R₅ - est choisi indépendamment dans le groupe OH ou un groupe OH protégé, ou un groupe monophosphate, ou un groupe diphosphate, ou un groupe triphosphate, ou un groupe alpha-thiotriphosphate.

3. Composés macromoléculaires selon la revendication 1, dans lesquels le composant Nuk comprend les structures suivantes : 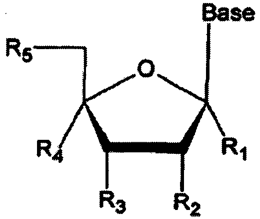 dans lesquelles
base - est choisie indépendamment dans le groupe comprenant l'adénine, ou la 7-désaza-adénine ou la guanine, ou la 7-désazaguanine, ou la thymine, ou la cytosine, ou l'uracile, ou leurs modifications aptes à entraîner des réactions enzymatiques,
R₁ - est H
R₂ - est choisi indépendamment dans le groupe H, OH, halogéno, NH₂, SH ou un groupe OH protégé
R₃ - est choisi indépendamment dans le groupe O, R₃₋₂-L, P(O)ₘ-R₃₋₂-L, dans lesquelles m est 1 ou 2, NH-R₃₋₂-L, S-R₃₋₂-L, Si-R₃₋₂-L ou dans lesquelles R₃₋₂ est le site de couplage du segment de liaison au nucléotide et L - est l'unité de couplage du segment de liaison (L),
R₄ - est H ou OH
R₅ - est choisi indépendamment dans le groupe OH, ou un groupe OH protégé, ou un groupe monophosphate, ou un groupe diphosphate, ou un groupe triphosphate, ou un groupe alpha-thiotriphosphate.

4. Composés macromoléculaires selon la revendication 1, dans lesquels le composant Nuk comprend les structures suivantes : 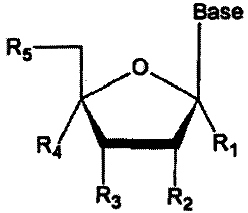 dans lesquelles
base - est choisie indépendamment dans le groupe comprenant l'adénine, ou la 7-désaza-adénine ou la guanine, ou la 7-désazaguanine, ou la thymine, ou la cytosine, ou l'uracile, ou leurs modifications aptes à entraîner des réactions enzymatiques,
R₁ - est H
R₂ - est choisi indépendamment dans le groupe H, OH, Hal, NH₂, SH ou un groupe OH protégé
R₃ - est choisi indépendamment dans le groupe H, OH, PO₃, SH, NH₂, O-R₃₋₁, P(O)ₘ-R₃₋₁ (m est égal à 1 ou 2), NH-R₃₋₁, S-R₃₋₁, Si-R₃₋₁, dans lesquelles R₃₋₁ est un groupe clivable de façon chimique, photochimique ou enzymatique,
R₅ - est choisi indépendamment dans le groupe O-, R₅₋₁-L ou P-(O)₃-, R₅₋₁-L (groupe monophosphate modifié) ou P-(O)₃-P-(O)₃-R₅₋₁-L (groupe diphosphate modifié) ou P-(O)₃-P-(O)₃-P-(O)₃-R₅₋₁-L (groupe triphosphate modifié), dans lesquelles R₅₋₁ est le site de couplage du segment de liaison au nucléotide et L - est l'unité de couplage du segment de liaison (L).

5. Composés macromoléculaires selon les revendications 1 à 4, dans lesquels l'unité de couplage du segment de liaison (L) comprend des éléments structurels suivants : R₆-NH-R₇, R₆-O-R₇, R₆-S-R₇, R₆-SS-R₇, R₆-CO-NH-R₇, R₆-NH-CO-R₇, R₆-CO-O-R₇, R₆-O-CO-R₇, R₆-CO-S-R₇, R₆-S-CO-R₇, R₆-P(O)₂-R₇, R₆-Si-R₇, R₆-(CH₂)ₙ-R₇, R₆-(CH₂)ₙ-R₇, R₆-A-(CH₂)ₙ-R₇, R₆-(CH₂)ₙ-B-R₇, R₆-(CH=CH-)ₙ-R₇, R₆-(A-CH=CH-)ₙ-R₇, R₆-(CH=CH=B-)ₙ-R₇, R₆-A-CH=CH-(CH₂-)ₙR₇, R₆-(-CH=CH-CH₂)ₙ-B-R₇, R₆-(C≡C-)ₙ-R₇, R₆-(A-C≡C-)ₙ-R₇, R₆-(C≡C-B-)ₙ-R₇, R₆-A-C≡C-(CH₂-)ₙ-R₇, R₆-(-C≡C-CH₂)ₙ-B-R₇, R₆-(-C≡C-CH₂-CH₂-)ₙ-B-R₇,
dans lesquels R₆ - est le composant Nuk, R₇ - est le radical du segment de liaison et A et B comprennent des éléments structurels suivants : -NH-, -O-, -S-, -SS-, -CO-NH-, -NH-CO-, -CO-O-, -O-CO-, -CO-S-, -S-CO-, -P(O)₂-, Si-, -(CH₂)ₙ-, un groupe photolabile, dans lesquelles n - est égal à 1 à 5.

6. Composés macromoléculaires selon les revendications 1 à 5, dans lesquels le composant du segment de liaison comprend un polymère hydrosoluble et ce polymère consiste en plusieurs segments et des monomères d'un segment sont identiques.

7. Composés macromoléculaires selon la revendication 6, dans lesquels le composant du segment de liaison comprend au moins un segment de polymères hydrosolubles choisi indépendamment dans le groupe suivant :
polyéthylène glycol (PEG), polysaccharides, dextrane, polyamides, polypeptides, polyphosphates, polyacétates, poly(alkylène glycols), copolymères d'éthylène glycol et de propylène glycol, alcools poly(oléfiniques), poly(vinylpyrrolidones), poly(hydroxyalkylméthacrylamides), poly(hydroxyalkylméthacrylates), poly(x-hydroxy-acides), acide poly-acrylique, poly-acrylamide, alcool poly(vinylique).

8. Composés macromoléculaires selon les revendications 1 à 7, dans lesquels un composant de segment de liaison a une longueur moyenne entre 50 à 100, 100 à 200, 200 à 500, 500 à 1000, 1000 à 2000, 2000 à 10 000, 10 000 et 50 000 atomes (atomes de chaîne).

9. Composés macromoléculaires selon les revendications 1 à 8, dans lesquels un composant de marqueur a une fonction de signalisation, de transmission de signal, une fonction catalytique ou affine.

10. Composés macromoléculaires selon les revendications 1 à 9, dans lesquels un composant de marqueur consiste en une unité de marqueur structurelle.

11. Composés macromoléculaires selon les revendications 1 à 9, dans lesquels un composant de marqueur consiste en plusieurs unités de marqueur structurelles liées à un composant de noyau.

12. Composés macromoléculaires selon les revendications 10 ou 11, dans lesquels une unité de marqueur structurelle comprend indépendamment l'un des éléments structurels suivants :
biotine, haptène, isotope radioactif, atome de terre rare, colorant, colorant fluorescent.

13. Composés macromoléculaires selon les revendications 10 ou 11, dans lesquels une unité de marqueur structurelle comprend indépendamment l'un des éléments suivants :
nanocristaux ou leurs modifications, protéines ou leurs modifications, chaînes d'acide nucléique ou leurs modifications, particules ou leurs modifications.

14. Composés macromoléculaires selon la revendication 13, dans lesquels une unité de marqueur structurelle de l'une des protéines suivantes comprend :
des enzymes ou leurs conjugués ou modifications,
des anticorps ou leurs conjugués ou modifications,
de la streptavidine ou ses conjugués ou modifications,
de l'avidine ou ses conjugués ou modifications

15. Composés macromoléculaires selon la revendication 13, dans lesquels une unité de marqueur structurelle comprend l'une des chaînes d'acide nucléique des sortes suivantes : ADN, ARN, PNA, dans lesquels la longueur des chaînes d'acide nucléique se situe entre 10 et 10 000 nucléotides.

16. Composés macromoléculaires selon les revendications 11 à 15, dans lesquels le composant de noyau du composant de marqueur comprend indépendamment l'un des éléments suivants : un polymère hydrosoluble du groupe comprenant : des polyamides (par exemple, polypeptides), de l'acide polyacrylique et leurs dérivés, des polyacrylamides et leurs dérivés, des alcools polyvinyliques et leurs dérivés, des polyacrylamides et leurs dérivés, des alcools polyvinyliques et leurs dérivés, des acides nucléiques et leurs dérivés, de la streptavidine ou de l'avidine et leurs dérivés, des dendrimères, dans lesquels ces éléments peuvent être linéaires ou ramifiés ou être ramifiés conjointement.

17. Composés macromoléculaires selon les revendications 1 à 9, 11 à 16, dans lesquels la liaison entre plusieurs unités de marqueur structurelles et le composant de noyau s'effectue de façon covalente ou affine.

18. Composés macromoléculaires selon les revendications 1 à 10, dans lesquels la liaison entre une unité de marqueur structurelle et le segment de liaison s'effectue de façon covalente ou affine.

19. Composés macromoléculaires selon les revendications 1 à 9, 11 à 17, dans lesquels la liaison entre le composant de noyau et le segment de liaison s'effectue de façon covalente ou affine

20. Composés macromoléculaires selon les revendications 1 à 19, dans lesquels seul un composant Nuk est lié à un composant de segment de liaison couplé au composant de noyau, dans lesquels la longueur du segment de liaison se situe entre 50 à 100, 100 à 200, 200 à 500, 500 à 1000, 1000 à 2000, 2000 à 5000 atomes.

21. Composés macromoléculaires selon les revendications 1 à 20, dans lesquels seul un composant Nuk avec un composant de segment de liaison couplé est lié au composant de marqueur, dans lesquels la longueur du segment de liaison se situe entre 50 à 100, 100 à 200, 200 à 500, 500 à 1000, 1000 à 2000, 2000 à 5000 atomes et le composant du segment de liaison comporte un ou plusieurs liaisons pouvant être clivées dans des conditions modérées.

22. Composés macromoléculaires selon les revendications 1 à 21, dans lesquels seul un composant Nuk avec un composant de segment de liaison couplé est lié au composant de marqueur, dans lesquels la longueur du segment de liaison se situe entre 50 à 100, 100 à 200, 200 à 500, 500 à 1000, 1000 à 2000, 2000 à 5000 atomes et une ou plusieurs parties de la macromolécule Nuk sont modifiées de telle sorte que seul un composant Nuk peut être intégré dans un brin de croissance.

23. Composés macromoléculaires selon les revendications 1 à 19, dans lesquels plusieurs composants Nuk sont couplés respectivement par un segment de liaison à un composant de marqueur, dans lesquels la longueur du composant du segment de liaison respectif se situe entre 50 à 100, 100 à 200, 200 à 500, 500 à 1000, 1000 à 2000, 2000 à 5000 atomes.

24. Composés macromoléculaires selon les revendications 1 à 19, 23, dans lesquels plusieurs composants Nuk sont couplés respectivement par un segment de liaison à un composant de marqueur, dans lesquels la longueur du composant de segment de liaison respectif se situe entre 50 à 100, 100 à 200, 200 à 500, 500 à 1000, 1000 à 2000, 2000 à 5000 atomes et le segment de liaison respectif comporte une ou plusieurs liaisons pouvant être clivées dans des conditions modérées.

25. Composés macromoléculaires selon les revendications 1 à 19, 23, 24 dans lesquels plusieurs composants Nuk sont couplés respectivement par un segment de liaison à un composant de marqueur, dans lesquels la longueur du composant de segment de liaison respectif se situe entre 50 à 100, 100 à 200, 200 à 500, 500 à 1000, 1000 à 2000, 2000 à 5000 atomes et une ou plusieurs parties de la macromolécule Nuk sont modifiées de telle sorte que seul un composant Nuk peut être intégré dans une chaîne d'acide nucléique en croissance.

26. Composés macromoléculaires selon la revendication 2 à 4, dans lesquels R₅ est un groupe triphosphate ou un groupe alpha-thiotriphosphate ou un groupe tétraphosphate.

27. Composés macromoléculaires selon les revendications 2 à 4, dans lesquels R₅ est un groupe monophosphate ou un groupe diphosphate.

28. Procédé de modification de chaînes d'acide nucléique, dans lequel pour le couplage, on utilise des macromolécules Nuk selon les revendications 1 à 27.

29. Procédé selon la revendication 28, dans lequel la modification s'effectue par couplage enzymatique et le mélange réactionnel comprend des composants suivants :
- au moins une sorte de macromolécules Nuk ou leurs étapes intermédiaires selon les revendications 1 à 25, dans lequel chaque sorte des macromolécules Nuk possède un marquage caractéristique pour celles-ci,
- au moins une population des chaînes d'acide nucléique,
- au moins une sorte d'enzyme pour le couplage des macromolécules Nuk sur les chaînes d'acide nucléique.

30. Procédé selon la revendication 28, dans lequel la modification s'effectue par un couplage enzymatique et le mélange réactionnel comprend des composants suivants :
- au moins une sorte de macromolécules Nuk ou leurs étapes intermédiaires selon les revendications 1 à 25, dans lequel chaque sorte des macromolécules Nuk possède un marquage caractéristique pour celles-ci,
- au moins une population des chaînes d'acide nucléique,
- au moins une sorte d'enzyme pour le couplage des macromolécules Nuk sur les chaînes d'acide nucléique
- au moins une autre sorte de nucléosides triphosphates.

31. Procédé selon les revendications 29, 30, dans lequel la sorte d'enzyme comprend, indépendamment, l'un des groupes suivants : ADN-polymérase, ARN-polymérase, transférase terminale.

32. Procédé selon la revendication 30, dans lequel l'« autre sorte » de nucléosides triphosphates est choisie indépendamment dans le groupe des : ribonucléosides triphosphates (ATP, GTP, UTP, CTP), des 2'-désoxyribonucléosides triphosphates (dATP, dUTP, dTTP, dCTP, dGTP), des 2',3'-didésoxynucléosides triphosphates (ddATP, ddGTP, ddUTP, ddCTP, ddTTP).

33. Procédé selon la revendication 32, dans lequel l'« autre sorte » de nucléosides triphosphates est constituée de nucléotides modifiés de façon classique avec un marquage, dans lequel ce marquage est choisi indépendamment dans le groupe comprenant : un colorant fluorescent, une biotine, un haptène, un élément radioactif.

34. Procédé selon les revendications 28 à 33, dans lequel au moins deux populations indépendantes sont présentes sur des chaînes d'acide nucléique.

35. Procédé selon la revendication 34, dans lequel au moins l'une des populations des chaînes d'acide nucléique a une fonction d'amorce et au moins une population des chaînes d'acide nucléique a une fonction de matrice.

36. Procédé selon la revendication 28, dans lequel la modification s'effectue par un couplage chimique, dans lequel le couplage des macromolécules Nuk s'effectue sur des chaînes d'acide nucléique par couplage de phosphoro-amidite.

37. Procédé selon les revendications 28 à 36, dans lequel dans le procédé de marquage, on utilise des macromolécules Nuk qui permettent le couplage uniquement d'un seul composant Nuk dans le brin d'acide nucléique en croissance et l'intégration multiple est empêchée par des modifications sur le composant Nuk, et/ou le composant de segment de liaison et/ou le composant de marqueur.

38. Procédé selon la revendication 37, dans lequel l'autre couplage est empêché de façon réversible.

39. Procédé selon la revendication 37, dans lequel l'autre couplage est empêché de façon irréversible.

40. Procédé selon les revendications 28 à 36, dans lequel dans le procédé de marquage, on utilise des macromolécules Nuk qui permettent un couplage de plusieurs composants Nuk dans le brin d'acide nucléique en croissance.

41. Procédé selon les revendications 28 à 40, dans lequel les chaînes d'acide nucléique participant à la réaction sont couplées sur une phase solide et ont des positions adressables.

42. Procédé selon la revendication 41, dans lequel les chaînes d'acide nucléique représentent une population unitaire.

43. Procédé selon la revendication 41, dans lequel les chaînes d'acide nucléique représentent deux ou plusieurs populations différentes et chacune des populations a une position adressable sur la phase solide.

44. Procédé selon les revendications 41, 42, dans lequel le couplage de macromolécules Nuk sur une population s'effectue de façon unitaire, aux macromolécules fixées à la phase solide, dans lequel le composant de marqueur de la macromolécule Nuk reste après le couplage sur le brin d'acide nucléique prolongé et n'est pas séparé.

45. Procédé selon les revendications 41, 42, dans lequel le couplage des macromolécules Nuk à une population s'effectue de façon unitaire aux molécules d'acide nucléique fixées à la phase solide, dans lequel le composant de marqueur ou ses composants individuels avec ou sans composants de segment de liaison de la macromolécule Nuk sont séparés pendant le couplage ou après le couplage du composant Nuk intégré dans le brin d'acide nucléique en croissance.

46. Procédé selon les revendications 41, 43, dans lequel le couplage des macromolécules Nuk s'effectue dans un mélange réactionnel parallèlement à deux ou plusieurs populations différentes sur la molécule d'acide nucléique fixée à la phase solide, dans lequel ces populations ont respectivement des positions adressables différentes sur la phase solide et le composant de marqueur de la macromolécule Nuk reste après le couplage sur le brin d'acide nucléique prolongé et n'est pas séparé.

47. Procédé selon les revendications 41, 43, dans lequel le couplage des macromolécules Nuk s'effectue dans un mélange réactionnel parallèlement à deux ou plusieurs populations différentes de molécules d'acide nucléiques fixées à la phase solide, dans lequel ces populations ont des positions adressables différentes sur la phase solide et le composant de marqueur ou ses composants individuels avec ou sans composant de segment de liaison de la macromolécule Nuk sont séparés pendant le couplage ou après le couplage du composant Nuk.

48. Procédé selon les revendications 41 à 47, dans lequel les positions adressables avec des molécules d'acides nucléiques sont réparties sur la phase solide sous forme de taches sur une surface plane, dans lequel par tache, les molécules d'acide nucléiques sont présentes de façon unitaire.

49. Procédé selon les revendications 41 à 47, dans lequel les positions adressables sont fixée avec des molécules d'acide nucléique aux billes ou particules, dans lequel par bille, les molécules d'acide nucléique sont présentes de façon unitaire.

50. Procédé selon les revendications 41 à 47, dans lequel les positions adressables avec des molécules d'acide nucléique sont réparties dans un système à récipients multiples, tel que des plaques de microtitrage ou des plaques de nanotitrage ou des plaques de picotitrage, dans lequel dans un récipient du système à récipient multiple, les molécules d'acide nucléique sont présentes de façon unitaire.

51. Procédé selon les revendications 28 à 35 et 37 à 50, qui comprend les étapes suivantes :
a) mise à disposition d'au moins une population de chaînes d'acide nucléique à brin simple (NSK)
b) hybridation d'amorces sur ces chaînes d'acide nucléique, dans lequel il se forme des complexes de NSK-amorce
c) incubation d'au moins une sorte de macromolécule Nuk selon les revendications 1 à 25 conjointement avec une sorte de polymérase selon la revendication 31 avec les complexes de NSK-amorce obtenus dans les étapes a et b, dans des conditions qui permettent l'intégration de macromolécules Nuk complémentaires, dans lequel chaque sorte de macromolécule Nuk possède un marquage caractéristique de celles-ci,
d) élimination des macromolécules Nuk non intégrées des complexes de NSK-amorce
e) détection des signaux des macromolécules Nuk intégrées dans les complexes NSK-amorce
f) élimination des composants de segment de liaison et des composants de marqueur des macromolécules Nuk intégrées dans les complexes NSK-amorce
g) lavage des complexes NSK-amorce
éventuellement, répétition des étapes (c) à (g).

52. Procédé selon les revendications 28 à 40, dans lequel les chaînes d'acide nucléique sont couplées sur une phase solide dans un ordre aléatoire.

53. Procédé selon les revendications 28 à 41, 52 pour l'analyse de séquence parallèle de séquences d'acide nucléique (chaînes d'acide nucléique, NSK), dans lequel on
génère des fragments (NSKF) à brin simple présentant une longueur d'environ 50 à 1000 nucléotides, qui peuvent représenter des séquences partielles se chevauchant d'une séquence complète, on
lie les NSKF en utilisant une amorce unitaire ou plusieurs amorces différentes sous la forme de complexes de NSKF-amorce sur une surface réactionnelle dans un ordre aléatoire, on
réalise une réaction de construction cyclique du brin complémentaire de NSKF en utilisant une ou plusieurs polymérases, en ce que
a) aux complexes de NSKF-amorce liés sur la surface, on ajoute une solution qui contient une ou plusieurs polymérases et une à quatre macromolécules Nuk qui ont un composant de marqueur marqué avec des colorants fluorescents, dans lequel lors de l'utilisation concomitante d'au moins deux macromolécules Nuk, les colorants fluorescents se trouvant respectivement sur le composant de marqueur sont choisis de telle sorte que les macromolécules Nuk utilisées peuvent être distinguées les unes des autres par une mesure de signaux fluorescents différents, dans lequel les macromolécules Nuk sont modifiées d'un point de vue structurel, de telle sorte que la polymérase n'est pas capable, après l'insertion d'une telle macromolécule Nuk dans un brin complémentaire en croissance, d'intégrer une autre macromolécule Nuk dans le même brin, dans lequel les composants de segment de liaison peuvent être clivés avec le composant du marqueur, on
b) incube la phase stationnaire obtenue à l'étape a) dans des conditions qui sont appropriées pour prolonger les brins complémentaires, dans lequel les brins complémentaires sont prolongés respectivement d'une macromolécule Nuk, on
c) lave la phase stationnaire obtenue à l'étape b) dans des conditions qui sont appropriées pour éliminer des macromolécules Nuk non intégrées dans un brin complémentaire, on
d) détecte les macromolécules Nuk individuelles intégrées dans des brins complémentaires par mesure du signal caractéristique pour le colorant fluorescent respectif, dans lequel on détermine de façon concomitante la position relative des signaux fluorescents individuels sur la surface réactionnelle, on
e) clive le composant du segment de liaison pour générer des (NT ou) NSKF non marqués et le composant de marqueur est clivé du composant Nuk joint au brin complémentaire, on
f) lave la phase stationnaire obtenue à l'étape e) dans des conditions qui sont appropriées pour éliminer le composant de marqueur, on
répète éventuellement plusieurs fois les étapes a) à f),
dans lequel on détermine la position relative de complexe NSKF-amorce individuels sur la surface réactionnelle et la séquence de ces NSKF par affectation spécifique des cycles consécutifs dans l'étape d) aux signaux de fluorescence des macromolécules Nuk détectés sur les positions respectives.

54. Procédé selon la revendication 53, **caractérisé en ce que** l'on répète plusieurs fois les étapes a) à f) de réaction de construction cyclique, dans lequel on utilise dans chaque cycle uniquement une sorte de macromolécules Nuk respective.

55. Procédé selon les revendications 53, **caractérisé en ce que** l'on répète plusieurs fois les étapes a) à f) de la réaction de construction cyclique, dans lequel on utilise dans chaque cycle respectivement deux sortes de macromolécules Nuk marquées différemment.

56. Procédé selon la revendication 53, **caractérisé en ce que** l'on répète plusieurs fois les étapes a) à f) de la réaction de construction cyclique, dans lequel on utilise dans chaque cycle respectivement quatre sortes de macromolécules Nuk marquées différemment.

57. Procédé selon la revendication 53, **caractérisé en ce que** les variantes de NSK sont une séquence de référence connue et l'on répète plusieurs fois les étapes a) à f) de la réaction de construction cyclique, dans lequel on utilise dans les cycles de manière alternative respectivement deux sortes de macromolécules Nuk marquées différemment et deux NT non marqués et l'on détermine les séquences complètes par comparaison avec la séquence de référence.

58. Procédé selon les revendications 53 à 57, **caractérisé en ce que** l'on introduit dans les NSKF respectivement un site de liaison d'amorce (PBS), dans lequel on introduit dans les NSK à double brin sur les deux brins individuels complémentaires, respectivement un PBS et dans lequel les sites de liaison d'amorce présentent pour tous les NSKF respectivement des séquences identiques ou différentes.

59. Procédé selon les revendications 53 à 57, **caractérisé en ce que** l'on met en contact les NSKF avec des amorces dans une solution dans des conditions qui sont appropriées pour l'hybridation de l'amorce aux sites de liaison de l'amorce (PBS) des NSKF, dans lequel les amorces présentent des séquences identiques ou différentes les unes des autres, et on lie ensuite les complexes NSKF-amorce formés sur la surface réactionnelle.

60. Procédé selon les revendications 53 à 57, **caractérisé en ce que** l'on immobilise tout d'abord les NSKF sur la surface réactionnelle et on met seulement ensuite les amorces en contact dans des conditions qui sont appropriées pour l'hybridation des amorces aux sites de liaison d'amorce (PBS) des NSKF, dans lequel des complexes de NSKF-amorce sont formés, dans lequel les amorces présentent des séquences identiques ou différentes les unes des autres.

61. Procédé selon les revendications 53 à 60, dans lequel on réalise en parallèle la réaction de construction sur 10 à 100 000 populations de séquences différentes.

62. Procédé selon les revendications 53 à 60, dans lequel on réalise en parallèle la réaction de construction sur 100 000 à 100 000 000 populations de séquences différentes.

63. Procédé selon les revendications 28 à 62, dans lequel les séquences de la chaîne d'acide nucléique sont déterminées.

64. Procédé selon les revendications 28 à 63, dans lequel le composant de marqueur est marqué de façon fluorescente.

65. Procédé selon les revendications 41 à 64, dans lequel la phase solide est choisie indépendamment dans le groupe suivant : silicone, verre, céramique, matière plastique, gels ou leurs modifications.

66. Kit qui comprend les composés macromoléculaires selon les revendications 1 à 25, et comprend éventuellement d'autres composants du groupe suivant : au moins une sorte de polymérases et au moins une autre sorte de nucléoside triphosphates ou de 2'-désoxy-nucléoside triphosphates.

67. Procédés dans lesquels on utilise des composés macromoléculaires selon les revendications 1 à 25 dans des réactions enzymatiques, dans lesquels des enzymes des groupes suivants sont incluses : polymérases, ligases, nucléases (endonucléases ou exonucléases).

68. Procédés dans lesquels on utilise des composés macromoléculaires selon les revendications 26 et 27, dans lesquels des enzymes des groupes suivants sont incluses : polymérases, ligases, nucléases (endonucléases ou exonucléases).
